# EUROPEAN PATENT APPLICATION

(11) **EP 4 129 406 A1**
(43) Date of publication of application: **08.02.2023**
(21) Application number: 21775678.2
(22) Date of filing: 24.03.2021
(51) Int. Cl.: A61P 29/00

(54) **NOVEL BENZIMIDAZOLE DERIVATIVE**

(30) Priority: 25.03.2020 JP 2020054552; 25.12.2020 JP 2020217098
(71) Applicant: Carna Biosciences, Inc., Kobe-shi, Hyogo 650-0047 (JP)
(72) Inventor: KIYOI, Takao, Kobe-shi, Hyogo 650-0047 (JP); MATSUMOTO, Hirokazu, Kobe-shi, Hyogo 650-0047 (JP); TAKAMATSU, Shiori, Kobe-shi, Hyogo 650-0047 (JP); SAWA, Masaaki, Kobe-shi, Hyogo 650-0047 (JP)
(74) Representative: Tostmann, Holger Carl
(86) International application number: PCT/JP2021/012359
(87) International publication number: WO 2021/193756

(57) **Abstract**

A benzimidazole derivative represented by formula (I) (wherein A¹ to A³ and R¹ to R⁶ are as described in the description) or a pharmaceutically acceptable salt thereof, which inhibits activation of STING pathway and, therefore, which is useful as a prophylactic or therapeutic medicine for inflammatory diseases, autoimmune diseases, cancer, etc.

## Description

### Technical Field

The present invention relates to medicaments, particularly to novel benzimidazole derivatives or pharmaceutically acceptable salts thereof having an inhibitory effect on STING pathway activation.

### Background Technology

STING (STimulator of Interferon Genes) plays an important role in biological defense mechanisms as a molecule that induces an innate immune response against various RNA virus and DNA virus infections. STING binds to a ligand such as cyclic GMP-AMP (cGAMP), which is a cyclic dinucleotide produced by cyclic GMP-AMP synthase (cGAS), activates TANK-binding kinase 1 (TBK1), and induces type I IFN production via the transcription factor IRF3 (Non-Patent Literature 1).

In recent years, it has been reported that STING is also activated by tumor-derived self-DNA, mitochondrial DNA, etc., and induces a pro-inflammatory response, and is attracting increasing attention as a drug discovery target for cancer and autoimmune diseases (Non-Patent Literature 2).

Human STING is encoded by a gene called Tmem173, and an autoinflammatory disease called infantile-onset STING-associated vasculitis (SAVI: STING-Associated Vasculopathy with onset in infancy) has been reported as a genetic disease caused by mutation of this Tmem173. In SAVI patients, STING is constantly activated due to mutation, and excessive inflammation causes abnormal antibody production and skin and lung tissue damage (Non-Patent Literature 3). It has also been reported that activating mutations in STING occur in patients with autoinflammatory genetic diseases such as familial lupus frostbite and familial lupus-like syndrome (Non-Patent Literature 4).

In addition, it is known that when self-DNA accumulates in cells due to defective DNA degradation, constant activation of the STING pathway occurs, causing autoimmune diseases. Aicardi-Goutieres syndrome (AGS) is also considered to be one of them, and it has been reported that the symptoms are suppressed when STING is deficient in this disease model mouse (Non-Patent Literature 5).

In systemic lupus erythematosus (SLE), autoantibodies called antinuclear antibodies, especially anti-DNA antibodies, are excessively produced, which is thought to cause an excessive immune response. Recently, however, it has become clear that activation of the STING pathway induces interferon production, which is important in the pathology of SLE. That is, it has been reported that cGAMP contained in patient peripheral blood is correlated with the pathological condition score, and interferon induction by cGAMP in patient serum is suppressed in STING-deficient cells (Non-Patent Literature 6 and 7).

Since STING is involved in various immune responses *in vivo,* it has also been reported that STING is involved in many diseases. For example, in studies of sepsis in which organ damage is caused by systemic inflammation due to pathogen infection, it has been reported that the symptoms are alleviated when STING is deficient in sepsis model mice. (Non-Patent Literatures 8 and 9) Studies using model mice have also revealed the involvement of STING in inflammatory diseases such as nonalcoholic steatohepatitis (NASH), liver fibrosis, acute pancreatitis, and polyarthritis. (Non-Patent Literatures 10, 11, 12, and 13) Furthermore, patients with Parkinson's disease, a neurodegenerative disease, have been shown to have increased levels of inflammatory cytokines due to disruption of mitochondrial homeostasis, and it has been reported that these abnormalities are ameliorated by deficient STING in model mice (Non-Patent Literatures 14 and 15).

Therefore, inhibitors of STING pathway activation are useful in treating various inflammatory and immune diseases in which the STING pathway is involved.

### Citation List

### Non-Patent Literature

Non-Patent Literature 1: Paludan, S. R. and Bowie, A. G., Immunity, 2013, 38(5), 870-880
Non-Patent Literature 2: Motwani M., et al., Nat. Rev. Genet., 2019, 20(11), 657-674
Non-Patent Literature 3: Liu, Y. et al., N. Engl. J. Med., 2014, 371(6), 507-518
Non-Patent Literature 4: Jeremiah, N., et al., J. Clin. Invest., 2014, 124(12), 5516-5520
Non-Patent Literature 5: Mackenzie, K. J., et al., ENBO J., 2016, 35(8), 831-844
Non-Patent Literature 6: An, J., et al., Arthritis Rheumatol., 2017, 69(4), 800-807
Non-Patent Literature 7: Kato, Y., et al., Ann. Rheum. Dis., 2018, 77(10), 1507-1515
Non-Patent Literature 8: Zeng, L., et al., Sci. Transl. Med., 2017, 9 (412)
Non-Patent Literature 9: Hu, Q., et al., EBio Medicine, 2019, 41, 497-508
Non-Patent Literature 10: Yu, Y., et al., J. Clin. Invest., 2019, 129(2), 546-555
Non-Patent Literature 11: Iracheta-Vellve, A., et al., J. Biol. Chem., 2016, 291(52), 26794-26805
Non-Patent Literature 12: Maekawa, H., et al., Cell Rep., 2019, 29(5), 1261-1273. e6
Non-Patent Literature 13: Ahn, J., et al., Proc. Natl. Acad. Sci. U.S.A. 2012, 109(47), 19386-19391
Non-Patent Literature 14: Andrea, A. and Chen, Z. J., Science, 2019, 363 (6431)
Non-Patent Literature 15: Sliter, D. A., et al., Nature, 2018, 561(7722), 258-262

### SUMMARY OF THE INVENTION

### Problems to be Solved by the Invention

The object of the present invention is to provide a medicament, particularly a novel benzimidazole derivative or a pharmaceutically acceptable salt thereof having an activity-inhibiting action on STING pathway activation.

### Means for Solving the Problems

The object of the present invention is achieved by the following (1) to (9). A benzimidazole derivative or a pharmaceutically acceptable salt thereof represented by
(1) Formula (I): 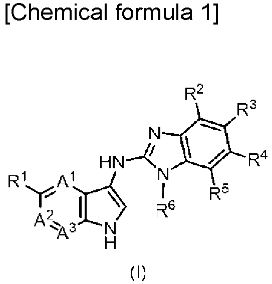 (where A¹ represents a nitrogen atom or C-R⁷, A² represents a nitrogen atom or C-R⁸, A³ represents a nitrogen atom or C-R⁹,
   R¹ is a hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted alkoxy group, a substituted or unsubstituted aryl group, a substituted or unsubstituted carbamoyl group, a 4-morpholine carbonyl group, a cyano group, a carboxy group or an alkoxycarbonyl group,
   R², R³, R⁴ and R⁵ are each independently and optionally selected from the group consisting of a hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted alkoxy group, a substituted or unsubstituted aryl group, a substituted or unsubstituted heteroaryl group, a substituted or unsubstituted heterocyclo group, a substituted or unsubstituted aryloxy group, a substituted or unsubstituted cycloalkyloxy group, a substituted or unsubstituted heteroaryloxy group, a substituted or unsubstituted heterocyclooxy group, a substituted or unsubstituted arylsulfonyl group, a substituted or unsubstituted carbamoyl group, a substituted or unsubstituted amino group, a substituted or unsubstituted aminosulfonyl group, a cyano group, a carboxy group, an alkoxycarbonyl group and a nitro group, wherein R² and R³, or R³ and R⁴, or R⁴ and R⁵ may be bonded to each other to form a ring,
   R⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted heteroaryl group, a substituted or unsubstituted heterocyclo group, or a substituted or unsubstituted amino group,
   R⁷, R⁸ and R⁹ are each independently and optionally selected from the group consisting of a hydrogen atom, a halogen atom, a substituted or unsubstituted alkyl group, a substituted or unsubstituted alkenyl group, a substituted or unsubstituted cycloalkenyl group, a substituted or unsubstituted alkoxy group, a substituted or unsubstituted aryl group, a substituted or unsubstituted heteroaryl group, a hydroxyl group, a substituted or unsubstituted amino group and a substituted or unsubstituted carbamoyl group.)
(2) The benzimidazole derivative or a pharmaceutically acceptable salt thereof according to (1) above, wherein in formula (I), 1) A' is C- R⁷, A² is C-R⁸, A³ is C-R⁹; 2) A' is a nitrogen atom, A² is C-R⁸, A³ is C-R⁹; 3) A' is C-R⁷, A² is a nitrogen atom, A³ is C-R⁹; 4) A¹ is C-R⁷, A² is C-R⁸, A³ is a nitrogen atom, or 5) both A¹ and A³ represent a nitrogen atom and A² represents C-R⁸.
(3) The benzimidazole derivative or a pharmaceutically acceptable salt thereof according to (1) or (2) above, wherein in formula (I), A² represents C-R⁸, 1) A¹ is C-R⁷, A³ is C-R⁹; 2) A¹ is a nitrogen atom, A³ is C-R⁹; or 3) A¹ represents C-R⁷ and A³ represents a nitrogen atom.
(4) The benzimidazole derivative or a pharmaceutically acceptable salt thereof according to (1) or (2) above, wherein in the above formula (1), A¹, A² and A³ are represented by C-R⁷, C-R⁸ and C-R⁹, respectively.
(5) The benzimidazole derivative or a pharmaceutically acceptable salt thereof according to (1) or (2) above, wherein in the above formula (I), A¹ is a nitrogen atom and A² and A³ are C-R⁸ and C-R⁹, respectively.
(6) The benzimidazole derivative or a pharmaceutically acceptable salt thereof according to (1) or (2) above, wherein in the above formula (I), A¹ is C-R⁷, A² is a nitrogen atom, and A³ is C-R⁹.
(7) The benzimidazole derivative or a pharmaceutically acceptable salt thereof according to (1) or (2) above, wherein in the above formula (I), A¹ is C-R⁷, A² is C-R⁸, and A³ is a nitrogen atom.
(8) The benzimidazole derivative or a pharmaceutically acceptable salt thereof according to (1) or (2) above, wherein in the above formula (I), both A¹ and A³ are nitrogen atoms and A² is C-R⁸.
(9) The compounds of Examples 1 to 288 or pharmaceutically acceptable salts thereof described below.

### Effects of the Invention

The present inventors have made various studies to solve the above problems, and as a result, the novel benzimidazole derivative represented by the formula (I) or a pharmaceutically acceptable salt thereof exhibits excellent STING pathway activation inhibitory activity, and have completed the present invention. The compounds provided by the present invention are effective as prophylactic or therapeutic pharmaceuticals (pharmaceutical compositions) for diseases known to be associated with STING-mediated cell responses, such as inflammatory diseases, autoimmune diseases, cancer, and the like. In addition, by combining with therapeutic agents for other inflammatory diseases, autoimmune diseases, and cancer, an effect on immune response and the like can be expected, and it is useful as therapeutic pharmaceuticals (pharmaceutical compositions). Furthermore, it is useful as a STING inhibitor and as a reagent for experimental research.

### Brief Description of the Drawing

[FIG. 1] shows an example of the IL-6 production inhibitory effect on the STING agonist-stimulated mouse model of the representative compounds (Test Example 2)
[FIG. 2] shows an example of the IFN-β production inhibitory effect on the STING agonist-stimulated mouse model of the representative compounds (Test Example 2)
[FIG. 3] shows an example of the TNF-α production inhibitory effect on the STING agonist-stimulated mouse model of the representative compounds (Test Example 2) Best Mode for Carrying Out the Invention

Hereinafter, the present invention will be described in detail.

The novel benzimidazole derivatives of the present invention are compounds represented the general formula (I): 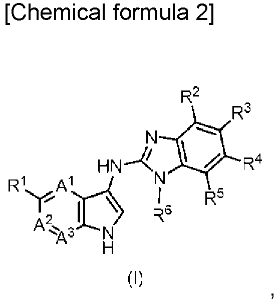 having a basic skeleton in which a bicyclic nitrogen-containing heteroaryl ring is substituted at the 2nd position of the benzimidazole ring via a nitrogen atom.

More specifically, in formula (I) above, A¹ represents a nitrogen atom or C-R⁷, A² represents a nitrogen atom or C-R⁸, and A³ represents a nitrogen atom or C-R⁹.

Furthermore, R¹ can be a hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted alkoxy group, a substituted or unsubstituted aryl group, a substituted or unsubstituted carbamoyl group, a 4-morpholine carbonyl group, a cyano group, a carboxy group or an alkoxycarbonyl group.

R², R³, R⁴ and R⁵ are each independently and optionally selected from a group consisting of the hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted alkoxy group, a substituted or unsubstituted aryl group, a substituted or unsubstituted heteroaryl group, a substituted or unsubstituted heterocyclo group, a substituted or unsubstituted aryloxy group, a substituted or unsubstituted cycloalkyloxy group, a substituted or unsubstituted heteroaryloxy group, a substituted or unsubstituted heterocyclooxy group, a substituted or unsubstituted arylsulfonyl group, a substituted or unsubstituted carbamoyl group, a substituted or unsubstituted amino group, a substituted or unsubstituted aminosulfonyl group, a cyano group, a carboxy group, an alkoxycarbonyl group or a nitro group. R² and R³, or R³ and R⁴, or R⁴ and R⁵ may be bonded to each other to form a ring.

R⁶ may be a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted heteroaryl group, a substituted or unsubstituted heterocyclo group, or a substituted or unsubstituted amino group.

R⁷, R⁸ and R⁹ may be each independently and optionally selected from the group consisting of a hydrogen atom, a halogen atom, a substituted or unsubstituted alkyl group, a substituted or unsubstituted alkenyl group, a substituted or unsubstituted cycloalkenyl group, a substituted or unsubstituted alkoxy group, a substituted or unsubstituted aryl group, a substituted or unsubstituted heteroaryl group, a hydroxyl group, a substituted or unsubstituted amino group or a substituted or unsubstituted carbamoyl group.

One embodiment of the benzimidazole derivative of the present invention in formula (I) include a benzimidazole derivative or a pharmaceutically acceptable salt, wherein A¹, A² and A³ are represented by C-R⁷, C-R⁸ and C-R⁹, respectively.

Another embodiment of the benzimidazole derivative of the present invention in formula (I) include a benzimidazole derivative or a pharmaceutically acceptable salt, wherein A¹ is a nitrogen atom; A² and A³ are respectively C-R⁸ and C-R⁹.

Another embodiment of the benzimidazole derivative of the present invention in formula (I) include a benzimidazole derivative or a pharmaceutically acceptable salt, wherein A¹ is C-R⁷, A² is a nitrogen atom, and A³ is C-R⁹.

Another embodiment of the benzimidazole derivative of the present invention in formula (I) include a benzimidazole derivative or a pharmaceutically acceptable salt, wherein A¹ is C-R⁷, A² is C-R⁸, and A³ is a nitrogen atom.

Another embodiment of the benzimidazole derivative of the present invention in formula (I) include a benzimidazole derivative or a pharmaceutically acceptable salt, wherein A¹ and A³ are both nitrogen atoms and A² is C-R⁸.

Another embodiment of the benzimidazole derivative of the present invention in formula (I) include the compounds of Examples 1 to 288 below or pharmaceutically acceptable salts thereof.

The terms used in this specification are explained below.

A "halogen atom" represents a fluorine atom, a chlorine atom, a bromine atom or an iodine atom unless otherwise specified.

An unsubstituted alkyl group (also simply referred to as an alkyl group) means a linear or branched saturated hydrocarbon group (C1-4 alkyl) having 1 to 4 carbon atoms unless otherwise specified. Specific examples include methyl, ethyl, propyl, isopropyl, n-butyl, isobutyl, sec-butyl, tert-butyl and the like.

An unsubstituted alkenyl group (also referred to simply as an alkenyl group) means a linear or branched hydrocarbon group (C2-6 alkenyl) having 2 to 6 carbon atoms and having at least one carbon-carbon double bond. Specific examples include vinyl group, allyl group, 1-propenyl group, isopropenyl group and 2-methylallyl group.

An unsubstituted alkynyl group (also referred to simply as an alkynyl group) means a linear or branched hydrocarbon group (C2-6 alkynyl) having 2 to 6 carbon atoms and having at least one carbon-carbon triple bond. Specific examples include ethynyl group, 1-propynyl, 2-propynyl, 1-butenyl, 2-butenyl and 3-butenyl groups.

An unsubstituted cycloalkyl group (also referred to simply as a cycloalkyl group) means a cyclic alkyl group having 3 to 7 carbon atoms (C3-7 cycloalkyl), specifically cyclopropyl, cyclobutyl, cyclopentyl, cyclohexyl, cycloheptyl and the like.

An unsubstituted cycloalkenyl group (also simply referred to as a cycloalkenyl group) means a cyclic hydrocarbon group having 3 to 7 carbon atoms (C3-7 cycloalkenyl) having at least one double bond. Specific examples include cyclopropenyl, cyclobutenyl, cyclopentenyl, cyclohexenyl, cycloheptenyl and the like.

An unsubstituted alkoxy group (also simply referred to as an alkoxy group) is a monovalent substituent (C1-4 alkoxy) in which the alkyl group is bonded to a substituted position via an oxygen atom (-O-). Specific examples include methoxy, ethoxy, n-propoxy, isopropoxy, n-butoxy, t-butoxy and the like.

An unsubstituted cycloalkyloxy group (also simply referred to as a cycloalkyloxy group) is a monovalent substituent in which the cycloalkyl group is a monovalent substituent (C3-7 cycloalkyloxy) bonded to the substituent position through an oxygen atom (-O-). Specific examples include cyclopropyloxy, cyclobutyloxy, cyclopentyloxy, cyclohexyloxy, cycloheptyloxy and the like.

An unsubstituted aryl group (also referred to simply as an aryl group) means a monocyclic or bicyclic aryl group having 6 to 14 carbon atoms, such as phenyl, naphthyl, and indenyl.

An unsubstituted heteroaryl group (also referred to simply as a heteroaryl group) is a 5- to 10-membered monocyclic or bicyclic heteroaryl group containing 1 to 4 heteroatoms independently selected from the group consisting of a sulfur atom, an oxygen atom and a nitrogen atom. Specific examples include a bicyclic heteroaryl group, specifically pyrrolyl, furanyl, thienyl, imidazolyl, pyrazolyl, oxazolyl, thiazolyl, triazolyl, tetrazolyl, pyridyl, pyrazinyl, pyrimidinyl, pyridazinyl, thiazinyl, triazinyl, indolyl, isoindolyl, indazolyl, benzimidazolyl, benzothiazolyl, benzofuranyl, quinolyl, isoquinolyl, imidazopyridyl, benzopyranyl and the like.

In the present specification, 5- or 6-membered monocyclic nitrogen-containing heteroaryl groups such as pyrrolyl, imidazolyl, pyrazolyl, triazolyl, tetrazolyl, pyridyl, pyrazinyl, pyrimidinyl, pyridazinyl, triazinyl and the like are preferably used.

An unsubstituted aryloxy group (also simply referred to as an aryloxy group) is a monovalent substituent in which the aryl group is bonded to the substituted position via an oxygen atom (-O-). Specific examples include phenoxy, naphthyloxy, indenyloxy and the like.

An unsubstituted heteroaryloxy group (also referred to simply as a heteroaryloxy group) is a monovalent substituent in which the heteroaryl group is bonded to a substituted position via an oxygen atom (-O-). Specific examples include furanyloxy, thienyloxy, pyrrolyloxy, imidazolyloxy, pyrazolyloxy, oxazolyloxy, thiazolyloxy, triazolyloxy, tetrazolyloxy, pyridyloxy, pyrazinyloxy, pyrimidinyloxy, pyridazinyloxy, and the like.

An unsubstituted heterocyclo group (also referred to simply as a heterocyclo group) means a 3- to 8-membered saturated or partially saturated heterocyclic group containing at least one heteroatom selected from a nitrogen atom, a sulfur atom and an oxygen atom. Specific examples include morpholino, piperazinyl, tetrahydrofuryl, tetrahydropyranyl, pyrrolidyl and the like.

An unsubstituted heterocyclooxy group (also simply referred to as a heterocyclooxy group) is a monovalent substituent in which the heterocyclo group is bonded to a substitution position via an oxygen atom (-O-). Specific examples include methylpiperidinyloxy, oxetanyloxy, pyranyloxy and the like.

An unsubstituted arylsulfonyl group (also referred to simply as an arylsulfonyl group) is a monovalent substituent in which the aryl group is bonded via a sulfonyl (-SO2-). Specific examples include benzenesulfonyl, naphthalenesulfonyl, and the like.

Alkoxy of alkoxycarbonyl is the same as the above alkoxy.

In the above formula (I), the ring portion of "R² and R³, or R³ and R⁴, or R⁴ and R⁵ that may be bonded to each other to form a ring" contains at least one heteroatom selected from a nitrogen atom and an oxygen atom. An optionally substituted saturated or unsaturated hetero 5-membered or hetero 6-membered ring is exemplified. A specific example is 1,4-dioxane condensed with an aromatic ring.

Substituents for the above terms will now be described.

A halogen atom is exemplified as the substituent of the substituted alkyl group, and one or a plurality of the same or different halogen atoms may be substituted at any position. Specifically, a trifluoromethyl group is exemplified as a substituted alkyl group.

Other substituents of the substituted alkyl group include the hydroxyl group, methoxy group, dimethylamino group, cyclopropyl group, dimethylcarbamoyl group, cyano group, morphonyl group and the like. One or more of the same or different substituents, including the halogen atom, may be substituted at any position of the alkyl group.

The substituents of the substituted alkenyl group, substituted alkynyl group, and substituted alkoxy group are the same as those of the substituted alkyl group.

Examples of substituents on the substituted amino group include the alkyl groups described above, and one alkyl group or two identical or two different alkyl groups may be substituted. Specifically, a dimethylamino group is exemplified as a substituted amino group.

Examples of substituents on the substituted carbamoyl group include the alkyl groups described above, and one alkyl group or two identical or two different alkyl groups may be substituted. In addition, a phenyl group and a propargyl group can be cited as specific examples of substituents other than the alkyl group.

Substituents examples of substituted cycloalkyl, substituted cycloalkenyl, substituted aryl and substituted heteroaryl include a halogen atom, a substituted or unsubstituted alkyl group, a substituted or unsubstituted amino group, a nitro group, a cyano group or a methylsulfonyl group. Substituents for substituted aryloxy, substituted heteroaryloxy and substituted arylsulfonyl are the same as those for substituted aryl, substituted heteroaryl and substituted aryl, respectively.

In the compound of the present invention represented by the formula (1), the definitions and preferred ranges of R¹ to R⁹ are as follows, but the technical scope of the present invention is not limited to the ranges of the compounds listed below.

Examples of R¹ include a hydrogen atom, a halogen atom, an optionally substituted C1-4 alkyl (for example, C1-4 alkyl optionally substituted with halogen), a C2-6 alkenyl, a C2-6 alkynyl, a C3-7 cycloalkyl, a C3-7 cycloalkenyl, a C1-4 alkoxy, a phenyl, a C1-4 alkoxycarbonyl, a carboxy, a cyano, a phenylcarbamoyl, a 2-propynylcarbamoyl, a 4-morpholine carbonyl and the like. The halogenated C1-4 alkyl is exemplified by trifluoromethyl.

R² to R⁵ may each independently and optionally include a hydrogen atom, a halogen atom, an optionally substituted C1-4 alkyl (for example, optionally substituted with halogen and/or C1-4 alkoxy), an optionally substituted C2-6 alkenyl (for example, C1-4 alkoxy, optionally substituted with halogen and/or cyclopropyl), an optionally substituted C2-6 alkynyl (for example, C1-4 alkoxy, optionally substituted with cyclopropyl and/or dimethylamino), a C3-7 cycloalkyl (for example, cyclopropyl), an optionally substituted C1-4 alkoxy (for example, optionally substituted with C1-4 alkoxy), a C3-7 cycloalkyloxy (for example, cyclohexyloxy), an optionally substituted phenyl (for example, optionally substituted with halogen or morpholinomethyl), an optionally substituted phenoxy (methylsulfonyl, dimethylamino, cyano, optionally substituted with a halogen atom and/or dimethylaminomethyl), an optionally substituted monocyclic nitrogen-containing heteroaryl (for example, pyridyl, methylpyrazolyl, etc.), an optionally substituted monocyclic nitrogen-containing heteroaryloxy (for example, pyridyloxy, methylpyrazyloxy, etc.), a heterocyclo (for example, dihydropyranyl, tetrahydropyranyl, piperidyl), a heterocyclooxy (for example, oxetanyloxy, tetrahydropyranyloxy, piperidyl) oxy, etc.), a substituted aminosulfonyl (for example, N-methyl, N-phenylaminosulfonyl, etc.), a dimethylcarbamoyl, a benzyloxy, a cyano, a nitro, a carboxy, a methoxycarbonyl, and the like. Here, the halogenated C1-4 alkyl includes trifluoromethyl, and the halogenated C1-4 alkoxy includes trifluoromethyloxy.

R² and R³, R³ and R⁴, or R⁴ and R⁵ may be bonded to each other to form a ring, and the ring formed is exemplified by 1,4-dioxane condensed with an aromatic ring.

R⁶ may be a hydrogen atom, an optionally substituted C1-4 alkyl (for example, optionally substituted with hydroxy, cyclopropyl, C1-4 alkoxy, dimethylamino, dimethylcarbamoyl or methylsulfonyl), a C2-6 alkenyl, a C2-6 alkynyl, a C3-7 cycloalkyl, an optionally substituted amino (for example, dimethylamino, methylamino, amino, etc.), an optionally substituted heteroaryl (for example, pyridyl, methylimidazolyl, etc.), or a heterocyclo (for example, oxetanyl, pyrrolidyl, etc.).

R⁷ to R⁹ may include a hydrogen atom, a halogen atom, a C1-4 alkyl, a C2-6 alkenyl, a C3-7 cycloalkyl, a C1-4 alkoxy, a phenyl, a monocyclic nitrogen-containing heteroaryl (for example, pyridyl, methylpyrazolyl, etc.), a hydroxy, a dimethylamino, or a dimethylcarbamoyl.

Compound (I) of the present invention may have isomers depending on, for example, the type of substituent. In this specification, although the chemical structures of only one form of the isomers are described, the present invention also includes all isomers (geometric isomers, optical isomers, tautomers, etc.) that can occur structurally, and also includes single isomers and mixtures thereof.

In the present invention, the "hydrogen atom" includes ¹H and ²H (D). Deuterium conversion products obtained by converting any one or two or more ¹H of the compounds represented by formula (I) to ²H (D) are also included in the compounds represented by formula (I).

In addition, pharmaceutically acceptable salts of the compound (I) of the present invention include inorganic acid salts with hydrochloric acid, sulfuric acid, carbonic acid, phosphoric acid and the like, and organic acid salts with formic acid, acetic acid, fumaric acid, maleic acid, methanesulfonic acid, p-toluenesulfonic acid and the like. In addition, alkali metal salts with sodium, potassium, etc., alkaline earth metal salts with magnesium, calcium, etc., organic amine salts with lower alkylamines and lower alcohol amines, etc., basic amino acid salts with lysine, arginine and ornithine etc., and ammonium salts and the like are also included in the present invention.

Compound (I) and its pharmaceutically acceptable salts of the present invention include both inner salts and solvates such as hydrates.

Compound (I) of the present invention and pharmaceutically acceptable salts thereof can be produced, for example, by the following methods. In the production methods shown below, if the defined group changes under the conditions of the method or is unsuitable to carry out the method, it can be easily produced by a method commonly used in organic synthetic chemistry, for example, by means of protecting or deprotecting a functional group [T. W. Greene, Protective Groups in Organic Synthesis 3rd Edition, John Wiley & Sons, Inc., 1999]. In addition, the order of reaction steps such as introduction of substituents can be changed as necessary.

The following general reaction schemes are used to detail the synthesis of the benzimidazole derivatives disclosed in the present invention. The compounds of the present invention of formula (I) disclosed herein can be prepared by the methods described in Schemes 1-5 below, as well as by general synthetic methods, as provided in the Examples. It can be produced by changing a general synthesis method, a commercially available starting material, a starting material that can be synthesized from a commercially available compound by a known method or a method analogous thereto, or a method well known to those skilled in the art.

Each variable depicted in the schemes below applies to all functional groups detailed in the compounds provided in this invention. Tautomers and solvates (for example, hydrates) of compounds of formula (I) are also included in the present invention.

Abbreviations and symbols used in the following description have the following meanings.
DMF: N, N-dimethylformamide
NMP: N-methylpyrrolidone
THF: Tetrahydrofuran
DMSO: Dimethylsulfoxide
Boc: Tert-butoxycarbonyl
Boc₂O: Di-tert-butyl dicarbonate
CDI: 1,1'-carbonyldiimidazole
EDCI/HCl: 1-ethyl-3-(3-dimethylaminopropyl) carbodiimide hydrochloride
DIPEA: N, N-diisopropylamine

### [Production method of compound (I) of the present invention]

The compounds of the present invention shown by formula (I) can be manufactured by, for example, Scheme 1 below: 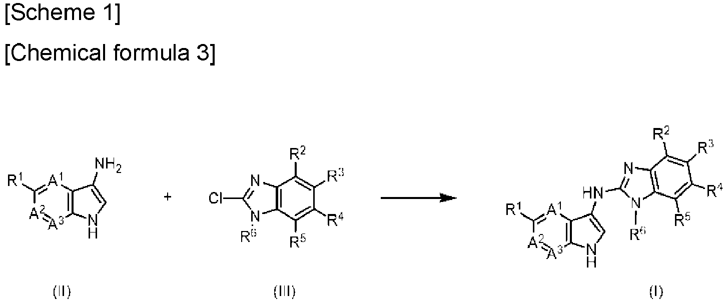 (where A¹, A², A³, R¹, R², R³, R⁴, R⁵ and R⁶ are each as defined above).

The compound (I) of the present invention can be produced by subjecting the benzimidazole skeleton to a nucleophilic substitution reaction using the compound (II). That is, compound (I) of the present invention can be obtained by reacting compound (II) with 0.5 to 5 molar equivalents of compound (III) in a solvent in the presence of an acid. Any solvent may be used as long as it is inert to the reaction, and is not particularly limited. For example, DMF, THF, NMP, 1,4-dioxane, ethanol, isopropanol, n-butanol, 2-butanol and the like can be used, but preferably NMP or 1,4-dioxane and the like can be used. The acid used in the reaction is not particularly limited, and an inorganic acid or an organic acid can be used. For example, hydrochloric acid, p-toluenesulfonic acid and the like are commonly used. The amount of the acid used can be an equivalent amount or an excess amount relative to compound (II). Preferably, in the case of hydrochloric acid, 4N hydrochloric acid/1,4-dioxane solution and the like are mentioned, and in the case of p-toluenesulfonic acid, it is 1 to 5 molar equivalents. The reaction can be carried out in the range of 0°C to 200°C for several minutes to several days. It can be carried out by reacting for 5 to 48 hours.

In addition, a compound represented by formula (I) can also be obtained under the same conditions as in Scheme 1 using a compound in which the amino group of compound (II) is protected with a protecting group that can be deprotected under acidic conditions, such as a Boc group.

Compound (II) used as a starting material in Scheme 1 is commercially available, or it can be prepared from compound (IV), for example, using Scheme 2 below. 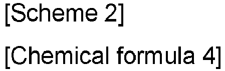 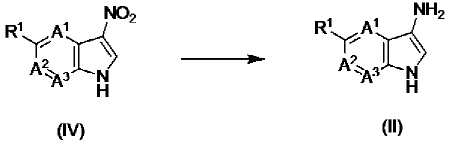 (where A¹, A², A³ and R¹ are the same as defined above).

Compound (II) can be produced by reducing the nitro group of compound (IV). That is, compound (II) can be obtained by subjecting compound (IV) in a solvent to a reduction method commonly used in synthetic organic chemistry, such as catalytic reduction using palladium carbon or the like, or metal reduction using tin, zinc, iron, or the like, to form a nitro group.

Also, a compound in which the amino group of compound (II) is protected with a Boc group can be obtained by reacting compound (IV) with Boc₂O in a solvent in the presence of a metal such as ammonium chloride and zinc.

Compound (IV) used as a starting material in Scheme 2 can be obtained as a commercial product, or can be prepared from compound (V) according to, for example, Scheme 3 below. 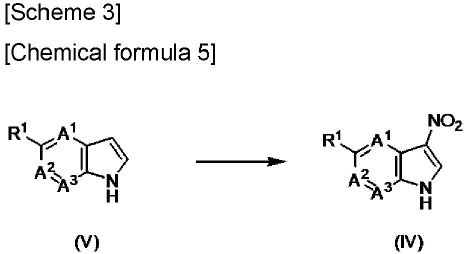 (where A¹, A², A³ and R¹ are the same as defined above).

The compound (IV) can be produced by nitrating compound (V). That is, the compound (IV) can be obtained by reacting compound (V) under nitration reaction conditions generally used in organic chemistry, such as under fuming nitric acid, mixed acid of concentrated sulfuric acid and nitric acid. Although the nitrating agent is not particularly limited, for example, 1 to 5 molar equivalents of potassium nitrate can be used in the presence of concentrated sulfuric acid. The reaction can be carried out at - 20°C to 50°C for several minutes to several days, but preferably at -20°C to 0°C for 10 minutes to 1 hour.

In addition, compound (IV) can also be produced by a known method or an analogous method [for example, Bioorg. Med. Chem. 2007, 15, 3248-3265 or Tetrahedron Letters 2012, 53, 4841-4842]. That is, compound (IV) can be obtained by reacting compound (V) with 1 to 5 molar equivalents of a nitrating agent and 1 to 5 molar equivalents of acid chloride in a solvent.

Compound (V) used as a starting material in Scheme 3 can be obtained as a commercial product, or can be produced by a known method or a method analogous thereto [for example, J. Org. Chem. 2010, 75, 11-15.].

Compound (III) used as a starting material in Scheme 1 can be obtained as a commercial product or can be prepared from compound (VI), for example, in Scheme 4 below. 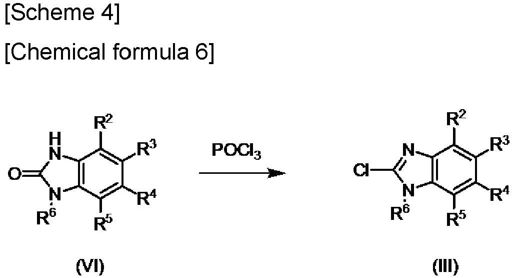 (where R², R³, R⁴, R⁵ and R⁶ are each as defined above).

The compound (III) can be produced by chlorinating compound (VI). That is, the compound (III) can be obtained by treating compound (VI) with a chlorinating agent such as phosphorus oxychloride, or optionally in the presence of a solvent. The reaction can be carried out in the range of 0°C to 200°C for several minutes to several days, but preferably at 70°C to 150°C for 1 hour to 24 hours.

The compound (VI) used as a starting material in Scheme 4 is obtained as a commercial product, or can be prepared from compound (VII), for example, in Scheme 5 below. 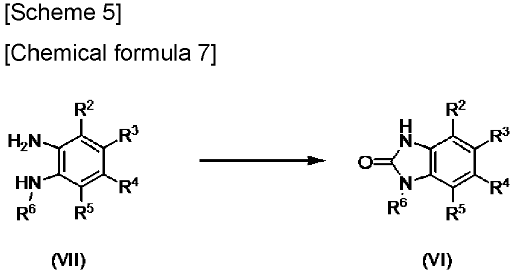 (where R², R³, R⁴, R5 and R⁶ are each as defined above).

The compound (VI) can be produced by converting two adjacent amino groups of compound (VII) into cyclic urea. That is, the compound (VI) can be obtained by reacting compound (VII) with a carbonylation reagent such as triphosgene or CDI in a solvent. Any solvent can be used as long as it is inert to the reaction. Dichloromethane, NMP, DMF, THF and the like can be used, but THF is preferably used. The reaction can be carried out in the range of 0°C to 150°C for several minutes to several days, but preferably at room temperature to 100°C for 10 minutes to 24 hours.

The compound (VII) used as a starting material in Scheme 5 is commercially available, or it can be produced by a known method or a method analogous thereto [for example, J. Med. Chem. 2011, 54, 7920-7933 and J.P. Org. Chem. 2017, 82, 9243-9252].

The compound of the present invention represented by formula (I) can be produced by the method shown in Scheme 1, as well as by the method shown in Scheme 6 below. 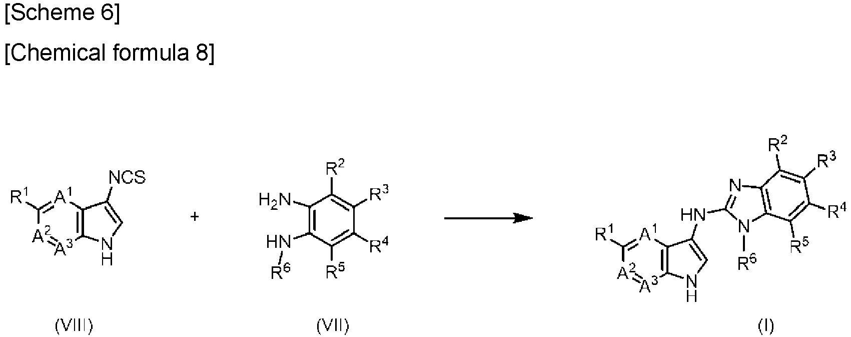 (where A¹, A², A³, R¹, R², R³, R⁴, R⁵ and R⁶ are each as defined above).

The compound (I) of the present invention can be produced by cyclizing a thiourea derivative obtained by reacting compound (VII) with compound (VIII). That is, the compound (I) of the present invention is a thiourea derivative obtained by reacting compound (VII) with 0.5 to 1.5 molar equivalents of compound (VIII) in a solvent. It can be obtained by a cyclization reaction using condensation conditions generally used in synthetic organic chemistry, for example, using 1 to 3 molar equivalents of a condensing agent such as EDCI/HCl. In the synthesis of the thiourea derivative, any solvent may be used as long as it is inert to the reaction, and chloroform, THF, DMF, NMP and the like can be used, but DMF can be preferably used. The reaction can be carried out in the range of 0°C to 100°C for several minutes to several days, but preferably at room temperature to 80°C for 10 minutes to 24 hours. The thiourea derivative obtained by the reaction can be used as it is for the next reaction without purification, but the purified product can also be subjected to the condensation cyclization reaction. In the condensation cyclization reaction, any solvent may be used as long as it is inert to the reaction, and is not particularly limited. For example, DMF, THF, NMP, etc. can be used, but preferably DMF can be used. The reaction can be carried out in the range of 0°C to 100°C for several minutes to several days. Preferably, the reaction can be carried out at room temperature to 80° C for 5 hours to 24 hours.

The compound (VIII) used as a starting material in Scheme 6 can be produced, for example, from compound (II) as shown in Scheme 7. 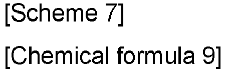 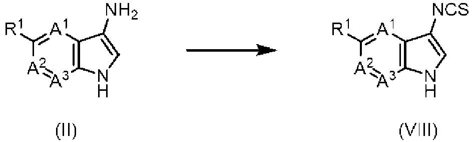 (where A¹, A², A³ and R¹ are the same as defined above)

The compound (VIII) can be produced by converting the amino group of compound (II) to an isothiocyanate group. That is, the compound (VIII) can be obtained by reacting compound (II) with 1 to 3 molar equivalents of an isothiocyanating reagent such as thiophosgene in the presence of 1 to 3 molar equivalents of a base such as DIPEA in a solvent. Any solvent can be used as long as it is inert to the reaction, and chloroform, THF and the like can be used, but THF is preferably used. The reaction can be carried out in the range of 0°C to room temperature for several minutes to several days, but preferably at room temperature for 10 minutes to 24 hours.

The compound (I) of the present invention having a desired functional group at a desired position can be obtained by appropriately combining the above methods and carrying out a method commonly used in organic synthetic chemistry (for example, alkylation reactions of the amino group, reactions that convert the carboxyl group to substituted or unsubstituted carboxamide group, cross-coupling reactions such as Suzuki-Miyaura reactions, and reduction of carbon-carbon double bond by hydrogenation reactions).

### [Use of compound (I) of the present invention]

Compound (I) or a pharmaceutically acceptable salt of the present invention can be prepared in the form of conventional pharmaceutical formulations (pharmaceutical compositions) suitable for oral, parenteral or topical administration.

Formulations for oral administration include solid formulations such as tablets, granules, powders and capsules, and liquid formulations such as syrups. These formulations can be prepared by conventional methods. Solid formulations can be prepared by using conventional pharmaceutical carriers such as lactose, starch such as corn starch, microcrystalline cellulose such as microcrystalline cellulose, hydroxypropylcellulose, calcium carboxymethylcellulose, talc, magnesium stearate, and the like. Capsules can be prepared by encapsulating the prepared granules or powder. A syrup can be prepared by dissolving or suspending the compound (I) of the present invention or a pharmaceutically acceptable salt thereof in an aqueous solution containing sucrose, carboxymethylcellulose and the like.

Formulations for parenteral administration include injections such as infusions. Injection formulations can also be prepared by conventional methods and include tonicity agents (for example, mannitol, sodium chloride, glucose, sorbitol, glycerol, xylitol, fructose, maltose, and mannose), stabilizers (for example, sodium sulfite and albumin), and preservatives (for example, benzyl alcohol and methyl p-oxybenzoate) as appropriate.

The dose of compound (I) of the present invention or a pharmaceutically acceptable salt can vary according to the severity of the disease, age and weight of the patient, dosage form, etc., but the usual range for adults is 1 mg to 1,000 mg per day, which can be administered in single doses or in 2 or 3 divided doses by the oral or parenteral route.

Compound (I) of the present invention or a pharmaceutically acceptable salt can also be used as a STING inhibitor and as a reagent for experiments and research.

In addition, the compound (I) of the present invention, which is radioactively labeled, can also be used as a molecular probe for PET.

### Embodiments

The present invention will be described in more detail with reference to examples and test examples below, but the present invention is not limited by these examples.

The compounds were identified by hydrogen nuclear magnetic resonance spectroscopy (¹H-NMR) and mass spectroscopy (MS). ¹H-NMR was measured at 400 MHz unless otherwise specified and exchangeable hydrogen may not be clearly observed depending on the compound and measurement conditions. In addition, br means a broad signal (broad). For HPLC preparative chromatography, a commercially available ODS column was used, and water/acetonitrile (containing formic acid) or water/methanol (containing formic acid) was used as an eluent for fractionation in the gradient mode.

### Embodiment 1

### Preparation of N-(5-bromo-1H-indol-3-yl)-1H-benzo[d]imidazole-2-amine

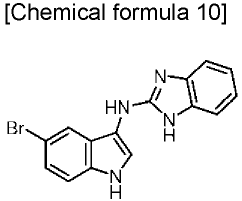

Tert-butyl 2-chloro-1H-benzo[d]imidazole-1-carboxylate (20 mg, 0.079 mmol) and 5-bromo-1H-indol-3-amine (18. 38 mg, 0.087 mmol) in 1,4-dioxane solution (1 mL) were added with 4N hydrochloric acid/1,4-dioxane solution (0.087 mmol), and the mixture was stirred at 120°C for 1.5 hours. After cooling the reaction mixture to room temperature, the solvent was distilled off under reduced pressure, and the residue was purified by silica gel chromatography (chloroform:methanol=10:0-9:1) to obtain the title compound (4 mg).

¹H NMR (400 MHz, DMSO-d₆) δ 11.02 (s, 1H), 10.71 (s, 1H), 9.06 (s, 1H ), 7.85 (d, J = 2.0 Hz, 1H), 7.82 (d, J = 2.5 Hz, 1H), 7.27 - 7.17 (m , 1H), 7.21 (dd, J = 8.6, 2.0 Hz, 3H), 6.93 (s, 2H); LCMS(m/z) 329.1 [M+H]⁺.

### Embodiment 2

### Preparation of N-(5-phenyl-1H-indol-3-yl)-1H-benzo[d]imidazole-2-amine

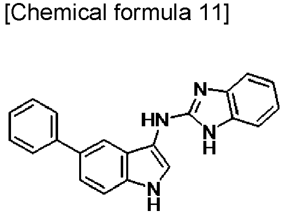

### (First step)

5-bromo-3-nitro-1H-indole (80 mg, 0.332 mmol), phenylboronic acid (50. 6mg, 0.415 mmol), and [1,1'-bis(diphenylphosphino)ferrocene] palladium (II) dichloride dichloromethane adduct (27.1 mg, 0.033 mmol) in 80% 1,4-dioxane aqueous suspension (2.5 mL) were added with sodium carbonate (106 mg, 0.996 mmol) and heated to reflux for 18 hours. After cooling the reaction mixture to room temperature, ethyl acetate and water were added to the reaction mixture to separate the organic layer. The aqueous layer was extracted with ethyl acetate, and the obtained organic layers were combined, washed with saturated brine, and dried over anhydrous sodium sulfate. The solvent was evaporated under reduced pressure, and the residue was purified by silica gel chromatography (hexane:ethyl acetate=1:0-0:1) to produce 3-nitro-5-phenyl-1H-indole (44 mg).

LCMS (m/z) 239.2 [M+H]⁺.

### (Second step)

3-nitro-5-phenyl-1H-indole (43 mg, 0.180 mmol) was dissolved in ethanol (2 mL), and 10% palladium carbon (10 mg) was added. The reaction was carried out at room temperature for 3 hours under a hydrogen atmosphere. After filtering the insoluble matter, the filtrate was concentrated to obtain 5-phenyl-1H-indol-3-amine (30 mg).

LCMS (m/z) 209.2 [M+H]⁺.

### (Third step)

5-phenyl-1H-indol-3-amine (29 mg, 0.139 mmol) and 2-chloro-1H-benzo[d]imidazole (25.5 mg, 0.167 mmol) were added to 4N hydrochloric acid/1,4-dioxane solution (1 mL) and stirred at 100°C for 2 hours. The reaction mixture was cooled to room temperature, and ethyl acetate and saturated sodium hydrogencarbonate aqueous solution were added, and the organic layer was separated. The aqueous layer was extracted with ethyl acetate, and the obtained organic layers were combined, washed with water and saturated brine, and dried over anhydrous sodium sulfate. The solvent was removed under reduced pressure and the residue was purified using HPLC preparative chromatography to give the title compound as the formate salt (1.1 mg).

¹H NMR (400 MHz, Methanol-d₄) δ 8.51 (s, 1H), 7.73 - 7. 70 (m, 1H), 7.6 2 - 7.57 (m, 2H), 7.53 - 7.46 (m, 3H), 7.39 - 7.33(m, 2H), 7.28 - 7.2 1 (m, 3H), 7.10 - 7.05 (m, 2H); LCMS(m/z) 325.2 [M+H]⁺.

### Embodiment 3

### Preparation of N-(5-bromo-1H-indol-3-yl)-5-phenoxy-1H-benzo[d]imidazole-2-amine

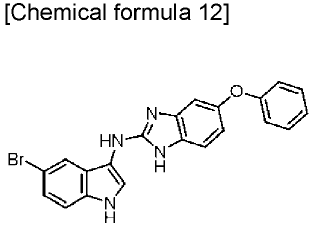

### (First step)

4-phenoxybenzene-1,2-diamine (426 mg, 2.13 mmol) and CDI (517 mg, 3.19 mmol) were added to THF (15 mL) and stirred for 0.5 hours at room temperature. Ethyl acetate was added to the reaction mixture, and the mixture was washed with a saturated aqueous sodium hydrogencarbonate solution. The obtained organic layer was dried over anhydrous sodium sulfate, and the solvent was distilled off under reduced pressure, and the residue was purified by silica gel chromatography (chloroform:methanol=1:0-0:1) to obtain 5-phenoxy-1,3-dihydro-2H-benzo[d]imidazole-2-one (280 mg).

LCMS (m/z) 227.2 [M+H]⁺.

### (Second step)

A mixture of 5-phenoxy-1,3-dihydro-2H-benzo[d]imidazole-2-one (280 mg, 1.24 mmol) and phosphorus oxychloride (4.6 mL) were stirred at 100°C for 2.5 hours. The reaction mixture was cooled to room temperature, and ice was added, and ethyl acetate and saturated aqueous sodium bicarbonate were added, and the organic layer was separated. The obtained organic layer was washed with saturated aqueous sodium hydrogencarbonate solution and saturated brine, and dried over anhydrous sodium sulfate. The solvent was distilled off under reduced pressure to obtain 2-chloro-5-phenoxy-1H-benzo[d]imidazole (280 mg).

LCMS (m/z) 245.2 [M+H]⁺.

### (Third step)

5-bromo-1H-indol-3-amine (12 mg, 0.057 mmol) and 2-chloro-5-phenoxy-1H-benzo[d]imidazole (13.9 mg, 0.057 mmol) were added to 4N hydrochloric acid/1,4-dioxane solution (1 mL) and stirred at 120°C for 1 hour. To complete the reaction, 5-bromo-1H-indol-3-amine (6 mg, 0.029 mmol) was further added and stirred at 120°C for 1 hour. The reaction mixture was cooled to room temperature, and the solvent was distilled off under reduced pressure, and the residue was purified by silica gel chromatography (hexane:ethyl acetate=1:0-0:1, then ethyl acetate:methanol=1:0-0:1) to obtain the title compound (8.3 mg).

¹H NMR (400 MHz, DMSO-d₆) δ 11. 03 (s, 1H), 10.75 - 10.65 (m, 1H), 9. 11 (s, 1H), 7.85 - 7.80 (m, 2H), 7.40 - 7.28 (m, 3H), 7.27 - 7.14 (m, 2H), 7.11 - 6. 99 (m, 1H), 6. 99 - 6.83 (m, 3H), 6.78 - 6. 59 (m, 1H);

LCMS(m/z) 421.2 [M+H]⁺.

### Embodiment 4

### Preparation of N-(5-bromo-1H-indol-3-yl)-5-chloro-1H-benzo[d]imidazole-2-amine

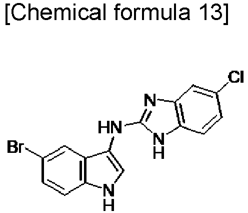

5-bromo-1H-indol-3-amine (30 mg, 0.142 mmol) and 2,5-dichloro-1H-benzo[d]imidazole (26.6 mg, 0.142 mmol) were added to 4N hydrochloric acid/1,4-dioxane solution (1.5 mL) and stirred overnight under reflux conditions. After cooling the reaction mixture to room temperature, the solvent was distilled off under reduced pressure, the residue was crudely purified by silica gel chromatography (chloroform:methanol=1:0-19:1). The mixture was further purified by HPLC preparative chromatography to obtain the title compound as the formate salt (23 mg).

¹H NMR (400 MHz, DMSO-d6) δ 11.06 (s, 1H), 10.94 - 10.84 (m, 1H), 9.2 1 (s, 1H), 8.15 (s, 1H), 7.82 (d, J = 1.9 Hz, 1H), 7.79 (d, J = 2.5 H z, 1H), 7.35 (d, J = 8.6 Hz, 1H), 7.26 - 7.11 (m, 3H), 6.92 (brs, 1H) ; LCMS(m/z) 363.1 [M+H]⁺.

### Embodiment 5

### Preparation of N-(5-bromo-1H-indol-3-yl)-4-methyl-1H-benzo[d]imidazole-2-amine

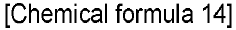

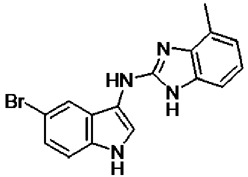

5-bromo-1H-indol-3-amine (30 mg, 0.142 mmol) and 2-chloro-4-methyl-1H-benzo[d]imidazole (22.5 mg, 0.135 mmol) were added to 4N hydrochloric acid/1,4-dioxane solution (3 mL) and stirred overnight under reflux conditions. After cooling the reaction mixture to room temperature, the solvent was distilled off under reduced pressure, the residue was crudely purified by silica gel chromatography (chloroform:methanol=1:0-9:1), and further purified by HPLC preparative chromatography to obtain the title compound as the formate salt (6 mg).

¹H NMR (400 MHz, DMSO-d6) δ 10.99 (s, 1H), 10.70 (s, 1H), 9.03 (s, 1H ), 8.20 (s, 1H), 7.86 - 7.80 (m, 2H), 7.34 (d, J = 8.6 Hz, 1H), 7.20 (dd, J = 8.6, 1.9 Hz, 1H), 7.02 (s, 1H), 6.77 (brs, 2H), 2.42 (s, 3H)

; LCMS(m/z) 343.1 [M+H]⁺.

### Embodiment 6

### Preparation of N-(5-bromo-1H-indol-3-yl)-5-methoxy-1H-benzo[d]imidazole-2-amine

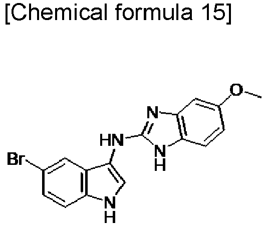

5-bromo-1H-indol-3-amine (30 mg, 0.142 mmol) and 2-chloro-5-methoxy-1H-benzo[d]imidazole (23.36 mg, 0.128 mmol) were added to 4N hydrochloric acid/1,4-dioxane solution (2 mL) and stirred overnight under reflux conditions. After cooling the reaction mixture to room temperature, the solvent was distilled off under reduced pressure, the residue was crudely purified by silica gel chromatography (chloroform:methanol=1:0-19:1), and further purified by HPLC preparative chromatography to obtain the title compound as the formate salt (16 mg).

¹H NMR (400 MHz, DMSO-d6) δ 10. 97 (s, 1H), 10.52 (brs, 1H), 9.01 (s, 1H), 8.16 (s, 1H), 7.86 (d, J = 1.9 Hz, 1H), 7.81 (d, J = 2.4 Hz, 1H) , 7.33 (d, J = 8.6 Hz, 1H), 7.20 (dd, J = 8.6, 1.9 Hz, 1H), 7.09 (s, 1H), 6.83 (s, 1H), 6.54 (s, 1H), 3.73 (s, 3H); LCMS(m/z) 357.1 [M+H]⁺.

### Embodiment 7

### Preparation of 5-bromo-N-(5-bromo-1H-indol-3-yl)-1H-benzo[d]imidazole-2-amine

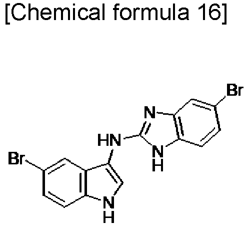

5-bromo-1H-indol-3-amine (30 mg, 0.142 mmol) and 5-bromo-2-chloro-1H-benzo[d]imidazole (29.6 mg, 0.128 mmol) were added to 4N hydrochloric acid/1,4-dioxane solution (2 mL) and stirred overnight under reflux conditions. After cooling the reaction mixture to room temperature, the solvent was distilled off under reduced pressure, the residue was crudely purified by silica gel chromatography (chloroform:methanol=1:0-9:1), and further purified by HPLC preparative chromatography to obtain the title compound as the formate salt (16 mg).

¹H NMR (400 MHz, DMSO-d6) δ 11.06 (s, 1H), 11.00 - 10.86 (m, 1H), 9.2 5 (s, 1H), 8.16 (s, 1H), 7.83 (d, J = 1.9 Hz, 1H), 7.79 (s, 1H), 7.39 - 7.31 (m, 2H), 7.21 (dd, J = 8.6, 1.9 Hz, 1H), 7.19 - 6.99 (m, 2H);

LCMS(m/z) 407.1 [M+H]⁺.

### Embodiment 8

### Preparation of N-(5-bromo-1H-indol-3-yl)-5-(trifluoromethyl)-1H-benzo [d]imidazole-2-amine

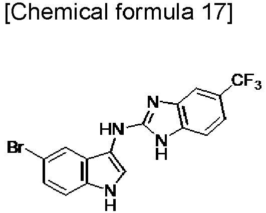

5-bromo-1H-indol-3-amine (30 mg, 0.142 mmol) and 2-chloro-5-(trifluoromethyl)-1H-benzo[d]imidazole (28.2 mg, 0.128 mmol) were added to 4N hydrochloric acid/1,4-dioxane solution (2 mL) and stirred overnight under reflux conditions. After cooling the reaction mixture to room temperature, the solvent was distilled off under reduced pressure, the residue was crudely purified by silica gel chromatography (chloroform:methanol=1:0-9:1), and further purified by HPLC preparative chromatography to obtain the title compound as the formate salt (20 mg).

¹H NMR (400 MHz, DMSO-d6) δ 11.27 - 11.07 (m, 2H), 9.44 - 9.37 (m, 1H ), 8.17 (s, 1H), 7.86 - 7.79 (m, 2H), 7.49 (d, J = 19.9 Hz, 1H), 7.41 - 7.32 (m, 2H), 7.31 - 7.19 (m, 2H); LCMS(m/z) 397.1 [M+H]⁺.

### Embodiment 9

### Preparation of N-(4-bromo-1H-indol-3-yl)-1H-benzo[d]imidazole-2-amine

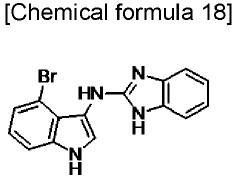

### (First step)

4-bromo-3-nitro-1H-indole (500 mg, 2.08 mmol) in methanol/water (10:3, 13 mL) was added with zinc dust (1.36 g, 20.83 mmol) and ammonium chloride (557 mg, 10.42 mmol) at 0°C and stirred at 0°C for 30 minutes. Boc₂O (545 mg, 2.50 mmol) was added and the mixture was stirred at room temperature for 2 hours. Ethyl acetate and water were added to the reaction mixture and the organic layer was separated. The aqueous layer was extracted with ethyl acetate, and the obtained organic layers were combined, washed with water and saturated brine, and dried over anhydrous sodium sulfate. The solvent was evaporated under reduced pressure to obtain tert-butyl (4-bromo-1H-indol-3-yl)carbamate (250 mg).

LCMS (m/z) 311.4 [M+H]⁺.

### (Second step)

A mixture of tert-butyl (4-bromo-1H-indol-3-yl)carbamate (200 mg, 0.64 mmol) and 4N hydrochloric acid/1,4-dioxane solution (4 mL) were added with 2-chloro-1H-benzo[d]imidazole (98 mg, 0. 64 mmol) and stirred at 120°C for 16 hours. The reaction mixture was cooled to room temperature, and the solvent was distilled off under reduced pressure. Saturated aqueous sodium bicarbonate solution was added to make it basic, and the mixture was extracted with 10% methanol/dichloromethane. The obtained organic layer was washed with saturated brine and dried over anhydrous sodium sulfate. After removing the solvent under reduced pressure, the residue was purified using HPLC preparative chromatography to obtain the title compound as the formate salt (11 mg).

¹H NMR (500 MHz, DMSO-d₆) *δ* 11.38 (s, 1H), 10.81 (brs, 1H), 8.20 (s, 1H), 7.69 (d, J = 1.5 Hz, 1H), 7.43 (dd, J = 0.6, 8.2 Hz, 1H), 7.20 - 7.02 (m, 5H), 6.91 - 6.78 (m, 2H); LCMS(m/z) 327.4 [M+H]⁺.

### Embodiment 10

### Preparation of N-(5-bromo-1H-pyrrolo[2,3-b]pyridine-3-yl)-5-phenoxy-1H-benzo[d]imidazole-2-amine

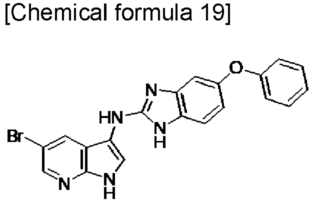

5-bromo-1H-pyrrolo[2,3-b]pyridine-3-amine (30 mg, 0.141 mmol) and 2-chloro-5-phenoxy-1H-benzo[d]imidazole (31.2 mg, 0.127 mmol) were added to a mixed solvent of 4N hydrochloric acid/1,4-dioxane solution and DMF (2:1,3 mL). The mixture was stirred overnight under reflux conditions. After cooling the reaction mixture to room temperature, the solvent was distilled off under reduced pressure, the residue was crudely purified by silica gel chromatography (chloroform:methanol=1:0-19:1), and further purified by HPLC preparative chromatography to obtain the title compound as the formate salt (13 mg).

¹H NMR (400 MHz, DMSO-d6) δ 11.61 (s, 1H), 11.11 - 10.92 (m, 1H), 9.3 7 (s, 1H), 8.32 (d, J = 2.2 Hz, 1H), 8.28 (d, J = 2.2 Hz, 1H), 8.19 ( s, 1H), 7.92 (s, 1H), 7.33 (t, J = 7.8 Hz, 2H), 7.29 - 7.15 (m, 1H), 7.08 - 7.01 (m, 1H), 6.98 - 6.84 (m, 3H), 6.74 - 6.62 (m, 1H); LCMS(m /z) 422.2 [M+H]⁺.

### Embodiment 11

### Preparation of N-(5-bromo-1H-pyrrolo[2,3-c]pyridine-3-yl)-5-phenoxy-1H-benzo[d]imidazole-2-amine

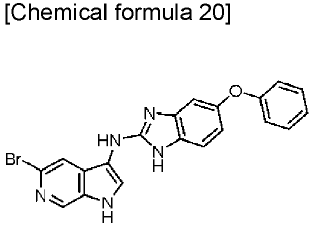

### (First step)

Potassium nitrate (180 mg, 1.776 mmol) was added to concentrated sulfuric acid (3 mL) and stirred at 0°C for 5 minutes. 5-bromo-1 H-pyrrolo[2,3-c]pyridine (250 mg, 1.269 mmol) was added, and the mixture was further stirred at 0°C for 30 minutes. Ice water was added to the reaction mixture, and the precipitated solid was collected by filtration and dried to obtain 5-bromo-3-nitro-1H-pyrrolo[2,3-c]pyridine (280 mg).

¹H NMR (400 MHz, DMSO-d6) δ 13.30 (s, 1H), 8.92 (d, J = 2.2 Hz, 1H), 8.74 (d, J = 1.0 Hz, 1H), 8. 13 (d, J = 1.0 Hz, 1H); LCMS(m/z) 242. 01 [M+H]⁺.

### (Second step)

5-bromo-3-nitro-1H-pyrrolo[2,3-c]pyridine (230 mg, 0.95 mmol) in acetic acid/concentrated hydrochloric acid mixed solution (1:1,6 mL) was added to tin (II) chloride (901 mg, 4.75 mmol) and stirred at room temperature for 35 minutes. A 2M sodium hydroxide aqueous solution was added to terminate the reaction, and the mixture was extracted with chloroform. After the organic layer was dried over anhydrous sodium sulfate, the solvent was distilled off under reduced pressure to obtain 5-bromo-1H-pyrrolo[2,3-c]pyridine-3-amine (50 mg).

¹H NMR (400 MHz, DMSO-d6) *δ* 10.79 (s, 1H), 8.33 (d, J = 1.0 Hz, 1H), 7.73 (t, J = 0.9 Hz, 1H), 6.91 (d, J = 2.3 Hz, 1H), 4.32 (s, 2H); LCM S(m/z) 212.07 [M+H]⁺.

### (Third step)

5-bromo-1H-pyrrolo[2,3-c]pyridine-3-amine (40 mg, 0.189 mmol) was added with 2-chloro-5-phenoxy-1H-benzo[d]imidazole in NMP solution (2 mL) (46.2 mg, 0.189 mmol) and p-toluenesulfonic acid monohydrate (53.8 mg, 0.283 mmol) and stirred for 2.5 hours at 120°C. After cooling the reaction mixture to room temperature, ethyl acetate was added, and the mixture was washed with a saturated aqueous sodium hydrogencarbonate solution. The obtained organic layer was dried over anhydrous sodium sulfate, and the solvent was distilled off under reduced pressure, and the obtained residue was purified by silica gel chromatography (chloroform:methanol=1:0-9:1) to obtain the title compound (30 mg).

¹H NMR (400 MHz, DMSO-d6) δ 11.52 (s, 1H), 10.90 (brs, 1H), 9.30 (s, 1H), 8.53 (d, J = 1.0 Hz, 1H), 8.11 (d, J = 2.5 Hz, 1H), 7.88 (t, J = 0.8 Hz, 1H), 7.38 - 7.27 (m, 2H), 7.23 (brs, 1H), 7.04 (t, J = 7.3 H z, 1H), 6.96 - 6.89 (m, 3H), 6.69 (brs, 1H); LCMS(m/z) 422.2 [M+H]⁺.

### Embodiment 12

### Preparation of N-(5-bromo-1H-pyrrolo[3,2-b]pyridine-3-yl)-5-phenoxy-1H-benzo[d]imidazole-2-amine

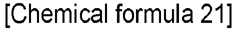

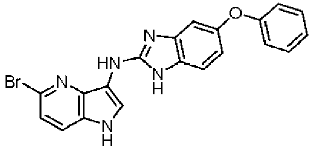

### (First step)

Potassium nitrate (180 mg, 1.776 mmol) was added to concentrated sulfuric acid (3 mL) and stirred at room temperature for 1 minute. The reaction solution was cooled to 0°C and 5-bromo-1H-pyrrolo[3,2-b]pyridine (250 mg, 1.269 mmol) was added and the mixture was stirred at 0°C for 25 minutes. Ice water was added to the reaction mixture, and the precipitated solid was collected by filtration and dried to obtain 5-bromo-3-nitro-1H-pyrrolo[3,2-b]pyridine (300 mg).

¹H NMR (400 MHz, DMSO-d6) δ 12. 97 (s, 1H), 8.88 (d, J = 3.7 Hz, 1H), 7.95 (d, J = 8.6 Hz, 1H), 7.54 (d, J = 8.6 Hz, 1H); LCMS(m/z) 244. 01 [M+H]⁺.

### (Second step)

5-bromo-3-nitro-1H-pyrrolo[3,2-b]pyridine (230 mg, 0.950 mmol) in acetic acid/concentrated hydrochloric acid mixed solution (1:1,6 mL) was added to tin (II) chloride (901 mg, 4.75 mmol) and stirred at room temperature for 30 minutes. A 2M sodium hydroxide aqueous solution was added to terminate the reaction, and the mixture was extracted with chloroform. After the organic layer was dried over anhydrous sodium sulfate, the solvent was distilled off under reduced pressure to obtain 5-bromo-1H-pyrrolo[3,2-b]pyridine-3-amine (160 mg).

¹H NMR (400 MHz, DMSO-d6) δ 10. 60 (s, 1H), 7.57 (d, J = 8.4 Hz, 1H), 7.12 (d, J = 8.4 Hz, 1H), 6.96 (d, J = 2.5 Hz, 1H), 4.10 (s, 2H); LCM S(m/z) 214.02 [M+H]⁺.

### (Third step)

5-bromo-1H-pyrrolo[3,2-b]pyridine-3-amine (33 mg, 0.156 mmol) was added with 2-chloro-5-phenoxy-1H-benzo[d]imidazole in NMP solution (1 mL) (38.1 mg, 0.156 mmol) and p-toluenesulfonic acid monohydrate (44. 4mg, 0.233 mmol) and stirred for 2.5 hours at 120 °C. After cooling the reaction mixture to room temperature, ethyl acetate was added, and the mixture was washed with a saturated aqueous sodium hydrogencarbonate solution. The obtained organic layer was dried over anhydrous sodium sulfate, and the solvent was distilled off under reduced pressure, and the obtained residue was purified by silica gel chromatography (chloroform:methanol=1:0-19:3) to obtain the title compound (20 mg).

¹H NMR (400 MHz, Methanol-d₄) δ 7.80 - 7.68 (m, 2H), 7.32 - 7.19 (m, 4H), 7.07 - 7.01 (m, 1H), 6.96 - 6.91 (m, 3H), 6.76 (d, J = 7.8 Hz, 1

H); LCMS(m/z) 422.2 [M+H]⁺.

### Embodiment 13

### Preparation of N-(5-bromo-1H-indol-3-yl)-5-(pyridine-3-yloxy)-1H-benzo[d]imidazole-2-amine

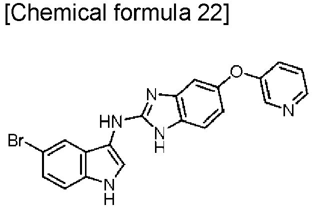

### (First step)

5-fluoro-2-nitroaniline (500 mg, 3.20 mmol) in DMF solution (10 mL) was added with pyridine-3-ol (609 mg, 6.41 mmol) and potassium carbonate (885 mg, 6.41 mmol) and stirred at 100°C for 4 hours. After cooling the reaction mixture to room temperature, ethyl acetate was added, and the mixture was washed with a saturated aqueous sodium hydrogencarbonate solution. After drying the obtained organic layer over anhydrous sodium sulfate, the solvent was distilled off under reduced pressure to obtain 2-nitro-5-(pyridine-3-yloxy)aniline (741 mg).

¹H NMR (400 MHz, DMSO-d6) δ 8.54 - 8.48 (m, 2H), 8.06 - 8.00 (m, 1H), 7.68 (ddd, J = 8.4, 2.8, 1.4 Hz, 1H), 7.54 (ddd, J = 8.4, 4.7, 0.7 H z, 1H), 7.49 (s, 2H), 6.39 (d, J = 2.7 Hz, 1H), 6.34 (dd, J = 9.4, 2. 7 Hz, 1H); LCMS(m/z) 232.11 [M+H]⁺.

### (Second step)

2-nitro-5-(pyridine-3-yloxy)aniline (740 mg, 3.20 mmol) was dissolved in ethanol (20 mL), and 10% palladium carbon (170 mg) was added. The mixture was stirred overnight at room temperature under a hydrogen atmosphere. Insoluble matters were filtered off, and the filtrate was concentrated under reduced pressure. In order to complete the reaction, the obtained mixture was dissolved in a mixed solvent of ethanol/ethyl acetate (2:1, 30 mL). 10% palladium on carbon (170 mg) was added, and the mixture was further stirred at room temperature under a hydrogen atmosphere overnight. After filtering the insoluble matter, the filtrate was concentrated under reduced pressure to obtain 4-(pyridine-3-yloxy)benzene-1,2-diamine (640 mg).

LCMS (m/z) 202.17 [M+H]⁺.

### (Third step)

4-(pyridine-3-yloxy)benzene-1,2-diamine (640 mg, 3.18 mmol) and CDI (774 mg, 4.77 mmol) were added to THF (20 mL) and stirred at room temperature for 2 hours. Ethyl acetate was added to the reaction mixture, and the mixture was washed with a saturated aqueous sodium hydrogencarbonate solution. The obtained organic layer was dried over anhydrous sodium sulfate, and the solvent was distilled off under reduced pressure. Ethanol was added to the obtained residue to suspend it, and the solid was collected by filtration. The solid was washed with ethanol and dried to obtain 5-(pyridine-3-yloxy)-1,3-dihydro-2H-benzo[d]imidazole-2-one (480 mg).

¹H NMR (400 MHz, DMSO-d6) δ 10.67 (d, J = 9.6 Hz, 2H), 8.36 - 8.27 (m , 2H), 7.37 (ddd, J = 8.5, 4.5, 0.8 Hz, 1H), 7.32 (ddd, J = 8.4, 2.9, 1.5 Hz, 1H), 6.94 (d, J = 8.2 Hz, 1H), 6.72 - 6.64 (m, 2H); LCMS(m/z ) 228.12 [M+H]⁺.

### (Fourth step)

5-(pyridine-3-yloxy)-1,3-dihydro-2H-benzo[d]imidazole-2-one (75 mg, 0.33 mmol) was added with phosphorus oxychloride (1.9 mL) and stirred at 100°C overnight. The reaction mixture was cooled to room temperature, and ice was added, and ethyl acetate and saturated aqueous sodium bicarbonate were added, and the organic layer was separated. The obtained organic layer was washed with saturated aqueous sodium hydrogencarbonate solution and saturated brine, and dried over anhydrous sodium sulfate. The solvent was evaporated under reduced pressure to obtain 2-chloro-5-(pyridine-3-yloxy)-1H-benzo[d]imidazole (60 mg).

LCMS (m/z) 246.06 [M+H]⁺.

### (Fifth step)

5-bromo-1H-indol-3-amine (51.5 mg, 0.244 mmol) in NMP solution (2 mL) was added with 2-chloro-5-(pyridine-3-yloxy)-1H-benzo[d]imidazole (60 mg, 0.244 mmol) and p-toluenesulfonic acid monohydrate (69.7 mg, 0.366 mmol) and stirred at 120°C for 3.5 hours. After cooling the reaction mixture to room temperature, ethyl acetate was added, and the mixture was washed with a saturated aqueous sodium hydrogencarbonate solution. The obtained organic layer was dried over anhydrous sodium sulfate, and the solvent was distilled off under reduced pressure, and the obtained residue was purified by amine-modified silica gel chromatography (chloroform:methanol=1:0-9:1) to obtain the title compound (12 mg).

¹H NMR (400 MHz, DMSO-d6) δ 11.08 - 11.01 (m, 1H), 10.81 - 10.71 (m, 1H), 9.18 - 9.12 (m, 1H), 8.41 - 8.20 (m, 2H), 7.85 (d, J = 2.5 Hz, 1 H), 7.81 (dd, J = 12.6, 2.5 Hz, 1H), 7.44 - 7.15 (m, 5H), 6.99 - 6.91 (m, 1H), 6.77 - 6.65 (m, 1H); LCMS(m/z) 422.2 [M+H]⁺

### Embodiment 14

### Preparation of N-(5-fluoro-1H-indol-3-yl)-1H-benzo[d]imidazole-2-amine

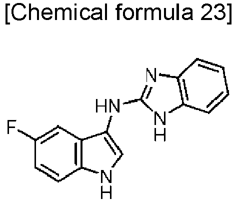

### (First step)

5-fluoro-1H-indole (1 g, 7.41 mmol) and silver nitrate (1.5 g, 8.89 mmol) in acetonitrile (15 mL) at 0°C were added with benzoyl chloride (1.24 g, 8.89 mmol) and stirred at room temperature for 2 hours. Insoluble matter was filtered through celite, and saturated aqueous sodium hydrogencarbonate solution was added to the filtrate, and the mixture was extracted with ethyl acetate. The obtained organic layer was washed with saturated brine and dried over anhydrous sodium sulfate. After the solvent was distilled off under reduced pressure, the residue was purified by silica gel chromatography (ethyl acetate:petroleum ether=15:85) to obtain 5-fluoro-3-nitro-1H-indole (1g).

LCMS (m/z) 181.1 [M+H]⁺.

### (Second step)

Tert-butyl (5-fluoro-1H-indol-3-yl)carbamate (1 g) was obtained by the same method as in the first step of Example 9 using 5-fluoro-3-nitro-1H-indole (1 g, 5.55 mmol). LCMS (m/z) 251.3 [M+H]⁺.

### (Third step)

Tert-butyl (5-fluoro-1H-indol-3-yl)carbamate (0.3 g, 1.2 mmol) in NMP (4 mL) was added with 2-chloro-1H-benzo[d]imidazole (0.146 g, 0.969 mmol) and p-toluenesulfonic acid monohydrate (0.309 g, 1.8 mmol) and stirred at 120°C for 4 hours. The reaction mixture was poured onto ice and the obtained solid was collected by filtration and dried under reduced pressure. The obtained crude product was purified using HPLC preparative chromatography to obtain the title compound (36 mg).

¹H NMR (500 MHz, DMSO-d₆) δ 10.90 (s, 1H), 10.80 (s, 1H), 8.98 (s, 1H) , 7.80 (d, J = 2.4 Hz, 1H), 7.38 - 7.33 (m, 2H), 7.26 - 7.11 (m, 2H), 6.97 - 6.83 (m, 3H); LCMS(m/z) 267.1 [M+H]⁺.

### Embodiment 15

### Preparation of N-(5-chloro-1H-indol-3-yl)-1H-benzo[d]imidazole-2-amine

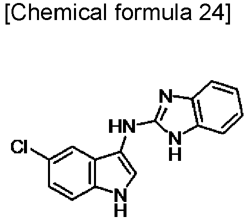

### (First step)

5-chloro-3-nitro-1H-indole (0.300 g, 1.54 mmol) was used to obtain tert-butyl (5-chloro-1H-indol-3-yl)carbamate (0.200 g) in the same manner as in the first step of Example 9 . LCMS (m/z) 267.4 [M+H]⁺.

### (Second step)

Tert-butyl (5-chloro-1H-indol-3-yl)carbamate (0.20 g, 0.75 mmol) in 4N hydrochloric acid/1,4-dioxane solution (2 mL) was added with 2-chloro-1H-benzo[d]imidazole (0.136 g, 0.90 mmol) and stirred at 70°C for 16 hours. The reaction mixture was cooled to room temperature, and basified with a saturated aqueous sodium hydrogencarbonate solution, and extracted with ethyl acetate. The obtained organic layer was washed with saturated brine and dried over anhydrous sodium sulfate. After evaporating the solvent under reduced pressure, the residue was crudely purified by silica gel chromatography (ethyl acetate:petroleum ether=3:7) and then purified by HPLC preparative chromatography to obtain the title compound as a formate salt (10 mg).

¹H NMR (500 MHz, DMSO-d₆) δ 10.98 (brs, 2H), 9.20 (s, 1H), 8.22 (s, 1H ), 7.84 (d, J = 2.4 Hz, 1H), 7.72 (d, J = 1.8 Hz, 1H), 7.38 (d, J = 8 .5 Hz, 1H), 7.21 (d, J = 4.0 Hz, 2H), 7.12 - 7.07 (m, 1H), 6.92 (dd, J = 3.1, 5.8 Hz, 2H); LCMS(m/z) 283.2 [M+H]⁺.

### Embodiment 16

### Preparation of N-(5-iodo-1H-indol-3-yl)-1H-benzo[d]imidazole-2-amine

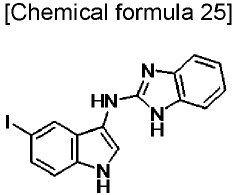

### (First step)

5-iodo-1H-indole (4 g, 16.46 mmol) was used to obtain 5-Iodo-3-nitro-1H-indole (2.7 g) by the same manner as in the first step of Example 14 .

LCMS (m/z) 289.1 [M+H]⁺.

### (Second step)

5-iodo-3-nitro-1H-indole (0.5 g, 1.74 mmol) was used to obtain tert-butyl (5-iodo-1H-indol-3-yl) carbamate (0.3 g) in the same manner as in the first step of Example 9. LCMS (m/z) 359.2 [M+H]⁺.

### (Third step)

Tert-butyl (5-iodo-1H-indol-3-yl)carbamate (0.141 g, 0.39 mmol) was used to obtain the title compound as a formate salt (10 mg) by the same method as in the third step of Example 14.

¹H NMR (500 MHz, DMSO-d₆) δ 11.85 (brs, 1H), 10. 90 (brs, 1H), 9.84 (br s, 1H), 8.42 (s, 1H), 8.15 (s, 1H), 7.84 (d, J = 2.4 Hz, 1H), 7.33 (d d, J = 1.4, 8.4 Hz, 1H), 7.25 - 7.15 (m, 3H), 6. 94 - 6.86 (m, 2H); LC

MS(m/z) 375.3 [M+H]⁺.

### Example 17

### Preparation of N-(7-bromo-1H-indol-3-yl)-1H-benzo[d]imidazole-2-amine

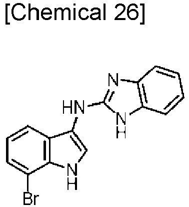

### (First step)

7-bromo-3-nitro-1H-indole (0.5 g, 2.08 mmol) was used to obtain a preparation of tert-butyl (7-bromo-1H-indol-3-yl)carbamate (0.25 g) in the same manner as in the first step of Example 9 .

LCMS (m/z) 313.2 [M+H]⁺.

### (Second step)

Tert-butyl (7-bromo-1H-indol-3-yl)carbamate (0.1 g, 0.32 mmol) was used to obtain the title compound (42 mg) in the same manner as in the third step of Example 14.

¹H NMR (500 MHz, DMSO-d₆) δ 11. 02 (s, 1H), 10.70 (brs, 1H), 9. 07 (s, 1 H), 7.83 (d, J = 2.4 Hz, 1H), 7.67 (d, J = 7.9 Hz, 1H), 7.34 (d, J = 7.5 Hz, 1H), 7.22 (brs, 2H), 6.98 - 6.89 (m, 3H); LCMS(m/z) 327. 1 [M+ H]⁺.

### Embodiment 18

### Preparation of N-(5-bromo-1H-indol-3-yl)-1-methyl-1H-benzo[d]imidazole-2-amine

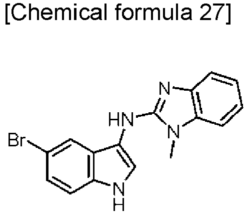

2-chloro-1-methyl-1H-benzo[d]imidazole (0.2 g, 1.2 mmol) and 5-bromo-1H-indol-3-amine (0.303 g, 1.44 mmol) was used to obtain the title compound (15 mg) in the same manner as in the third step of Example 14.

¹H NMR (500 MHz, DMSO-d₆) δ 11. 05 (brs, 1H), 8.56 (s, 1H), 8. 03 (d, J = 1.8 Hz, 1H), 7.94 (d, J = 2. 1 Hz, 1H), 7.35 (d, J = 8.5 Hz, 1H), 7. 29 - 7.24 (m, 2H), 7.20 (dd, J = 1.5, 8.5 Hz, 1H), 7.03 - 6.97 (m, 2H ), 3.74 (s, 3H); LCMS(m/z) 341.4 [M+H]⁺.

### Embodiment 19

### Preparation of 2-[(5-bromo-1H-indol-3-yl)amino]-1H-benzo[d]imidazole-5-carbonitrile

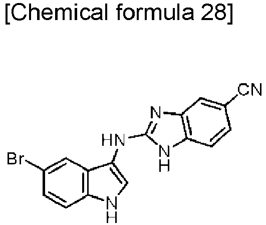

5-bromo-1H-indol-3-amine (35.7 mg, 0.169 mmol) in NMP solution (2 mL) was added with 2-chloro-1H-benzo[d]imidazole-5-carbonitrile (30 mg, 0.169 mmol) and p-toluenesulfonic acid monohydrate (48.2 mg, 0.253 mmol) and stirred at 120°C for 2.5 hours. After cooling the reaction mixture to room temperature, ethyl acetate was added, and the mixture was washed with a saturated aqueous sodium hydrogencarbonate solution. The obtained organic layer was dried over anhydrous sodium sulfate, and the solvent was distilled off under reduced pressure, and the obtained residue was crudely purified by silica gel chromatography (chloroform:methanol=1:0-9:1), and further purified by ion exchange column (SCX) to obtain the title compound (16 mg).

¹H NMR (400 MHz, DMSO-d6) δ 11.34 - 11. 09 (m, 2H), 9.43 (brs, 1H), 7. 80 (brs, 2H), 7.68 - 7.46 (m, 1H), 7.40 - 7.30 (m, 3H), 7.23 (dd, J = 8.6, 2. 0 Hz, 1H); LCMS(m/z) 352.1 [M+H]⁺.

### Embodiment 20

### Preparation of methyl 2-[(5-bromo-1H-indol-3-yl)amino]-1H-benzo [d]imidazole-4-carboxylate

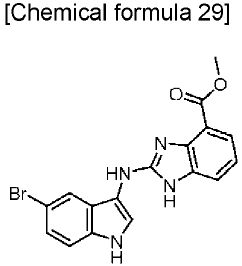

5-bromo-1H-indol-3-amine (88 mg, 0.418 mmol) and methyl 2-chloro-1H-benzo[d]imidazole-4-carboxylate (80 mg, 0.380 mmol) were added to a 4N hydrochloric acid/1,4-dioxane solution (3 mL) and stirred under reflux conditions for 2 hours. Furthermore, 5-bromo-1H-indol-3-amine (80 mg, 0.379 mmol) was added and stirred for 1 hour under reflux conditions. After cooling the reaction mixture to room temperature, the solvent was distilled off under reduced pressure, the residue was crudely purified by silica gel chromatography (chloroform:methanol=1:0-9:1), and further purified by HPLC preparative chromatography to obtain the title compound as the formate salt (5 mg).

¹H NMR (400 MHz, DMSO-d6) δ 11.00 (brs, 2H), 9.36 (brs, 1H), 8.35 (br s, 1H), 7.91 (d, J = 2.3 Hz, 1H), 7.79 (brs, 1H), 7.58 - 7.50 (m, 2H) , 7.36 (d, J = 8.7 Hz, 1H), 7.23 (dd, J = 8.7, 1.9 Hz, 1H), 7.10 (t, J = 7.8 Hz, 1H), 3.95 (s, 3H); LCMS(m/z) 387.1 [M+H]⁺.

### Embodiment 21

### Preparation of 2-bromo-N-(5-phenoxy-1H-benzo[d]imidazole-2-yl)-5H-pyrrolo[2,3-b] pyrazin-7-amine

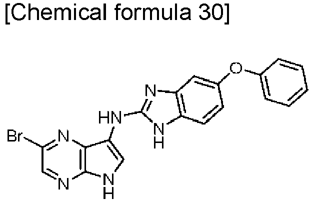

### (First step)

2-bromo-7-nitro-5H-pyrrolo[2,3-b]pyrazine (60 mg, 0.247 mmol) was dissolved in a mixed solvent of methanol/saturated aqueous ammonium chloride solution (2:1,3 mL), and added with zinc dust (161 mg, 2.469 mmol) and stirred at room temperature for 10 minutes. Further, Boc₂O (64.7 mg, 0.296 mmol) was added to the reaction solution and stirred at room temperature for 30 minutes. After the reaction mixture was diluted with ethyl acetate, it was filtered using celite, and the filtrate was concentrated under reduced pressure. The obtained residue was purified by silica gel chromatography (chloroform:methanol=1:0-19:1) to obtain tert-butyl (2-bromo-5H-pyrrolo[2,3-b] pyrazin-7-yl)carbamate (30 mg).

¹H NMR (400 MHz, DMSO-d6) δ 12. 13 (s, 1H), 9. 09 (s, 1H), 8.36 (s, 1H) , 7.92 (s, 1H), 1.45 (s, 9H).

### (Second step)

Tert-butyl (2-bromo-5H-pyrrolo[2,3-b]pyrazin-7-yl)carbamate (30 mg, 0.096 mmol) in NMP (2 mL) was added with 2-chloro-5-phenoxy-1H-benzo[d]imidazole (28. 1 mg, 0.115 mmol) and p-toluenesulfonic acid monohydrate (27.3 mg, 0.144 mmol) and stirred at 120°C for 2 hours. After cooling the reaction mixture to room temperature, ethyl acetate was added, and the mixture was washed with a saturated aqueous sodium hydrogencarbonate solution. The obtained organic layer was dried over anhydrous sodium sulfate, and the solvent was distilled off under reduced pressure, and the obtained residue was crudely purified by silica gel chromatography (chloroform:methanol=1:0-19:1), and further purified by ion exchange column (SCX) to obtain the title compound (19 mg).

¹H NMR (400 MHz, DMSO-d6) δ 12. 10 (brs, 1H), 10. 58 (brs, 1H), 9.57 (b rs, 1H), 8.41 (s, 1H), 8.37 (brs, 1H), 7.37 - 7.24 (m, 3H), 7.05 (t, J = 7.4 Hz, 1H), 6.97 (d, J = 2.3 Hz, 1H), 6.94 (d, J = 8.1 Hz, 2H), 6.71 (brs, 1H); LCMS(m/z) 421.2 [M+H]⁺.

### Embodiment 22

### Preparation of methyl 3-[(5-phenoxy-1H-benzo[d]imidazole-2-yl) amino]-1H-indole-5-carboxylate

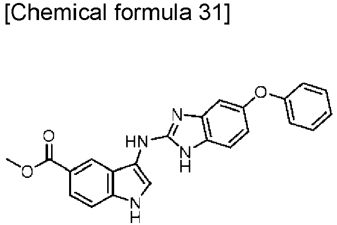

### (First step)

Silver nitrate (1.7 g, 10 mmol) was added with N-bromosuccinimide (1.78 g, 10 mmol) in acetonitrile (50 mL) and stirred at 80°C for 10 minutes. Methyl 1H-indole-5-carboxylate (1.75 g, 10 mmol) was added and the mixture was further stirred at 80°C for 3 hours. After the insoluble matter was filtered through celite, the filtrate was evaporated under reduced pressure, and the residue was purified by silica gel chromatography (chloroform:methanol=1:0-19:1) to obtain methyl 3-nitro-1H-indole-5-carboxylate (2.3 g).

¹H NMR (400 MHz, DMSO-d6) δ 12. 96 (s, 1H), 8.81 (d, J = 3.4 Hz, 1H), 8.77 - 8.72 (m, 1H), 7.95 (dd, J = 8.6, 1.7 Hz, 1H), 7. 68 (dd, J = 8. 6, 0.7 Hz, 1H), 3.90 (s, 3H); LCMS(m/z) 221. 17 [M+H]⁺.

### (Second step)

Methyl 3-nitro-1H-indole-5-carboxylate (100 mg, 0.454 mmol) was added to a mixed solvent of methanol/saturated aqueous ammonium chloride solution (2:1, 4.5 mL) and zinc dust (297 mg, 4. 54 mmol) and stirred at room temperature for 10 minutes. Boc₂O (119 mg, 0.545 mmol) was added to the reaction solution and stirred at room temperature for 1 hour. After the reaction mixture was diluted with ethyl acetate, it was filtered using celite, and the filtrate was concentrated under reduced pressure. The obtained residue was purified by silica gel chromatography (chloroform:methanol=1:0-97:3) to obtain methyl 3-[(tert-butoxycarbonyl)amino]-1H-indole-5-carboxylate (70 mg).

¹H NMR (400 MHz, DMSO-d6) δ 11. 13 (s, 1H), 9.41 (s, 1H), 8.61 (s, 1H) , 7.69 (dd, J = 8.6, 1.7 Hz, 1H), 7.52 (s, 1H), 7.39 - 7.33 (m, 1H), 3.84 (s, 3H), 1.49 (s, 9H).

### (Third step)

Methyl 3-[(tert-butoxycarbonyl)amino]-1H-indole-5-carboxylate (70 mg, 0.241 mmol) in NMP (2 mL) was added with 2-chloro-5-phenoxy-1H-benzo[d]imidazole (56 mg, 0.229 mmol) and p-toluenesulfonic acid monohydrate (68.8 mg, 0.362 mmol) and stirred for 2.5 hours. After cooling the reaction mixture to room temperature, ethyl acetate was added, and the mixture was washed with a saturated aqueous sodium hydrogencarbonate solution. The obtained organic layer was dried over anhydrous sodium sulfate, and the solvent was distilled off under reduced pressure. The residue was crudely purified by silica gel chromatography (chloroform:methanol=1:0-19:1) and further purified by amine-modified silica gel chromatography (chloroform:methanol=1:0-97:3) to obtain the title compound (2 mg).

¹H NMR (400 MHz, DMSO-d6) δ 11.23 - 11. 17 (m, 1H), 10.69 - 10.57 (m, 1H), 9.42 - 9.38 (m, 1H), 8.49 (brs, 1H), 7.95 - 7.88 (m, 1H), 7.77 - 7.72 (m, 1H), 7.47 - 7.40 (m, 1H), 7.38 - 7.29 (m, 2H), 7.29 - 7.19 (m, 1H), 7.08 - 7.00 (m, 1H), 6.98 - 6.88 (m, 3H), 6.74 - 6.63 (m, 1H ), 3.86 (s, 3H); LCMS(m/z) 399.3 [M+H]⁺.

### Embodiment 23

### Preparation of N-(5-bromo-1H-indol-3-yl)-5-(phenylsulfonyl)-1H-benzo [d]imidazole-2-amine

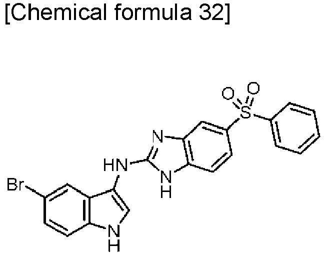

### (First step)

5-chloro-2-nitroaniline (500 mg, 2.9 mmol) and sodium benzenesulfinate (1.16 g, 5.79 mmol) were added to NMP (5 mL) and stirred at 150°C for 0.5 hours. Water was added under ice-cooling, and the precipitated solid was collected by filtration, washed with water, and dried under reduced pressure to obtain 2-nitro-5-(phenylsulfonyl)aniline (722 mg). LCMS (m/z) 279.2 [M+H]⁺.

### (Second step)

2-nitro-5-(phenylsulfonyl)aniline (600 mg, 2.156 mmol) was dissolved in ethanol (18 mL), and 10% palladium carbon (115 mg) was added. The reaction was carried out at room temperature for 18 hours under a hydrogen atmosphere. Insoluble matter was filtered off, and the filtrate was concentrated to obtain 4-(phenylsulfonyl)benzene-1,2-diamine (535 mg).

LCMS (m/z) 249.2 [M+H]+.

### (Third step)

4-(Phenylsulfonyl)benzene-1,2-diamine (535 mg, 2.155 mmol) was used to obtain 5-(phenylsulfonyl)-1,3-dihydro-2H-benzo[d]imidazole-2-one (493 mg) by the same method as in the first step of Example 3.

LCMS (m/z) 275.1 [M+H]+.

### (Fourth step)

5-(Phenylsulfonyl)-1,3-dihydr the Second step o-2H-benzo[d]imidazole-2-one (50 mg, 0.182 mmol) was used to obtain 2-chloro-5-(phenylsulfonyl)-1H-benzo[d]imidazole (25.7 mg) by the same method as in the second step of Example 3 .

LCMS (m/z) 293.1 [M+H]+.

### (Fifth step)

2-chloro-5-(phenylsulfonyl)-1H-benzo[d]imidazole (13.8 mg, 0.047 mmol) in 1,4-dioxane solution (1 mL) was added with 5-bromo-1H-indol-3-amine (10.45 mg, 0.049 mmol) and p-toluenesulfonic acid monohydrate (13.45 mg, 0.071 mmol) and stirred at 120°C for 1 hour. The reaction mixture was cooled to 0°C and the precipitated solid was collected by filtration and washed successively with 1N aqueous sodium hydroxide solution, water and ethyl acetate to obtain the title compound (3.7 mg).

¹H NMR (400 MHz, DMSO-d₆) δ 11. 50 (s, 1H), 7.95 - 7.91 (m, 2H), 7.80 ( d, J = 1.9 Hz, 1H), 7.79 - 7.56 (m, 4H), 7.48 - 7.40 (m, 3H), 7.28 (d , J = 8.5 Hz, 1H), 7.11 (d, J = 7.9 Hz, 2H); LCMS(m/z) 467.2 [M+H]⁺.

### Embodiment 24

### Preparation of 4-bromo-N-(5-bromo-1H-indol-3-yl)-1H-benzo[d]imidazole-2-amine

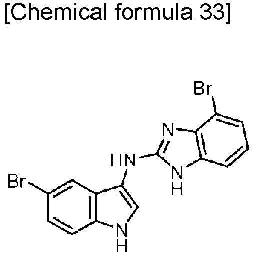

4-bromo-2-chloro-1H-benzo[d]imidazole (0.1 g, 0.43 mmol) was added with 5-bromo-1H-indol-3-amine (0.11 g, 0.52 mmol) to obtain the title compound (58 mg) in the same manner as in the third step of Example 11.

¹H NMR (400 MHz, DMSO-d₆) δ 11. 10 (s, 1H), 11.05 (s, 1H), 9.31 (s, 1H) , 7.85 - 7.79 (m, 2H), 7.37 (d, J = 8.8 Hz, 1H), 7.22 (dd, J = 2.0, 8 .6 Hz, 1H), 7.20 - 7.10 (m, 2H), 6.82 (t, J = 7.8 Hz, 1H); LCMS(m/z) 404.9 [M+H]⁺.

### Embodiment 25

### Preparation of N-(5-bromo-1H-indol-3-yl)-4-fluoro-1H-benzo[d]imidazole-2-amine

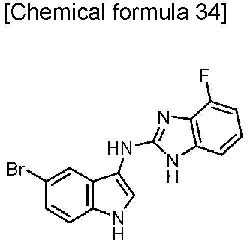

2-chloro-4-fluoro-1H-benzo[d]imidazole (0.1 g, 0.59 mmol) was added with 5-bromo-1H-indol-3-amine (0.148 g, 0. 7 mmol) to obtain the title compound (50 mg) in the same manner as in the third step of Example 11.

¹H NMR (500 MHz, DMSO-d₆) δ 11.06 (brs, 1H), 11.02 (brs, 1H), 9.21 (s, 1H), 7.92 - 7.78 (m, 2H), 7.36 (d, J = 8.5 Hz, 1H), 7.22 (dd, J = 1. 8, 8.5 Hz, 1H), 7.03 (d, J = 7.6 Hz, 1H), 6.92 - 6.73 (m, 2H); LCMS(m /z) 345.0 [M+H]⁺.

### Embodiment 26

### Preparation of N-(5-bromo-1H-indol-3-yl)-5-[3-(dimethylamino)phenoxy]-1H-benzo[d]imidazole-2-amine

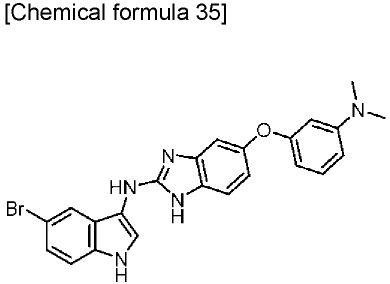

### (First step)

3-(dimethylamino)phenol (4.4 g, 32.05 mmol) was added with 5-fluoro-2-nitroaniline (5 g, 32.05 mmol) to obtain by 3-(3-amino-4-nitrophenoxy)-N,N-dimethylaniline (5 g) by the same method as in the first step of Example 13.

LCMS (m/z) 274.1 [M+H]⁺.

### (Second step)

3-(3-amino-4-nitrophenoxy)-N,N-dimethylaniline (4 g, 14.65 mmol) was dissolved in a mixed solvent of ethanol/water (60 mL, 2:1) and added with iron powder (4.08 g, 73.26 mmol) and ammonium chloride (7.84 g, 146.52 mmol) and stirred at 90°C for 2 hours. The reaction mixture was filtered through celite, and the filtrate was diluted with water and then extracted with ethyl acetate. The obtained organic layer was dried over anhydrous sodium sulfate, and the solvent was distilled off under reduced pressure. The residue was purified by silica gel chromatography (ethyl acetate:petroleum ether=1:1) to obtain 4-[3-(dimethylamino)phenoxy]benzene-1,2-diamine (3 g).

LCMS (m/z) 244.4 [M+H]⁺.

### (Third step)

4-[3-(dimethylamino)phenoxy]benzene-1,2-diamine (2.8 g, 11.52 mmol) in THF (30 mL) was added with CDI (2.8 g, 17.28 mmol) and stirred at 60°C for 4 hours. The reaction mixture was diluted with saturated brine and extracted with ethyl acetate. The obtained organic layer was dried over anhydrous sodium sulfate, and the solvent was distilled off under reduced pressure. The residue was purified by silica gel chromatography (ethyl acetate:petroleum ether=3:2) to obtain 5-[3-(dimethylamino)phenoxy]-1,3-dihydro-2H-benzo[d]imidazole-2-one (3 g).

LCMS (m/z) 270.4 [M+H]⁺.

### (Fourth step)

5-[3-(dimethylamino)phenoxy]-1,3-dihydro-2H-benzo[d]imidazole-2-one (0.5 g, 1.86 mmol) was added with phosphorus oxychloride (6.93 mL) and N,N-dimethylaniline (0.2 mL) and stirred at 100°C for 16 hours. The reaction mixture was concentrated under reduced pressure, and the residue was made basic by adding a saturated aqueous solution of sodium bicarbonate and extracted with 10% methanol/dichloromethane. The obtained organic layer was dried over anhydrous sodium sulfate, and the solvent was distilled off under reduced pressure. The residue was purified by silica gel chromatography (ethyl acetate: petroleum ether=1:1) to obtain 3-[(2-chloro-1H-benzo[d]imidazol-5-yl)oxy]-
N,N-dimethylaniline (0.45 g).

LCMS (m/z) 288.5 [M+H]⁺.

### (Fifth step)

3-[(2-chloro-1H-benzo[d]imidazol-5-yl)oxy]-N,N-dimethylaniline (0.3 g, 1.04 mmol) was added with 5-bromo-1H-indol-3-amine (0.33 g, 1.57 mmol) to obtain the title compound (37 mg) in the same manner as in the third step of Example 11.

¹H NMR (500 MHz, Methanol-d₄) δ 7.63 (d, J = 2.0 Hz, 1H), 7.46 (s, 1H) , 7.33 (d, J = 8.5 Hz, 1H), 7.24 (dd, J = 1.8, 8.5 Hz, 1H), 7.18 - 7. 15 (m, 1H), 7. 08 (t, J = 8.1 Hz, 1H), 6. 89 (s, 1H), 6. 72 - 6.65 (m, 1 H), 6.44 (dd, J = 2.3, 8.4 Hz, 1H), 6. 35 (t, J = 2.4 Hz, 1H), 6.23 (d d, J = 1.5, 8.0 Hz, 1H), 2.86 (s, 6H); LCMS(m/z) 462.0 [M+H]⁺.

### Embodiment 27

### Preparation of 3-[(5-phenoxy-1H-benzo[d]imidazole-2-yl)amino]-1H-indole-5-carboxylic acid

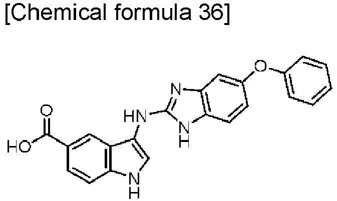

### (First step)

Methyl 3-nitro-1H-indole-5-carboxylate (2.3 g, 10.45 mmol) was added to a mixed solvent of THF/1,4-dioxane/2N aqueous sodium hydroxide solution (1:1:1, 60 mL) and stirred at 60°C for 5 hours. 2N Hydrochloric acid was added to the reaction mixture, and the organic layer was concentrated under reduced pressure. The suspension is filtered and the solid is washed with water and dried to obtain 3-nitro-1H-indole-5-carboxylic acid (1.34 g). LCMS (m/z) 205.07 [MH]⁻.

### (Second step)

3-nitro-1H-indole-5-carboxylic acid (1.34 g, 6 5 mmol) was dissolved in a mixed solvent of methanol/saturated aqueous ammonium chloride solution (35:26, 61 mL), and added with zinc dust (4.25 g, 65.0 mmol) and stirred at room temperature for 15 minutes. Boc₂O (1.7 g, 7.8 mmol) was added to the reaction mixture and stirred at room temperature for 1 hour. After the reaction mixture was diluted with ethyl acetate, it was filtered using celite, and the filtrate was concentrated under reduced pressure. The obtained residue was purified by silica gel chromatography (chloroform:methanol=1:0-7:3) to obtain 3-[(tert-butoxycarbonyl)amino]-1H-indole-5-carboxylic acid (570 mg).

LCMS (m/z) 275.10 [MH]⁻.

### (Third step)

3-[(tert-butoxycarbonyl)amino]-1H-indole-5-carboxylic acid (100 mg, 0.362 mmol) and 2-chloro-5-phenoxy-1H-benzo[d]imidazole (81 mg, 0.329 mmol) were added to a mixed solvent of 4N hydrochloric acid/1,4-dioxane solution and DMF (6:1, 3.5 mL) and stirred under reflux for 6 hours. After cooling the reaction mixture to room temperature, the solvent was distilled off under reduced pressure, the obtained residue was crudely purified by silica gel chromatography (chloroform:methanol=1:0-9:1), and further purified by HPLC preparative chromatography to obtain the title compound (2.7 mg).

¹H NMR (400 MHz, DMSO-d6) δ 11. 13 (brs, 1H), 10.77 - 10.59 (m, 1H), 9 .38 (brs, 1H), 8.47 (brs, 1H), 7.92 - 7.83 (m, 1H), 7.73 (d, J = 8.6 Hz, 1H), 7.44 - 7.30 (m, 3H), 7.29 - 7.18 (m, 1H), 7. 08 - 7. 00 (m, 1H ), 6.98 - 6.88 (m, 3H), 6.74 - 6.61 (m, 1H); LCMS(m/z) 385.2 [M+H]⁺.

### Embodiment 28

### Preparation of morpholino {3-[(5-phenoxy-1H-benzo[d]imidazole-2-yl)amino]-1H-indol-5-yl}methanone

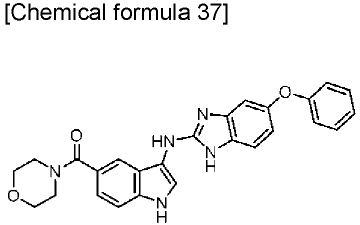

3-[(5-phenoxy-1H-benzo[d]imidazole-2-yl)amino]-1H-indole-5-carboxylic acid (10 mg, 0.026 mmol) in pyridine solution (0.5 mL) was added with morpholine (2.72 mg, 0.029 mmol) and EDCI/HCl (5. 49 mg, 0.031 mmol) and stirred at room temperature for 6 hours. Ethyl acetate was added to the reaction mixture, and the mixture was washed with a saturated ammonium chloride aqueous solution. The obtained organic layer was dried over anhydrous sodium sulfate, and the solvent was distilled off under reduced pressure, and the residue was crudely purified by amine-modified silica gel chromatography (chloroform:methanol=1:0-19:1) to obtain the title compound (4 mg).

¹H NMR (400 MHz, DMSO-d6) δ 11. 07 - 11. 01 (m, 1H), 10.80 - 10.66 (m, 1H), 9.21 - 9.15 (m, 1H), 7.84 - 7.75 (m, 2H), 7.41 (d, J = 8.4 Hz, 1 H), 7.36 - 7.29 (m, 2H), 7.26 - 7.14 (m, 2H), 7. 07 - 6. 99 (m, 1H), 6. 97 - 6.84 (m, 3H), 6.72 - 6.60 (m, 1H), 3.63 - 3.50 (m, 8H); LCMS(m/z ) 454.3 [M+H]⁺.

### Embodiment 29

### Preparation of 3-[(5-phenoxy-1H-benzo[d]imidazole-2-yl)amino]-N-(prop-2-yn-1-yl)-1H-indole-5-carboxamide

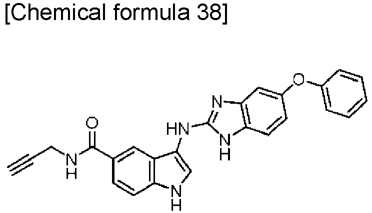

3-[(5-phenoxy-1H-benzo[d]imidazole-2-yl)amino]-1H-indole-5-carboxylic acid (50 mg, 0.13 mmol) in a pyridine solution (1 mL) was added with propargylamine (10.75 mg, 0.195 mmol) and EDCI/HCl (37.4 mg, 0.195 mmol) and stirred at room temperature for 22 hours. Ethyl acetate was added to the reaction mixture and washed with water. The obtained organic layer was dried over anhydrous sodium sulfate, and the solvent was distilled off under reduced pressure. The residue was crudely purified by amine-modified silica gel chromatography (chloroform:methanol=1:0-9:1) and further purified by silica gel chromatography (chloroform:methanol=1:0-9:1) to obtain the title compound (2.5 mg).

¹H NMR (400 MHz, DMSO-d6) δ 11.10 - 11.04 (m, 1H), 10.66 - 10.56 (m, 1H), 9.21 - 9.19 (m, 1H), 8.77 - 8.72 (m, 1H), 8.29 (brs, 1H), 7.85 - 7.79 (m, 1H), 7.67 - 7.62 (m, 1H), 7.39 (d, J = 8.7 Hz, 1H), 7.36 - 7.29 (m, 2H), 7.27 - 7.16 (m, 1H), 7. 09 - 6. 99 (m, 1H), 6. 96 - 6.89 ( m, 3H), 6.72 - 6.58 (m, 1H), 4.09 - 4.03 (m, 2H), 3.09 (t, J = 2.4 Hz , 1H); LCMS(m/z) 422.2 [M+H]⁺.

### Embodiment 30

### Preparation of 3-[(5-phenoxy-1H-benzo[d]imidazole-2-yl)amino]-N-phenyl-1H-indole-5-carboxamide

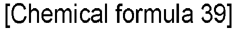

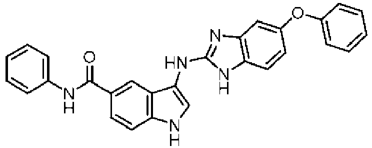

3-[(5-phenoxy-1H-benzo[d]imidazole-2-yl)amino]-1H-indole-5-carboxylic acid (46 mg, 0.12 mmol) in a pyridine solution (1 mL) was added with aniline (16 72 mg, 0.18 mmol) and EDCI/HCl (34.4 mg, 0.18 mmol) and stirred at room temperature for 16 hours. Ethyl acetate was added to the reaction mixture and washed with water. The obtained organic layer was dried over anhydrous sodium sulfate, and the solvent was distilled off under reduced pressure. The residue was crudely purified by amine-modified silica gel chromatography (chloroform:methanol=1:0-19:1) and further purified by silica gel chromatography (chloroform:methanol=1:0-19:1) to obtain the title compound (4 mg).

¹H NMR (400 MHz, DMSO-d6) δ 11. 14 - 11.08 (m, 1H), 10.69 - 10.58 (m, 1 H), 10. 15 (s, 1H), 9.29 - 9.25 (m, 1H), 8.39 (d, J = 1.5 Hz, 1H), 7.9 2 - 7.74 (m, 4H), 7.46 (d, J = 8.6 Hz, 1H), 7.38 - 7.28 (m, 4H), 7.28 - 7.18 (m, 1H), 7.11 - 7. 00 (m, 2H), 6. 98 - 6.89 (m, 3H), 6.73 - 6.6 1 (m, 1H); LCMS(m/z) 460.2 [M+H]⁺.

### Embodiment 31

### Preparation of N-(5-bromo-1H-pyrrolo[3,2-b]pyridine-3-yl)-5-(trifluoromethyl)-1H-benzo[d]imidazole-2-amine

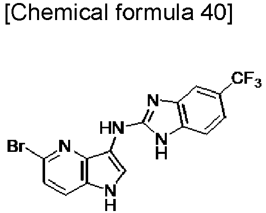

### (First step)

5-bromo-3-nitro-1H-pyrrolo[3,2-b]pyridine (100 mg, 0.413 mmol) was dissolved in a mixed solvent of methanol/saturated aqueous ammonium chloride solution (2:1, 3 mL), and added with zinc dust (270 mg, 4.13 mmol) and stirred at room temperature for 10 minutes. Boc₂O (108 mg, 0.496 mmol) was added to this mixture and stirred at room temperature for 1 hour. After the reaction mixture was diluted with ethanol, it was filtered using celite, and the filtrate was concentrated under reduced pressure. The obtained residue was diluted with ethyl acetate and washed with saturated aqueous sodium hydrogen carbonate solution. After drying the obtained organic layer over anhydrous sodium sulfate, the solvent was evaporated under reduced pressure to obtain tert-butyl (5-bromo-1H-pyrrolo[3,2-b]pyridine-3-yl)carbamate (120 mg).

### (Second step)

Tert-butyl (5-bromo-1H-pyrrolo[3,2-b]pyridine-3-yl)carbamate (40 mg, 0.128 mmol) and 2-chloro-5-(trifluoromethyl)-1H-benzo[d]imidazole (28. 3 mg, 0.128 mmol) were added to 4N hydrochloric acid/1,4-dioxane solution (2 mL), and the mixture was stirred under reflux conditions for 1.5 hours. To complete the reaction, DMF (1 mL) was added to the mixture and stirred under reflux conditions for a further 2.5 hours. After cooling the reaction mixture to room temperature, the solvent was distilled off under reduced pressure, and the residue was crudely purified by amine-modified silica gel chromatography (chloroform:methanol=1:0-97:3), and further purified by HPLC preparative chromatography to obtain the title compound as the formate salt (8 mg).

¹H NMR (400 MHz, DMSO-d6) δ 11.32 (brs, 1H), 10.92 - 10.76 (m, 1H), 9 .61 - 9.48 (m, 1H), 8.24 - 8.17 (m, 2H), 7.78 (d, J = 8.5 Hz, 1H), 7. 63 - 7.53 (m, 1H), 7.43 (brd, J = 8.3 Hz, 1H), 7.33 - 7.21 (m, 2H); L CMS(m/z) 398.1 [M+H]⁺.

### Embodiment 32

### Preparation of N-(5-bromo-1H-pyrrolo[3,2-b]pyridine-3-yl)-5-chloro-1H-benzo[d]imidazole-2-amine

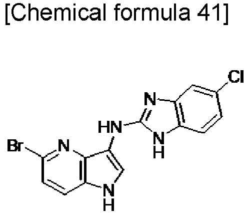

Tert-butyl (5-bromo-1H-pyrrolo[3,2-b]pyridine-3-yl)carbamate (40 mg, 0.128 mmol) and 2,5-dichloro-1H-benzo[d]imidazole (23.96 mg, 0.128 mmol) were added to 4N hydrochloric acid/1,4-dioxane solution (2 mL), and the mixture was stirred under reflux conditions for 1.5 hours. To complete the reaction, DMF (1 mL) was added to the mixture and stirred under reflux conditions for a further 2.5 hours. After cooling the reaction mixture to room temperature, the solvent was distilled off under reduced pressure, and the residue was crudely purified by amine-modified silica gel chromatography (chloroform:methanol=1:0-97:3), and further purified by HPLC preparative chromatography to obtain the title compound as the formate salt (9 mg).

¹H NMR (400 MHz, DMSO-d6) δ 11.28 (brs, 1H), 10.66 - 10.55 (m, 1H), 9 .37 (brs, 1H), 8.23 (s, 1H), 8.18 (brs, 1H), 7.77 (d, J = 8.5 Hz, 1H) , 7.34 - 7.23 (m, 3H), 6.95 (dd, J = 18.4, 8.3 Hz, 1H); LCMS(m/z) 364 . 0 [M+H]⁺.

### Embodiment 33

### Preparation of N-(5-bromo-1H-indol-3-yl)-5-fluoro-1H-benzo[d]imidazole-2-amine

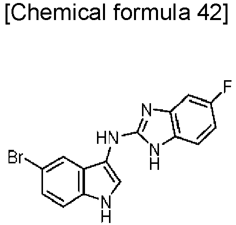

2-chloro-5-fluoro-1H-benzo[d]imidazole (0.1 g, 0.59 mmol) was added with 5-bromo-1H-indol-3-amine (0.148 g, 0.7 mmol) to obtain the title compound (40 mg) in the same manner as in the third step of Example 14.

¹H NMR (400 MHz, DMSO-d₆) δ 11. 07 - 10. 97 (m, 1H), 10.83 - 10.69 (m, 1 H), 9.14 - 9. 04 (m, 1H), 7.89 - 7.76 (m, 2H), 7.37 - 7.29 (m, 1H), 7. 24 - 7.17 (m, 1H), 7. 14 - 7. 11 (m, 1H), 7. 03 - 6. 93 (m, 1H), 6.82 - 6 .64 (m, 1H); LCMS(m/z) 345. 0 [M+H]⁺.

### Embodiment 34

### Preparation of methyl 2-[(5-bromo-1H-pyrrolo[3,2-b]pyridine-3-yl)amino]-1H-benzo[d]imidazole-5-carboxylate

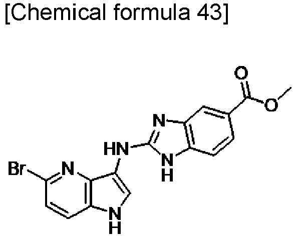

Tert-butyl (5-bromo-1H-pyrrolo[3,2-b]pyridine-3-yl)carbamate (45 1 mg, 0.144 mmol) in NMP solution (2 mL) was added with methyl 2-chloro-1H-benzo[d]imidazole-5-carboxylate (32 mg, 0.152 mmol) and p-toluenesulfonic acid monohydrate (43.3 mg, 0.228 mmol) and stirred at 120°C for 5 hours. After cooling the reaction mixture to room temperature, a saturated aqueous sodium hydrogencarbonate solution was added, and the precipitated solid was collected by filtration. The obtained crude product was crudely purified by amine-modified silica gel chromatography (chloroform:methanol=1:0-97:3) and further purified by HPLC preparative chromatography to obtain the title compound as a formate (4 mg).

¹H NMR (400 MHz, DMSO-d6) δ 11.33 (brs, 1H), 10.95 - 10.71 (m, 1H), 9 .61 - 9.43 (m, 1H), 8.30 (brs, 1H), 8.23 - 8.17 (m, 1H), 7.93 - 7.84 (m, 1H), 7.78 (d, J = 8.5 Hz, 1H), 7.69 - 7.60 (m, 1H), 7.37 - 7.27 ( m, 2H), 3.83 (s, 3H); LCMS(m/z) 388.1 [M+H]⁺.

### Embodiment 35

### Preparation of N-(5-chloro-1H-pyrrolo[3,2-b]pyridine-3-yl)-5-phenoxy-1H-benzo[d]imidazole-2-amine

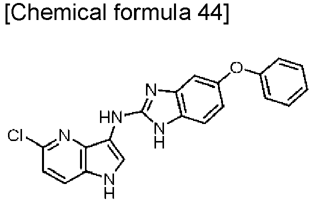

### (First step)

5-chloro-3-nitro-1H-pyrrolo[3,2-b]pyridine (50 mg, 0.253 mmol) was dissolved in a mixed solvent of methanol/saturated ammonium chloride aqueous solution (2:1, 3 mL), and added with zinc dust (165 mg, 2.53 mmol) and stirred at room temperature for 10 minutes. Boc₂O (66.3 mg, 0.304 mmol) was added and the mixture was stirred at room temperature for 2 hours. After diluting the reaction mixture with ethyl acetate, it was filtered using celite, and the filtrate was concentrated under reduced pressure to obtain tert-butyl (5-chloro-1H-pyrrolo[3,2-b]pyridine-3-yl)carbamate (60 mg).

### (Second step)

Tert-butyl (5-chloro-1H-pyrrolo[3,2-b]pyridine-3-yl)carbamate (60 mg, 0.224 mmol) in NMP solution (1.5 mL) was added with 2-chloro-5-phenoxy-1H-benzo[d]imidazole (43.9 mg, 0.179 mmol) and p-toluenesulfonic acid monohydrate (63.9 mg, 0.336 mmol) and stirred at 120°C for 3 hours. After cooling the reaction mixture to room temperature, a saturated aqueous sodium hydrogencarbonate solution was added, and the precipitated solid was collected by filtration. The obtained crude product was crudely purified by amine-modified silica gel chromatography (chloroform:methanol=1:0-19:1) and further purified by silica gel chromatography (chloroform:methanol=1:0-19:1) to obtain the title compound (11 mg).

¹H NMR (400 MHz, DMSO-d6) δ 11.23 (s, 1H), 10.53 - 10.42 (m, 1H), 9.2 8 (s, 1H), 8.20 (brs, 1H), 7.85 (d, J = 8.4 Hz, 1H), 7.33 (dd, J = 8. 5, 7.3 Hz, 2H), 7.27 (brd, J = 8.4 Hz, 1H), 7.18 (d, J = 8.5 Hz, 1H), 7.04 (t, J = 7.4 Hz, 1H), 7.00 - 6.90 (m, 3H), 6.74 - 6.61 (m, 1H);

LCMS(m/z) 376.1 [M+H]⁺.

### Embodiment 36

### Preparation of N-(5-methoxy-1H-pyrrolo[3,2-b]pyridine-3-yl)-5-phenoxy-1H-benzo[d]imidazole-2-amine

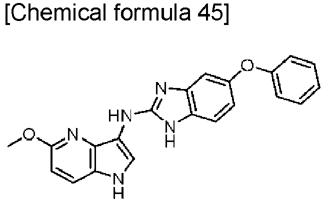

### (First step)

Concentrated sulfuric acid (1.5 mL) was added with potassium nitrate (75 mg, 0.742 mmol) and stirred at room temperature for 1 minute. After cooling the reaction mixture to -20°C, 5-methoxy-1H-pyrrolo[3,2-b]pyridine (100 mg, 0.675 mmol) was added and stirred at -20°C for 30 minutes. Ice water was added to the reaction mixture, and 28% aqueous ammonia was added to make it basic. The precipitated solid was collected by filtration and dried to obtain 5-methoxy-3-nitro-1H-pyrrolo[3,2-b]pyridine (87 mg).

¹H NMR (400 MHz, DMSO-d6) δ 8.62 (s, 1H), 7.87 (d, J = 8.8 Hz, 1H), 6 . 78 (d, J = 8.9 Hz, 1H), 3. 94 (s, 3H); LCMS(m/z) 194. 13 [M+H]⁺.

### (Second step)

5-Methoxy-3-nitro-1H-pyrrolo[3,2-b]pyridine (87 mg, 0.45 mmol) was added to a mixed solvent of methanol/saturated aqueous ammonium chloride solution (2:1, 4.5 mL) and added with zinc dust (294 mg, 4. 5 mmol) and stirred at room temperature for 10 minutes. Boc₂O (118 mg, 0.54 mmol) was added to the reaction mixture and stirred at room temperature for 20 minutes. After the reaction mixture was diluted with ethanol, it was filtered using celite, and the filtrate was concentrated under reduced pressure. The obtained residue was purified by silica gel chromatography (chloroform:methanol=1:0-97:3) to obtain tert-butyl 5-methoxy-1H-pyrrolo[3,2-b]pyridine-3-yl)carbamate (60 mg).

### (Third step)

Tert-butyl (5-methoxy-1H-pyrrolo[3,2-b]pyridine-3-yl)carbamate (60 mg, 0.228 mmol) in NMP solution (1.5 mL) was added with 2-chloro-5-phenoxy-1H-benzo[d]imidazole (39 mg, 0.16 mmol) and p-toluenesulfonic acid monohydrate (65 mg, 0.342 mmol) and stirred at 120°C for 3. 5 hours. After cooling the reaction mixture to room temperature, a saturated aqueous sodium hydrogencarbonate solution was added, and the precipitated solid was collected by filtration. The obtained crude product was crudely purified by silica gel chromatography (chloroform:methanol=1:0-19:1) and further purified by ion exchange column (SCX) to obtain the title compound (8 mg).

¹H NMR (400 MHz, DMSO-d6) δ 11. 02 (brs, 1H), 7.83 (brs, 1H), 7.74 (d, J = 8.7 Hz, 1H), 7.37 - 7.29 (m, 2H), 7.25 (d, J = 8.4 Hz, 1H), 7.04 (t, J = 7. 4 Hz, 1H), 6.97 - 6.90 (m, 3H), 6.70 (brd, J = 8.4 Hz, 1H) , 6.61 (d, J = 8.7 Hz, 1H), 3.90 (s, 3H); LCMS(m/z) 372.2 [M+H]⁺.

### Embodiment 37

### Preparation of 2-[(5-bromo-1H-pyrrolo[3,2-b]pyridine-3-yl)amino]-1H-benzo[d]imidazole-5-carboxylic acid

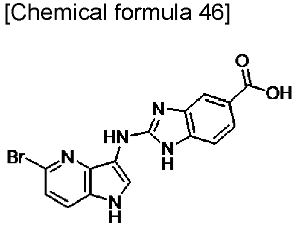

Tert-butyl (5-bromo-1H-pyrrolo[3,2-b]pyridine-3-yl)carbamate (127 mg, 0.407 mmol) and 2-chloro-1H-benzo[d]imidazole-5-carboxylic acid (76 mg, 0.387 mmol) were added to 4N hydrochloric acid/1,4-dioxane solution (3 mL), and the mixture was stirred under reflux conditions for 2.5 hours. To complete the reaction, DMF (1 mL) was added to the mixture and stirred under reflux conditions for a further 1.5 hours. After cooling the reaction mixture to room temperature, the solvent was distilled off under reduced pressure, and the residue was crudely purified by silica gel chromatography (chloroform:methanol=1:0-0:1), and further purified by HPLC preparative chromatography to obtain the title compound as the formate salt (4 mg).

¹H NMR (400 MHz, DMSO-d6) δ 11.37 - 11.28 (m, 1H), 10.92 - 10.76 (m, 1H), 9.55 - 9.39 (m, 1H), 8.45 (s, 1H), 8.23 - 8.17 (m, 1H), 7.86 (br s, 1H), 7.78 (d, J = 8.4 Hz, 1H), 7.67 - 7.59 (m, 1H), 7.33 - 7.26 (m , 2H); LCMS(m/z) 374.0 [M+H]⁺.

### Embodiment 38

### Preparation of N-(7-chloro-1H-pyrrolo[3,2-b]pyridine-3-yl)-5-phenoxy-1H-benzo[d]imidazole-2-amine

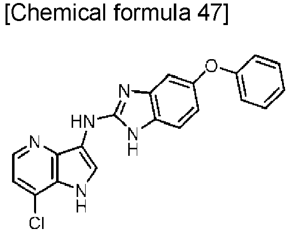

### (First step)

Concentrated sulfuric acid (1.5 mL) was added with potassium nitrate (93 mg, 0.918 mmol) and stirred at room temperature for 1 minute. After cooling the reaction mixture to 0°C, 7-chloro-1H-pyrrolo[3,2-b]pyridine (100 mg, 0.655 mg) was added and stirred at 0°C for 1 hour. Ice water was added to the reaction mixture, and 28% aqueous ammonia was added to make it basic. The precipitated solid was collected by filtration and dried to obtain 7-chloro-3-nitro-1H-pyrrolo[3,2-b]pyridine (68 mg).

¹1H NMR (400 MHz, DMSO-d6) δ 13.44 (s, 1H), 8.93 (s, 1H), 8.56 (d, J = 5.1 Hz, 1H), 7.56 (d, J = 5.1 Hz, 1H); LCMS(m/z) 198.08 [M+H]⁺.

### (Second step)

7-chloro-3-nitro-1H-pyrrolo[3,2-b]pyridine (68 mg, 0.344 mmol) was dissolved in a mixed solvent of methanol/saturated aqueous ammonium chloride solution (2:1,3 mL), and added with zinc dust (225 mg, 3.44 mmol) and stirred at room temperature for 10 minutes. Boc₂O (90 mg, 0.413 mmol) was added to the reaction mixture and stirred for 1.5 hours. After the reaction mixture was diluted with ethanol, it was filtered using celite, and the filtrate was concentrated under reduced pressure. The obtained residue was purified by silica gel chromatography (chloroform:methanol=1:0-49:1) to obtain tert-butyl (7-chloro-1H-pyrrolo[3,2-b]pyridine-3-yl)carbamate (45 mg).

¹H NMR (400 MHz, DMSO-d6) δ 11. 58 (s, 1H), 8.78 (s, 1H), 8.25 (d, J = 5.0 Hz, 1H), 7.71 (s, 1H), 7.28 (d, J = 5.0 Hz, 1H), 1.46 (s, 9H).

### (Third step)

Tert-butyl (7-chloro-1H-pyrrolo[3,2-b]pyridine-3-yl)carbamate (40 mg, 0.149 mmol) was added with 2-chloro-5-phenoxy-1H-benzo[d]imidazole in NMP solution (1 mL) (29.2 mg, 0.120 mmol) and p-toluenesulfonic acid monohydrate (42.6 mg, 0.224 mmol) and stirred at 120°C for 2 hours. After cooling the reaction mixture to room temperature, a saturated aqueous sodium hydrogencarbonate solution was added, and the precipitated solid was collected by filtration. The obtained solid was crudely purified by silica gel chromatography (chloroform:methanol=1:0-19:1) and further purified by ion exchange column (SCX) to obtain the title compound (14 mg).

¹H NMR (400 MHz, DMSO-d6) δ 11.63 (brs, 1H), 9.56 (brs, 1H), 8.30 (d, J = 5.0 Hz, 1H), 8.16 (d, J = 2.7 Hz, 1H), 7.49 (brs, 1H), 7.37 - 7. 27 (m, 4H), 7.08 - 7.02 (m, 1H), 6.99 (d, J = 2.3 Hz, 1H), 6.97 - 6.9 0 (m, 2H), 6.72 (brd, J = 8.3 Hz, 1H); LCMS(m/z) 376.1 [M+H]⁺.

### Embodiment 39

### Preparation of N-(5-bromo-1H-indol-3-yl)-1-methyl-6-phenoxy-1H-benzo[d]imidazole-2-amine

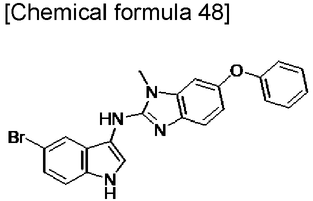

### (First step)

2-nitro-5-phenoxyaniline (100 mg, 0.434 mmol) in DMF (1 mL) under ice cooling was added with 60% sodium hydride (19.11 mg, 0.478 mmol) and stirred for 10 minutes. Methyl iodide (67.8 mg, 0.478 mmol) in DMF (1 mL) was added to the mixture and stirred at room temperature for 10 minutes. The reaction mixture was diluted with water and extracted with ethyl acetate. The obtained organic layer was washed with saturated brine and dried over anhydrous sodium sulfate, and the solvent was evaporated under reduced pressure to obtain N-methyl-2-nitro-5-phenoxyaniline (106 mg).

LCMS (m/z) 245.1 [M+H]⁺.

### (Second step)

N-methyl-2-nitro-5-phenoxyaniline (102 mg, 0.59 mmol) was used to obtain N1-methyl-5-phenoxybenzene-1,2-diamine (90 mg) by the same method as in the second step of Example 23.

LCMS (m/z) 215.2 [M+H]⁺.

### (Third step)

N1-methyl-5-phenoxybenzene-1,2-diamine (86.5 mg, 0.403 mmol) was used to obtain 1-methyl-6-phenoxy-1,3-dihydro-2H-benzo[d]imidazole-2-one (55.2 mg) by the same method as in the first step of Example 3.

LCMS (m/z) 241.2 [M+H]⁺.

### (Fourth step)

1-methyl-6-phenoxy-1,3-dihydro-2H-benzo[d]imidazole-2-one (53 mg, 0.221 mmol) was used to obtain 2-chloro-1-methyl-6-phenoxy-1H-benzo[d]imidazole (32.4 mg) by the same method as in the second step of Example 3 .

LCMS (m/z) 259.1 [M+H]⁺.

### (Fifth step)

2-chloro-1-methyl-6-phenoxy-1H-benzo[d]imidazole (6 mg, 0.023 mmol) and 5-bromo-1H-indol-3-amine (5.38 mg, 0.026 mmol) were added to 4N hydrochloric acid/1,4-dioxane solution (0.504 mL) and stirred at 120°C for 48 hours. After cooling the reaction mixture to 0°C, the precipitated solid was collected by filtration, washed with ethyl acetate, and purified by HPLC preparative chromatography to obtain the title compound as a formate salt (1. 9 mg).

¹H NMR (400 MHz, DMSO-d₆) 811.01 (s, 1H), 8.58 (s, 1H), 8.24 (s, 1H), 8.03 (d, J = 2.0 Hz, 1H), 7.93 (d, J = 2.4 Hz, 1H), 7.36 - 7.30 (m, 3H), 7.27 (d, J = 8.3 Hz, 1H), 7.21 (dd, J = 8.5, 2.0 Hz, 1H), 7.08 - 7.01 (m, 2H), 6.95 - 6.90 (m, 2H), 6.73 (dd, J = 8.5, 2.4 Hz, 1H), 3 .70 (s, 3H); LCMS(m/z) 433.1 [M+H]⁺.

### Embodiment 40

### Preparation of N-(5-bromo-1H-pyrrolo[3,2-b]pyridine-3-yl)-1-methyl-6-phenoxy-1H-benzo[d]imidazole-2-amine

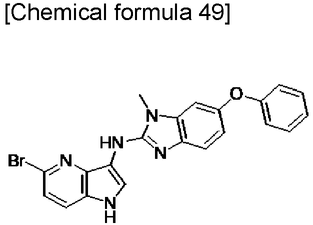

2-chloro-1-methyl-6-phenoxy-1H-benzo[d]imidazole (5 mg, 0.019 mmol) and tert-butyl (5-bromo-1H-pyrrolo[3,2-b]pyridine-3-yl)carbamate (6.64 mg, 0.021 mmol) were added to 4N hydrochloric acid/1,4-dioxane solution (0.5 mL) and stirred at 120°C for 6 days. After cooling the reaction mixture to room temperature, the solvent was distilled off under reduced pressure, and the residue was purified using HPLC preparative chromatography to obtain the title compound as a formate salt (1. 3 mg).

¹H NMR (400 MHz, DMSO-d₆) δ 11.32 (s, 1H), 8.74 (s, 1H), 8.39 (s, 1H), 8.22 (d, J = 2.7 Hz, 1H), 7.78 (d, J = 8.4 Hz, 1H), 7.36 - 7.30 (m, 2H), 7.28 (dd, J = 8.4, 7.2 Hz, 2H), 7. 08 (d, J = 2.4 Hz, 1H), 7.06 - 7.01 (m, 1H), 6.96 - 6.90 (m, 2H), 6.73 (dd, J = 8.4, 2.4 Hz, 1H), 3 .71 (s, 3H); LCMS(m/z) 434.2 [M+H]⁺.

### Embodiment 41

### Preparation of N-(5-bromo-1H-pyrrolo[3,2-b]pyridine-3-yl)-1-methyl-5-phenoxy-1H-benzo[d]imidazole-2-amine

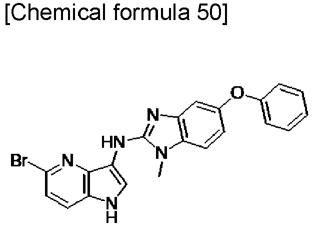

### (First step)

2-nitro-4-phenoxyaniline (230 mg, 1 mmol) was used to obtain N-methyl-2-nitro-4-phenoxyaniline (241 mg) by the same method as in the first step of Example 39. LCMS (m/z) 245.2 [M+H]⁺.

### (Second step)

N-methyl-2-nitro-4-phenoxyaniline (237 mg, 0.97 mmol) was used to obtain N1-methyl-4-phenoxybenzene-1,2-diamine (193 mg) by the same method as in the second step of Example 23.

LCMS (m/z) 215.2 [M+H]⁺.

### (Third step)

N1-methyl-4-phenoxybenzene-1,2-diamine (190 mg, 0.887 mmol) was used to obtain 1-methyl-5-phenoxy-1,3-dihydro-2H-benzo[d]imidazole-2-one (142 mg) by the same method as in the third step of Example 26.

LCMS (m/z) 241.2 [M+H]⁺.

### (Fourth step)

1-methyl-5-phenoxy-1,3-dihydro-2H-benzo[d]imidazole-2-one (140 mg, 0.583 mmol) was used to obtain 2-chloro-1-methyl-5-phenoxy-1H-benzo[d]imidazole (68 mg) by the same method as in the second step of Example 3.

LCMS (m/z) 259.2 [M+H]⁺.

### (Fifth step)

2-chloro-1-methyl-5-phenoxy-1H-benzo[d]imidazole (12 mg, 0.046 mmol) and tert-butyl (5-bromo-1H-pyrrolo[3,2-b]pyridine-3-yl)carbamate (15. 93 mg, 0.051 mmol) were used to obtain the title compound as the formate salt (2.5 mg) by the same method as the third step of Example 14 .

¹H NMR (400 MHz, DMSO-d₆) δ 11.33 (s, 1H), 8.79 (s, 1H), 8.30 (s, 1H), 8.18 (d, J = 2.7 Hz, 1H), 7.77 (d, J = 8.5 Hz, 1H), 7.35 - 7.25 (m, 4H), 7.06 - 7. 00 (m, 1H), 6.96 - 6.88 (m, 3H), 6.73 (dd, J = 8.4, 2.3 Hz, 1H), 3.75 (s, 3H) ; LCMS(m/z) 434.2 [M+H]⁺.

### Embodiment 42

### Preparation of N-(5-bromo-1H-pyrrolo[3,2-b]pyridine-3-yl)-1-methyl-6 -(trifluoromethyl)-1H-benzo[d]imidazole-2-amine

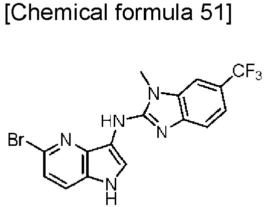

### (First step)

2-chloro-1-nitro-4-(trifluoromethyl)benzene (400 mg, 1.773 mmol) was added with 2M methylamine/THF solution (2. 22 mL) and stirred at room temperature for 18 hours. The reaction mixture was diluted with water and then extracted with ethyl acetate. The obtained organic layer was washed with saturated brine and dried over anhydrous sodium sulfate, and the solvent was distilled off under reduced pressure. To complete the reaction, the obtained residue was again treated with a 2M methylamine/THF solution (2. 22 mL) in order to obtain N-methyl-2-nitro-5-(trifluoromethyl)aniline (379 mg) by a similar reaction.

LCMS(m/z) 221.3[M+H]⁺.

### (Second step)

N-methyl-2-nitro-5-(trifluoromethyl)aniline (377 mg, 1.712 mmol) was dissolved in ethanol (6 mL), and 10% palladium carbon (91 mg) was added, and the reaction was carried out at room temperature for 2 hours under a hydrogen atmosphere. The insoluble matter was filtered off, and the filtrate was concentrated to obtain N1-methyl-5-(trifluoromethyl)benzene-1,2-diamine (300 mg).

LCMS (m/z) 191.1 [M+H]⁺.

### (Third step)

N1-methyl-5-(trifluoromethyl)benzene-1,2-diamine (297 mg, 1.562 mmol) in THF (10 mL) was added with CDI (380 mg, 2.343 mmol) in THF (10 mL) and stirred at 70°C for 24 hours. The reaction mixture was diluted with water and extracted with ethyl acetate. The obtained organic layer was washed with saturated brine and dried over anhydrous sodium sulfate, and the solvent was distilled off under reduced pressure. The residue was purified by silica gel chromatography (hexane:ethyl acetate=1:0-0:1) to obtain 1-methyl-6-(trifluoromethyl)-1,3-dihydro-2H-benzo[d]imidazole-2-one (202 mg).

LCMS (m/z) 217.1 [M+H]⁺.

### (Fourth step)

1-methyl-6-(trifluoromethyl)-1,3-dihydro-2H-benzo[d]imidazole-2-one (201 mg, 0.93 mmol) was used to obtain 2-chloro-1-methyl-6-(trifluoromethyl)-1H-benzo[d]imidazole (103 mg) by the same method as the second step of Example 3.

LCMS (m/z) 235.1 [M+H]⁺.

### (Fifth step)

2-chloro-1-methyl-6-(trifluoromethyl)-1H-benzo[d]imidazole (15 mg, 0.064 mmol) was added with tert-butyl (5-bromo-1H-pyrrolo[3,2-b]pyridine-3-yl)carbamate (21.95 mg, 0.07 mmol) in NMP solution (0.3 mL) and p-toluenesulfonic acid monohydrate (18.24 mg, 0.096 mmol) and stirred at 120°C for 1 hour. After cooling the reaction mixture to room temperature, it was purified by silica gel chromatography (hexane:ethyl acetate=1:0-0:1, then ethyl acetate:methanol=1:0-0:1), followed by ion exchange column (SCX) to obtain the title compound.(7.3 mg).

¹H NMR (400 MHz, DMSO-d₆) *δ*11.41 (s, 1H), 9.02 (s, 1H), 8.18 (d, J = 2.7 Hz, 1H), 7.79 (d, J = 8.5 Hz, 1H), 7.66 (s, 1H), 7.43 - 7.26 (m, 3H), 3.82 (s, 3H) ; LCMS(m/z) 410.2 [M+H]⁺.

### Embodiment 43

### Preparation of N-(5-bromo-1H-pyrrolo[3,2-b]pyridine-3-yl)-1-methyl-5-(trifluoromethyl)-1H-benzo[d]imidazole-2-amine

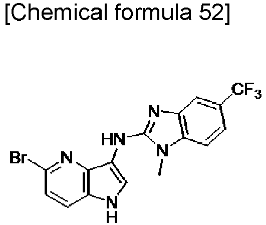

### (First step)

1-methyl-5-(trifluoromethyl)-1,3-dihydro-2H-benzo[d]imidazole-2-one (316 mg, 1.462 mmol) was used to obtain 2-chloro-1-methyl-5-(trifluoromethyl)-1H-benzo[d]imidazole (246 mg) by the same method as the second step of Example 3.

LCMS (m/z) 235.1 [M+H]⁺.

### (Second step)

2-chloro-1-methyl-5-(trifluoromethyl)-1H-benzo[d]imidazole (11 mg, 0.047 mmol) was added with tert-butyl (5-bromo-1H-pyrrolo[3,2-b]pyridine-3-yl)carbamate (16.10 mg, 0.052 mmol) in NMP solution (0.3 mL) with p-toluenesulfonic acid monohydrate (13.38 mg, 0.07 mmol) and stirred at 120°C for 1 hour. After cooling the reaction mixture to room temperature, it was treated with an ion exchange column (SCX) and purified using HPLC preparative chromatography to obtain the title compound as a formate salt (1.5 mg).

¹H NMR (400 MHz, DMSO-d₆) *δ*11.42 (s, 1H), 9.01 (s, 1H), 8.52 (s, 1H), 8.20 (d, J = 2.7 Hz, 1H), 7.79 (d, J = 8.4 Hz, 1H), 7.55 (s, 1H), 7. 46 (d, J = 8.3 Hz, 1H), 7.35 - 7.32 (m, 1H), 7.30 (d, J = 8.5 Hz, 1H) , 3.81 (s, 3H); LCMS(m/z) 410.1 [M+H]⁺.

### Embodiment 44

### Preparation of N-(5-chloro-1H-pyrrolo[3,2-b]pyridine-3-yl)-1-methyl-6-phenoxy-1H-benzo[d]imidazole-2-amine

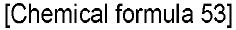

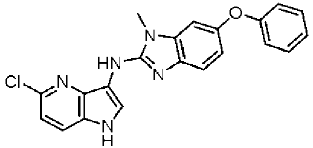

2-chloro-1-methyl-6-phenoxy-1H-benzo[d]imidazole (18 mg, 0.07 mmol) was added with tert-butyl (5-chloro-1H-pyrrolo[3,2-b]pyridine-3-yl)carbamate (20.49 mg, 0.077 mmol) in NMP solution (0.5 mL) with p-toluenesulfonic acid monohydrate (19.85 mg, 0.104 mmol) and stirred at 120°C for 1 hour. After cooling the reaction mixture to room temperature, a saturated aqueous sodium hydrogencarbonate solution was added, and the mixture was extracted with chloroform. The obtained organic layer was washed with water and saturated brine, and dried over anhydrous sodium sulfate. The solvent was evaporated under reduced pressure, and the residue was purified by silica gel chromatography (hexane:ethyl acetate=1:0-0:1, then ethyl acetate:methanol=1:0-0:1) to obtain the title compound (7.4 mg).

¹H NMR (400 MHz, DMSO-d₆) *δ*11.30 (s, 1H), 8.74 (s, 1H), 8.24 (d, J = 2.7 Hz, 1H), 7.86 (d, J = 8.4 Hz, 1H), 7.36 - 7.30 (m, 2H), 7.27 (d, J = 8.4 Hz, 1H), 7.18 (d, J = 8.4 Hz, 1H), 7.08 (d, J = 2.3 Hz, 1H), 7.07 - 7.00 (m, 1H), 6.95 - 6.90 (m, 2H), 6.73 (dd, J = 8.4, 2.3 Hz, 1H), 3.71 (s, 3H); LCMS(m/z) 390.2 [M+H]⁺.

### Embodiments 45 to 47

The following example compounds [Table 1] were produced using corresponding starting materials, according to the method described in Example 44 above, and, if necessary, by appropriately combining methods commonly used in synthetic organic chemistry. In addition, the physicochemical data of each compound are shown in [Table 2].

**[Table 1]**

| **Embodi ments** | **Structural formula** | **Compound name** |
|---|---|---|
| **45** | 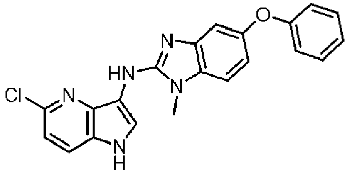 | **N-(5-Chloro-1H-pyrrolo[3,2-b]pyri dine-3-yl)-1-methyl-5-phenoxy-1 H-benzo[d]imidazole-2-amine** |
| **46** | 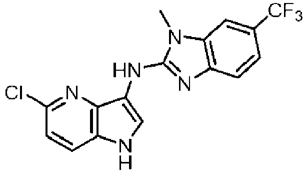 | **N-(5-Chloro-1H-pyrrolo[3,2-b]pyri dine-3-yl)-1-methyl-6-(trifluorome thyl)-1H-benzo[d]imidazole-2-ami ne** |
| **47** | 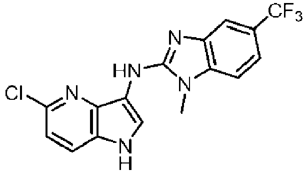 | **N-(5-Chloro-1H-pyrrolo[3,2-b]pyri dine-3-yl)-1-methyl-5-(trifluorome thyl)-1H-benzo[d]imidazole-2-ami ne** |

**[Table 2]**

| Embodi ments | ¹H-NMR δ (ppm) | LCMS m/z [M+H]⁺ |
|---|---|---|
| **45** | (DMSO-d6) *δ* 11.33 (s, 1H), 8.81 (s, 1H), 8.20 (s, 1H), 7 .86 (d, J = 8.5 Hz, 1H), 7.39 - 7.23 (m, 3H), 7.18 (d, J = 8.5 Hz, 1H), 7.03 (t, J = 7.3 Hz, 1H), 6.97 - 6.84 (m, 3H) , 6.78 - 6.70 (m, 1H), 3.76 (s, 3H). | 390.2 |
| **46** | (DMSO-d6) *δ* 11.39 (s, 1H), 9.02 (s, 1H), 8.20 (d, J = 2. 7 Hz, 1H), 7.87 (d, J = 8.5 Hz, 1H), 7.66 (d, J = 1.7 Hz, 1H), 7.41 - 7.36 (m, 1H), 7.35 - 7.30 (m, 1H), 7.19 (d, J = 8.5 Hz, 1H), 3.82 (s, 3H). | 366.1 |
| **47** | (DMSO-d6) *δ* 11.40 (d, J = 2.1 Hz, 1H), 9.01 (s, 1H), 8. 22 (d, J = 2.7 Hz, 1H), 7.87 (d, J = 8.5 Hz, 1H), 7.55 (s, 1H), 7.46 (d, J = 8.2 Hz, 1H), 7.38 - 7.29 (m, 1H), 7.19 ( d, J = 8.5 Hz, 1H), 3.81 (s, 3H). | 366.1 |

### Embodiment 48

### Preparation of N-[5-(Cyclohex-1-en-1-yl)-1H-pyrrolo[3,2-b]pyridine-3-yl]-5-phenoxy-1H-benzo[d]imidazole-2-amine

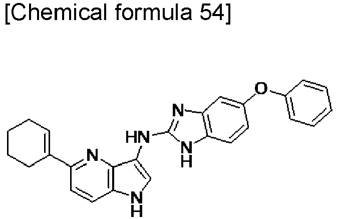

N-(5-bromo-1H-pyrrolo[3,2-b]pyridine-3-yl)-5-phenoxy-1H-benzo[d]imidazole-2-amine (75 mg, 0.178 mmol) was added to a mixed solvent of 1,4-dioxane/water (10:1, 2.2 mL) with 1-cyclohexen-1-yl-boronic acid (33.7 mg, 0.268 mmol), and bis[di-tert-butyl(4-dimethylaminophenyl)phosphine]dichloropalladium (II) (12.64 mg, 0.018 mmol) and cesium fluoride (81 mg, 0.535 mmol), and reacted at 100°C for 30 minutes using a microwave reactor. After cooling the reaction mixture to room temperature, the solvent was concentrated under reduced pressure. The obtained residue was crudely purified by silica gel chromatography (chloroform:methanol=1:0-0:1) and further purified by HPLC preparative chromatography to obtain the title compound as a formate salt (2 mg).

¹H NMR (400 MHz, Methanol-d4) δ 8.49 (s, 1H), 7.83 (d, J = 8.7 Hz, 1H ), 7.56 (s, 1H), 7.48 (d, J = 8.7 Hz, 1H), 7.40 - 7.33 (m, 2H), 7.29 (d, J = 8.5 Hz, 1H), 7.15 - 7. 08 (m, 1H), 7. 04 - 6.99 (m, 2H), 6.94 - 6.87 (m, 2H), 6.66 - 6.59 (m, 1H), 2.69 - 2.63 (m, 2H), 2.31 - 2.24 (m, 2H), 1.85 - 1.75 (m, 2H), 1.75 - 1.66 (m, 2H); LCMS(m/z) 422.2 [M +H]⁺.

### Embodiment 49

### Preparation of N-(6-bromo-1H-pyrrolo[3,2-b]pyridine-3-yl)-5-phenoxy-1H-benzo [d]imidazole-2-amine

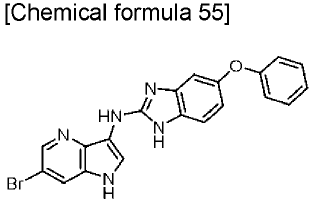

### (First step)

6-bromo-3-nitro-1H-pyrrolo[3,2-b]pyridine (301 mg, 1.244 mmol) was dissolved in a mixed solvent of methanol/saturated aqueous ammonium chloride solution (2:1, 15 mL), and added with zinc dust (813 mg, 12.44 mmol) and stirred at room temperature for 10 minutes. Boc₂O (326 mg, 1.492 mmol) was added to this mixture and stirred at room temperature for 1 hour. After the reaction mixture was diluted with ethanol, it was filtered using celite, and the filtrate was concentrated under reduced pressure. The obtained residue was purified by silica gel chromatography (chloroform:methanol=1:0-97:3) to obtain tert-butyl (6-bromo-1H-pyrrolo[3,2-b]pyridine-3-yl)carbamate (250 mg).

¹H NMR (400 MHz, DMSO-d6) δ 11. 15 (s, 1H), 8.74 (s, 1H), 8.34 (d, J = 2.0 Hz, 1H), 7.96 (d, J = 2.0 Hz, 1H), 7.68 (s, 1H), 1.45 (s, 9H).

### (Second step)

Tert-butyl (6-bromo-1H-pyrrolo[3,2-b]pyridine-3-yl)carbamate (150 mg, 0.707 mmol) was added with 2-chloro-5-phenoxy-1H-benzo[d]imidazole in NMP solution (3 mL) (156 mg, 0.637 mmol) and p-toluenesulfonic acid monohydrate (202 mg, 1.061 mmol) and stirred at 120°C for 2 hours. After cooling the reaction mixture to room temperature, saturated aqueous sodium hydrogen carbonate solution was added, and the mixture was extracted with ethyl acetate. The organic layer was concentrated under reduced pressure, and the obtained residue was purified by amine-modified silica gel chromatography (chloroform:methanol=1:0-19:1) to obtain the title compound (20 mg).

¹H NMR (400 MHz, DMSO-d6) δ 11. 19 (brs, 1H), 10.61 (brs, 1H),9.48 (br s, 1H), 8.40 (d, J = 2.0 Hz, 1H), 8.12 (s, 1H), 8.04 (d, J = 2.0 Hz, 1H), 7.38 - 7.31 (m, 2H), 7.28 (d, J = 8.4 Hz, 1H), 7.05 (t, J = 7.4 Hz, 1H), 7.00 - 6.90 (m, 3H), 6.71 (brd, J = 8.3 Hz, 1H); LCMS(m/z) 4 20.1 [M+H]⁺.

### Embodiment 50

### Preparation of N-(5-cyclohexyl-1H-pyrrolo[3,2-b]pyridine-3-yl)-5-phenoxy-1H-benzo[d]imidazole-2-amine

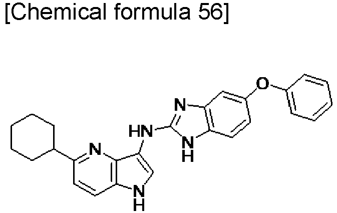

N-[5-(cyclohex-1-en-1-yl)-1H-pyrrolo[3,2-b]pyridine-3-yl]-5-phenoxy-1H-benzo[d]imidazole-2-amine (16 mg, 0.038 mmol) was dissolved in a mixed solvent of ethanol/ethyl acetate (1:1, 3 mL), and 10% palladium carbon (4.04 mg) was added, and the mixture was stirred at room temperature under a hydrogen atmosphere for 1.5 hours, and further stirred at 50°C overnight. Insoluble matters were filtered off, and the filtrate was concentrated under reduced pressure. To complete the reaction, the obtained residue was dissolved in ethanol solvent (1 mL) and washed with 10% palladium on carbon (4.04 mg), and the mixture was stirred overnight at room temperature under a hydrogen atmosphere. The insoluble matter was filtered off, the filtrate was concentrated under reduced pressure, and the residue was purified using HPLC preparative chromatography to obtain the title compound as a formate salt (0.35 mg).

¹H NMR (400 MHz, Methanol-d4) δ 8.55 (s, 1H), 7.79 (d, J = 8.5 Hz, 1H ), 7.62 (s, 1H), 7.37 - 7.30 (m, 2H), 7.27 (d, J = 8.5 Hz, 1H), 7.17 (d, J = 8.5 Hz, 1H), 7.09 - 7.01 (m, 1H), 7.00 - 6.96 (m, 3H), 6. 80 ( dd, J = 8.4, 2.3 Hz, 1H), 2.94 - 2.82 (m, 1H), 2.12 - 2.03 (m, 1H), 1 .95 - 1.87 (m, 3H), 1.85 - 1.76 (m, 2H), 1.76 - 1.63 (m, 2H), 1.58 - 1.45 (m, 1H), 1.40 - 1.30 (m, 1H); LCMS(m/z) 422.3 [M-H]⁻.

### Embodiment 51

### Preparation of 5-phenoxy-N-(6-phenyl-1H-pyrrolo[3,2-b]pyridine-3-yl)-1H-benzo [d]imidazole-2-amine

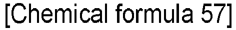

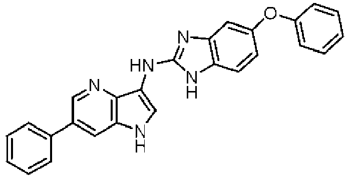

N-(6-bromo-1H-pyrrolo[3,2-b]pyridine-3-yl)-5-phenoxy-1H-benzo[d]imidazole-2-amine (12 mg, 0.029 mmol) was added to a mixed solvent of 1,4-dioxane/water (10:1,0.55 mL) with phenylboronic acid (5.22 mg, 0.043 mmol), and bis[di-tert-butyl(4-dimethylaminophenyl)phosphine]dichloropalladium (II) (12.02 mg, 0.00286 mmol) and cesium fluoride (13.01 mg, 0.086 mmol), and reacted at 100°C for 30 minutes using a microwave reactor. After cooling the reaction mixture to room temperature, the solvent was concentrated under reduced pressure. The obtained residue was purified by silica gel chromatography (chloroform:methanol=1:0-19:1) to obtain the title compound (7 mg).

¹H NMR (400 MHz, DMSO-d6) δ 11. 08 - 11.02 (m, 1H), 10.74 - 10.65 (m, 1H), 9.32 (s, 1H), 8.65 (s, 1H), 8.12 (brd, J = 13.5 Hz, 1H), 7.98 (d , J = 2.0 Hz, 1H), 7.79 - 7.72 (m, 2H), 7.51 (dd, J = 8.3, 7.0 Hz, 2H ), 7.43 - 7.37 (m, 1H), 7.36 - 7.24 (m, 3H), 7.04 (t, J = 7.4 Hz, 1H) , 7.01 - 6.90 (m, 3H), 6.68 (dd, J = 23.8, 8.8 Hz, 1H); LCMS(m/z) 418 .2 [M+H]⁺.

### Embodiment 52

### Preparation of N-(7-methyl-1H-pyrrolo[3,2-b]pyridine-3-yl)-5-phenoxy-1H-benzo [d]imidazole-2-amine

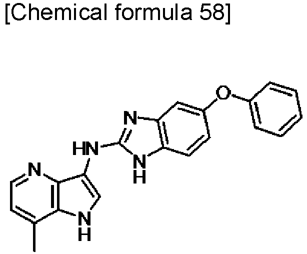

### (First step)

Concentrated sulfuric acid (1.5 mL) was added with potassium nitrate (107 mg, 1.059 mmol) and stirred at room temperature for 1 minute, and cooled to 0°C. 7-methyl-1H-pyrrolo[3,2-b]pyridine (100 mg, 0.757 mmol) was added to the reaction mixture and stirred at 0°C for 40 minutes. Ice water was added to the reaction mixture, and 28% aqueous ammonia was added to make it basic. The precipitated solid was collected by filtration and dried to obtain 7-methyl-3-nitro-1H-pyrrolo[3,2-b]pyridine (115 mg).

¹H NMR (400 MHz, DMSO-d6) δ 12.93 (s, 1H), 8.84 (s, 1H), 8.45 (d, J = 4.8 Hz, 1H), 7. 19 (dd, J = 4.8, 0.9 Hz, 1H), 2.55 (d, J = 0.9 Hz, 3H ); LCMS(m/z) 178.09 [M+H]⁺.

### (Second step)

7-methyl-3-nitro-1H-pyrrolo[3,2-b]pyridine (114 mg, 0.643 mmol) was added to a mixed solvent of methanol/saturated aqueous ammonium chloride solution (2:1, 4.5 mL) with zinc dust (421 mg, 6.43 mmol) and stirred at room temperature for 10 minutes. Boc₂O (169 mg, 0.772 mmol) was added to this mixture and stirred at room temperature for 1 hour. The reaction mixture was diluted with methanol, filtered through celite, and the filtrate was concentrated under reduced pressure. The obtained residue was purified by amine-modified silica gel chromatography (chloroform:methanol=1:0-97:3) to obtain tert-butyl (7-methyl-1H-pyrrolo[3,2-b]pyridine-3-yl)carbamate (120 mg).

LCMS (m/z) 248.16 [M+H]⁺.

### (Third step)

Tert-butyl (7-methyl-1H-pyrrolo[3,2-b]pyridine-3-yl)carbamate (110 mg, 0.445 mmol) was added with 2-chloro-5-phenoxy-1H-benzo[d]imidazole in NMP solution (2 mL) (76 mg, 0.311 mmol) and p-toluenesulfonic acid monohydrate (127 mg, 0. 667 mmol) and stirred at 120°C for 2.5 hours. After cooling the reaction mixture to room temperature, a saturated aqueous sodium hydrogencarbonate solution was added, and the precipitated solid was collected by filtration. The obtained solid was crudely purified by amine-modified silica gel chromatography (chloroform:methanol=1:0-19:1) and further purified by HPLC preparative chromatography to obtain the title compound as a formate salt (44 mg).

¹H NMR (400 MHz, Methanol-d4) δ 8.42 (brs, 1H), 8.28 (d, J = 4.8 Hz, 1H), 7. 81 (s, 1H), 7.36 - 7.31 (m, 2H), 7.29 (d, J = 8.6 Hz, 1H), 7.1 5 (dd, J = 4.9, 0.9 Hz, 1H), 7.12 - 7.04 (m, 1H), 7.01 - 6.92 (m, 3H) , 6.86 (dd, J = 8.6, 2.3 Hz, 1H), 2.65 (d, J = 0.8 Hz, 3H); LCMS(m/z) 356.2 [M+H]⁺.

### Embodiment 53

### Preparation of N-(5-methyl-1H-pyrrolo[3,2-b]pyridine-3-yl)-5-phenoxy-1H-benzo [d]imidazole-2-amine

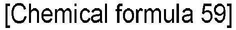

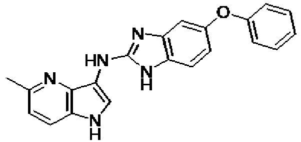

### (First step)

Tert-butyl 5-bromo-1H-pyrrolo[3,2-b]pyridine-3-yl)carbamate (125 mg, 0.4 mmol) in 1,4-dioxane solution (3 mL) was added with trimethylboroxine (75 mg, 0.601 mmol), and potassium carbonate (221 mg, 1.602 mmol), and [1,3-bis(2,6-diisopropylphenyl)imidazole-2-ylidene](3-chloropyridyl)palladium (II) dichloride (27.2 mg, 0.04 mmol) and stirred at 130°C for 1.5 hours using a microwave reactor. After cooling the reaction mixture to room temperature, the solvent was concentrated under reduced pressure, and the obtained residue was purified by amine-modified silica gel chromatography (chloroform:methanol=1:0-97:3) to obtain (5-methyl-1H-pyrrolo[ tert-butyl 3,2-b]pyridine-3-yl)carbamate (35 mg).

### (Second step)

Tert-butyl (5-methyl-1H-pyrrolo[3,2-b]pyridine-3-yl)carbamate (35 mg, 0.142 mmol) in NMP solution (1.5 mL) was added with 2-chloro-5-phenoxy-1H-benzo[d]imidazole (27.7 mg, 0.113 mmol) and p-toluenesulfonic acid monohydrate (40.4 mg, 0.212 mmol) and stirred at 120°C for 2 hours. After cooling the reaction mixture to room temperature, a saturated aqueous sodium hydrogencarbonate solution was added, and the precipitated solid was collected by filtration. The obtained crude product was crudely purified by amine-modified silica gel chromatography (chloroform:methanol=1:0-97:3) and further purified by HPLC preparative chromatography to obtain the title compound as a formate (8 mg).

¹H NMR (400 MHz, DMSO-d6) δ 10.82 - 10.80 (m, 1H), 9.07 (brs, 1H), 8. 19 (s, 1H), 7.99 (d, J = 2.7 Hz, 1H), 7.66 (d, J = 8.3 Hz, 1H), 7.38 - 7.29 (m, 2H), 7.26 (d, J = 8.3 Hz, 1H), 7.07 - 7.00 (m, 2H), 6.96 ( d, J = 2.4 Hz, 1H), 6.95 - 6.90 (m, 2H), 6.66 (dd, J = 8.3, 2.4 Hz, 1 H), 2.59 (s, 3H); LCMS(m/z) 356.1 [M+H]⁺.

### Embodiment 54

### Preparation of 5-phenoxy-N-(7-phenyl-1H-pyrrolo[3,2-b]pyridine-3-yl)-1H-benzo [d]imidazole-2-amine

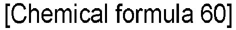

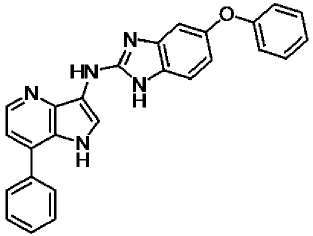

### (First step)

7-chloro-3-nitro-1H-pyrrolo[3,2-b]pyridine (95 mg, 0.481 mmol) was dissolved in a mixed solution of 1,4-dioxane solution/water (2:1, 3 mL) with phenylboronic acid (88 mg, 0.721 mmol), and sodium carbonate (204 mg, 1.923 mmol), and tetrakis(triphenylphosphine)palladium (0) (111 mg, 0.096 mmol), and reacted at 150°C for 30 minutes using a microwave reactor. After cooling the reaction mixture to room temperature, the solvent was concentrated under reduced pressure, and the obtained residue was crudely purified by amine-modified silica gel chromatography (chloroform:methanol=1:0-4:1) to obtain an intermediate. This intermediate was dissolved in a mixed solvent of methanol/saturated aqueous ammonium chloride solution (2:1, 4.5 mL) and zinc dust (301 mg, 4.60 mmol) was added, and after stirring at room temperature for 10 minutes, Boc₂O (120 mg, 0.552 mmol) was added and the mixture was further stirred at room temperature for 40 minutes. The solvent was evaporated under reduced pressure, and the residue was purified by amine-modified silica gel chromatography (hexane:ethyl acetate=1:0-1:1) to obtain tert-butyl (7-phenyl-1H-pyrrolo[3,2-b]pyridine-3-yl)carbamate (90 mg).

### (Second step)

Tert-butyl (7-phenyl-1H-pyrrolo[3,2-b]pyridine-3-yl)carbamate (90 mg, 0.291 mmol) was added with 2-chloro-5-phenoxy-1H-benzo[d]imidazole (49. 8 mg, 0.204 mmol) and p-toluenesulfonic acid monohydrate (83 mg, 0.436 mmol) and stirred at 120°C for 2 hours. After cooling the reaction mixture to room temperature, a saturated aqueous sodium hydrogencarbonate solution was added, and the precipitated solid was collected by filtration. The obtained solid was crudely purified by amine-modified silica gel chromatography (chloroform:methanol=1:0-97:3) and further purified by silica gel chromatography (chloroform:methanol=1:0-97:3) to obtain the title compound (23 mg).

¹H NMR (400 MHz, DMSO-d6) δ 11.01 (s, 1H), 10.72 (brs, 1H), 9.35 (s, 1H), 8.43 (d, J = 4.8 Hz, 1H), 8.09 (d, J = 2.6 Hz, 1H), 7.82 - 7.75 (m, 2H), 7.66 - 7.57 (m, 2H), 7.59 - 7.49 (m, 1H), 7.36 - 7.31 (m, 2H ), 7.29 (d, J = 8.7 Hz, 1H), 7.26 (d, J = 4.8 Hz, 1H), 7.07 - 7.01 (m , 1H), 7.00 (d, J = 2.3 Hz, 1H), 6.96 - 6.91 (m, 2H), 6.69 (brd, J = 8.4 Hz, 1H); LCMS(m/z) 418.2 [M+H]⁺.

### Embodiment 55

### Preparation of 5-phenoxy-N-[5-(trifluoromethyl)-1H-pyrrolo[3,2-b]pyridine-3-yl]-1H-benzo[d]imidazole-2-amine

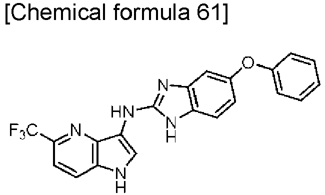

### (First step)

Potassium nitrate (243 mg, 2.407 mmol) was added to concentrated sulfuric acid (5 mL) and stirred at room temperature for 1 minute. The reaction mixture was cooled to 0°C, and 5-(trifluoromethyl)-1H-pyrrolo[3,2-b]pyridine (320 mg, 1.719 mmol) was added and stirred at 0°C for 1.5 hours. Ice water was added to the reaction mixture, and the precipitated solid was collected by filtration and dried to obtain 3-nitro-5-(trifluoromethyl)-1H-pyrrolo[3,2-b]pyridine (360 mg).

¹H NMR (400 MHz, DMSO-d6) δ 9. 06 (s, 1H), 8.22 (d, J = 8.5 Hz, 1H), 7 .82 (d, J = 8.6 Hz, 1H); LCMS(m/z) 232.06 [M+H]⁺.

### (Second step)

3-nitro-5-(trifluoromethyl)-1H-pyrrolo[3,2-b]pyridine (360 mg, 1.558 mmol) was dissolved in a mixed solvent of methanol/saturated aqueous ammonium chloride solution (2:1, 15 mL), and zinc dust (1.02 g, 15.58 mmol) was added and stirred at room temperature for 10 minutes. Boc₂O (408 mg, 1.869 mmol) was added to the mixture and stirred at room temperature for 50 minutes. After completion of the reaction, the solvent was distilled off under reduced pressure, the residue was purified by amine-modified silica gel chromatography (chloroform:methanol=1:0-97:3) to obtain [5-(trifluoromethyl)-1H-pyrrolo[3, tert-butyl 2-b]pyridine-3-yl]carbamate (310 mg).

### (Third step)

Tert-butyl [5-(trifluoromethyl)-1H-pyrrolo[3,2-b]pyridine-3-yl]carbamate (53 mg, 0.176 mmol) in NMP solution (1.5 mL) was added with 2-chloro-5-phenoxy-1H-benzo[d]imidazole (30.1 mg, 0.123 mmol) and p-toluenesulfonic acid monohydrate (50.2 mg, 0.264 mmol) and stirred at 120°C for 2.5 hours. After cooling the reaction mixture to room temperature, a saturated aqueous sodium hydrogencarbonate solution was added, and the precipitated solid was collected by filtration. The obtained crude product was purified by amine-modified silica gel chromatography (chloroform:methanol=1:0-19:1) to obtain the title compound (22 mg).

¹H NMR (400 MHz, DMSO-d6) δ 11.48 - 11.42 (m, 1H), 10.61 - 10.53 (m, 1H), 9.24 (s, 1H), 8.43 - 8.36 (m, 1H), 8.04 - 7.97 (m, 1H), 7.62 (dd , J = 8.4, 0.8 Hz, 1H), 7.39 - 7.25 (m, 3H), 7.08 - 7.03 (m, 1H), 7.0 2 - 6.97 (m, 1H), 6.96 - 6.91 (m, 2H), 6.75 - 6.65 (m, 1H); LCMS(m/z) 410.2 [M+H]⁺.

### Embodiment 56

### Preparation of N-(5-chloro-1H-indol-3-yl)-1-methyl-5-(trifluoromethyl)-1H-benzo [d]imidazole-2-amine

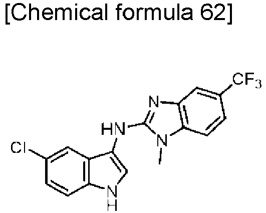

### (First step)

5-chloro-1H-indole-3-amine hydrochloride (600 mg, 2.95 mmol) in THF (15 mL) was added thiophosgene (374 mg, 3.25 mmol) and DIPEA (764 mg, 5.91 mmol) was added and stirred at room temperature for 30 minutes. Ethyl acetate was added to the reaction mixture, and the mixture was washed with water and saturated brine in that order. The obtained organic layer was dried over anhydrous sodium sulfate, and the solvent was distilled off under reduced pressure to obtain 5-chloro-3-isothiocyanato-1H-indole (617 mg).

¹H NMR (400 MHz, DMSO-d6) δ 11.77 (s, 1H), 7.84 (d, J = 2.9 Hz, 1H), 7.58 (d, J = 2.0 Hz, 1H), 7.48 (d, J = 8.7 Hz, 1H), 7.23 (dd, J = 8.7 , 2.0 Hz, 1H); LCMS(m/z) 207.1 [M-H]⁻.

### (Second step)

N1-methyl-4-(trifluoromethyl)benzene-1,2-diamine (356 mg, 1.87 mmol) in DMF solution (10 mL) of 5-chloro-3-isothiocyanato-1H-indole (411 mg, 1.97 mmol) was added and stirred at 50°C for 1.5 hours. After cooling the reaction mixture to room temperature, ethyl acetate was added, and the mixture was washed with water and saturated brine in that order. The obtained organic layer was dried over anhydrous sodium sulfate, the solvent was distilled off under reduced pressure, and the obtained residue was crudely purified by amine-modified silica gel chromatography (chloroform:methanol=1:0-4:1) to obtain the thiourea compound (413 mg). The obtained thiourea compound (410 mg, 1.03 mmol) was dissolved in DMF (8 mL), EDCl-HCl (296 mg, 1.54 mmol) was added to the solution, and the mixture was stirred at 50°C for 2 hours. In order to complete the reaction, the mixture was further stirred at 60°C for 1.5 hours. After cooling the reaction mixture to room temperature, ethyl acetate was added, and the mixture was washed with water and saturated brine in that order. The obtained organic layer was dried over anhydrous sodium sulfate, the solvent was distilled off under reduced pressure, and the obtained residue was purified by silica gel chromatography (hexane:ethyl acetate=1:0-1:1) to obtain the title compound (273 mg).

¹H NMR (400 MHz, DMSO-d6) δ 11. 07 (s, 1H), 8.80 (s, 1H), 7.92 (d, J = 2.5 Hz, 1H), 7.85 (dt, J = 2.2, 0.6 Hz, 1H), 7.56 - 7.54 (m, 1H), 7. 45 (d, J = 8.3 Hz, 1H), 7.40 (dd, J = 8.6, 0.6 Hz, 1H), 7.35 - 7.31 ( m, 1H), 7.11 (dd, J = 8.5, 2.1 Hz, 1H), 3.80 (s, 3H); LCMS(m/z) 365.1 [M+H]⁺.

### Embodiment 57

### Preparation of N-(5-chloro-1H-indol-3-yl)-1-cyclopropyl-5-(trifluoromethyl)-1H-benzo [d]imidazole-2-amine

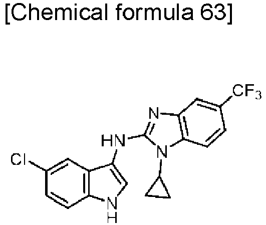

N1-cyclopropyl-4-(trifluoromethyl)benzene-1,2-diamine (607 mg, 2.81 mmol) was added to a DMF solution (10 mL) of 5-chloro-3-isothiocyanato-1H-indole (617 mg, 2.96 mmol) and stirred at 50°C for 3 hours. After cooling the reaction mixture to room temperature, ethyl acetate was added, and the mixture was washed with water and saturated aqueous sodium chloride solution. The obtained organic layer was dried over anhydrous sodium sulfate, the solvent was distilled off under reduced pressure, and the obtained residue was crudely purified by silica gel chromatography (chloroform:methanol=1:0-19:1) to obtain a thiourea compound (758 mg). The obtained thiourea compound (758 mg, 1.78 mmol) was dissolved in DMF (10 mL), EDCl-HCl (513 mg, 68 mmol) was added and stirred at 60°C for 1.5 hours. After cooling the reaction mixture to room temperature, ethyl acetate was added, and the mixture was washed with water and saturated brine in that order. The obtained organic layer was dried over anhydrous sodium sulfate, the solvent was distilled off under reduced pressure, and the obtained residue was subjected to silica gel chromatography (hexane:ethyl acetate=1:0-1:1) and amine-modified silica gel chromatography (hexane: ethyl acetate = 1:0-0:1, and further purified with chloroform:methanol = 1:0-49:1). The obtained crude product was suspended and washed with diethyl ether to obtain the title compound (295 mg).

¹H NMR (400 MHz, DMSO-d6) δ 11. 09 (s, 1H), 8.65 (s, 1H), 7.84 (d, J = 2.4 Hz, 1H), 7.74 (d, J = 2. 1 Hz, 1H), 7. 52 - 7.50 (m, 1H), 7.48 - 7 .43 (m, 1H), 7.41 (dd, J = 8.6, 0.6 Hz, 1H), 7.34 - 7.30 (m, 1H), 7. 1 1 (dd, J = 8.7, 2.1 Hz, 1H), 3.30 - 3.26 (m, 1H), 1.37 - 1. 27 (m, 2H) , 1.09 - 1.00 (m, 2H); LCMS(m/z) 391.1 [M+H]⁺.

### Embodiment 58

### Preparation of N-(5-chloro-1H-pyrrolo[3,2-b]pyridine-3-yl)-6-(4-fluorophenoxy)-1-methyl-1H-benzo[d]imidazole-2-amine

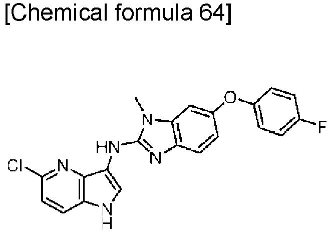

### (First step)

(5-chloro-1H-pyrrolo[3,2-b]pyridine-3-yl)carbamate tert-butyl (4.5 g, 16.81 mmol) was added with 4N hydrochloric acid/ethyl acetate solution (60 mL), and the mixture was stirred at room temperature for 2 hours. The reaction suspension is filtered and the solid is dried to give 5-chloro-1H-pyrrolo[3,2-b]pyridine-3-amine hydrochloride (3.4 g) was obtained.

¹H NMR (400 MHz, DMSO-d6) δ 11.90 (s, 1H), 10.26 (s, 3H), 7.97 (d, J = 8.6 Hz, 1H), 7.86 (d, J = 3.1 Hz, 1H), 7.31 (d, J = 8.6 Hz, 1H); LC MS(m/z) 168.0 [M+H]⁺.

### (Second step)

5-chloro-1H-pyrrolo[3,2-b]pyridine-3-amine hydrochloride (3.4 g, 16.66 mmol) in THF (100 mL) was added thiophosgene (2.11 g, 18.33 mmol) and DIPEA (4.31g, 33.3 mmol) was added, and the mixture was stirred at room temperature for 1.5 hours. Ethyl acetate was added to the residue obtained by distilling off the solvent under reduced pressure, and the mixture was washed with water and saturated brine in that order. After drying the obtained organic layer over anhydrous sodium sulfate, the solvent was distilled off under reduced pressure to obtain 5-chloro-3-isothiocyanato-1H-pyrrolo[3,2-b]pyridine (3.49 g).

¹H NMR (400 MHz, DMSO-d6) δ 12.01 (s, 1H), 8.06 (d, J = 3.2 Hz, 1H), 7.94 (d, J = 8.5 Hz, 1H), 7.31 (d, J = 8.6 Hz, 1H); LCMS(m/z) 210.0 [ M+H]⁺.

### (Third step)

5-fluoro-N-methyl-2-nitroaniline (1 g, 5. 88 mmol) in DMF (20 mL), 4-fluorophenol (988 mg, 8.82 mmol) and potassium carbonate (1.625g, 11.75 mmol) was added and stirred overnight at 95°C. After cooling the reaction mixture to room temperature, it was diluted with water. The precipitated solid was filtered, washed with water, and dried to obtain 5-(4-fluorophenoxy)-N-methyl-2-nitroaniline (954 mg).

¹H NMR (400 MHz, DMSO-d6) δ 8.32 - 8.26 (m, 1H), 8.10 (d, J = 9.4 Hz, 1H), 7.35 - 7.28 (m, 2H), 7.27 - 7.20 (m, 2H), 6.35 (d, J = 2.5 Hz, 1H), 6.20 (dd, J = 9.5, 2.6 Hz, 1H), 2.85 (d, J = 5.0 Hz, 3H); LCMS(m /z) 263.0 [M+H]⁺.

### (Fourth step)

5-(4-fluorophenoxy)-N-methyl-2-nitroaniline (954 mg, 3.64 mmol) was dissolved in a mixed solvent of ethanol (10 mL) and ethyl acetate (10 mL), 10% palladium on carbon (116 mg) was added, and the mixture was stirred at room temperature for 3 hours under a hydrogen atmosphere. Insoluble matter was filtered off, and the filtrate was concentrated to obtain 5-(4-fluorophenoxy)-N1-methylbenzene-1,2-diamine (845 mg).

¹H NMR (400 MHz, DMSO-d6) δ 7.17 - 7.06 (m, 2H), 6.93 - 6.84 (m, 2H), 6.55 - 6.48 (m, 1H), 6.12 - 6.04 (m, 2H), 4.83 (q, J = 5.0 Hz, 1H), 4.35 (s, 2H), 2.65 (d, J = 5.0 Hz, 3H); LCMS(m/z) 233.1 [M+H]⁺.

### (Fifth step)

5-chloro-3-isothiocyanato-1H-pyrrolo[3,2-b]pyridine (66.2 mg , 0.316 mmol) in DMF (4 mL) was added with 5-(4-fluorophenoxy)-N1-methylbenzene 1,2-diamine (110 mg, 0.474 mmol), EDCl-HCl (121 mg, 0.631 mmol), pyridine (250 mg, 3.16 mmol) and stirred at 100°C for 1 hour. After cooling the reaction mixture to room temperature, ethyl acetate was added, and the mixture was washed with water and saturated brine in that order. After drying the obtained organic layer with anhydrous sodium sulfate, the solvent was distilled off under reduced pressure. The obtained residue was crudely purified by amine-modified silica gel chromatography (chloroform:methanol=1:0-97:3) and silica gel chromatography (chloroform:methanol=1:0-97:3), followed by ion exchange column (SCX) to obtain the title compound (14 mg).

¹H NMR (400 MHz, DMSO-d6) δ 11.32 (s, 1H), 8.75 (s, 1H), 8.22 (s, 1H) , 7.87 (d, J = 8.5 Hz, 1H), 7.26 (d, J = 8.4 Hz, 1H), 7.21 - 7.13 (m, 3H), 7.11 - 7.05 (m, 1H), 7.00 - 6.93 (m, 2H), 6.78 - 6.69 (m, 1H), 3.71 (s, 3H); LCMS(m/z) 408.1 [M+H]⁺.

### Embodiments 59-288

The following example compounds [Table 3] were obtained by using the corresponding raw materials (commercially available products, or compounds obtained by derivatizing the commercially available compounds by a known method or a method similar thereto), according to the method described in the above examples. Depending on the circumstances, it was produced by appropriately combining methods commonly used in synthetic organic chemistry. In addition, the physicochemical data of each compound are shown in [Table 4].

**[Table 3]**

| Embodiments | Structural formula | Compound name |
|---|---|---|
| 59 | 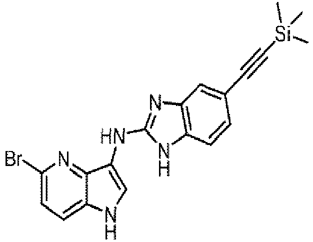 | N-(5-bromo-1H-pyrrolo[3,2-b]pyridine-3-yl)-5-[(trimethylsilyl)ethynyl]-1H-benzo[d]imidazole-2-amine |
| 60 | 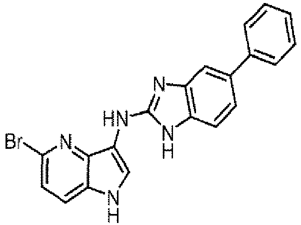 | N-(5-bromo-1H-pyrrolo[3,2-b]pyridine-3-yl)-5-phenyl-1H-benzo[d]imidazole-2-amine |
| 61 | 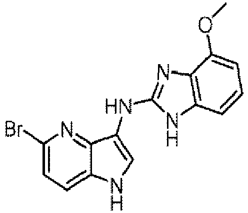 | N-(5-bromo-1H-pyrrolo[3,2-b]pyridine-3-yl)-4-methoxy-1H-benzo[d]imidazole-2-amine |
| 62 | 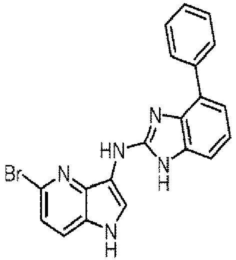 | N-(5-bromo-1H-pyrrolo[3,2-b]pyridine-3-yl)-4-phenyl-1H-benzo[d]imidazole-2-amine |
| 63 | 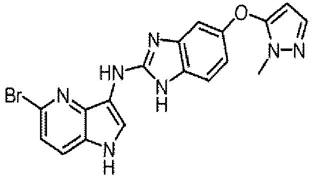 | N-(5-bromo-1H-pyrrolo[3,2-b]pyridine-3-yl)-5-[(1-methyl-1H-pyrazol-5-yl)oxy]-1H-benzo[d]imidazole-2-amine |
| 64 | 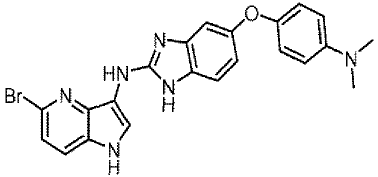 | N-(5-bromo-1H-pyrrolo[3,2-b]pyridine-3-yl)-5-[4-(dimethylamino)phenoxy]-1H-benzo[d]imidazole-2-amine |
| 65 | 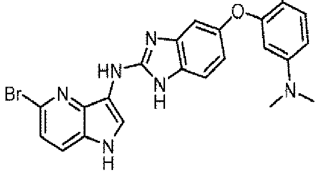 | N-(5-bromo-1H-pyrrolo[3,2-b]pyridine-3-yl)-5-[3-(dimethylamino)phenoxy]-1H-benzo[d]imidazole-2-amine |
| 66 | 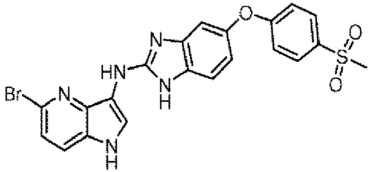 | N-(5-bromo-1H-pyrrolo[3,2-b]pyridine-3-yl)-5-[4-(methylsulfonyl)phenoxy]-1H-benzo[d]imidazole-2-amine |
| 67 | 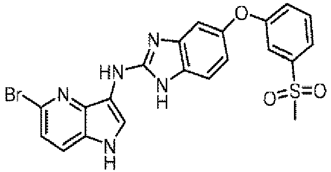 | N-(5-bromo-1H-pyrrolo[3,2-b]pyridine-3-yl)-5-[3-(methylsulfonyl)phenoxy]-1H-benzo[d]imidazole-2-amine |
| 68 | 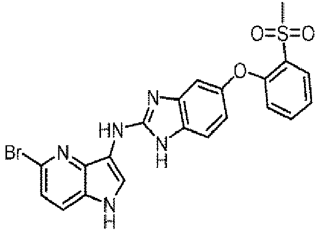 | N-(5-bromo-1H-pyrrolo[3,2-b]pyridine-3-yl)-5-[2-(methylsulfonyl)phenoxy]-1H-benzo[d]imidazole-2-amine |
| 69 | 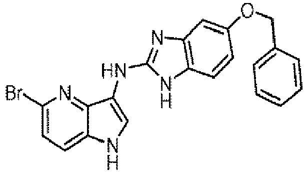 | 5-(benzyloxy)-N-(5-bromo-1H-pyrrolo[3,2-b]pyridine-3-yl)-1H-benzo[d]imidazole-2-amine |
| 70 | 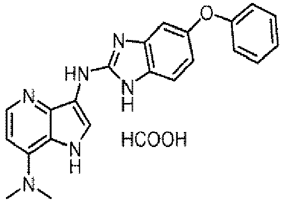 | N7,N7-dimethyl-N3-(5-phenoxy-1H-benzo[d]imidazole-2-yl)-1H-pyrrolo[3,2-b]pyridine-3,7-diamine formate |
| 71 | 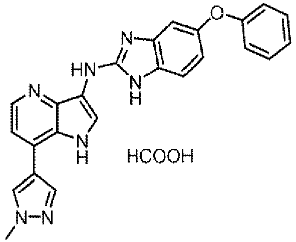 | N-[7-(1-methyl-1H-pyrazol-4-yl)-1H-pyrrolo[3,2-b]pyridine-3-yl]-5-phenoxy-1H-benzo[d]imidazole - 2-amine formate |
| 72 | 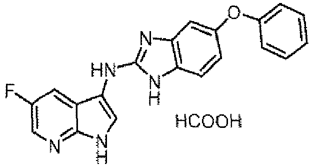 | N-(5-fluoro-1H-pyrrolo[2,3-b]pyridine-3-yl)-5-phenoxy-1H-benzo[d]imidazole-2-amine formate |
| 73 | 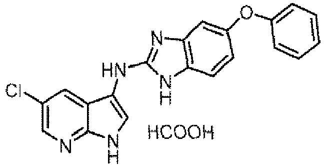 | N-(5-Chloro-1H-pyrrolo[2,3-b]pyridine-3-yl)-5-phenoxy-1H-benzo[d]imidazole-2-amine formate |
| 74 | 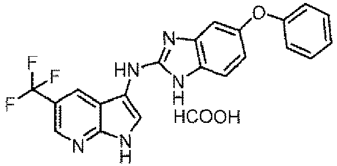 | 5-phenoxy-N-[5-(trifluoromethyl)-1H-pyrrolo[2,3-b]pyridine-3-yl]-1H-benzo[d]imidazole-2-amine formate |
| 75 | 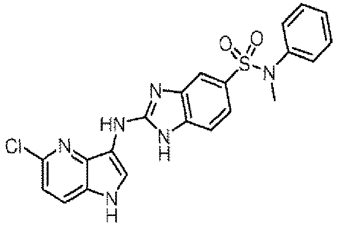 | 2-[(5-Chloro-1H-pyrrolo[3,2-b]pyridine-3-yl)amino]-N-methyl-N-phenyl-1H-benzo[d]imidazole-5-sulfonamide |
| 76 | 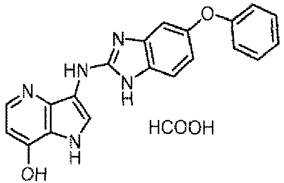 | 3-[(5-phenoxy-1H-benzo[d]imidazole-2-yl)amino]-1H-pyrrolo[3,2-b]pyridin-7-ol formate |
| 77 | 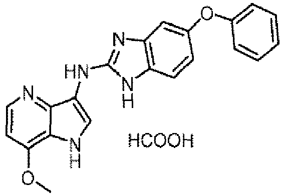 | N-(7-Methoxy-1H-pyrrolo[3,2-b]pyridine-3-yl)-5-phenoxy-1H-benzo[d]imidazole-2-amine formate |
| 78 | 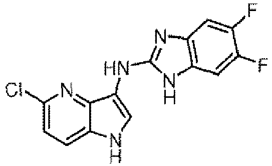 | N-(5-Chloro-1H-pyrrolo[3,2-b]pyridine-3-yl)-5,6-difluoro-1H-benzo[d]imidazole-2-amine |
| 79 | 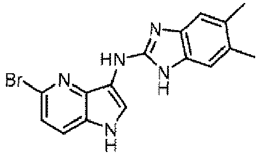 | N-(5-bromo-1H-pyrrolo[3,2-b]pyridine-3-yl)-5,6-dimethyl-1H-benzo[d]imidazole-2-amine |
| 80 | 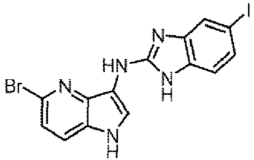 | N-(5-bromo-1H-pyrrolo[3,2-b]pyridine-3-yl)-5-iodo-1H-benzo[d]imidazole-2-amine |
| 81 | 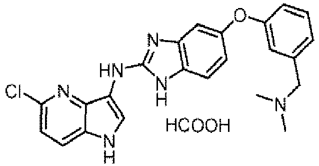 | N-(5-chloro-1H-pyrrolo[3,2-b]pyridine-3-yl)-5-{3-[(dimethylamino)methyl]phenoxy }-1H-benzo[d]imidazole- 2-amine formate |
| 82 | 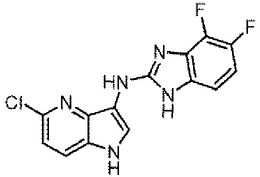 | N-(5-Chloro-1H-pyrrolo[3,2-b]pyridine-3-yl)-4,5-difluoro-1H-benzo[d]imidazole-2-amine |
| 83 | 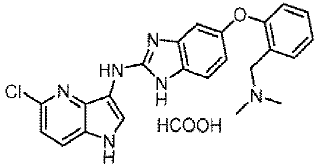 | N-(5-chloro-1H-pyrrolo[3,2-b]pyridine-3-yl)-5-{2-[(dimethylamino)methyl]phenoxy }-1H-benzo[d]imidazole- 2-amine formate |
| 84 | 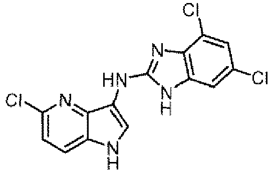 | 4,6-dichloro-N-(5-chloro-1H-pyrrolo[3,2-b]pyridine-3-yl)-1H-benzo[d]imidazole-2-amine |
| 85 | 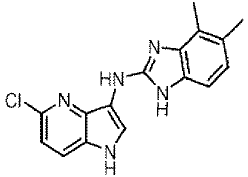 | N-(5-Chloro-1H-pyrrolo[3,2-b]pyridine-3-yl)-4,5-dimethyl-1H-benzo[d]imidazole-2-amine |
| 86 | 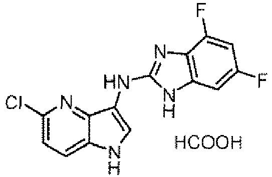 | N-(5-Chloro-1H-pyrrolo[3,2-b]pyridine-3-yl)-4,6-difluoro-1H-benzo[d]imidazole-2-amine formate |
| 87 | 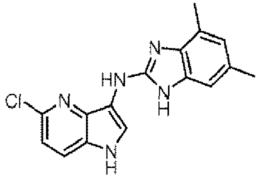 | N-(5-Chloro-1H-pyrrolo[3,2-b]pyridine-3-yl)-4,6-dimethyl-1H-benzo[d]imidazole-2-amine |
| 88 | 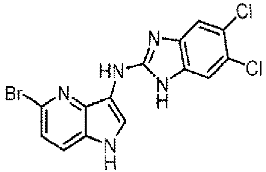 | N-(5-bromo-1H-pyrrolo[3,2-b]pyridine-3-yl)-5,6-dichloro-1H-benzo[d]imidazole-2-amine |
| 89 | 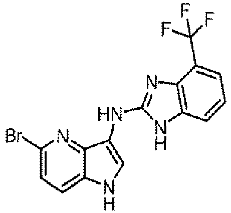 | N-(5-bromo1H-pyrrolo[3,2-b]pyridine-3-yl)-4-(trifluoromethyl)-1H-benzo[d]imidazole-2-amine |
| 90 | 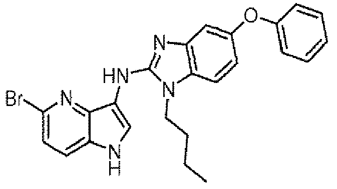 | N-(5-bromo-1H-pyrrolo[3,2-b]pyridine-3-yl)-1-butyl-5-phenoxy-1H-benzo[d]imidazole-2-amine |
| 91 | 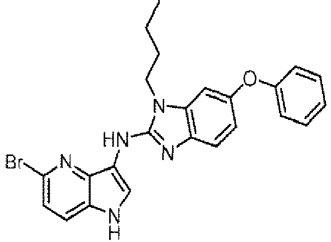 | N-(5-bromo-1H-pyrrolo[3,2-b]pyridine-3-yl)-1-butyl-6-phenoxy-1H-benzo[d]imidazole-2-amine |
| 92 | 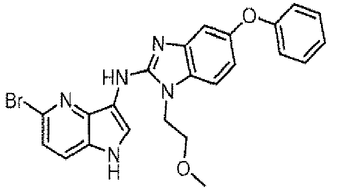 | N-(5-bromo-1H-pyrrolo[3,2-b]pyridine-3-yl)-1-(2-methoxyethyl)-5-phenoxy-1H-benzo[d]imidazole-2-amine |
| 93 | 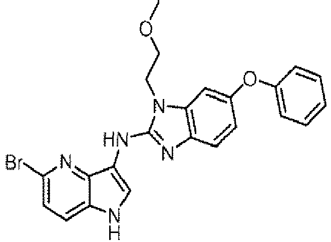 | N-(5-bromo-1H-pyrrolo[3,2-b]pyridine-3-yl)-1-(2-methoxyethyl)-6-phenoxy-1H-benzo[d]imidazole-2-amine |
| 94 | 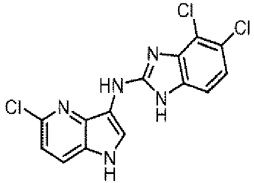 | 4,5-dichloro-N-(5-chloro-1H-pyrrolo[3,2-b]pyridine-3-yl)-1H-benzo[d]imidazole-2-amine |
| 95 | 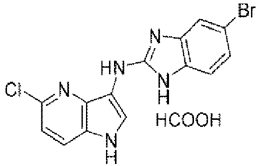 | 5-bromo-N-(5-chloro-1H-pyrrolo[3,2-b]pyridine-3-yl)-1H-benzo[d]imidazole-2-amine formate |
| 96 | 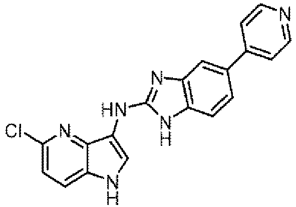 | N-(5-Chloro-1H-pyrrolo[3,2-b]pyridine-3-yl)-5-(pyridine-4-yl)-1H-benzo[d]imidazole-2-amine |
| 97 | 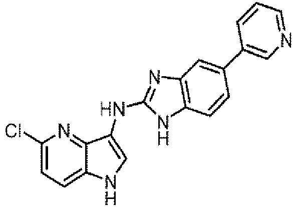 | N-(5-Chloro-1H-pyrrolo[3,2-b]pyridine-3-yl)-5-(pyridine-3-yl)-1H-benzo[d]imidazole-2-amine |
| 98 | 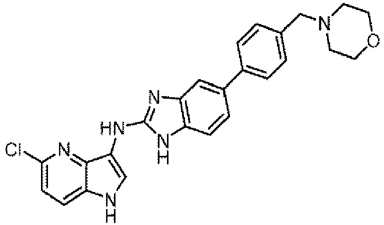 | N-(5-Chloro-1H-pyrrolo[3,2-b]pyridine-3-yl)-5-[4-(morpholinomethyl)phenyl]-1H-benzo[d]imidazole-2-amine |
| 99 | 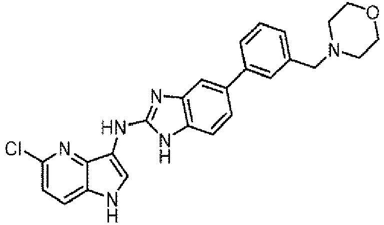 | N-(5-Chloro-1H-pyrrolo[3,2-b]pyridine-3-yl)-5-[3-(morpholinomethyl)phenyl]-1H-benzo[d]imidazole-2-amine |
| 100 | 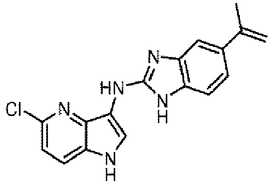 | N-(5-chloro-1H-pyrrolo[3,2-b]pyridine-3-yl)-5-(prop-1-en-2-yl)-1H-benzo[d]imidazole-2-amine |
| 101 | 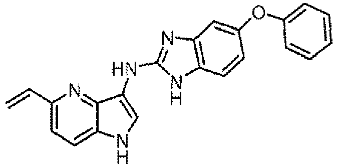 | 5-phenoxy-N-(5-vinyl-1H-pyrrolo[3,2-b]pyridine-3-yl)-1H-benzo[d]imidazole-2-amine |
| 102 | 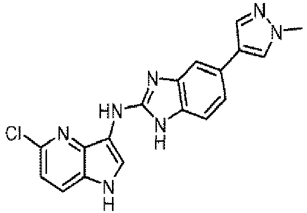 | N-(5-chloro-1H-pyrrolo[3,2-b]pyridine-3-yl)-5-(1-methyl-1H-pyrazol-4-yl)-1H-benzo[d]imidazole-2-amine |
| 103 | 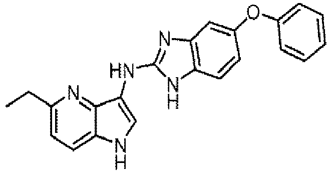 | N-(5-ethyl-1H-pyrrolo[3,2-b]pyridine-3-yl)-5-phenoxy-1H-benzo[d]imidazole-2-amine |
| 104 | 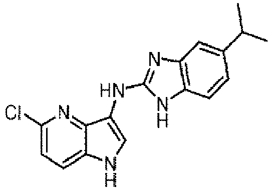 | N-(5-Chloro-1H-pyrrolo[3,2-b]pyridine-3-yl)-5-isopropyl-1H-benzo[d]imidazole-2-amine |
| 105 | 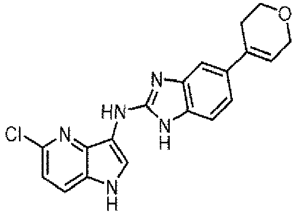 | N-(5-chloro-1H-pyrrolo[3,2-b]pyridine-3-yl)-5-(3,6-dihydro-2H-pyran-4-yl)-1H-benzo[d] imidazole-2-amine |
| 106 | 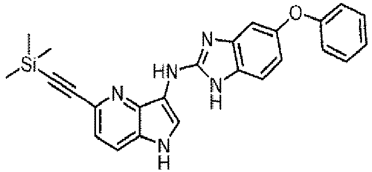 | 5-phenoxy-N-{5-[(trimethylsilyl)ethynyl]-1H-pyrrolo[3,2-b]pyridine-3-yl}-1H-benzo[d]imidazole-2-amine |
| 107 | 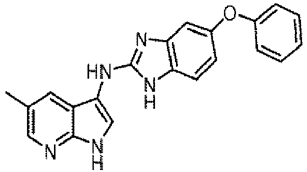 | N-(5-methyl-1H-pyrrolo[2,3-b]pyridine-3-yl)-5-phenoxy-1H-benzo[d]imidazole-2-amine |
| 108 | 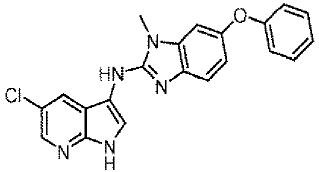 | N-(5-Chloro-1H-pyrrolo[2,3-b]pyridine-3-yl)-1-methyl-6-phenoxy-1H-benzo[d]imidazole-2-amine |
| 109 | 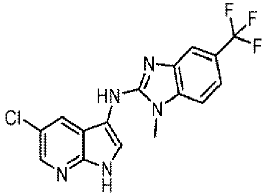 | N-(5-Chloro-1H-pyrrolo[2,3-b]pyridine-3-yl)-1-methyl-5-(trifluoromethyl)-1H-benzo[d]imidazole-2-amine |
| 110 | 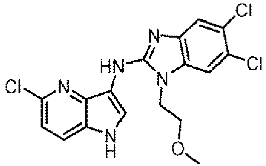 | 5,6-dichloro-N-(5-chloro-1H-pyrrolo[3,2-b]pyridine-3-yl)-1-(2-methoxyethyl)-1H-benzo[d]imidazole-2-amine |
| 111 | 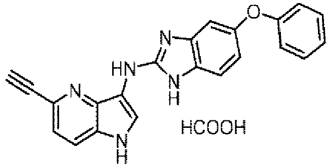 | N-(5-ethynyl-1H-pyrrolo[3,2-b]pyridine-3-yl)-5-phenoxy-1H-benzo[d]imidazole-2-amine formate |
| 112 | 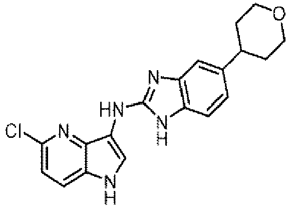 | N-(5-chloro-1H-pyrrolo[3,2-b]pyridine-3-yl)-5-(tetrahydro-2H-pyran-4-yl)-1H-benzo[d]imidazole-2-amine |
| 113 | 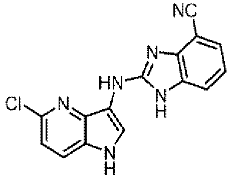 | 2-[(5-Chloro-1H-pyrrolo[3,2-b]pyridine-3-yl)amino]-1H-benzo[d]imidazole-4-carbonitrile |
| 114 | 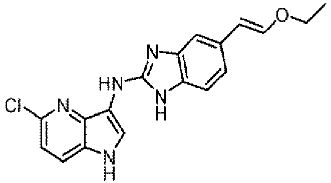 | (E)-N-(5-chloro-1H-pyrrolo[3,2-b]pyridine-3-yl)-5-(2-ethoxyvinyl)-1H-benzo[d]imidazole-2-amine |
| 115 | 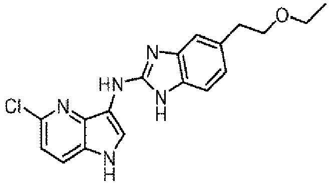 | N-(5-Chloro-1H-pyrrolo[3,2-b]pyridine-3-yl)-5-(2-ethoxyvinyl)-1H-benzo[d]imidazole-2-amine |
| 116 | 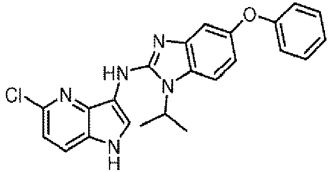 | N-(5-Chloro-1H-pyrrolo[3,2-b]pyridine-3-yl)-1-isopropyl-5-phenoxy-1H-benzo[d]imidazole-2-amine |
| 117 | 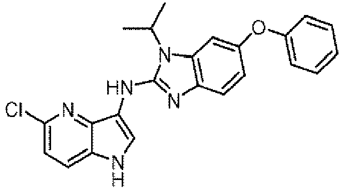 | N-(5-Chloro-1H-pyrrolo[3,2-b]pyridine-3-yl)-1-isopropyl-6-phenoxy-1H-benzo[d]imidazole-2-amine |
| 118 | 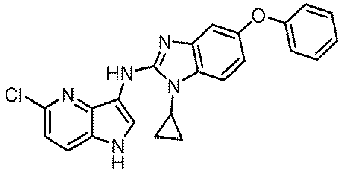 | N-(5-Chloro-1H-pyrrolo[3,2-b]pyridine-3-yl)-1-cyclopropyl-5-phenoxy-1H-benzo[d]imidazole-2-amine |
| 119 | 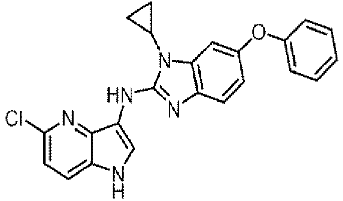 | N-(5-Chloro-1H-pyrrolo[3,2-b]pyridine-3-yl)-1-cyclopropyl-6-phenoxy-1H-benzo[d]imidazole-2-amine |
| 120 | 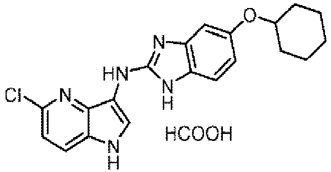 | N-(5-Chloro-1H-pyrrolo[3,2-b]pyridine-3-yl)-5-(cyclohexyloxy)-1H-benzo[d]imidazole-2-amine formate |
| 121 | 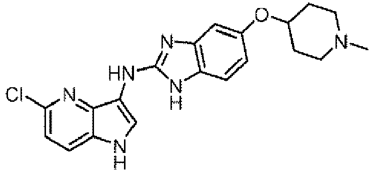 | N-(5-Chloro-1H-pyrrolo[3,2-b]pyridine-3-yl)-5-[(1-methylpiveridin-4-yl)oxy]-1H-benzo[d]imidazole- 2-amine |
| 122 | 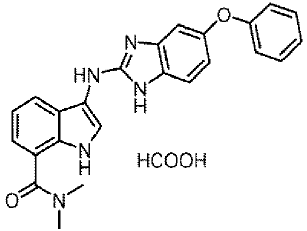 | N,N-dimethyl-3-[(5-phenoxy-1H-benzo[d]imidazole-2-yl)amino]-1H-indole-7-carboxamide formate |
| 123 | 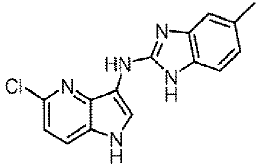 | N-(5-chloro-1H-pyrrolo[3,2-b]pyridine-3-yl)-5-methyl-1H-benzo[d]imidazole-2-amine |
| 124 | 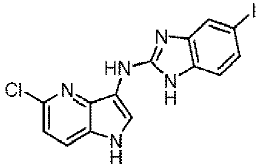 | N-(5-Chloro-1H-pyrrolo[3,2-b]pyridine-3-yl)-5-iodo-1H-benzo[d]imidazole-2-amine |
| 125 | 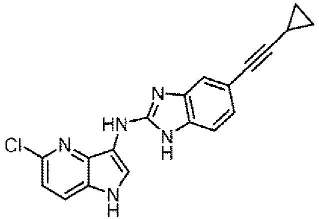 | N-(5-Chloro-1H-pyrrolo[3,2-b]pyridine-3-yl)-5-(cyclopropylethynyl)-1H-benzo[d]imidazole-2-amine |
| 126 | 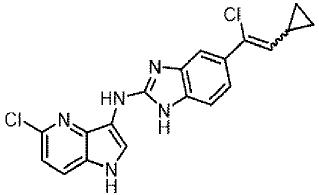 | N-(5-chloro-1H-pyrrolo[3,2-b]pyridine-3-yl)-5-(1-chloro-2-cyclopropylvinyl)-1H-benzo[d]imidazole-2-amine |
| 127 | 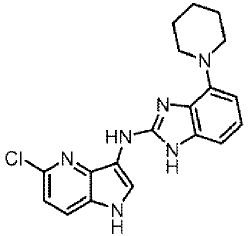 | N-(5-Chloro-1H-pyrrolo[3,2-b]pyridine-3-yl)-4-(piperidine-1-yl)-1H-benzo[d]imidazole-2-amine |
| 128 | 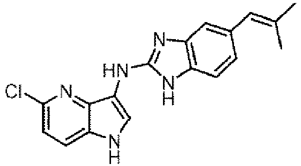 | N-(5-chloro-1H-pyrrolo[3,2-b]pyridine-3-yl)-5-(2-methylprop-1-en-1-yl)-1H-benzo[d]imidazole-2-amine |
| 129 | 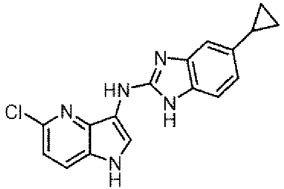 | N-(5-Chloro-1H-pyrrolo[3,2-b]pyridine-3-yl)-5-cyclopropyl-1H-benzo[d]imidazole-2-amine |
| 130 | 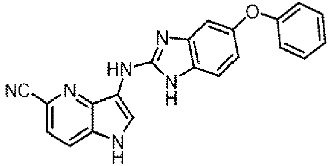 | 3-[(5-phenoxy-1H-benzo[d]imidazole-2-yl)amino]-1H-pyrrolo[3,2-b]pyridine-5-carbonitrile |
| 131 | 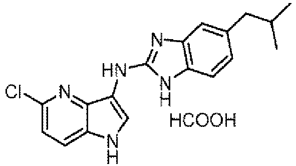 | N-(5-Chloro-1H-pyrrolo[3,2-b]pyhdine-3-yl)-5-isobutyl-1H-benzo[d]imidazole-2-amine formate |
| 132 | 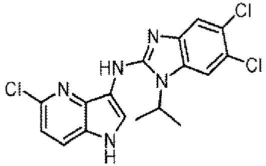 | 5,6-dichloro-N-(5-chloro-1H-pyrrolo[3,2-b]pyridine-3-yl)-1-isopropyl-1H-benzo[d]imidazole-2-amine |
| 133 | 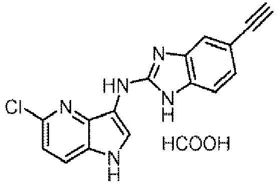 | N-(5-Chloro-1H-pyrrolo[3,2-b]pyridine-3-yl)-5-ethynyl-1H-benzo[d]imidazole-2-amine formate |
| 134 | 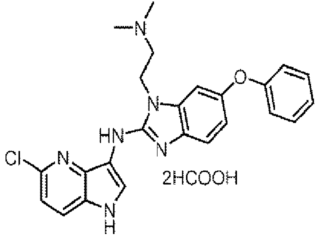 | N-(5-Chloro-1H-pyrrolo[3,2-b]pyridine-3-yl)-1-[2-(dimethylamino)ethyl]-6-phenoxy-1H-benzo[d]imidazole -2-amine diformate |
| 135 | 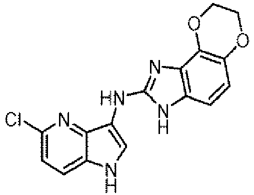 | N-(5-chloro-1H-pyrrolo[3,2-b]pyridine-3-yl)-7,8-dihydro-3H-[1,4]dioxino[2',3':3, 4]benzo[1,2-d] imidazole-2-amine |
| 136 | 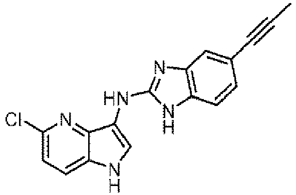 | N-(5-Chloro-1H-pyrrolo[3,2-b]pyridine-3-yl)-5-(prop-1-yn-1-yl)-1H-benzo[d]imidazole-2-amine |
| 137 | 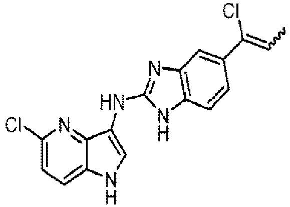 | N-(5-Chloro-1H-pyrrolo[3,2-b]pyridine-3-yl)-5-(1-chloroprop-1-en-1-yl)-1H-benzo[d]imidazole-2-amine |
| 138 | 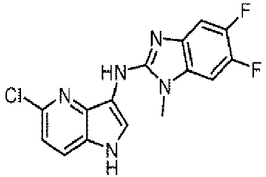 | N-(5-Chloro-1H-pyrrolo[3,2-b]pyridine-3-yl)-5,6-difluoro-1-methyl-1H-benzo[d]imidazole-2-amine |
| 139 | 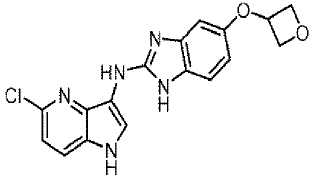 | N-(5-Chloro-1H-pyrrolo[3,2-b]pyridine-3-yl)-5-(oxetan-3-yloxy)-1H-benzo[d]imidazole-2-amine |
| 140 | 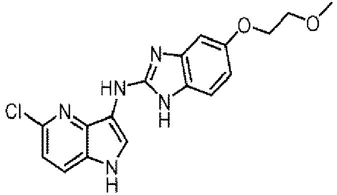 | N-(5-Chloro-1H-pyrrolo[3,2-b]pyridine-3-yl)-5-(2-methoxyethoxy)-1H-benzo[d]imidazole-2-amine |
| 141 | 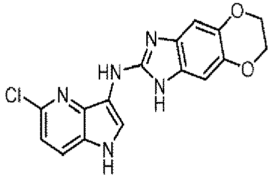 | N-(5-chloro-1H-pyrrolo[3,2-b]pyridine-3-yl)-6,7-dihydro-1H-[1,4]dioxino[2',3':4,5]benzo[1,2-d] imidazole-2-amine |
| 142 | 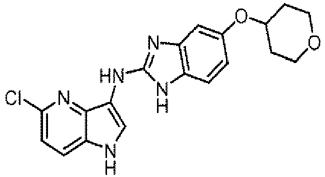 | N-(5-Chloro-1H-pyrrolo[3,2-b]pyridine-3-yl)-5-[(tetrahydro-2H-pyran-4-yl)oxy)-1H-benzo[d] imidazole-2-amine |
| 143 | 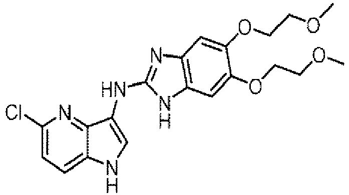 | N-(5-Chloro-1H-pyrrolo[3,2-b]pyridine-3-yl)-5,6-bis(2-methoxyethoxy)-1H-benzo[d]imidazole-2-amine |
| 144 | 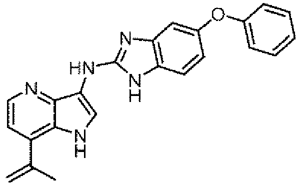 | 5-phenoxy-N-[7-(prop-1-en-2-yl)-1H-pyrrolo[3,2-b]pyridine-3-yl]-1H-benzo[d]imidazole-2-amine |
| 145 | 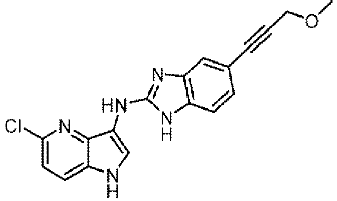 | N-(5-Chloro-1H-pyrrolo[3,2-b]pyridine-3-yl)-5(3-methoxyprop-1-yn-1-yl)-1H-benzo[d]imidazole-2-amine |
| 146 | 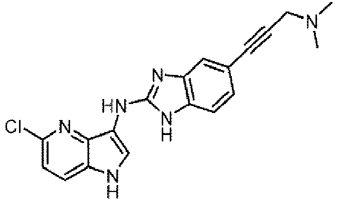 | N-(5-Chloro-1H-pyrrolo[3,2-b]pyridine-3-yl)-5-[3-(dimethylamino)prop-1-yn-1-yl]-1H-benzo[d]imidazole-2-amine |
| 147 | 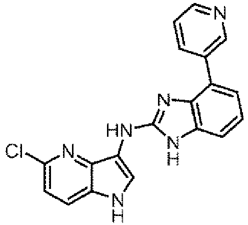 | N-(5-Chloro-1H-pyrrolo[3,2-b]pyridine-3-yl)-4-(pyridine-3-yl)-1H-benzo[d]imidazole-2-amine |
| 148 | 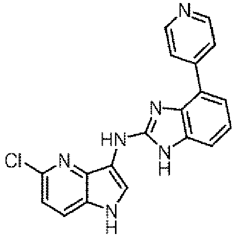 | N-(5-Chloro-1H-pyrrolo[3,2-b]pyridine-3-yl)-4-(pyridine-4-yl)-1H-benzo[d]imidazole-2-amine |
| 149 | 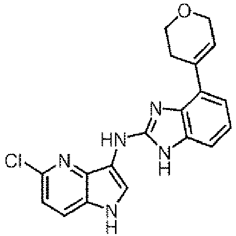 | N-(5-chloro-1H-pyrrolo[3,2-b]pyridine-3-yl)-4-(3,6-dihydro-2H-pyran-4-yl)-1H-benzo[d] imidazole-2-amine |
| 150 | 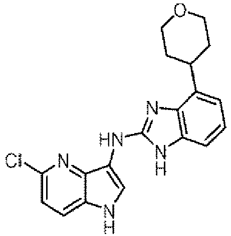 | N-(5-chloro-1H-pyrrolo[3,2-b]pyridine-3-yl)-4-(tetrahydro-2H-pyran-4-yl)-1H-benzo[d]imidazole-2-amine |
| 151 | 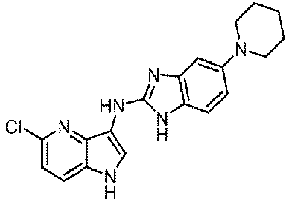 | N-(5-Chloro-1H-pyrrolo[3,2-b]pyridine-3-yl)-5-(piperidine-1-yl)-1H-benzo[d]imidazole-2-amine |
| 152 | 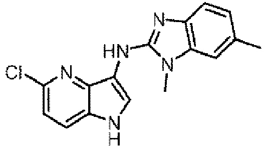 | N-(5-Chloro-1H-pyrrolo[3,2-b]pyridine-3-yl)-1,6-dimethyl-1H-benzo[d]imidazole-2-amine |
| 153 | 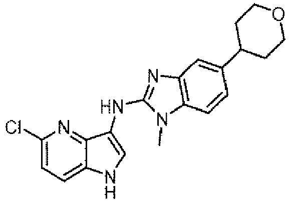 | N-(5-chloro-1H-pyrrolo[3,2-b]pyridine-3-yl)-1-methyl-5-(tetrahydro-2H-pyran-4-yl)-1H-benzo[d] imidazole-2-amine |
| 154 | 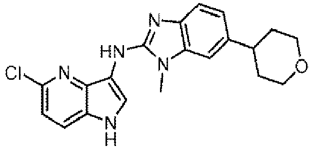 | N-(5-chloro-1H-pyrrolo[3,2-b]pyridine-3-yl)-1-methyl-6-(tetrahydro-2H-pyran-4-yl)-1H-benzo[d] imidazole-2-amine |
| 155 | 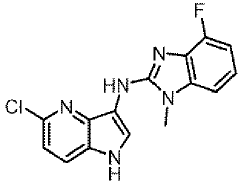 | N-(5-Chloro-1H-pyrrolo[3,2-b]pyridine-3-yl)-4-fluoro-1-methyl-1H-benzo[d]imidazole-2-amine |
| 156 | 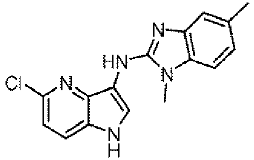 | N-(5-Chloro-1H-pyrrolo[3,2-b]pyridine-3-yl)-1,5-dimethyl-1H-benzo[d]imidazole-2-amine |
| 157 | 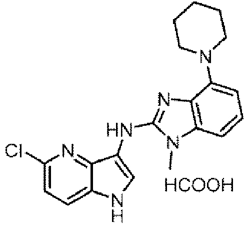 | N-(5-chloro-1H-pyrrolo[3,2-b]pyridine-3-yl)-1-methyl-4-(piperidine-1-yl)-1H-benzo[d]imidazole-2-amine formate |
| 158 | 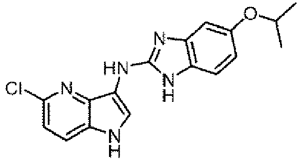 | N-(5-Chloro-1H-pyrrolo[3,2-b]pyridine-3-yl)-5-isopropoxy-1H-benzo[d]imidazole-2-amine |
| 159 | 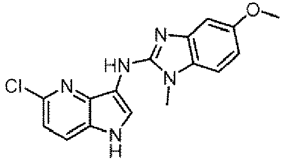 | N-(5-Chloro-1H-pyrrolo[3,2-b]pyridine-3-yl)-5-methoxy-1-methyl-1H-benzo[d]imidazole-2-amine |
| 160 | 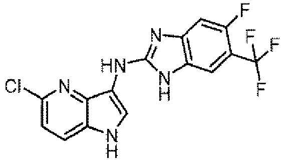 | N-(5-Chloro-1H-pyrrolo[3,2-b]pyridine-3-yl)-5-fluoro-6-(trifluoromethyl)-1H-benzo[d]imidazole-2-amine |
| 161 | 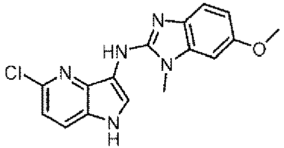 | N-(5-Chloro-1H-pyrrolo[3,2-b]pyridine-3-yl)-6-methoxy-1-methyl-1H-benzo[d]imidazole-2-amine |
| 162 | 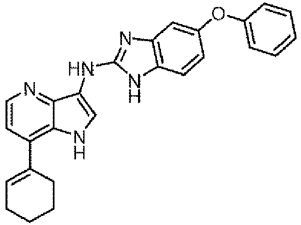 | N-[7-(Cyclohex-1-en-1-yl)-1H-pyrrolo[3,2-b]pyridine-3-yl]-5-phenoxy-1H-benzo[d]imidazole-2-amine |
| 163 | 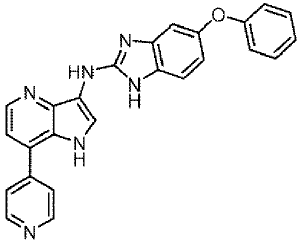 | 5-phenoxy-N-[7-(pyridine-4-yl)-1H-pyrrolo[3,2-b]pyridine-3-yl]-1H-benzo[d]imidazole-2-amine |
| 164 | 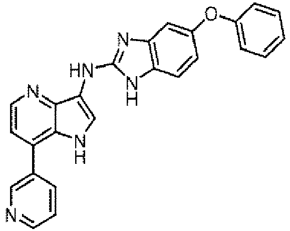 | 5-phenoxy-N-[7-(pyridine-3-yl)-1H-pyrrolo[3,2-b]pyridine-3-yl]-1H-benzo[d]imidazole-2-amine |
| 165 | 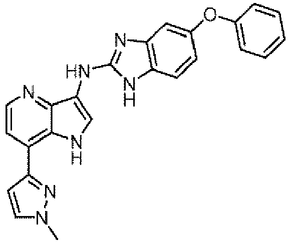 | N-[7-(1-methyl-1H-pyrazol-3-yl)-1H-pyrrolo[3,2-b]pyridine-3-yl]-5-phenoxy-1H-benzo[d]imidazole -2-amine |
| 166 | 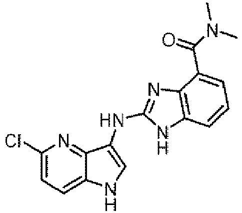 | 2-[(5-Chloro-1H-pyrrolo[3,2-b]pyridine-3-yl)amino]-N,N-dimethyl-1H-benzo[d]imidazole-4-carboxamide |
| 167 | 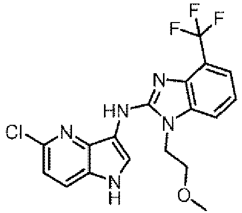 | N-(5-Chloro-1H-pyrrolo[3,2-b]pyridine-3-yl)-1-(2-methoxyethyl)-4-(trifluoromethyl)-1H-benzo[d]imidazole-2-amine |
| 168 | 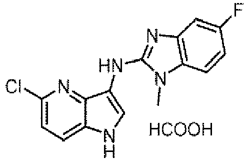 | N-(5-Chloro-1H-pyrrolo[3,2-b]pyridine-3-yl)-5-fluoro-1-methyl-1H-benzo[d]imidazole-2-amine formate |
| 169 | 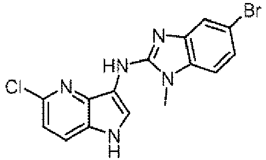 | 5-bromo-N-(5-chloro-1H-pyrrolo[3,2-b]pyridine-3-yl)-1-methyl-1H-benzo[d]imidazole-2-amine |
| 170 | 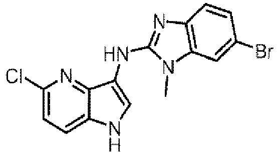 | 6-bromo-N-(5-chloro-1H-pyrrolo[3,2-b]pyridine-3-yl)-1-methyl-1H-benzo[d]imidazole-2-amine |
| 171 | 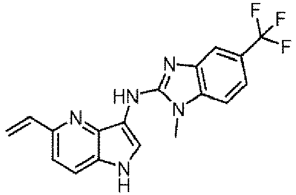 | 1-methyl-5-(trifluoromethyl)-N-(5-vinyl-1H-pyrrolo[3,2-b]pyridine-3-yl)-1H-benzo[d]imidazole-2-amine |
| 172 | 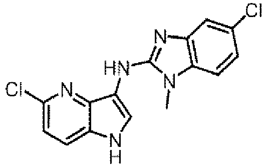 | 5-Chloro-N-(5-chloro-1H-pyrrolo[3,2-b]pyridine-3-yl)-1-methyl-1H-benzo[d]imidazole-2-amine |
| 173 | 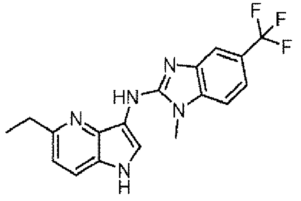 | N-(5-ethyl-1H-pyrrolo[3,2-b]pyridine-3-yl)-1-methyl-5-(trifluoromethyl)-1H-benzo[d]imidazole-2-amine |
| 174 | 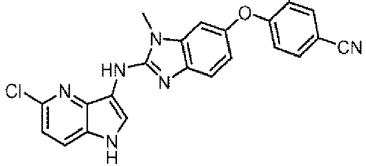 | 4-({2-[(5-chloro-1H-pyrrolo[3,2-b]pyridine-3-yl)amino]-1-methyl-1H-benzo[d]imidazole-6-yl} Oxy)benzonitrile |
| 175 | 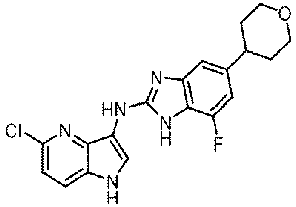 | N-(5-Chloro-1H-pyrrolo[3,2-b]pyridine-3-yl)-7-fluoro-5-(tetrahydro-2H-pyran-4-yl)-1H-benzo[d]imidazole-2-amine |
| 176 | 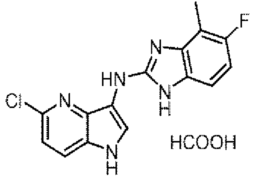 | N-(5-Chloro-1H-pyrrolo[3,2-b]pyridine-3-yl)-5-fluoro-4-methyl-1H-benzo[d]imidazole-2-amine formate |
| 177 | 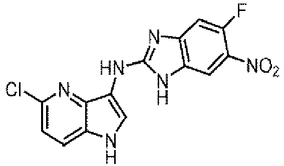 | N-(5-Chloro-1H-pyrrolo[3,2-b]pyridine-3-yl)-5-fluoro-6-nitro-1H-benzo[d]imidazole-2-amine |
| 178 | 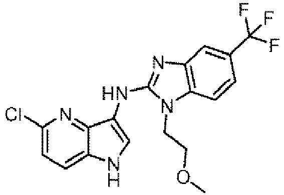 | N-(5-chloro-1H-pyrrolo[3,2-b]pyridine-3-yl)-1-(2-methoxyethyl)-5-(trifluoromethyl)-1H-benzo[d]imidazole-2-amine |
| 179 | 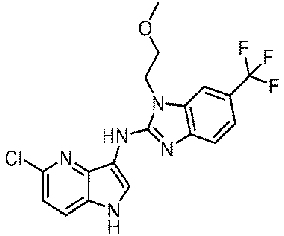 | N-(5-chloro-1H-pyrrolo[3,2-b]pyridine-3-yl)-1-(2-methoxyethyl)-6-(trifluoromethyl)-1H-benzo[d]imidazole-2-amine |
| 180 | 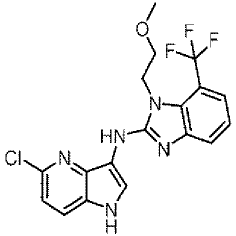 | N-(5-chloro-1H-pyrrolo[3,2-b]pyridine-3-yl)-1-(2-methoxyethyl)-7-(trifluoromethyl)-1H-benzo[d]imidazole-2-amine |
| 181 | 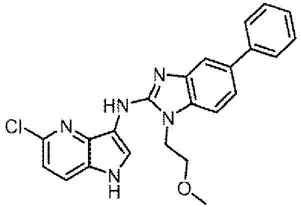 | N-(5-chloro-1H-pyrrolo[3,2-b]pyridine-3-yl)-1-(2-methoxyethyl)-5-phenyl)-1H-benzo[d]imidazole-2-amine |
| 182 | 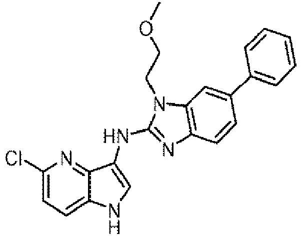 | N-(5-Chloro-1H-pyrrolo[3,2-b]pyridine-3-yl)-1-(2-methoxyethyl)-6-phenyl)-1H-benzo[d]imidazole-2-amine |
| 183 | 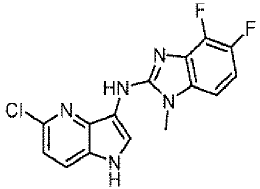 | N-(5-Chloro-1H-pyrrolo[3,2-b]pyridine-3-yl)-4,5-difluoro-1-methyl-1H-benzo[d]imidazole-2-amine |
| 184 | 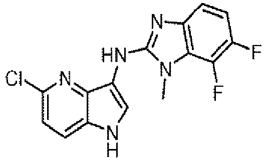 | N-(5-Chloro-1H-pyrrolo[3,2-b]pyridine-3-yl)-6,7-difluoro-1-methyl-1H-benzo[d]imidazole-2-amine |
| 185 | 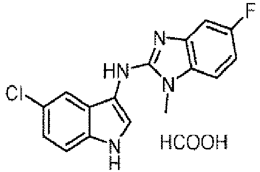 | N-(5-Chloro-1H-indol-3-yl)-5-fluoro-1-methyl-1H-benzo[d]imidazole-2-amine formate |
| 186 | 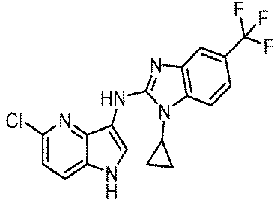 | N-(5-Chloro-1H-pyrrolo[3,2-b]pyridine-3-yl)-1-cyclopropyl-5-(trifluoromethyl)-1H-benzo[d]imidazole-2-amine |
| 187 | 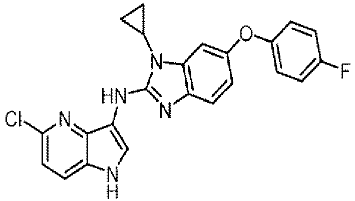 | N-(5-chloro-1H-pyrrolo[3,2-b]pyridine-3-yl)-1-cyclopropyl-6-(4-fluorophenoxy)-1H-benzo[d]imidazole-2-amine |
| 188 | 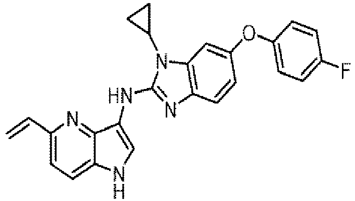 | 1-Cyclopropyl-6-(4-fluorophenoxy)-N-(5-vinyl-1H-pyrrolo[3,2-b]pyridine-3-yl)-1H-benzo[d]imidazole-2-amine |
| 189 | 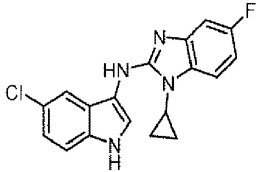 | N-(5-Chloro-1H-indol-3-yl)-1-cyclopropyl-5-fluoro-1H-benzo[d]imidazole-2-amine |
| 190 | 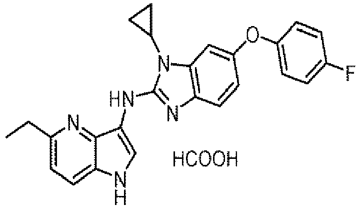 | 1-Cyclopropyl-N-(5-ethyl-1H-pyrrolo[3,2-b]pyridine-3-yl)-6-(4-fluorophenoxy)-1H-benzo[d]imidazole-2-amine formate |
| 191 | 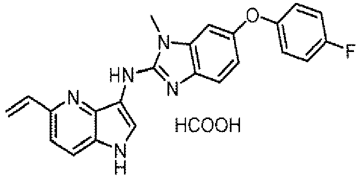 | 6-(4-fluorobuenoxy)-1-methyl-N-(5-vinyl-1H-pyrrolo[3,2-b]pyridine-3-yl)-1H-benzo[d]imidazole-2-amine formate |
| 192 | 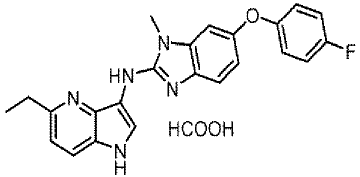 | N-(5-ethyl-1H-pyrrolo[3,2-b]pyridine-3-yl)-6-(4-fluorophenoxy)-1-methyl-1H-benzo[d]imidazole-2-amine formate |
| 193 | 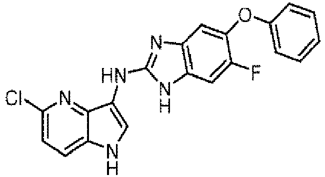 | N-(5-Chloro-1H-pyrrolo[3,2-b]pyridine-3-yl)-6-fluoro-5-phenoxy-1H-benzo[d]imidazole-2-amine |
| 194 | 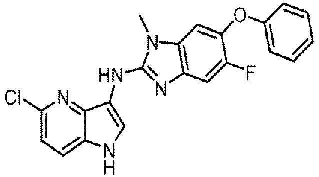 | N-(5-Chloro-1H-pyrrolo[3,2-b]pyridine-3-yl)-5-fluoro-1-methyl-6-phenoxy-1H-benzo[d]imidazole-2-amine |
| 195 | 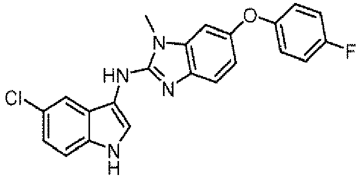 | N-(5-Chloro-1H-indol-3-yl)-6-(4-fluorophenoxy)-1-methyl-1H-benzo[d]imidazole-2-amine |
| 196 | 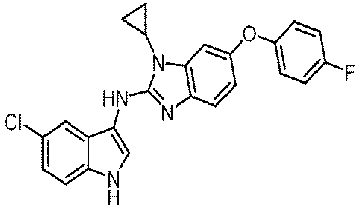 | N-(5-Chloro-1H-indol-3-yl)-1-cyclopropyl-6-(4-fluorophenoxy)-1H-benzo[d]imidazole-2-amine |
| 197 | 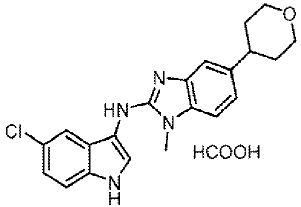 | N-(5-Chloro-1 H-indol-3-yl)-1-methyl-5-(tetrahydro-2H-pyran-4-yl)-1 H-benzo[d]imidazole-2-amine formate |
| 198 | 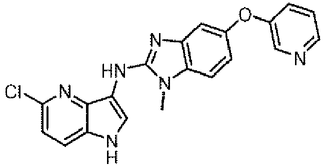 | N-(5-chloro-1H-pyrrolo[3,2-b]pyridine-3-yl)-1-methyl-5-(pyridine-3-yloxy)-1H-benzo[d]imidazole-2-amine |
| 199 | 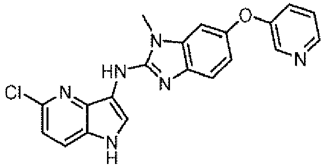 | N-(5-chloro-1H-pyrrolo[3,2-b]pyridine-3-yl)-1-methyl-5-(pyridine-3-yloxy)-1H-benzo[d]imidazole-2-amine |
| 200 | 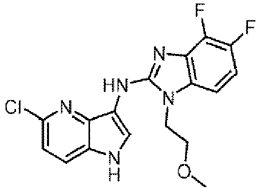 | N-(5-chloro-1H-pyrrolo[3,2-b]pyridine-3-yl)-4,5-difluoro-1-(2-methoxyethyl)-1H-benzo[d]imidazole-2-amine |
| 201 | 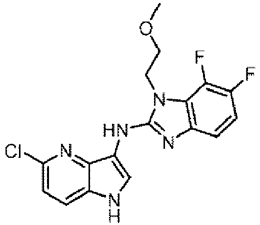 | N-(5-chloro-1H-pyrrolo[3,2-b]pyridine-3-yl)-6,7-difluoro-1-(2-methoxyethyl)-1H-benzo[d]imidazole-2-amine |
| 202 | 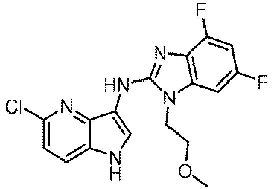 | N-(5-chloro-1H-pyrrolo[3,2-b]pyridine-3-yl)-4,6-difluoro-1-(2-methoxyethyl)-1H-benzo[d]imidazole-2-amine |
| 203 | 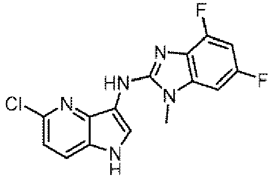 | N-(5-Chloro-1H-pyrrolo[3,2-b]pyridine-3-yl)-4,6-difluoro-1-methyl-1H-benzo[d]imidazole-2-amine |
| 204 | 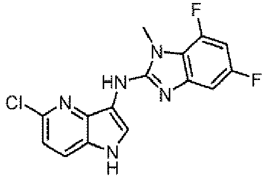 | N-(5-Chloro-1H-pyrrolo[3,2-b]pyridine-3-yl)-5,7-difluoro-1-methyl-1H-benzo[d]imidazole-2-amine |
| 205 | 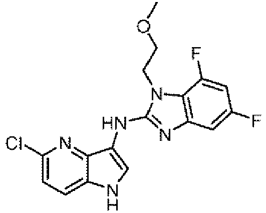 | N-(5-chloro-1H-pyrrolo[3,2-b]pyridine-3-yl)-5,7-difluoro-1-(2-methoxyethyl)-1H-benzo[d]imidazole-2-amine |
| 206 | 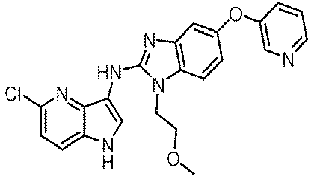 | N-(5-chloro-1H-pyrrolo[3,2-b]pyridine-3-yl)-1-(2-methylxhetyl)-5-(pyridine-3-yloxy)-1H-benzo[d]imidazole-2-amine |
| 207 | 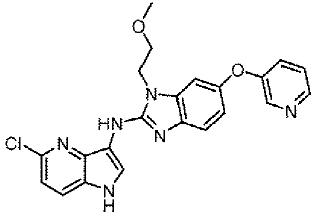 | N-(5-Chloro-1H-pyrrolo[3,2-b]pyridine-3-yl)-1-(2-methylxhetyl)-6-(pyridine-3-yloxy)-1H-benzo[d]imidazole-2-amine |
| 208 | 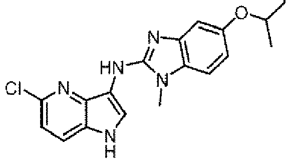 | N-(5-Chloro-1H-pyrrolo[3,2-b]pyridine-3-yl)-5-isopropoxy-1-methyl-1H-benzo[d]imidazole-2-amine |
| 209 | 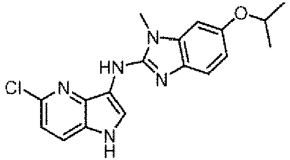 | N-(5-Chloro-1H-pyrrolo[3,2-b]pyridine-3-yl)-6-isopropoxy-1-methyl-1H-benzo[d]imidazole-2-amine |
| 210 | 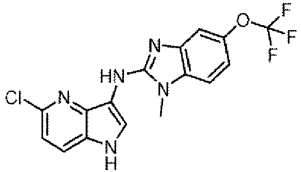 | N-(5-Chloro-1H-pyrrolo[3,2-b]pyridine-3-yl)-1-methyl-5-(trifluoromethoxy)-1H-benzo[d]imidazole-2-amine |
| 211 | 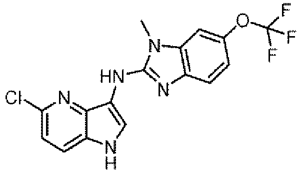 | N-(5-Chloro-1H-pyrrolo[3,2-b]pyridine-3-yl)-1-methyl-6-(trifluoromethoxy)-1H-benzo[d]imidazole-2-amine |
| 212 | 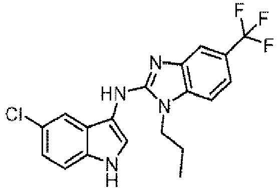 | N-(5-Chloro-1H-indol-3-yl)-1-propyl-5-(trifluoromethyl)-1H-benzo[d]imidazo-2-amine |
| 213 | 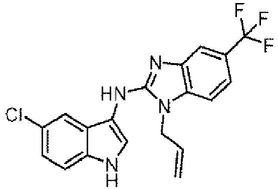 | 1-allyl-N-(5-chloro-1H-indol-3-yl)-5-(trifluoromethyl)-1H-benzo[d]imidazole-2-amine |
| 214 | 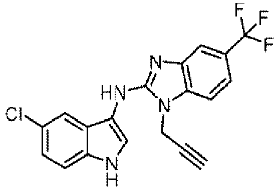 | N-(5-Chloro-1 H-indol-3-yl)-1-(pro-2-yl)-5-(trifluoromethyl)-1H-benzo[d]imidazole-2-amine |
| 215 | 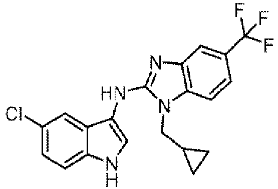 | N-(5-Chloro-1 H-indol-3-yl)-1-(cyclopropylmethyl)-5-(trifluoromethyl)-1 H-benzo[d]imidazole-2-amine |
| 216 | 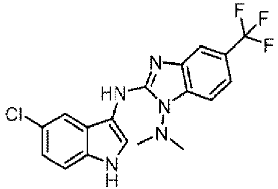 | N2-(5-Chloro-1H-indol-3-yl)-N1,N1-dimethyl-5-(trifluoromethyl)-1H-benzo[d]imidazole-1,2-diamine |
| 217 | 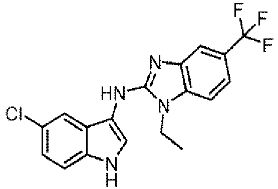 | N-(5-Chloro-1H-indol-3-yl)-1-ethyl-5-(trifluoromethyl)-IH-benzo[d]imidazole-2-amine |
| 218 | 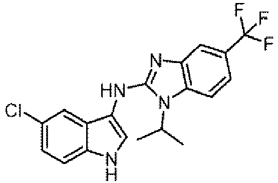 | N-(5-Chloro-1H-indol-3-yl)-1-isopropyl-5-(trifluoromethyl)-1H-benzo[d]imidazole-2-amine |
| 219 | 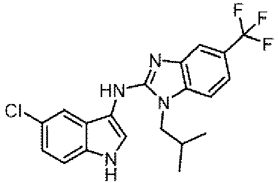 | N-(5-Chloro-1H-indol-3-yl)-1-isopropyl-5-(trifluoromethyl)-1H-benzo[d]imidazole-2-amine |
| 220 | 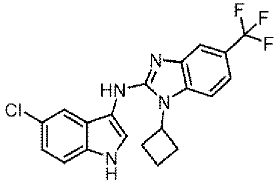 | N-(5-Chloro-1H-indol-3-yl)-1-isopropyl-5-(trifluoromethyl)-1H-benzo[d]imidazole-2-amine |
| 221 | 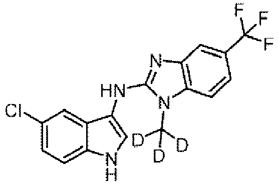 | N-(5-Chloro-1H-indol-3-yl)-1-(methyl-d3)-5-(trifluoromethyl)-1H-benzo[d]imidazole-2-amine |
| 222 | 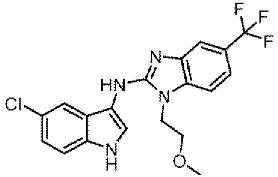 | N-(5-Chloro-1H-indol-3-yl)-1-(2-methoxyethyl)-5-(trifluoromethyl)-1H-benzo[d]imidazole-2-amine |
| 223 | 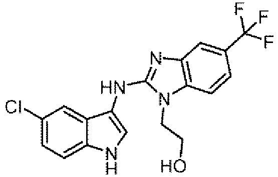 | 2-{2-[(5-chloro-1H-indol-3-yl)amino]-5-(trifluoromethyl)-1H-benzo[d]imidazole-1-yl}ethanol |
| 224 | 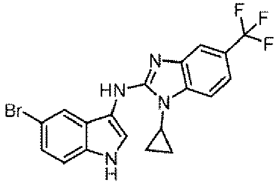 | N-(5-bromo-1H-indol-3-yl)-1-cyclopropyl-5-(trifluoromethyl)-1H-benzo[d]imidazole-2-amine |
| 225 | 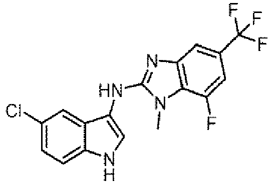 | N-(5-Chloro-1H-indol-3-yl)-7-fluoro-1-methyl-5-(trifluoromethyl)-1H-benzo[d]imidazole-2-amine |
| 226 | 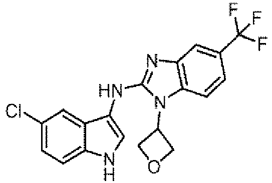 | N-(5-Chloro-1H-indol-3-yl)-1-(oxetan-3-yl)-5-(trifluoromethyl)-1H-benzo[d]imidazole-2-amine |
| 227 | 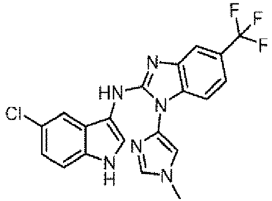 | N-(5-Chloro-1H-indol-3-yl)-1-(1-methyl-1H-imidazol-4-yl)-5-(trifluoromethyl)-1H-benzo[d]imidazole-2-amine |
| 228 | 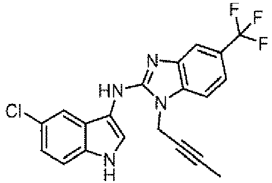 | 1-(But-2-yn-1-yl)-N-(5-rolo-1H-indol-3-yl)-5-(trifluoromethyl)-1H-benzo[d]imidazole-2-amine |
| 229 | 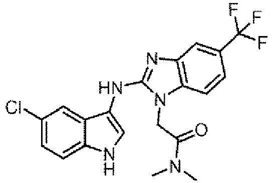 | 2-{2-[(5-chloro-1H-indol-3-yl)amino]-5-(trifluoromethyl)-1H-benzo[d]imidazole-1-yl}-N,N-dimethyl acetamide |
| 230 | 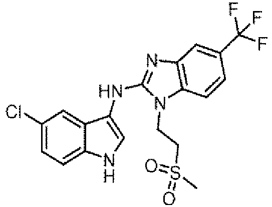 | N-(5-Chloro-1H-indol-3-yl)-1-[2-(methylsulfonyl)ethyl]-5-(trifluoromethyl)-1H-benzo[d]imidazole-2-amine |
| 231 | 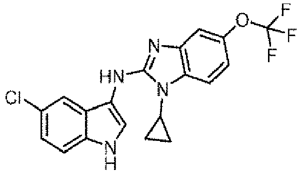 | N-(5-Chloro-1H-indol-3-yl)-1-cyclopropyl-5-(trifluoromethoxy)-1H-benzo[d]imidazole-2-amine |
| 232 | 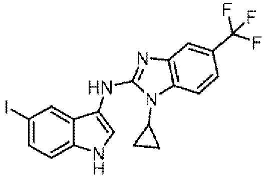 | 1-Cyclopropyl-N-(5-iodo-1H-indol-3-yl)-5-(trifluoromethyl)-1H-benzo[d]imidazole-2-amine |
| 233 | 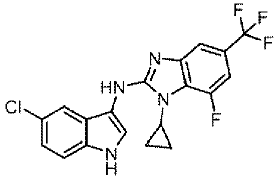 | N-(5-Chloro-1H-indol-3-yl)-1-cyclopropyl-7-fluoro-5-(trifluoromethyl)-1H-benzo[d]imidazole-2-amine |
| 234 | 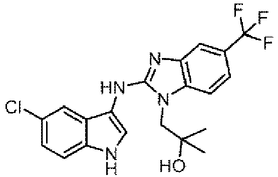 | 1-{2-[(5-chloro-1H-indol-3-yl)amino]-5-(trifluoromethyl)-1H-benzo[d]imidazole-1-yl}-2-methylpropane-2-ol |
| 235 | 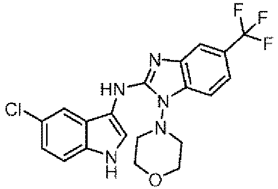 | N-(5-Chloro-1H-indol-3-yl)-1-morpholino-5-(trifluoromethyl)-1H-benzo[d]imidazole-2-amine |
| 236 | 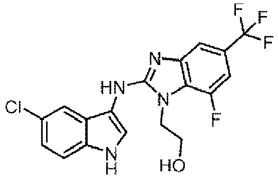 | 2-{2-[(5-chloro-1H-indol-3-yl)amino]-7-fluoro-5-(trifluoromethyl)-1H-benzo[d]imidazole-1-yl}ethane-1-ol |
| 237 | 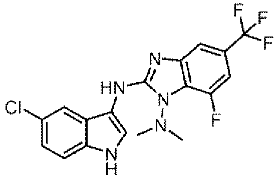 | N2-(5-Chloro-1H-indol-3-yl)-7-fluoro-N1,N1-dimethyl-5-(trifluoromethyl)-1H-benzo[d]imidazole-1,2-diamine |
| 238 | 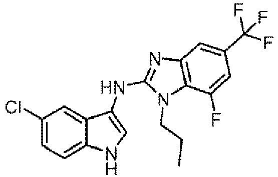 | N-(5-Chloro-1H-indol-3-yl)-7-fluoro-1-propyl-5-(trifluoromethyl)-1H-benzo[d]imidazole-2-amine |
| 239 | 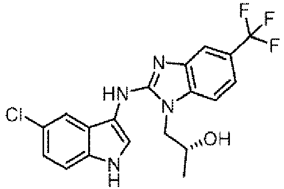 | (R)-1-{2-[(5-chloro-1H-indol-3-yl)amino]-5-(trifluoromethyl)-1H-benzo[d]imidazole-1-yl}propan-2-ol |
| 240 | 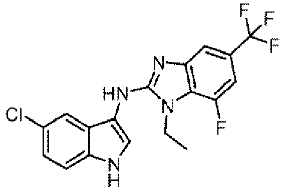 | N-(5-Chloro-1H-indol-3-yl)-1-ethyl-7-fluoro)-5-(trifluoromethyl)-1H-benzo[d]imidazole-2-amine |
| 241 | 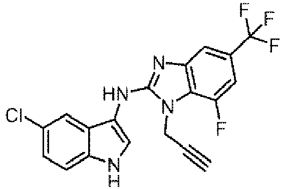 | N-(5-Chloro-1H-indol-3-yl)-7-fluoro-1-(prop-2-yn-1-yl)-5-(trifluoromethyl)-1H-benzo[d]imidazole-2-amine |
| 242 | 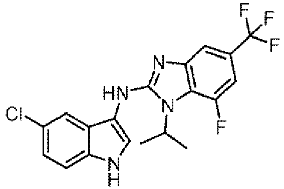 | N-(5-Chloro-1H-indol-3-yl)-7-fluoro-1-isopropyl-5-(trifluoromethyl)-1H-benzo[d]imidazole-2-amine |
| 243 | 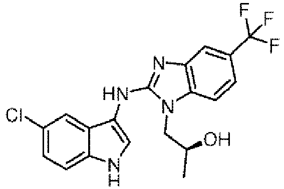 | (S)-1-{2-[(5-chloro-1H-indol-3-yl)amino]-5-(trifluoromethyl)-1H-benzo[d]imidazole-1-yl}propan-2-ol |
| 244 | 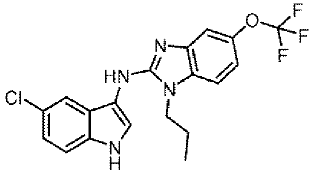 | N-(5-Chloro-1H-indol-3-yl)-1-propyl-5-(trifluoromethoxy)-1H-benzo[d]imidazole-2-amine |
| 245 | 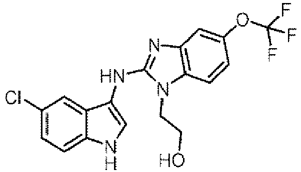 | 2-{2-[(5-Chloro-1H-indol-3-yl)amino]-5-(trifluoromethoxy)-1H-benzo[d]imidazole-1-yl}ethane-1-ol |
| 246 | 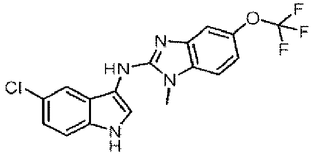 | N-(5-Chloro-1H-indol-3-yl)-1-methyl-5-(trifluoromethoxy)-1H-benzo[d]imidazole-2-amine |
| 247 | 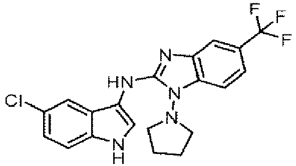 | N-(5-Chloro-1H-indol-3-yl)-1-(pyrrolidin-1-yl)-5-(trifluoromethyl)-1H-benzo[d]imidazole-2-amine |
| 248 | 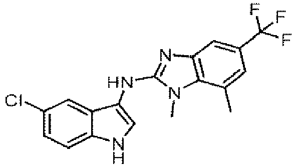 | N-(5-Chloro-1H-indol-3-yl)-1,7-dimethyl-5-(trifluoromethyl)-1H-benzo[d]imidazole-2-amine |
| 249 | 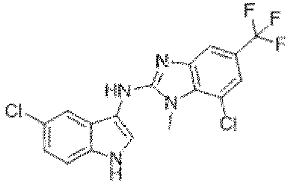 | 7-Chloro-N-(5-chloro-1H-indol-3-yl)-1-methyl-5-(trifluoromethyl)-1H-benzo[d]imidazole-2-amine |
| 250 | 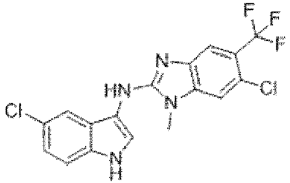 | 6-Chloro-N-(5-chloro-1H-indol-3-yl)-1-methyl-5-(trifluoromethyl)-1H-benzo[d]imidazole-2-amine |
| 251 | 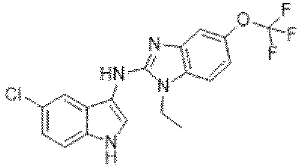 | N-(5-Chloro-1H-indol-3-yl)-1-ethyl-5-(trifluoromethoxy)-1H-benzo[d]imidazole-2-amine |
| 252 | 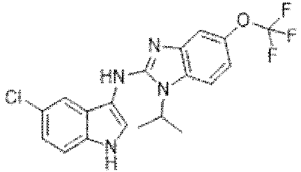 | N-(5-Chloro-1H-indol-3-yl)-1-isopropyl-6-(trifluoromethoxy)-1H-benzo[d]imidazole-2-amine |
| 253 | 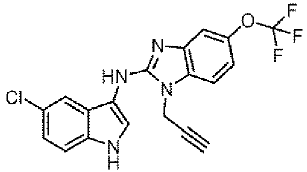 | N-(5-Chloro-1H-indol-3-yl)-1-(prop-2-yn-1-yl)-5-(trifluoromethoxy)-1H-benzo[d]imidazole-2-amine |
| 254 | 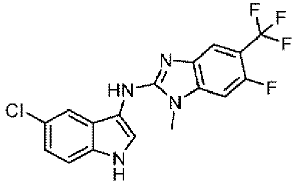 | N-(5-Chloro-1H-indol-3-yl)-6-fluoro-1-methyl-5-(trifluoromethyl)-1H-benzo[d]imidazole-2-amine |
| 255 | 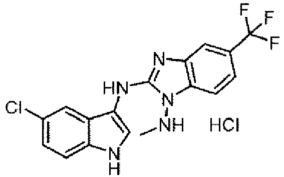 | N2-(5-Chloro-1H-indol-3-yl)-N1-methyl-5-(trifluoromethyl)-1H-benzo[d]imidazole-1,2-diamine hydrochloride |
| 256 | 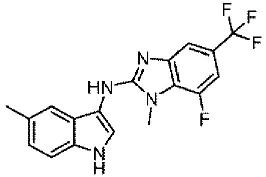 | 7-fluoro-1-methyl-N-(5-methyl-1H-indol-3-yl)-5-(trifluoromethyl)-1H-benzo[d]imidazole-2-amine |
| 257 | 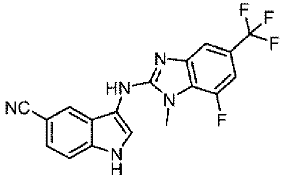 | 3-{[7-fluoro-1-methyl-5-(trifluoromethyl)-1H-benzo[d]imidazole-2-yl]amino}-1H-indole-5-carbonitrile |
| 258 | 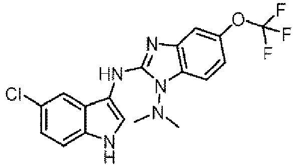 | N2-(5-Chloro-1H-indol-3-yl)-N1,N1-dimethyl-5-(trifluoromethoxy)-1H-benzo[d]imidazole-1,2-diamine |
| 259 | 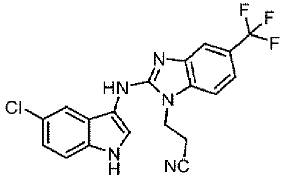 | 3-{2-[(5-chloro-1H-indol-3-yl)amino]-5-(trifluoromethyl)-1H-benzo[d]imidazole-1-yl]propanenitrile |
| 260 | 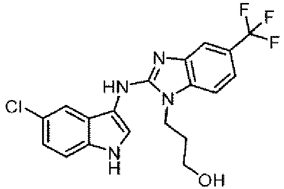 | 3-{2-[(5-Chloro-1H-indol-3-yl)amino]-5-(trifluoromethyl)-1H-benzo[d]imidazole-1-yl]propan-1-ol |
| 261 | 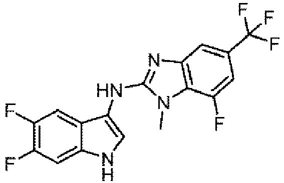 | N-(5,6-difluoro-1H-indol-3-yl)-7-fluoro-1-methyl-5-(trifluoromethyl)-1H-benzo[d]imidazole-2-amine |
| 262 | 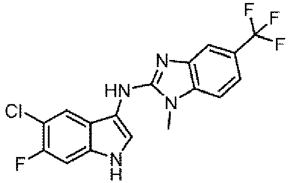 | N-(5-Chloro-6-fluoro-1H-indol-3-yl)-1-methyl-5-(trifluoromethyl)-1H-benzo[d]imidazole-2-amine |
| 263 | 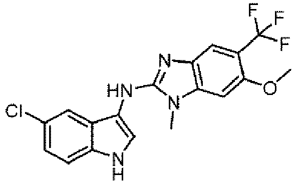 | N-(5-Chloro-1H-indol-3-yl)-6-methoxy-1-methyl-5-(trifluoromethyl)-1H-benzo[d]imidazole-2-amine |
| 264 | 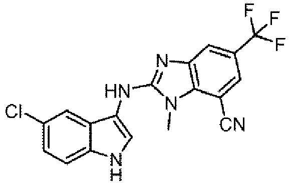 | 2-[(5-Chloro-1H-indol-3-yl)amino]-1-methyl-5-(trifluoromethyl)-1H-benzo[d]imidazole-7-carbonitrile |
| 265 | 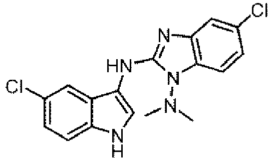 | 5-Chloro-N2-(5-chloro-1H-indol-3-yl)-N1,N1-dimethyl-1H-benzo[d]imidazole-1,2-diamine |
| 266 | 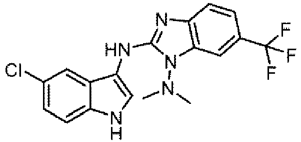 | N2-(5-Chloro-1H-indol-3-yl)-N1,N1-dimethyl-6-(trifluoromethyl)-1H-benzo[d]imidazole-1,2-diamine |
| 267 | 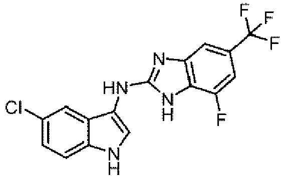 | N-(5-Chloro-1H-indol-3-yl)-7-fluoro-5-(trifluoromethyl)-1H-benzo[d]imidazole-2-amine |
| 268 | 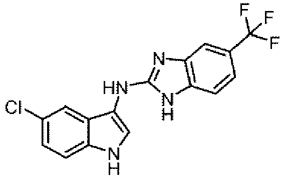 | N-(5-Chloro-1H-indol-3-yl)-5-(trifluoromethyl)-1H-benzo[d]imidazole-2-amine |
| 269 | 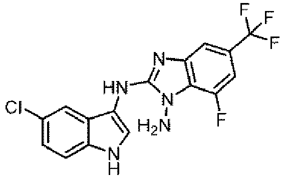 | N2-(5-Chloro-1H-indol-3-yl)-7-fluoro-5-(trifluoromethyl)-1H-benzo[d]imidazole-1,2-diamine |
| 270 | 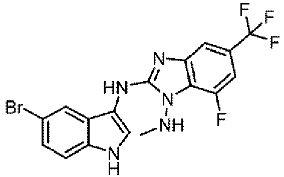 | N2-(5-Chloro-1H-indol-3-yl)-7-fluoro-N1-methyl-5-(trifluoromethyl)-1H-benzo[d]imidazole-1,2-diamine |
| 271 | 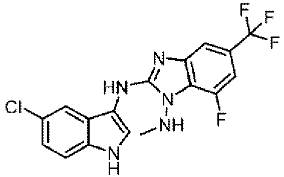 | N2-(5-Chloro-1H-indol-3-yl)-7-fluoro-N1-methyl-5-(trifluoromethyl)-1H-benzo[d]imidazole-1,2-diamine |
| 272 | 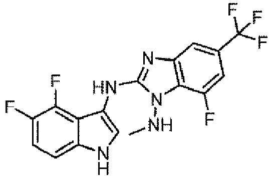 | N2-(4,5-difluoro-1H-indol-3-yl)-7-fluoro-N1-methyl-5-(trifluoromethyl)-1H-benzo[d]imidazole-1,2-diamine |
| 273 | 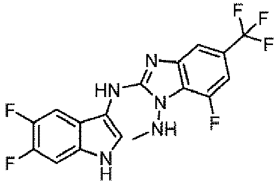 | N2-(5,6-difluoro-1H-indol-3-yl)-7-fluoro-N1-methyl-5-(trifluoromethyl)-1H-benzo[d]imidazole-1,2-diamine |
| 274 | 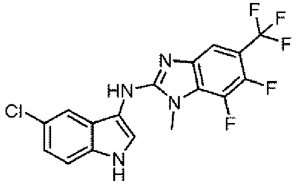 | N-(5-Chloro-1H-indol-3-yl)-6,7-difluoro-1-methyl-5-(trifluoromethyl)-1H-benzo[d]imidazole-2-amine |
| 275 | 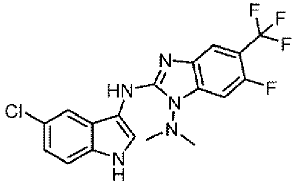 | N2-(5-Chloro-1H-indol-3-yl)-6-fluoro-N 1,N1-dimethyl-5-(trifluoromethyl)-1H-benzo[d]imidazole-1,2-didiamine |
| 276 | 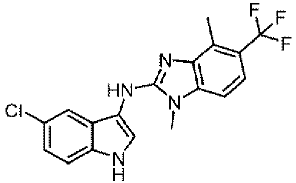 | N-(5-Chloro-1H-indol-3-yl)-1,4-dimethyl-5-(trifluoromethyl)-1H-benzo[d]imidazole-2-amine |
| 277 | 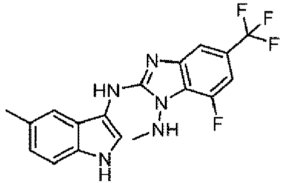 | 7-fluoro-N1-methyl-N2-(5-methyl-1H-indol-3-yl)-5-(trifluoromethyl)-1H-benzo[d]imidazole-1,2-diamine |
| 278 | 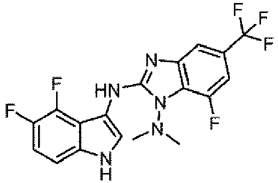 | N2-(4,5-difluoro-1H-indol-3-yl)-7-fluoro-N1,N1-dimethyl-5-(trifluoromethyl)-1H-benzo[d]imidazole-1,2-diamine |
| 279 | 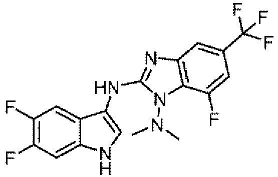 | N2-(5,6-difluoro-1H-indol-3-yl)-7-fluoro-N1,N1-dimethyl-5-(trifluoromethyl)-1H-benzo[d]imidazole-1,2-diamine |
| 280 | 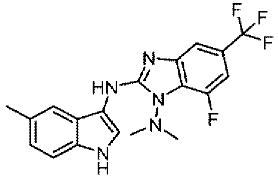 | 7-fluoro-N1,N1-dimethyl-N2-(5-methyl-1H-indol-3-yl)-5-(trifluoromethyl)-1H-benzo[d]imidazole-1,2-diamine |
| 281 | 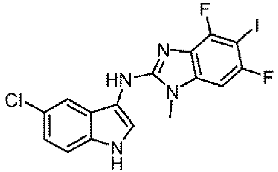 | N-(5-Chloro-1H-indol-3-yl)-4,6-difluoro-5-iodo-1-methyl-1H-benzo[d]imidazole-1,2-amine |
| 282 | 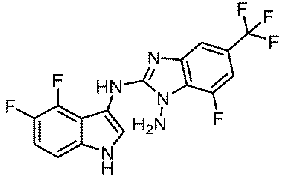 | N2-(4,5-difluoro-1H-indol-3-yl)-7-fluoro-5-(trifluoromethyl)-1H-benzo[d]imidazole-1,2-diamine |
| 283 | 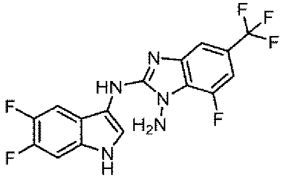 | N2-(5,6-difluoro-1H-indol-3-yl)-7-fluoro-5-(trifluoromethyl)-1H-benzo[d]imidazole-1,2-diamine |
| 284 | 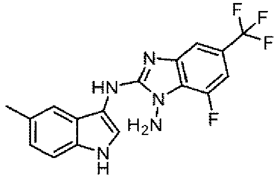 | 7-fluoro-N2-(5-methyl-1H-indol-3-yl)-5-(trifluoromethyl)-1H-benzo[d]imidazole-1,2-diamine |
| 285 | 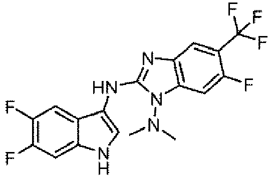 | N2-(5,6-difluoro-1H-indol-3-yl)-6-fluoro-N1,N1-dimethyl-5-(trifluoromethyl)-1H-benzo[d]imidazole-1,2-amine |
| 286 | 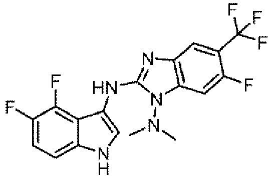 | N2-(4,5-difluoro-1H-indol-3-yl)-6-fluoro-N1,N1-dimethyl-5-(trifluoromethyl)-1H-benzo[d]imidazole-1,2-amine |
| 287 | 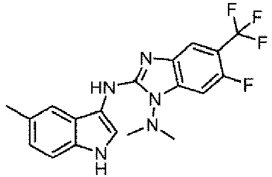 | 6-fluoro-N1,N1-dimethyl-N2-(5-methyl-1H-indol-3-yl)-5-(trifluoromethyl)-1H-benzo[d]imidazole-1,2-amine |
| 288 | 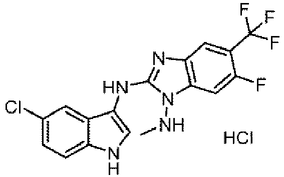 | N2-(5-Chloro-1H-indol-3-yl)-6-fluoro-N1-methyl-5-(trifluoromethyl)-1H-benzo[d]imidazole-1,2-diamine hydrochloride |

**[Table 4]**

| Embodi ments | ¹H-NMR δ (ppm) | LCMS m/z [M+H]⁺ |
|---|---|---|
| 59 | (DMSD-d6) δ = 11.27 (br. s, 1H), 10.74 - 10.58 (m, 1H), 9.38 (s, 1H), 8.18 (s, 1H), 7.77 (d, J = 8.6 Hz, 1H), 7.34 (s, 1H), 7.29 (d, J = 8.6 Hz, 1H), 7.23 (d, J = 8.1 Hz, 1H), 7.05 (br. s, 1H) , 0.23 (s, 9H). | 424.0 |
| 60 | (DMSO-d6) δ = 11.25 (s, 1H), 10.53 (s, 1H), 9. 28 (s, 1H), 8.24 (s, 1H), 7.78 (d, J = 8.3 Hz, 1 H), 7.68 - 7.61 (m, 2H), 7.57 (d, J = 1.5 Hz, 1 H), 7.46 - 7.40 (m, 2H), 7.35 (t, J = 7.5 Hz, 1H ), 7.30 - 7.23 (m, 3H). | 404.0 |
| 61 | (DMSO-d6) δ = 11.19 (s, 1H), 10.57 - 10.43 (m, 1H), 9.12 - 8.77 (m, 1H), 8.25 - 8.16 (m, 1H), 7.80 - 7.74 (m, 1H), 7.32 - 7.25 (m, 1H), 6.99 - 6.83 (m, 2H), 6.65 - 6.55 (m, 1H), 3.95 - 3.87 (m, 3H). | 358.0 |
| 62 | (DMSD-d6) δ 11.32 - 11.15 (m, 1H), 10.78 - 10. 56 (m, 1H), 9.37 - 8.76 (m, 1H), 8.25 - 8.20 (m , 1H), 8.19 - 8.12 (m, 1H), 7.80 - 7.73 (m, 1H), 7.71 - 7.63 (m, 1H), 7.60 - 7.53 (m, 1H), 7.51 - 7.39 (m, 2H), 7.39 - 7.21 (m, 3H), 7.16 - 6.9 9 (m, 1H). | 404.0 |
| 63 | (DMSO-d6) δ 11.25 (br.s, 1H), 10.54 (br.s, 1H), 9.29 (s, 1H), 8.18 (s, 1H), 7.76 (d, J = 8.6 Hz, 1H), 7.35 - 7.21 (m, 3H), 7.06 (s, 1H), 6.82 - 6 .75 (m, 1H), 5.57 (s, 1H), 3.68 (s, 3H). | 424.0 |
| 64 | (500 MHz, DMSO-d6) δ 11.25 - 11.20 (m, 1H), 10.43 - 10.35 (m, 1H), 9.23 - 9.19 (m, 1H), 8.2 2 - 8.15 (m, 1H), 7.75 (d, J = 8.5 Hz, 1H), 7.3 0 - 7.26 (m, 1H), 7.23 - 7.15 (m, 1H), 6.90 - 6. 80 (m, 3H), 6.77 - 6.72 (m, 2H), 6.63 - 6.57 (m , 1H), 2.85 (s, 6H). | 463.0 |
| 65 | (DMSO-d6) δ = 11.30 - 11.21 (m, 1H), 10.48 - 10.43 (m, 1H), 9.27 - 9.22 (m, 1H), 8.22 - 8.16 (m, 1H), 7.76 (d, J = 8.6 Hz, 1H), 7.31 - 7.21 ( m, 2H), 7.12 - 7.05 (m, 1H), 6.98 - 6.90 (m, 1H ), 6.70 - 6.62 (m, 1H), 6.45 - 6.39 (m, 1H), 6.3 5 - 6.29 (m, 1H), 6.18 - 6.11 (m, 1H), 2.85 (s, 6H). | 463.0 |
| 66 | (DMSO-d6) δ = 11.25 (br. s, 1H), 10.62 (br. s, 1H), 9.33 (s, 1H), 8.20 (s, 1H), 7.87 (d, J = 8.8 Hz, 2H), 7.77 (d, J = 8.1 Hz, 1H), 7.39 - 7.27 (m, 2H), 7.15 - 7.00 (m, 3H), 6.75 (s, 1H), 3.17 (s, 3H). | 498.2 |
| 67 | (DMSO-d6) δ = 11.25 (br. s, 1H), 10.56 (br. s, 1H), 9.31 (s, 1H), 8.20 (s, 1H), 7.77 (d, J = 8.4 Hz, 1H), 7.64 - 7.60 (m, 2H), 7.37 - 7.28 (m, 4H), 7.07 (br. s, 1H), 6.76 - 6.72 (m, 1H), 3.21 (s, 3H). | 498.2 |
| 68 | (DMSO-d6) δ = 11.30 - 11.25 (m, 1H), 10.63 - 10.57 (m, 1H), 9.33 - 9.30 (m, 1H), 8.23 - 8.15 (m, 1H), 7.91 (d, J = 7.8 Hz, 1H), 7.77 (d, J = 8.6 Hz, 1H), 7.63 - 7.58 (m, 1H), 7.35 - 7.23 ( m, 3H), 7.14 - 7.08 (m, 1H), 6.93 - 6.75 (m, 2H ), 3.40 (s, 3H). | 497.9 |
| 69 | (TRIFLUOROACETIC ACID-d) δ 8.51 (d, J = 8.4 Hz, 1H), 8.31 (s, 1H), 7.85 (d, J = 8.1 Hz, 1H ), 7.41 - 7.25 (m, 6H), 7.11 (d, J = 11.2 Hz, 2 H), 5.18 (s, 2H). | 434.0 |
| 70 | (DMSO-d6) δ 10.57 (s, 1H), 9.10 (s, 1H), 8.29 ( s, 2H), 8.05 (d, J = 5.3 Hz, 1H), 7.83 (s, 1H), 7.37 - 7.29 (m, 2H), 7.26 (d, J = 8.4 Hz, 1H), 7.07 - 6.99 (m, 1H), 6.97 (d, J = 2.3 Hz, 1H), 6.95 - 6.91 (m, 2H), 6.66 (dd, J = 8.4, 2.4 Hz, 1H), 6.42 (d, J = 5.4 Hz, 1H), 3.09 (s, 6H). | 385.3 |
| 71 | (DMSO-d6) δ 10.89 - 10.59 (m, 2H), 9.26 (s, 1 H), 8.49 (s, 1H), 8.32 (d, J = 4.8 Hz, 1H), 8.28 (s, 1H), 8.20 - 8.17 (m, 1H), 8.08 (d, J = 2.7 Hz, 1H), 7.39 - 7.26 (m, 4H), 7.04 (tt, J = 7.3, 1.2 Hz, 1H), 6.99 (d, J = 2.3 Hz, 1H), 6.96 - 6 .90 (m, 2H), 6.73 - 6.65 (m, 1H), 3.97 (s, 3H). | 422.3 |
| 72 | (DMSO-d6) δ 11.54 (s, 1H), 11.04 - 10.85 (m, 1 H), 9.25 (s, 1H), 8.25 - 8.21 (m, 1H), 8.18 (s, 1 H), 7.96 - 7.85 (m, 2H), 7.39 - 7.28 (m, 2H), 7 .27 - 7.13 (m, 1H), 7.08 - 7.02 (m, 1H), 6.99 - 6.81 (m, 3H), 6.78 - 6.52 (m, 1H). | 360.2 |
| 73 | (Methanol-d4) δ 8.49 (s, 1H), 8.21 (d, J = 2.3 Hz, 1H), 7.97 (d, J = 2.3 Hz, 1 H), 7.65 (s, 1H), 7.33 - 7.23 (m, 2H), 7.19 (d, J = 8.4 Hz, 1H), 7.05 - 6.97 (m, 1H), 6.95 - 6.88 (m, 3H), 6.73 (dd, J = 8.5, 2.3 Hz, 1H). | 376.2 |
| 74 | (DMSO-d6) δ 11.90 (s, 1H), 11.20 - 11.00 (m, 1H), 9.67 (s, 1H), 8.60 - 8.55 (m, 2H), 8.24 (s, 1H), 8.13 - 8.07 (m, 1H), 7.37 - 7.18 (m, 3H), 7.08 - 7.02 (m, 1H), 6.99 - 6.86 (m, 3H), 6.75 - 6.55 (m, 1H). | 410.2 |
| 75 | (DMSO-d6) δ 11.33 (s, 1H), 10.95 - 10.78 (m, 1 H), 9.69 - 8.53 (m, 1H), 8.20 - 8.16 (m, 1H), 7. 88 - 7.84 (m, 1H), 7.44 (d, J = 1.9 Hz, 1H), 7. 38 - 7.23 (m, 5H), 7.19 (d, J = 8.5 Hz, 1H), 7. 13 - 7.00 (m, 2H), 3.10 (s, 3H). | 453.2 |
| 76 | (DMSO-d6) δ 11.86 (brs, 1H), 11.45 (s, 1H), 11. 13 (brs, 1H), 9.02 (brs, 1H), 8.26 (s, 1H), 7.60 - 7.54 (m, 1H), 7.40 - 7.25 (m, 3H), 7.17 (brs, 1H), 7.06 - 7.00 (m, 1H), 6.94 - 6.85 (m, 3H), 6.69 - 6.60 (m, 1H), 5.94 - 5.86 (m, 1H). | 358.2 |
| 77 | (DMSO-d6) δ 11.12 (d, J = 2.8 Hz, 1H), 9.20 (b rs, 1H), 8.26 (s, 1H), 8.22 (d, J = 5.3 Hz, 1H), 7.90 (d, J = 2.8 Hz, 1H), 7.36 - 7.29 (m, 2H), 7.26 (d, J = 8.4 Hz, 1H), 7.06 - 7.00 (m, 1H), 6.97 (d, J = 2.3 Hz, 1H), 6.95 - 6.89 (m, 2H), 6.82 (d, J = 5.4 Hz, 1H), 6.66 (dd, J = 8.4, 2.4 Hz, 1H), 4.00 (s, 3H). | 372.3 |
| 78 | (DMSO-d6) δ 11.27 (s, 1H), 10.60 (brs, 1H), 9.4 4 (s, 1H), 8.17 (d, J = 2.7 Hz, 1H), 7.85 (d, J = 8.5 Hz, 1H), 7.32 - 7.25 (m, 2H), 7.18 (d, J = 8.5 Hz, 1H). | 320.0 |
| 79 | (DMSO-d6) δ = 11.17 (s, 1H), 10.23 (s, 1H), 9. 00 (s, 1H), 8.19 (d, J = 2.0 Hz, 1H), 7.75 (d, J = 8.6 Hz, 1H), 7.27 (d, J = 8.6 Hz, 1H), 7.09 (s, 1H), 7.04 (s, 1H), 2.24 (s, 6H). | 356.2 |
| 80 | (DMSO-d6) δ = 11.26 (s, 1H), 10.66 - 10.51 (m, 1H), 9.35 (s, 1H), 8.21 - 8.14 (m, 1H), 7.77 (d , J = 8.3 Hz, 1H), 7.63 - 7.57 (m, 1H), 7.30 - 7.20 (m, 2H), 7.14 - 7.06 (m, 1H). | 453.8 |
| 81 | (Methanol-d4) δ 8.53 (s, 1H), 7.87 (s, 1H), 7.83 (d, J = 8.5 Hz, 1H), 7.34 (t, J = 7.8 Hz, 1H), 7 .25 - 7.19 (m, 2H), 7.07 - 7.03 (m, 1H), 7.01 - 6.94 (m, 3H), 6.75 (dd, J = 8.5, 2.3 Hz, 1H), 3. 82 (s, 2H), 2.51 (s, 6H). | 433.3 |
| 82 | (Methanol-d4) δ 7.86 (s, 1H), 7.82 (d, J = 8.5 Hz, 1H), 7.18 (d, J = 8.6 Hz, 1H), 6.97 - 6.81 (m, 2H). | 320.0 |
| 83 | (DMSO-d6) δ 11.23 (brs, 1H), 10.50 - 10.39 (m, 1H), 9.27 (brs, 1H), 8.26 (s, 1H), 8.24 - 8.15 ( m, 1H), 7.84 (d, J = 8.5 Hz, 1H), 7.45 - 7.42 ( m, 1H), 7.29 - 7.14 (m, 3H), 7.10 - 7.05 (m, 1H ), 6.90 - 6.75 (m, 2H), 6.68 - 6.56 (m, 1H), 3.4 8 (s, 2H), 2.19 (s, 6H). | 433.2 |
| 84 | (DMSO-d6) δ 11.32 (s, 1 H), 10.84 (s, 1H), 9.62 (s, 1H), 8.22 (d, J = 2.7 Hz, 1H), 7.88 (d, J = 8.5 Hz, 1H), 7.28 (d, J = 2.0 Hz, 1H), 7.20 (d, J = 8.5 Hz, 1H), 7.10 (d, J = 1.9 Hz, 1H). | 352.0 |
| 85 | (Methanol-d4) δ 7.86 (s, 1H), 7.82 (d, J = 8.5 Hz, 1H), 7.20 (d, J = 8.5 Hz, 1H), 7.00 (d, J = 7.9 Hz, 1H), 6.84 (d, J = 8.0 Hz, 1H), 2.41 (s, 3H), 2.32 (s, 3H). | 312.1 |
| 86 | (Methanol-d4) δ 8.26 (s, 1H), 7.89 (s, 1H), 7.83 (d, J = 8.5 Hz, 1H), 7.21 (d, J = 8.5 Hz, 1H), 6.82 (ddd, J = 8.7, 2.3, 0.7 Hz, 1H), 6.64 (td, J = 10.5, 2.3 Hz, 1H). | 320.0 |
| 87 | (Methanol-d4) δ 7.85 (s, 1H), 7.82 (d, J = 8.5 Hz, 1H), 7.20 (d, J = 8.5 Hz, 1H), 6.92 (s, 1H), 6.67 (s, 1H), 2.43 (s, 3H), 2.34 (brs, 3H). | 312.1 |
| 88 | (DMSO-d6) δ = 11.32 (br. s, 1H), 10.74 (br. s, 1H), 9.54 (s, 1H), 8.16 (s, 1H), 7.78 (d, J = 8.6 ). Hz, 1H), 7.47 (s, 2H), 7.30 (d, J = 8.6 Hz, 1H | 395.9 |
| 89 | (DMSO-d6) δ 11.31 (br.s, 1H), 10.92 (br.s, 1H), 9.55 (br.s, 1H), 8.19 (s, 1H), 7.80 (d, J = 8.5 H z, 1H), 7.51 (br.s, 1H), 7.31 (d, J = 8.5 Hz, 1H ), 7.26 (d, J = 7.6 Hz, 1H), 7.05 (br.s, 1H). | 395.9 |
| 90 | (DMSO-d6) δ 11.35 (s, 1H), 8.71 (s, 1H), 8.14 ( s, 1H), 7.80 - 7.73 (m, 1 H), 7.36 - 7.23 (m, 4H ), 7.03 (t, J = 7.3 Hz, 1H), 6.94 - 6.88 (m, 3H) , 6.70 (dd, J = 8.4, 2.3 Hz, 1H), 4.26 (t, J = 7. 4 Hz, 2H), 1.79 - 1.67 (m, 2H), 1.46 - 1.31 (m, 2H), 0.95 (t, J = 7.4 Hz, 3H). | 476.0 |
| 91 | (DMSO-d6) δ 11.34 (s, 1H), 8.67 (s, 1H), 8.18 ( s, 1H), 7.81 - 7.74 (m, 1H), 7.39 - 7.21 (m, 4H ), 7.08 (d, J = 2.3 Hz, 1H), 7.03 (t, J = 7.4 Hz, 1H), 6.95 - 6.90 (m, 2H), 6.71 (dd, J = 8.3, 2. 3 Hz, 1H), 4.24 (t, J = 7.3 Hz, 2H), 1.75 - 1.63 (m, 2H), 1.41 - 1.27 (m, 2H), 0.90 (t, J = 7.4 Hz, 3H). | 476.0 |
| 92 | (DMSO-d6) δ 11.27 (s, 1H), 8.95 (s, 1H), 8.17 ( s, 1H), 7.76 (d, J = 8.4 Hz, 1H), 7.37 - 7.25 ( m, 4H), 7.04 (t, J = 7.4 Hz, 1H), 6.98 (d, J = 2 .3 Hz, 1H), 6.95 - 6.89 (m, 2H), 6.73 (dd, J = 8.4, 2.3 Hz, 1 H), 4.42 (t, J = 5.0 Hz, 2H), 3.78 (t, J = 4.9 Hz, 2H), 3.43 (s, 3H). | 478.0 |
| 93 | (DMSO-d6) δ 11.26 (s, 1H), 8.93 (s, 1H), 8.20 ( s, 1H), 7.77 (d, J = 8.5 Hz, 1H), 7.38 - 7.26 ( m, 4H), 7.15 (d, J = 2.3 Hz, 1H), 7.07 - 7.00 ( m, 1H), 6.96 - 6.91 (m, 2H), 6.75 (dd, J = 8.4, 2.3 Hz, 1H), 4.39 (t, J = 4.9 Hz, 2H), 3.73 (t, J = 4.8 Hz, 2H), 3.40 (s, 3H). | 478.0 |
| 94 | (DMSO-d6) δ 11.33 (s, 1H), 10.86 (s, 1H), 9.60 (s, 1H), 8.22 (s, 1H), 7.88 (d, J = 8.5 Hz, 1H), 7.25 - 7.17 (m, 2H), 7.14 - 7.07 (m, 1H). | 352.0 |
| 95 | (DMSO-d6) δ 11.26 (s, 1H), 10.66 - 10.52 (m, 1 H), 9.39 (s, 1H), 8.23 (s, 1H), 8.18 (brs, 1H), 7. 85 (d, J = 8.5 Hz, 1H), 7.47 - 7.40 (m, 1H), 7. 25 - 7.15 (m, 2H), 7.08 - 7.03 (m, 1H). | 364.0 |
| 96 | (DMSO-d6) δ 11.27 (s, 1H), 10.64 - 10.60 (m, 1 H), 9.39 (s, 1H), 8.60 - 8.53 (m, 2H), 8.26 - 8. 21 (m, 1H), 7.86 (d, J = 8.5 Hz, 1H), 7.77 - 7. 66 (m, 3H), 7.49 - 7.37 (m, 2H), 7.19 (d, J = 8 .5 Hz, 1H). | 361.1 |
| 97 | (DMSO-d6) δ 11.28 - 11.23 (m, 1H), 10.59 - 10. 55 (m, 1H), 9.37 - 9.33 (m, 1H), 8.90 - 8.86 (m , 1H), 8.51 - 8.48 (m, 1H), 8.21 - 8.23 (m, 1H), 8.09 - 8.01 (m, 1H), 7.88 - 7.84 (m, 1H), 7.65 - 7.61 (m, 1H), 7.48 - 7.42 (m, 1H), 7.42 - 7.37 (m, 1H), 7.37 - 7.27 (m, 1H), 7.19 (d, J = 8.5 Hz, 1H). | 361.1 |
| 98 | (DMSO-d6) δ 11.26 - 11.22 (m, 1H), 10.52 - 10. 48 (m, 1H), 9.31 - 9.28 (m, 1H), 8.26 - 8.23 (m , 1H), 7.88 - 7.83 (m, 1H), 7.64 - 7.54 (m, 3H) , 7.39 - 7.31 (m, 3H), 7.31 - 7.21 (m, 1H), 7.1 8 (d, J = 8.4 Hz, 1H), 3.62- 3.56 (m, 4H), 3.49 (s, 2H), 2.38 (brs, 4H). | 459.2 |
| 99 | (DMSO-d6) δ 11.24 (brs, 1H), 10.53 - 10.47 (m, 1H), 9.34 - 9.29 (m, 1H), 8.26 - 8.24 (m, 1H), 7.89 - 7.82 (m, 1H), 7.59 - 7.50 (m, 3H), 7.41 - 7.32 (m, 2H), 7.31 - 7.20 (m, 2H), 7.19 (d, J = 8.4 Hz, 1H), 3.61 - 3.56 (m, 4H), 3.55 - 3.52 (m, 2H), 2.40 (brs, 4H). | 459.1 |
| 100 | (DMSO-d6) δ 11.24 (s, 1H), 10.49 (brs, 1H), 9.3 0 (s, 1H), 8.22 (d, J = 2.7 Hz, 1H), 7.85 (d, J = 8.5 Hz, 1H), 7.43 (d, J = 1.7 Hz, 1H), 7.24 ( d, J = 8.2 Hz, 1H), 7.18 (d, J = 8.5 Hz, 1H), 7 .18 (brs, 1H), 5.34 (d, J = 1.8 Hz, 1H), 4.97 (t, J = 1.6 Hz, 1H), 2.14 (s, 3H). | 324.2 |
| 101 | (Methanol-d4) δ 7.79 (d, J = 8.5 Hz, 1H), 7.75 (s, 1H), 7.45 (d, J = 8.5 Hz, 1H), 7.32 - 7.25 ( m, 2H), 7.22 (d, J = 8.5 Hz, 1H), 7.04 - 6.98 ( m, 1H), 6.98 - 6.90 (m, 4H), 6.74 (dd, J = 8.5, 2.3 Hz, 1H), 6.11 (dd, J = 17.7, 1.1 Hz, 1H), 5. 44 (dd, J = 11.0, 1.1 Hz, 1H). | 368.2 |
| 102 | (DMSO-d6) δ 11.21 (s, 1H), 10.44 - 10.37 (m, 1 H), 9.23 - 9.15 (m, 1H), 8.24 - 8.21 (m, 1H), 8. 03 - 7.97 (m, 1H), 7.87 - 7.83 (m, 1H), 7.79 - 7.73 (m, 1H), 7.51 - 7.41 (m, 1H), 7.28 - 7.10 (m, 3H), 3.86 (s, 3H). | 364.1 |
| 103 | (DMSO-d6) δ 11.32 - 10.98 (m, 1H), 10.87 (brs, 1H), 9.08 (brs, 1H), 7.95 (brs, 1H), 7.70 (d, J = 8.3 Hz, 1H), 7.36 - 7.29 (m, 2H), 7.26 (d, J = 8.4 Hz, 1H), 7.10 - 7.01 (m, 2H), 6.99 - 6.8 9 (m, 3H), 6.67 (brs, 1H), 2.88 (q, J = 7.6 Hz, 2H), 1.32 (t, J = 7.5 Hz, 3H). | 370.2 |
| 104 | (DMSO-d6) δ 11.25 (brs, 1H), 9.22 (brs, 1H), 8. 20 (d, J = 2.7 Hz, 1H), 7.85 (d, J = 8.5 Hz, 1H ), 7.21 - 7.14 (m, 3H), 6.89 - 6.83 (m, 1H), 2.9 8 - 2.86 (m, 1H), 1.23 (d, J = 6.9 Hz, 6H). | 326.1 |
| 105 | (Methanol-d4) δ 7.89 (s, 1H), 7.82 (d, J = 8.5 Hz, 1H), 7.32 (brs, 1H), 7.20 (d, J = 8.5 Hz, 1 H), 7.20 (brs, 1H), 7.17 - 7.11 (m, 1H), 6.11 - 6.05 (m, 1H), 4.34 - 4.27 (m, 2H), 3.91 - 3.90 ( m, 2H), 2.59 - 2.53 (m, 2H). | 366.2 |
| 106 | (Methanol-d4) δ 7.91 (s, 1H), 7.79 (d, J = 8.4 Hz, 1H), 7.36 (d, J = 8.4 Hz, 1H), 7.32 - 7.26 (m, 2H), 7.24 - 7.20 (m, 1H), 7.04 - 6.98 (m, 1 H), 6.96 - 6.90 (m, 3H), 6.77 - 6.71 (m, 1H), 0 .27 (s, 9H). | 438.3 |
| 107 | (DMSO-d6) δ 11.69 - 11.10 (m, 1H), 10.80 - 1 0.61 (m, 1H), 9.08 - 8.44 (m, 1H), 8.12 - 8.06 (m, 1H), 7.84 - 7.47 (m, 2H), 7.36 - 7.27 (m, 2 H), 7.25 - 7.09 (m, 1H), 7.07 - 6.98 (m, 1H), 6 .96 - 6.78 (m, 3H), 6.75 - 6.52 (m, 1H), 2.40 - 2.31 (m, 3H). | 356.2 |
| 108 | (DMSO-06) δ 11.60 (s, 1H), 8.74 (s, 1H), 8.35 ( d, J = 2.3 Hz, 1H), 8.22 (d, J = 2.3 Hz, 1H), 8 .05 (d, J = 2.5 Hz, 1H), 7.39 - 7.27 (m, 3H), 7 .08 (d, J = 2.3 Hz, 1H), 7.07 - 7.01 (m, 1H), 6 .97 - 6.91 (m, 2H), 6.74 (dd, J = 8.4, 2.3 Hz, 1H), 3.70 (s, 3H). | 390.2 |
| 109 | (DMSO-d6) δ 11.69 (s, 1H), 8.97 (s, 1H), 8.34 ( d, J = 2.4 Hz, 1 H), 8.24 (d, J = 2.3 Hz, 1H), 8 .04 (d, J = 2.5 Hz, 1H), 7.59 (d, J = 1.7 Hz, 1 H), 7.47 (d, J = 8.3 Hz, 1H), 7.34 (dd, J = 8.4, 1.7 Hz, 1H), 3.80 (s, 3H). | 366.1 |
| 110 | (Methanol-d4) δ 8.00 (s, 1H), 7.82 (d, J = 8.5 Hz, 1H), 7.48 (s, 1H), 7.39 (s, 1H), 7.20 (d, J = 8.5 Hz, 1H), 4.39 (t, J = 4.9 Hz, 2H), 3.84 (t, J = 4.9 Hz, 2H), 3.47 (s, 3H). | 410.0 |
| 111 | (Methanol-d4) δ 8.42 (s, 1H), 7.92 (s, 1H), 7.86 (d, J = 8.4 Hz, 1H), 7.43 (d, J = 8.4 Hz, 1H), 7.34 - 7.28 (m, 2H), 7.26 (d, J = 8.5 Hz, 1H), 7.08 - 7.01 (m, 1H), 6.98 - 6.91 (m, 3H), 6.81 (dd, J = 8.5, 2.3 Hz, 1H), 3.61 (s, 1H). | 366.1 |
| 112 | (Methanol-d4) δ 7.88 (s, 1H), 7.82 (d, J = 8.5 Hz, 1H), 7.22 - 7.17 (m, 2H), 7.15 (d, J = 1.6 Hz, 1H), 6.94 (dd, J = 8.1, 1.7 Hz, 1H), 4.08 - 4.01 (m, 2H), 3.61 - 3.53 (m, 2H), 2.87 - 2.77 (m, 1H), 1.88 - 1.74 (m, 4H). | 368.1 |
| 113 | (DMSO-d6) δ 11.37 (d, J = 2.7 Hz, 1H), 10.94 ( s, 1H), 9.72 (s, 1H), 8.21 (d, J = 2.7 Hz, 1H), 7.89 (d, J = 8.5 Hz, 1H), 7.51 (dd, J = 7.8, 1.1 Hz, 1H), 7.35 (dd, J = 7.8, 1.1 Hz, 1H), 7.20 ( d, J = 8.5 Hz, 1H), 7.02 (t, J = 7.8 Hz, 1H). | 309.0 |
| 114 | (DMSO-d6) δ 11.20 (s, 1H), 10.32 (s, 1H), 9.18 - 9.10 (m, 1H), 8.23 - 8.18 (m, 1H), 7.87 - 7.8 2 (m, 1H), 7.25 - 7.11 (m, 3H), 7.10 - 7.01 (m, 1H), 6.93 - 6.83 (m, 1H), 5.91 - 5.84 (m, 1H), 3.86 (q, J = 7.0 Hz, 2H), 1.30 - 1.22 (m, 3H). | 354.2 |
| 115 | (DMSO-d6) δ 11.19 (s, 1H), 10.37 - 10.31 (m, 1 H), 9.15 - 9.08 (m, 1H), 8.22 - 8.20 (m, 1H), 7. 86 - 7.82 (m, 1H), 7.22 - 7.11 (m, 3H), 6.87 - 6.77 (m, 1H), 3.60 - 3.53 (m, 2H), 3.48 - 3.41 (m, 2H), 2.82 (t, J = 7.3 Hz, 2H), 1.14 - 1.08 ( m, 3H). | 356.2 |
| 116 | (DMSO-d6) δ = 11.29 (br. s, 1H), 8.72 (s, 1H), 8.18 (s, 1H), 7.84 (d, J = 8.6 Hz, 1H), 7.46 (d, J = 8.6 Hz, 1H), 7.35 - 7.27 (m, 2H), 7.16 (d, J = 8.3 Hz, 1H), 7.07 - 6.99 (m, 1H), 6.96 - 6. 89 (m, 3H), 6.61 (dd, J = 2.4, 8.6 Hz, 1H), 5.0 6 - 4.96 (m, 1H), 1.59 (d, J = 6.8 Hz, 6H). | 418.1 |
| 117 | (DMSO-d6) δ = 11.28 (br. s, 1H), 8.68 (s, 1H), 8.21 (s, 1H), 7.85 (d, J = 8.3 Hz, 1H), 7.38 - 7 .31 (m, 2H), 7.30 - 7.26 (m, 1H), 7.20 - 7.13 ( m, 2H), 7.07 - 7.01 (m, 1H), 6.97 - 6.91 (m, 2H ), 6.71 (dd, J = 2.3, 8.4 Hz, 1H), 5.02 - 4.96 ( m, 1H), 1.54 (d, J = 6.8 Hz, 6H). | 418.1 |
| 118 | (DMSO-d6) δ = 11.31 (br. s, 1H), 8.19 - 8.14 ( m, 2H), 7.86 (d, J = 8.1 Hz, 1H), 7.35 - 7.27 ( m, 3H), 7.19 (d, J = 8.6 Hz, 1H), 7.07 - 7.00 ( m, 1H), 6.95 (d, J = 2.3 Hz, 1H), 6.94 - 6.89 ( m, 2H), 6.74 (dd, J = 8.4, 2.3 Hz, 1H), 3.30 - 3.28 (m, 1H), 1.32 - 1.24 (m, 2H), 1.08 - 1.02 ( m, 2H), | 416.1 |
| 119 | (DMSO-d6) δ = 11.30 (br. s, 1H), 8.21 (s, 1H), 8.13 (s, 1H), 7.87 (d, J = 8.6 Hz, 1H), 7.37 - 7 .32 (m, 2H), 7.29 (d, J = 8.3 Hz, 1H), 7.19 (d, J = 8.3 Hz, 1H), 7.08 - 7.01 (m, 2H), 6.98 - 6. 92 (m, 2H), 6.74 (dd, J = 2.3, 8.4 Hz, 1H), 3.3 0 - 3.24 (m, 1H), 1.28 - 1.20 (m, 2H), 1.05 - 0. 96 (m, 2H). | 416.1 |
| 120 | (DMSO-d6) δ 11.25 - 11.16 (m, 1H), 10.30 - 10. 24 (m, 1H), 9.16 - 9.08 (m, 1H), 8.40 (s, 1H), 8 .23 - 8.18 (m, 1H), 7.84 (d, J = 8.5 Hz, 1H), 7. 17 (d, J = 8.5 Hz, 1H), 7.15 - 7.08 (m, 1H), 6. 93 - 6.86 (m, 1H), 6.61 - 6.53 (m, 1H), 4.25 - 4.11 (m, 1H), 1.97 - 1.86 (m, 2H), 1.18 - 1.65 (m, 2H), 1.56 - 1.20 (m, 6H). | 382.2 |
| 121 | (DMSO-d6) δ 11.26 - 11.14 (m, 1H), 10.26 (s, 1H), 9.16 - 9.10 (m, 1H), 8.22 - 8.18 (m, 1H), 7.86 - 7.82 (m, 1H), 7.20 - 7.09 (m, 2H), 6.94 - 6.88 (m, 1H), 6.62 - 6.51 (m, 1H), 4.32 - 4.1 2 (m, 1H), 2.70 - 2.57 (m, 2H), 2.18 (s, 3H), 2. 18 - 2,08 (m, 2H), 1.97 - 1.85 (m, 2H), 1.68 - 1.55 (m, 2H). | 397.2 |
| 122 | (Methanol-d4) δ 8.52 (s, 1H), 7.66 - 7.58 (m, 1 H), 7.51 (s, 1H), 7.31 - 7.25 (m, 2H), 7.24 - 7. 21 (m, 1H), 7.19 - 7.11 (m, 2H), 7.05 - 6.96 ( m, 1H), 6.94 - 6.87 (m, 3H), 6.76 - 6.68 (m, 1 H), 3.19 (brs, 3H), 3.05 (brs, 3H). | 412.4 |
| 123 | (DMSO-d6) δ 11.19 (s, 1H), 10.34 - 10.28 (m, 1 H), 9.14 - 9.06 (m, 1H), 8.23 - 8.18 (m, 1H), 7 .87 - 7.80 (m, 1H), 7.22 - 7.05 (m, 3H), 6.82 - 6.71 (m, 1H), 2.34 (s, 3H). | 298.1 |
| 124 | (DMSO-d6) δ = 11.25 (br. s, 1 H), 10.60 (br. s, 1H), 9.37 (br. s, 1H), 8.18 (s, 1H), 7.85 (d, J = 8.3 Hz, 1H), 7.60 (s, 1H), 7.26 - 7.20 (m, 1H), 7.18 (d, J = 8.3 Hz, 1H), 7.11 (d, J = 8.3 Hz, 1H). | 409.9 |
| 125 | (DMSO-d6) δ = 11.24 (br. s, 1H), 10.58 - 10.46 (m, 1H), 9.31 (br. s, 1H), 8.19 (d, J = 2.7 Hz, 1H), 7.85 (d, J = 8.6 Hz, 1H), 7.25 (d, J = 1.0 Hz, 1H), 7.21 - 7.15 (m, 2H), 7.00 - 6.91 (m, 1 H), 1.54 - 1.48 (m, 1H), 0.88 - 0.81 (m, 2H), 0. 73 - 0.66 (m, 2H). | 348.1 |
| 126 | (DMSO-d6) δ 11.34 - 11.20 (m, 1H), 10.69 - 10 .48 (m, 1H), 9.48 - 9.25 (m, 1H), 8.26 - 8.18 ( m, 1H), 7.92 - 7.78 (m, 1H), 7.55 - 7.07 (m, 4 H), 5.78 - 5.31 (m, 1H), 1.96 - 1.47 (m, 1H), 0 .96 - 0.71 (m, 2H), 0.67 - 0.46 (m, 2H). | 384.1 |
| 127 | (TRIFLUOROACETIC ACID-d) δ 8.59 (d, J = 8.8 Hz, 1H), 8.34 (s, 1H), 7.72 (d, J = 8.8 Hz, 1H ), 7.60 (d, J = 8.0 Hz, 3H), 3.87 (d, J = 11.9 H z, 2H), 3.68 (t, J = 11.9 Hz, 2H), 2.19 - 1.95 ( m, 6H). | 367.2 |
| 128 | (DMSO-d6) δ 11.21 (s, 1H), 10.42 - 10.35 (m, 1 H), 9.24 - 9.16 (m, 1H), 8.24 - 8.19 (m, 1H), 7. 88 - 7.81 (m, 1H), 7.30 - 7.17 (m, 1H), 7.21 - 7.14 (m, 2H), 6.89 - 6.77 (m, 1H), 6.32 - 6.28 (m, 1H), 1.90 - 1.84 (m, 6H). | 338.2 |
| 129 | (DMSO-d6) δ 11.22 - 11.16 (m, 1H), 10.31 - 10. 25 (m, 1H), 9.16 - 9.09 (m, 1H), 8.23 - 8.18 (m , 1H), 7.87 - 7.80 (m, 1H), 7.20 - 7.09 (m, 2H) , 7.01 - 6.95 (m, 1H), 6.78 - 6.66 (m, 1 H), 1.9 8 - 1.88 (m, 1H), 0.94 - 0.84 (m, 2H), 0.67 - 0. 56 (m, 2H). | 324.2 |
| 130 | (DMSO-d6) δ 11.57 - 11.51 (m, 1H), 10.54 - 10. 46 (m, 1H), 9.58 - 9.53 (m, 1H), 8.43 - 8.35 ( m, 1H), 8.00 - 7.94 (m, 1H), 7.75 - 7.68 (m, 1H ), 7.38 - 7.26 (m, 3H), 7.08 - 6.97 (m, 2H), 6. 97 - 6.89 (m, 2H), 6.76 - 6.64 (m, 1H). | 367.2 |
| 131 | (Methanol-d4) δ 8.51 (s, 1H), 7.91 - 7.84 (m, 2 H), 7.28 - 7.22 (m, 1H), 7.22 - 7.16 (m, 1H), 7. 12 - 7.07 (m, 1H), 6.98 - 6.91 (m, 1H), 2.57 - 2.50 (m, 2H), 1.94 - 1.80 (m, 1H), 0.94 - 0.88 ( m, 6H). | 340.2 |
| 132 | (DMSO-06) δ 11.37 (d, J = 2.8 Hz, 1H), 8.94 (s , 1H), 8.15 (d, J = 2.9 Hz, 1 H), 7.86 (d, J = 8. 5 Hz, 1H), 7.69 (s, 1H), 7.45 (s, 1H), 7.18 (d, J = 8.5 Hz, 1H), 5.06 - 4.95 (m, 1H), 1.57 (d, J = 6.8 Hz, 6H). | 396.2 |
| 133 | (DMSO-d6) δ 11.27 (s, 1H), 10.72 - 10.56 (m, 1 H), 9.44 - 9.35 (m, 1H), 8.28 (s, 1H), 8.22 - 8. 17 (m, 1H), 7.89 - 7.82 (m, 1H), 7.39 - 7.34 (m , 1H), 7.28 - 7.21 (m, 1H), 7.21 - 7.15 (m, 1H), 7.12 - 7.02 (m, 1H), 3.93 - 3.87 (m, 1H). | 308.0 |
| 134 | (Methanol-d4) δ 8.54 (s, 2H), 7.99 (s, 1H), 7.75 (d, J = 8.5 Hz, 1H), 7.33 - 7.26 (m, 3H), 7.15 (d, J = 8.5 Hz, 1H), 7.05 - 6.99 (m, 1H), 6.98 (d, J = 2.2 Hz, 1H), 6.96 - 6.91 (m, 2H), 6.78 (dd, J = 8.5, 2.3 Hz, 1H), 4.26 - 4.17 (m, 2H), 2.91 - 2.80 (m, 2H), 2.53 (s, 6H). | 447.1 |
| 135 | (DMSO-d6) δ 11.20 - 11.13 (m, 1H), 10.47 - 10. 20 (m, 1H), 9.12 - 8.64 (m, 1H), 8.24 - 8.14 (m , 1H), 7.88 - 7.80 (m, 1H), 7.21 - 7.13 (m, 1H), 6.84 - 6.69 (m, 1H), 6.55 - 6.42 (m, 1H), 4.37 - 4.19 (m, 4H). | 342.1 |
| 136 | (500 MHz, DMSO-d6) δ = 11.24 (br. s, 1H), 10. 59 - 10.45 (m, 1H), 9.34 - 9.27 (m, 1H), 8.20 ( d, J = 2.4 Hz, 1H), 7.85 (d, J = 8.2 Hz, 1H), 7 .27 (s, 1H), 7.22 - 7.16 (m, 2H), 7.03 - 6.93 (m , 1H), 2.02 (s, 3H). | 322.1 |
| 137 | (DMSO-d6) δ 11.34 - 11.20 (m, 1H), 10.64 - 10. 49 (m, 1H), 9.42 - 9.29 (m, 1H), 8.25 - 8.17 (m , 1H), 7.89 - 7.82 (m, 1H), 7.58 - 6.90 (m, 4H) , 6.34 - 5.92 (m, 1H), 1.96 - 1.66 (m, 3H). | 358.0 |
| 138 | (DMSO-d6) δ 11.33 (s, 1H), 8.86 (s, 1H), 8.18 ( d, J = 2.7 Hz, 1H), 7.86 (d, J = 8.5 Hz, 1H), 7 .44 (dd, J = 10.9, 7.4 Hz, 1H), 7.31 - 7.22 (m, 1H), 7.18 (d, J = 8.4 Hz, 1H), 3.73 (s, 3H). | 334.1 |
| 139 | (DMSO-d6) δ 11.22 - 11.14 (m, 1H), 10.32 - 10. 27 (m, 1H), 9.21 - 9.14 (m, 1H), 8.25 - 8.17 (m , 1H), 7.88 - 7.81 (m, 1H), 7.21 - 7.13 (m, 2H) , 6.75 - 6.65 (m, 1H), 6.52 - 6.41 (m, 1H), 5.28 - 5.15 (m, 1H), 4.98 - 4.86 (m, 2H), 4.62 - 4. 54 (m, 2H). | 356.2 |
| 140 | (Methanol-d4) δ 7.87 (s, 1H), 7.82 (d, J = 8.5 Hz, 1H), 7.19 (d, J = 8.5 Hz, 1H), 7.13 (d, J = 8.6 Hz, 1H), 6.89 (d, J = 2.3 Hz, 1H), 6.68 (dd, J = 8.6, 2.4 Hz, 1H), 4.15 - 4.05 (m, 2H), 3.7 8 - 3.71 (m, 2H), 3.43 (s, 3H). | 358.1 |
| 141 | (DMSO-d6) δ 11.16 (s, 1H), 10.10 (brs, 1H), 9.0 9 (s, 1H), 8.18 (d, J = 2.7 Hz, 1H), 7.83 (d, J = 8.5 Hz, 1H), 7.16 (d, J = 8.4 Hz, 1H), 6.76 ( s, 2H), 4.18 (s, 4H). | 342.1 |
| 142 | (DMSO-d6) δ 11.22 - 11.14 (m, 1H), 10.30 - 10. 24 (m, 1H), 9.15 - 9.07 (m, 1H), 8.23 - 8.16 (m , 1H), 7.87 - 7.80 (m, 1H), 7.20 - 7.09 (m, 2H), 6.98 - 6.93 (m, 1H), 6.66 - 6.54 (m, 1H), 4.46 - 4.38 (m, 1H), 3.91 - 3.81 (m, 2H), 3.51 - 3. 43 (m, 2H), 1.99 - 1.92 (m, 2H), 1.62 - 1.55 (m , 2H). | 384.2 |
| 143 | (DMSO-d6) δ 11.15 (d, J = 2.7 Hz, 1H), 10.19 ( s, 1H), 9.06 (s, 1H), 8.19 (d, J = 2.7 Hz, 1H), 7.83 (d, J = 8.5 Hz, 1H), 7.16 (d, J = 8.5 Hz, 1H), 6.99 - 6.94 (m, 2H), 4.10 - 4.00 (m, 4H), 3.69 - 3.62 (m, 4H), 3.33 (s, 6H). | 432.3 |
| 144 | (500 MHz, TRIFLUOROACETIC ACID-d) δ 8.42 ( d, J = 6.2 Hz, 1H), 8.26 (s, 1H), 7.63 (d, J = 6 .3 Hz, 1H), 7.33 - 7.25 (m, 3H), 7.12 - 7.08 (m , 1H), 7.05 (dd, J = 8.9, 2.2 Hz, 1H), 7.00 (d, J = 2.2 Hz, 1H), 6.97 - 6.91 (m, 2H), 5.88 - 5. 86 (m, 1H), 5.76 (br.s, 1H), 2.32 (br.s, 3H). | 382.2 |
| 145 | (500 MHz, DMSO-d6) δ = 11.28 - 11.25 (m, 1H ), 10.69 - 10.54 (m, 1H), 9.44 - 9.32 (m, 1H), 8 .20 (d, J = 1.8 Hz, 1H), 7.85 (d, J = 8.5 Hz, 1 H), 7.35 (s, 1H), 7.25 (d, J = 7.9 Hz, 1H), 7.18 (d, J = 8.5 Hz, 1H), 7.10 - 7.01 (m, 1H), 4.31 (s, 2H), 3.34 (s, 3H). | 352.1 |
| 146 | (500 MHz, DMSO-d6) δ = 11.25 (br. s, 1H), 10. 55 (br. s, 1H), 9.36 (br. s, 1H), 8.23 - 8.17 (m, 1H), 7.85 (d, J = 8.5 Hz, 1H), 7.33 (s, 1H), 7.2 4 (d, J = 7.9 Hz, 1H), 7.18 (d, J = 8.5 Hz, 1H) , 7.07 - 7.00 (m, 1H), 3.43 (s, 2H), 2.25 (s, 6H) | 365.1 |
| 147 | (500 MHz, DMSO-d6) δ = 11.28 (br. s, 1H), 10. 70 (s, 1H), 9.44 - 9.38 (m, 2H), 8.56 - 8.48 (m , 2H), 8.20 (d, J = 2.4 Hz, 1H), 7.86 (d, J = 8. 2 Hz, 1H), 7.54 - 7.48 (m, 1H), 7.35 - 7.29 (m, 2H), 7.19 (d, J = 8.2 Hz, 1H), 7.06 (t, J = 7.6 Hz, 1H). | 361.1 |
| 148 | (500 MHz, DMSO-d6) δ = 11.30 (s, 1H), 10.71 ( s, 1H), 9.45 (s, 1H), 8.65 (d, J = 6.0 Hz, 2H), 8.26 - 8.21 (m, 3H), 7.86 (d, J = 8.5 Hz, 1H), 7.42 (d, J = 7.3 Hz, 1H), 7.36 (d, J = 7.6 Hz, 1H), 7.19 (d, J = 8.5 Hz, 1H), 7.06 (t, J = 7.2 Hz, 1H). | 361.1 |
| 149 | (500 MHz, DMSO-d6) δ = 11.25 - 11.14 (m, 1H) , 10.67 - 10.46 (m, 1H), 9.37 - 8.67 (m, 1H), 8 .23 - 8.17 (m, 1H), 7.88 - 7.80 (m, 1H), 7.34 - 7.10 (m, 3H), 7.04 - 6.86 (m, 2H), 4.38 - 4.28 ( m, 2H), 3.93 - 3.87 (m, 2H), 2.74 - 2.68 (m, 1 H), 2.57 - 2.51 (m, 1H). | 366.1 |
| 150 | (500 MHz,TRIFLUOROACETIC ACID-d) δ 8.58 ( d, J = 8.7 Hz, 1H), 8.33 (s, 1H), 7.69 (d, J = 8 .8 Hz, 1H), 7.35 (t, J = 7.9 Hz, 1H), 7.29 (d, J = 7.8 Hz, 1H), 7.18 (d, J = 8.0 Hz, 1H), 4.30 ( dd, J = 11.7, 4.2 Hz, 2H), 3.71 (t, J = 12.0 Hz, 2H), 3.12 (s, 1H), 2.11 - 1.99 (m, 2H), 1.90 - 1.84 (m, 2H). | 368.1 |
| 151 | (500 MHz, DMSO-d6) δ = 11.21 - 11.10 (m, 1H) , 10.22 - 10.12 (m, 1H), 9.06 - 9.00 (m, 1H), 8 .20 (d, J = 2.7 Hz, 1H), 7.83 (d, J = 8.5 Hz, 1 H), 7.17 - 7.08 (m, 2H), 6.90 (d, J = 2.1 Hz, 1 H), 6.70 - 6.59 (m, 1H), 3.0.3 -2.98 (m, 4H), 1. 68 - 1.63 (m, 4H), 1.53 - 1.48 (m, 2H). | 367.1 |
| 152 | (DMSO-d6) δ 11.26 (s, 1H), 8.64 (s, 1H), 8.24 ( d, J = 2.8 Hz, 1H), 7.85 (d, J = 8.5 Hz, 1H), 7 .20 - 7.12 (m, 2H), 7.07 (s, 1H), 6.86 - 6.79 ( m, 1H), 3.71 (s, 3H), 2.39 (s, 3H). | 312.2 |
| 153 | (DMSC3-d6} δ 11.28 (s, 1H), 8.67 (s, 1H), 8.24 ( d, J = 2.8 Hz, 1H), 7.85 (d, J = 8.5 Hz, 1H), 7 .22 - 7.13 (m, 3H), 6.90 (dd, J = 8.1, 1.7 Hz, 1H), 4.00 - 3.91 (m, 2H), 3.71 (s, 3H), 3.40 (s, 2H), 2.83 - 2.72 (m, 1H), 1.76 - 1.66 (m, 4H). | 382.2 |
| 154 | (DMSO-d6) δ 11.27 (d, J = 2.7 Hz, 1H), 8.65 (s , 1H), 8.24 (d, J = 2.7 Hz, 1H), 7.85 (d, J = 8. 5 Hz, 1H), 7.24 - 7.10 (m, 3H), 6.91 (dd, J = 8 .1, 1.7 Hz, 1H), 4.01 - 3.94 (m, 2H), 3.73 (s, 3 H), 3.50 - 3.41 (m, 2H), 2.87 - 2.76 (m, 1H), 1 .79 - 1.69 (m, 4H). | 382.2 |
| 155 | (DMSO-dfi) δ 11.34 (s, 1H), 8.87 (s, 1H), 8.23 ( d, J = 2.7 Hz, 1H), 7.87 (d, J = 8.5 Hz, 1H), 7 .18 (d, J = 8.5 Hz, 1H), 7.13 (dd, J = 8.0, 0.9 Hz, 1H), 6.97 (td, J = 8.0, 4.8 Hz, 1H), 6.89 - 6.79 (m, 1H), 3.76 (s, 3H). | 316.1 |
| 156 | (DMSO-d6) δ 11.27 (s, 1H), 8.65 (s, 1H), 8.23 ( d, J = 2.7 Hz, 1H), 7.85 (d, J = 8.5 Hz, 1H), 7 .17 (d, J = 8.4 Hz, 1H), 7.13 (d, J = 8.0 Hz, 1 H), 7.10 - 7.07 (m, 1H), 6.82 (d, J = 8.0 Hz, 1 H), 3.71 (s, 3H), 2.35 (s, 3H). | 312.1 |
| 157 | (Methanol-d4) δ 8.40 (s, 1H), 8.26 (s, 1H), 7.87 (d, J = 8.5 Hz, 1H), 7.24 (d, J = 8.5 Hz, 1H), 7.11 (t, J = 7.9 Hz, 1H), 7.02 (d, J = 7.9 Hz, 1 H), 6.80 (d, J = 7.8 Hz, 1H), 4.58 (brs, 4H), 3. 79 (s, 3H), 1.86 - 1.76 (m, 4H), 1.69 - 1.61 (m, 2H). | 381.2 |
| 158 | (DMSO-d6) δ 11.21 - 11.13 (m, 1H), 10.29 - 10. 22 (m, 1H), 9.13 - 9.06 (m, 1H), 8.20 (s, 1H), 7 .87 - 7.80 (m, 1H), 7.20 - 7.08 (m, 2H), 6.92 - 6.85 (m, 1 H), 6.60 - 6.48 (m, 1 H), 4.55 - 4.43 ( m, 1H), 1.28 - 1.22 (m, 6H). | 342.1 |
| 159 | (DMSO-d6) δ 11.28 (s, 1 H), 8.66 (s, 1H), 8.22 ( d, J = 2.8 Hz, 1H), 7.85 (d, J = 8.4 Hz, 1H), 7 .17 (d, J = 8.4 Hz, 1H), 7.13 (d, J = 8.6 Hz, 1 H), 6.88 (d, J = 2.4 Hz, 1H), 6.61 (dd, J = 8.5, 2.4 Hz, 1H), 3.73 (s, 3H), 3.70 (s, 3H). | 328.1 |
| 160 | (DMSO-d6) δ 11.34 (s, 1H), 10.79 (br.s, 1H), 9. 73 - 9.59 (m, 1H), 8.18 (s, 1H), 7.87 (d, J = 8. 5 Hz, 1H), 7.55 (d, J = 6.4 Hz, 1H), 7.35 - 7.2 5 (m, 1H), 7.19 (d, J = 8.5 Hz, 1H). | 370.1 |
| 161 | (DMSO-d6) δ 11.24 (s, 1H), 8.60 (s, 1H), 8.24 ( d, J = 2.8 Hz, 1H), 7.84 (d, J = 8.4 Hz, 1H), 7 .20 - 7.13 (m, 2H), 6.91 (d, J = 2.4 Hz, 1H), 6. 62 (dd, J = 8.5, 2.5 Hz, 1H), 3.78 (s, 3H), 3.71 (s, 3H). | 328.1 |
| 162 | (500 MHz, DMSO-d6) δ 10.75 - 10.57 (m, 2H), 9.20 (s, 1H), 8.31 - 8.26 (m, 1H), 8.04 - 7.97 ( m, 1H), 7.37 - 7.24 (m, 3H), 7.06 - 6.88 (m, 5 H), 6.73 - 6.61 (m, 1H), 6.33 (br.s, 1H), 2.49 - 2.45 (m, 2H), 2.33 - 2.27 (m, 2H), 1.84 - 1.77 ( m, 2H), 1.74 - 1.67 (m, 2H). | 422.4 |
| 163 | (500 MHz, DMSO-d6) δ 11.18 (s, 1H), 10.73 - 1 0.56 (m, 1H), 9.39 (br.s, 1H), 8.82 - 8.77 (m, 2 H), 8.50 - 8.46 (m, 1H), 8.14 (s, 1H), 7.81 - 7. 76 (m, 2H), 7.38 - 7.27 (m, 4H), 7.07 - 7.02 (m , 1H), 7.00 (s, 1H), 6.96 - 6.91 (m, 2H), 6.70 ( br.s, 1H). | 419.3 |
| 164 | (500 MHz, TRIFLUOROACETIC ACID-d) δ 9.38 ( d, J = 2.0 Hz, 1H), 9.09 (d, J = 5.9 Hz, 1H), 9 .03 (dt, J = 8.3, 1.7 Hz, 1H), 8.74 (d, J = 6.1 Hz, 1H), 8.43 (s, 1H), 8.38 (dd, J = 8.3, 5.9 Hz , 1H), 7.94 (d, J = 6.1 Hz, 1H), 7.31 - 7.25 (m, 3H), 7.12 - 7.04 (m, 2H), 6.99 (d, J = 2.3 Hz, 1H), 6.96 - 6.91 (m, 2H). | 419.3 |
| 165 | (500 MHz, TRIFLUOROACETIC ACID-d) δ 8.43 ( d, J = 6.3 Hz, 1H), 8.27 (s, 1H), 7.88 (d, J = 6 .2 Hz, 1H), 7.66 (d, J = 2.4 Hz, 1H), 7.33 - 7. 23 (m, 3H), 7.13 - 7.07 (m, 2H), 7.04 (dd, J = 8.9, 2.3 Hz, 1H), 6.99 (d, J = 2.3 Hz, 1H), 6.97 - 6.89 (m, 2H), 4.09 (s, 3H). | 422.4 |
| 166 | (500 MHz, DMSO-d6) δ = 11.27 - 11.21 (m, 1H ), 10.80 - 10.67 (m, 1H), 9.41 - 8.95 (m, 1H), 8.24 - 8.11 (m, 1H), 7.85 (d, J = 8.2 Hz, 1H), 7.44 - 7.26 (m, 1H), 7.18 (d, J = 8.2 Hz, 1H), 7.09 - 7.01 (m, 1H), 6.94 (br. s, 1H), 3.12 - 2.8 7 (m, 6H). | 355.1 |
| 167 | (500 MHz, DMSO-d6) δ = 11.27 (s, 1H), 9.26 (s , 1H), 8.32 (d, J = 2.7 Hz, 1H), 7.88 (d, J = 8. 5 Hz, 1H), 7.62 (d, J = 7.9 Hz, 1H), 7.33 (d, J = 7.6 Hz, 1H), 7.20 (d, J = 8.5 Hz, 1H), 7.13 (t , J = 7.8 Hz, 1H), 4.52 (t, J = 4.7 Hz, 2H), 3.7 8 (t, J = 4.7 Hz, 2H), 3.39 (s, 3H). | 410.2 |
| 168 | (DMSO-d6) δ 11.33 (d, J = 2.8 Hz, 1H), 8.81 (b rs, 1H), 8.19 (brs, 2H), 7.86 (d, J = 8.5 Hz, 1H ), 7.23 (dd, J = 8.6, 4.8 Hz, 1H), 7.18 (d, J = 8.4 Hz, 1H), 7.05 (dd, J = 10.0, 2.5 Hz, 1H), 6. 86 - 6.76 (m, 1H), 3.74 (s, 3H). | 316.1 |
| 169 | (DMSO-d6) δ 11.35 (s, 1H), 8.88 (s, 1H), 8.19 ( d, J = 2.4 Hz, 1H), 7.87 (d, J = 8.5 Hz, 1H), 7 .41 (d, J = 1.8 Hz, 1H), 7.25 (d, J = 8.3 Hz, 1 H), 7.19 (d, J = 8.4 Hz, 1H), 7.13 (dd, J = 8.4, 1.9 Hz, 1H), 3.75 (s, 3H). | 378.0 |
| 170 | (DMSO-d6) δ 11.35 (s, 1H), 8.86 (s, 1H), 8.21 ( d, J = 2.8 Hz, 1H), 7.87 (d, J = 8.5 Hz, 1H), 7 .52 (d, J = 1.9 Hz, 1H), 7.24 - 7.10 (m, 3H), 3 .75 (s, 3H). | 378.0 |
| 171 | (DMSO-d6) δ 11.10 (d, J = 2.8 Hz, 1H), 8.82 (s , 1H), 8.13 (d, J = 2.8 Hz, 1H), 7.76 (d, J = 8. 5 Hz, 1H), 7.58 - 7.53 (m, 1H), 7.46 (d, J = 8. 2 Hz, 1H), 7.42 (d, J = 8.4 Hz, 1H), 7.35 - 7.2 9 (m, 1H), 6.93 (dd, J = 17.6, 10.9 Hz, 1H), 6. 14 (dd, J = 17.7, 1.6 Hz, 1H), 5.37 (dd, J = 10 .9, 1.5 Hz, 1H), 3.82 (s, 3H). | 358.2 |
| 172 | (DMSO-d6) δ 11.34 (s, 1H), 8.87 (s, 1H), 8.19 ( d, J = 2.8 Hz, 1H), 7.86 (d, J = 8.5 Hz, 1H), 7 .31 - 7.24 (m, 2H), 7.18 (d, J = 8.5 Hz, 1H), 7 .01 (dd, J = 8.4, 2.0 Hz, 1H), 3.74 (s, 3H). | 332.1 |
| 173 | (DMSO-d6) δ 10.95 (d, J = 2.7 Hz, 1H), 8.74 (s , 1H), 8.06 (d, J = 2.8 Hz, 1H), 7.70 (d, J = 8. 3 Hz, 1H), 7.56 - 7.51 (m, 1H), 7.44 (d, J = 8. 2 Hz, 1H), 7.35 - 7.28 (m, 1H), 7.06 (d, J = 8. 4 Hz, 1H), 3.81 (s, 3H), 2.85 (q, J = 7.6 Hz, 2 H), 1.28 (t, J = 7.6 Hz, 3H). | 360.2 |
| 174 | (DMSO-d6) δ 11.32 (d, J = 2.8 Hz, 1H), 8.79 (s , 1H), 8.23 (d, J = 2.8 Hz, 1H), 7.86 (d, J = 8. 5 Hz, 1H), 7.82 - 7.76 (m, 2H), 7.32 (d, J = 8. 4 Hz, 1H), 7.22 - 7.15 (m, 2H), 7.08 - 7.01 (m , 2H), 6.80 (dd, J = 8.4, 2.3 Hz, 1H), 3.73 (s, 3H). | 415.1 |
| 175 | (DMSO-d6) δ 11.28 - 11.19 (m, 1H), 10.60 (s, 1 H), 9.28 (s, 1H), 8.20 (d, J = 2.6 Hz, 1H), 7.85 (d, J = 8.4 Hz, 1H), 7.18 (d, J = 8.5 Hz, 1H), 7.01 (d, J = 1.4 Hz, 1H), 6.72 (dd, J = 12.3, 1. 4 Hz, 1H), 3.97 - 3.82 (m, 2H), 3.48 - 3.41 (m, 2H), 2.83 - 2.73 (m, 1H), 1.76 - 1.58 (m, 4H). | 386.1 |
| 176 | (DMSO-d6) δ 11.22 (s, 1H), 10.42 (s, 1H), 9.24 (s, 1H), 8.25 (d, J = 2.7 Hz, 1H), 7.85 (d, J = 8.4 Hz, 1H), 7.18 (d, J = 8.5 Hz, 1H), 7.06 - 7. 00 (m, 1H), 6.73 - 6.66 (m, 1H), 2.39 (s, 3H). | 316.0 |
| 177 | (DMSO-d6) δ 11.43 (s, 1H), 11.01 (br.s, 1H), 9. 98 (br.s, 1H), 8.17 (s, 1H), 8.00 (d, J = 6.9 Hz, 1H), 7.88 (d, J = 8.5 Hz, 1 H), 7.30 (d, J = 12. 7 Hz, 1H), 7.21 (d, J = 8.5 Hz, 1H). | 347.0 |
| 178 | (DMSO-d6) δ = 11.33 (br.s, 1H), 9.10 (s, 1H), 8 .22 (d, J = 2.7 Hz, 1H), 7.87 (d, J = 8.6 Hz, 1 H), 7.58 (br.s, 1H), 7.51 (d, J = 8.3 Hz, 1H), 7. 36 - 7.30 (m, 1H), 7.19 (d, J = 8.6 Hz, 1H), 4. 51 (t, J = 4.9 Hz, 2H), 3.78 (t, J = 4.9 Hz, 2H) , 3.38 (s, 3H). | 410.2 |
| 179 | (DMSO-d6) δ = 11.33 (s, 1H), 9.15 (s, 1H), 8.2 1 (d, J = 2.8 Hz, 1H), 7.86 (d, J = 8.6 Hz, 1H) , 7.72 (s, 1H), 7.43 (d, J = 8.0 Hz, 1H), 7.34 ( d, J = 8.0 Hz, 1H), 7.19 (d, J = 8.3 Hz, 1H), 4 .51 (t, J = 4.6 Hz, 2H), 3.79 (t, J = 4.8 Hz, 2H ), 3.40 (s, 3H). | 410.2 |
| 180 | (DMSO-d6) δ = 11.30 (br. s, 1H), 9.28 (s, 1H), 8.19 (br. s, 1H), 7.87 (d, J = 8.6 Hz, 1H), 7.67 (d, J = 7.8 Hz, 1H), 7.37 (d, J = 7.3 Hz, 1H), 7.26 - 7.18 (m, 2H), 4.45 (t, J = 4.4 Hz, 2H), 3 .86 (t, J = 4.5 Hz, 2H), 3.50 (s, 3H). | 410.2 |
| 181 | (500 MHz, DMSO-d6) δ = 11.27 (br. s, 1H), 8.9 3 (s, 1H), 8.25 (d, J = 2.5 Hz, 1H), 7.88 (d, J = 8.5 Hz, 1H), 7.69 - 7.64 (m, 2H), 7.60 (d, J = 1.5 Hz, 1H), 7.47 - 7.39 (m, 3H), 7.35 - 7.29 (m, 2H), 7.21 (d, J = 8.5 Hz, 1H), 4.45 (t, J = 4.7 Hz, 2H), 3.80 (t, J = 4.9 Hz, 2H), 3.41 (s, 3H). | 418.3 |
| 182 | (500 MHz, DMSO-d6) δ = 11.26 (s, 1H), 8.94 (s , 1H), 8.25 (s, 1H), 7.86 (d, J = 8.2 Hz, 1H), 7. 74 - 7.69 (m, 2H), 7.66 (d, J = 1.2 Hz, 1H), 7. 48 - 7.43 (m, 2H), 7.40 - 7.34 (m, 2H), 7.32 - 7.28 (m, 1H), 7.18 (d, J = 8.2 Hz, 1H), 4.51 (t, J = 4.9 Hz, 2H), 3.81 (t, J = 4.9 Hz, 2H), 3.43 (s, 3H). | 418.3 |
| 183 | (500 MHz, DMSO-d6) δ 11.37 (s, 1H), 8.97 (s, 1H), 8.20 (s, 1H), 7.88 (d, J = 8.5 Hz, 1H), 7.1 9 (d, J = 8.5 Hz, 1H), 7.08 (dd, J = 8.7, 3.7 H z, 1H), 6.99 (ddd, J = 11.6, 8.7, 7.1 Hz, 1H), 3 .75 (s, 3H). | 334.2 |
| 184 | (500 MHz, DMSO-d6) δ 11.35 (br.s, 1H), 8.89 ( s, 1H), 8.16 (br.s, 1H), 7.86 (d, J = 8.4 Hz, 1H ), 7.18 (d, J = 8.4 Hz, 1H), 7.06 - 6.94 (m, 2H ), 3.91 (s, 3H). | 334.2 |
| 185 | (DMSO-d6) δ 12.72 (brs, 1H), 11.02 (s, 1H), 8.6 3 (s, 1H), 8.13 (s, 1H), 7.89 (d, J = 2.5 Hz, 1H ), 7.84 (d, J = 2.1 Hz, 1H), 7.39 (dd, J = 8.7, 0.5 Hz, 1H), 7.26 - 7.18 (m, 1H), 7.13 - 7.02 ( m, 2H), 6.86 - 6.76 (m, 1H), 3.73 (s, 3H). | 315.0 |
| 186 | (DMSO-d6) δ 11.37 (s, 1H), 8.42 (s, 1H), 8.19 ( s, 1H), 7.88 (d, J = 8.5 Hz, 1H), 7.58 - 7.53 ( m, 1 H), 7.51 - 7.45 (m, 1H), 7.38 - 7.31 (m, 1 H), 7.20 (d, J = 8.5 Hz, 1H), 3.31 - 3.30 (m, 1 H), 1.36 - 1.27 (m, 2H), 1.10 - 1.02 (m, 2H). | 392.2 |
| 187 | (DMSO-d6) δ 11.30 (s, 1H), 8.20 (d, J = 2.7 Hz , 1H), 8.17 - 8.12 (m, 1H), 7.87 (d, J = 8.5 Hz , 1H), 7.28 (d, J = 8.4 Hz, 1H), 7.25 - 7.12 (m , 3H), 7.04 - 6.94 (m, 3H), 6.73 (dd, J = 8.4, 2 .4 Hz, 1H), 3.30 - 3.25 (m, 1H), 1.29 - 1.20 (m , 2H), 1.04 - 0.97 (m, 2H). | 434.1 |
| 188 | (DMSO-d6) δ 11.01 (d, J = 2.7 Hz, 1H), 8.11 (d , J = 2.6 Hz, 1H), 7.93 (s, 1H), 7.76 (d, J = 8. 5 Hz, 1H), 7.39 (d, J = 8.5 Hz, 1H), 7.31 (d, J = 8.4 Hz, 1H), 7.23 - 7.13 (m, 2H), 7.03 - 6.8 9 (m, 4H), 6.74 (dd, J = 8.4, 2.4 Hz, 1H), 6.19 (dd, J = 17.6, 1.7 Hz, 1H), 5.38 (dd, J = 10.8, 1.7 Hz, 1H), 1.32 - 1.22 (m, 2H), 1.09 - 1.00 ( m, 2H). | 426.2 |
| 189 | (Methanol-d4) δ 7.49 - 7.44 (m, 2H), 7.35 (dd, J = 8.7, 0.7 Hz, 1H), 7.27 (dd, J = 8.7, 4.6 Hz, 1H), 7.13 - 7.06 (m, 1H), 6.92 - 6.85 (m, 1H), 6.81 - 6.72 (m, 1H), 3.23 - 3.15 (m, 1H), 1.34 - 1.28 (m, 2H), 1.16 - 1.10 (m, 2H). | 341.1 |
| 190 | (Methanol-d4) δ 8.46 (s, 1H), 7.90 (s, 1H), 7.84 (d, J = 8.4 Hz, 1H), 7.24 (d, J = 8.5 Hz, 1H), 7.19 (d, J = 8.5 Hz, 1H), 7.13 - 7.04 (m, 3H), 7.01 - 6.93 (m, 2H), 6.79 (dd, J = 8.5, 2.3 Hz, 1H), 3.28 - 3.22 (m, 1H), 2.93 (q, J = 7.6 Hz, 2H), 1.40 - 1.27 (m, 5H), 1.23 - 1.15 (m, 2H). | 428.3 |
| 191 | (Methanol-d4) δ 8.43 (brs, 1H), 7.94 (s, 1H), 7.8 7 (d, J = 8.6 Hz, 1H), 7.54 (d, J = 8.6 Hz, 1H) 7.27 (d, J = 8.5 Hz, 1H), 7.13 - 7.03 (m, 3H) , 7.03 - 6.89 (m, 3H), 6.83 (dd, J = 8.5, 2.3 H z, 1H), 6.09 (dd, J = 17.7, 0.9 Hz, 1H), 5.48 (d d, J = 11.1, 0.9 Hz, 1H), 3.78 (s, 3H). | 400.2 |
| 192 | (Methanol-d4) δ 8.44 (brs, 1H), 7.90 (s, 1H), 7.8 6 (d, J = 8.4 Hz, 1H), 7.27 (d, J = 8.6 Hz, 1H) , 7.21 (d, J = 8.5 Hz, 1H), 7.13 - 7.03 (m, 3H) , 7.02 - 6.94 (m, 2H), 6.84 (dd, J = 8.6, 2.3 H z, 1H), 3.78 (s, 3H), 2.94 (q, J = 7.7 Hz, 2H), 1.36 (t, J = 7.6 Hz, 3H). | 402.2 |
| 193 | (DMSO-d6) δ 11.27 - 11.23 (m, 1H), 10.58 - 10. 49 (m, 1H), 9.40 - 9.36 (m, 1H), 8.21 - 8.17 (m , 1H), 7.89 - 7.84 (m, 1H), 7.37 - 7.29 (m, 2H) , 7.29 - 7.23 (m, 1H), 7.20 - 7.16 (m, 1H), 7.14 - 7.08 (m, 1H), 7.07 - 7.01 (m, 1H), 6.94 - 6. 88 (m, 2H). | 394.1 |
| 194 | (DMSO-d6) δ 11.37 - 11.33 (m, 1H), 8.84 (s, 1 H), 8.19 (d, J = 2.7 Hz, 1H), 7.86 (d, J = 8.5 Hz, 1H), 7.36 - 7.30 (m, 2H), 7.30 - 7.23 (m, 2H), 7.18 (d, J = 8.5 Hz, 1H), 7.07 - 7.01 (m, 1H), 6.92 - 6.87 (m, 2H), 3.72 (s, 3H). | 408.1 |
| 195 | (DMSO-d6) δ 10.99 (s, 1H), 8.56 (s, 1H), 7.93 ( d, J = 2.5 Hz, 1H), 7.87 (d, J = 2.1 Hz, 1H), 7 .39 (d, J = 8.6 Hz, 1H), 7.26 (d, J = 8.4 Hz, 1 H), 7.21 - 7.13 (m, 2H), 7.10 (dd, J = 8.6, 2.1 Hz, 1H), 7.04 (d, J = 2.3 Hz, 1H), 7.01 - 6.93 (m, 2H), 6.71 (dd, J = 8.4, 2.4 Hz, 1H), 3.69 (s , 3H). | 407.1 |
| 196 | (DMSO-d6) δ 11.00 (s, 1H), 8.41 (s, 1H), 7.85 ( d, J = 2.5 Hz, 1H), 7.76 (d, J = 2.1 Hz, 1H), 7 .39 (d, J = 8.7 Hz, 1H), 7.22 (d, J = 8.4 Hz, 1 H), 7.21 - 7.14 (m, 2H), 7.10 (dd, J = 8.6, 2.1 Hz, 1H), 7.02 - 6.95 (m, 3H), 6.70 (dd, J = 8.4 , 2.4 Hz, 1H), 3.22 - 3.15 (m, 1H), 1.27 - 1.21 (m, 2H), 1.02 - 0.94 (m, 2H). | 433.2 |
| 197 | (Methanol-d4) δ 8.51 (s, 1H), 7.55 - 7.49 (m, 2 H), 7.43 - 7.36 (m, 1H), 7.28 (d, J = 8.2 Hz, 1 H), 7.18 - 7.07 (m, 3H), 4.07 - 3.99 (m, 2H), 3 .77 (s, 3H), 3.62 - 3.50 (m, 2H), 2.88 - 2.81 ( m, 1H), 1.84 - 1.73 (m, 4H). | 381.2 |
| 198 | (DMSO-d6) δ 11.32 (s, 1H), 8.81 (s, 1H), 8.31 ( dd, J = 2.9, 0.7 Hz, 1H), 8.26 (dd, J = 4.6, 1.4 Hz, 1H), 8.19 (d, J = 2.8 Hz, 1H), 7.85 (d, J = 8.4 Hz, 1H), 7.34 (ddd, J = 8.4, 4.6, 0.7 Hz, 1H), 7.31 - 7.24 (m, 2H), 7.17 (d, J = 8.4 Hz, 1H), 7.00 (d, J = 2.3 Hz, 1 H), 6.78 (dd, J = 8. 4, 2.3 Hz, 1H), 3.76 (s, 3H). | 391.2 |
| 199 | (DMSO-d6) δ 11.30 (d, J = 2.8 Hz, 1H), 8.76 (s , 1H), 8.34 (dd, J = 2.8, 0.7 Hz, 1H), 8.27 (dd, J = 4.6, 1.4 Hz, 1H), 8.24 (d, J = 2.8 Hz, 1H), 7.86 (d, J = 8.4 Hz, 1H), 7.36 (ddd, J = 8.5, 4. 6, 0.8 Hz, 1H), 7.31 - 7.27 (m, 2H), 7.18 (d, J = 8.5 Hz, 1H), 7.15 (d, J = 2.3 Hz, 1H), 6.78 ( dd, J = 8.4, 2.4 Hz, 1 H), 3.72 (s, 3H). | 391.2 |
| 200 | (500 MHz, DMSO-d6) δ 11.31 (s, 1H), 9.11 - 9. 05 (m, 1H), 8.21 - 8.16 (m, 1H), 7.89 - 7.83 (m , 1H), 7.21 - 7.11 (m, 2H), 7.03 - 6.95 (m, 1H) , 4.47 - 4.41 (m, 2H), 3.78 - 3.72 (m, 2H), 3.40 - 3.35 (m, 3H). | 378.2 |
| 201 | (500 MHz, DMSO-d6) δ 11.34 - 11.30 (m, 1H), 8.93 (s, 1H), 8.16 (d, J = 2.7 Hz, 1H), 7.86 (d, J = 8.5 Hz, 1H), 7.18 (d, J = 8.5 Hz, 1H), 7.10 - 6.97 (m, 2H), 4.54 (t, J = 5.2 Hz, 2H), 3.80 (t, J = 5.1 Hz, 2H), 3.37 (s, 3H). | 378.2 |
| 202 | (DMSO-06) δ 11.29 (br.s, 1H), 9.01 (br.s, 1H), 8.20 (s, 1H), 7.87 (br.s, 1H), 7.21 - 7.16 (m, 2 H), 6.86 (td, J = 10.7, 2.3 Hz, 1H), 4.44 (t, J = 4.9 Hz, 2H), 3.75 (t, J = 4.9 Hz, 2H), 3.39 (s, 3H). | 378.2 |
| 203 | (500 MHz, DMSO-d6) δ 11.33 (s, 1H), 8.91 (s, 1H), 8.21 (s, 1H), 7.87 (d, J = 8.4 Hz, 1H), 7.1 8 (d, J = 8.4 Hz, 1H), 7.14 (dd, J = 9.0, 2.3 H z, 1H), 6.84 (td, J = 10.7, 2.3 Hz, 1H), 3.75 (s, 3H). | 334.2 |
| 204 | (500 MHz, DMSO-d6) δ 11.37 (s, 1H), 8.93 (s, 1H), 8.12 (d, J = 2.8 Hz, 1H), 7.89 - 7.84 (m, 1H), 7.21 - 7.13 (m, 1H), 6.93 (dd, J = 9.5, 2.3 Hz, 1H), 6.83 - 6.77 (m, 1H), 3.89 - 3.85 (m, 3H). | 334.2 |
| 205 | (500 MHz, DMSO-d6) δ 11.34 (br.s, 1H), 8.97 ( br.s, 1H), 8.14 (br.s, 1H), 7.87 (br.s, 1H), 7.18 ( d, J = 8.5 Hz, 1H), 7.00 - 6.95 (m, 1H), 6.86 - 6.79 (m, 1H), 4.50 (t, J = 5.2 Hz, 2H), 3.78 (t, J = 5.1 Hz, 2H), 3.36 (s, 3H). | 378.2 |
| 206 | (500 MHz, DMSO-d6) δ 11.27 (s, 1H), 8.95 (s, 1H), 8.33 (d, J = 2.9 Hz, 1H), 8.27 (dd, J = 4. 6, 1.4 Hz, 1H), 8.19 (s, 1H), 7.85 (d, J = 8.4 H z, 1H), 7.39 - 7.32 (m, 2H), 7.31 - 7.27 (m, 1H) , 7.18 (d, J = 8.5 Hz, 1H), 7.04 (d, J = 2.4 Hz, 1H), 6.78 (dd, J = 8.5, 2.4 Hz, 1H), 4.44 (t, J = 5.0 Hz, 2H), 3.78 (t, J = 5.0 Hz, 2H), 3.41 (s , 3H). | 435.3 |
| 207 | (500 MHz, DMSO-d6) δ 11.25 (s, 1H), 8.91 (s, 1H), 8.34 (d, J = 2.9 Hz, 1H), 8.28 (dd, J = 4. 6, 1.4 Hz, 1H), 8.22 (s, 1H), 7.85 (d, J = 8.5 H z, 1H), 7.40 - 7.28 (m, 3H), 7.21 (d, J = 2.4 H z, 1H), 7.18 (d, J = 8.5 Hz, 1H), 6.80 (dd, J = 8.4, 2.4 Hz, 1H), 4.40 (t, J = 4.9 Hz, 2H), 3.73 (t, J = 4.9 Hz, 2H), 3.38 (s, 3H). | 435.3 |
| 208 | (500 MHz, DMSO-d6) δ 11.29 (s, 1H), 8.65 (s, 1H), 8.20 (s, 1H), 7.85 (d, J = 8.5 Hz, 1H), 7.1 7 (d, J = 8.5 Hz, 1H), 7.15 - 7.09 (m, 1H), 6.8 5 (s, 1H), 6.61 (d, J = 8.4 Hz, 1H), 4.54 - 4.46 (m, 1H), 3.70 (s, 3H), 1.24 (d, J = 6.0 Hz, 6H) | 356.2 |
| 209 | (500 MHz, DMSO-d6) δ 11.24 (s, 1H), 8.58 (s, 1H), 8.23 (s, 1H), 7.84 (d, J = 8.4 Hz, 1H), 7.1 9 - 7.12 (m, 2H), 6.89 (d, J = 2.4 Hz, 1H), 6.6 1 (dd, J = 8.4, 2.4 Hz, 1H), 4.59 - 4.52 (m, 1H ), 3.70 (s, 3H), 1.27 (d, J = 6.1 Hz, 6H). | 356.2 |
| 210 | (DMSO-dB) δ 11.36 (br.s, 1H), 8.91 (s, 1H), 8.1 9 (d, J = 2.7 Hz, 1H), 7.86 (d, J = 8.5 Hz, 1H) , 7.33 (d, J = 8.5 Hz, 1H), 7.22 (dd, J = 2.4, 1 .2 Hz, 1H), 7.18 (d, J = 8.5 Hz, 1H), 6.97 (ddd, J = 8.6, 2.3, 1.0 Hz, 1H), 3.77 (s, 3H). | 382.1 |
| 211 | (DMSO-d6) δ 11.34 (d, J = 2.9 Hz, 1H), 8.88 (s , 1H), 8.21 (d, J = 2.8 Hz, 1H), 7.86 (d, J = 8. 5 Hz, 1H), 7.39 - 7.33 (m, 1H), 7.30 (d, J = 8. 5 Hz, 1H), 7.18 (d, J = 8.4 Hz, 1H), 6.97 (ddd, J = 8.5, 2.5, 1.2 Hz, 1H), 3.77 (s, 3H). | 382.2 |
| 212 | (DMSO-d6) δ 11.10 (s, 1H), 8.72 (s, 1H), 7.88 ( d, J = 2.5 Hz, 1H), 7.75 (d, J = 2.1 Hz, 1H), 7 .53 (d, J = 1.7 Hz, 1H), 7.48 (d, J = 8.3 Hz, 1 H), 7.41 (dd, J = 8.6, 0.6 Hz, 1H), 7.34 - 7.27 (m, 1H), 7.11 (dd, J = 8.6, 2.1 Hz, 1H), 4.29 (t, J = 7.2 Hz, 2H), 1.86 - 1.72 (m, 2H), 0.94 (t, J = 7.4 Hz, 3H). | 393.1 |
| 213 | (DMSO-d6) δ 11.09 (s, 1H), 8.77 (s, 1H), 7.92 ( d, J = 2.6 Hz, 1H), 7.78 (d, J = 2.1 Hz, 1H), 7 .56 (s, 1H), 7.43 - 7.37 (m, 2H), 7.31 (d, J = 8 .0 Hz, 1H), 7.11 (dd, J = 8.6, 2.1 Hz, 1H), 6.09 - 5.95 (m, 1H), 5.25 - 5.17 (m, 1H), 5.07 - 4. 99 (m, 3H). | 391.1 |
| 214 | (DMSO-d6) δ 11.11 (s, 1H), 8.98 (s, 1H), 7.95 ( d, J = 2.5 Hz, 1H), 7.84 (d, J = 2.1 Hz, 1H), 7 .58 (d, J = 1.6 Hz, 1H), 7.53 (d, J = 8.2 Hz, 1 H), 7.41 (dd, J = 8.7, 0.6 Hz, 1H), 7.39 - 7.36 (m, 1H), 7.12 (dd, J = 8.6, 2.1 Hz, 1H), 5.28 (d , J = 2.5 Hz, 2H), 3.45 - 3.43 (m, 1H). | 389.1 |
| 215 | (DMSO-d6) δ 11.10 (s, 1H), 8.75 (s, 1H), 7.90 ( d, J = 2.5 Hz, 1H), 7.81 - 7.76 (m, 1H), 7.56 - 7.48 (m, 2H), 7.41 (dd, J = 8.6, 0.6 Hz, 1H), 7.34 - 7.27 (m, 1H), 7.11 (dd, J = 8.6, 2.1 Hz, 1H), 4.28 (d, J = 7.0 Hz, 2H), 1.37 - 1.26 (m, 1H), 0.57 - 0.45 (m, 4H). | 405.2 |
| 216 | (DMSO-d6) δ 11.07 (s, 1H), 8.73 (s, 1H), 7.89 - 7.83 (m, 2H), 7.73 (d, J = 8.2 Hz, 1H), 7.58 - 7.52 (m, 1H), 7.39 (dd, J = 8.7, 0.6 Hz, 1H), 7.31 - 7.27 (m, 1H), 7.10 (dd, J = 8.6, 2.1 Hz, 1H), 3.07 (s, 6H). | 394.1 |
| 217 | (DMSO-d6) δ 11.08 (s, 1H), 8.76 (s, 1H), 7.91 ( d, J = 2.5 Hz, 1H), 7.81 (d, J = 2.1 Hz, 1H), 7 .55 - 7.53 (m, 1H), 7.47 (d, J = 8.3 Hz, 1H), 7. 40 (d, J = 8.7 Hz, 1H), 7.34 - 7.29 (m, 1H), 7. 11 (dd, J = 8.6, 2.1 Hz, 1H), 4.36 (q, J = 7.1 Hz, 2H), 1.33 (t, J = 7.1 Hz, 3H). | 379.1 |
| 218 | (DMSO-d6) δ 11.06 (s, 1 H), 8.69 (s, 1H), 7.91 ( d, J = 2.5 Hz, 1H), 7.80 (d, J = 2.1 Hz, 1H), 7 .66 (d, J = 8.3 Hz, 1H), 7.55 (d, J = 1.8 Hz, 1 H), 7.40 (d, J = 8.6 Hz, 1H), 7.28 - 7.24 (m, 1 H), 7.10 (dd, J = 8.6, 2.1 Hz, 1H), 5.10 - 5.02 (m, 1H), 1.61 (d, J = 6.7 Hz, 6H). | 393.1 |
| 218 | (DMSO-d6) δ 11.11 (d, J = 1.9 Hz, 1H), 8.67 (s , 1H), 7.85 (d, J = 2.4 Hz, 1H), 7.70 (d, J = 2. 1 Hz, 1H), 7.52 (d, J = 1.7 Hz, 1H), 7.48 (d, J = 8.3 Hz, 1H), 7.41 (d, J = 8.6 Hz, 1H), 7.33 - 7.26 (m, 1H), 7.11 (dd, J = 8.6, 2.1 Hz, 1H), 4.16 (d, J = 7.6 Hz, 2H), 2.30 - 2.20 (m, 1H), 0.94 (d, J = 6.6 Hz, 6H). | 407.2 |
| 220 | (DMSO-d6) δ 11.06 (d, J = 2.6 Hz, 1H), 8.66 (s , 1H), 7.90 (d, J = 2.5 Hz, 1H), 7.80 (d, J = 2. 1 Hz, 1H), 7.76 (d, J = 8.4 Hz, 1H), 7.57 (d, J = 1.8 Hz, 1H), 7.40 (dd, J = 8.6, 0.6 Hz, 1H), 7.37 - 7.28 (m, 1H), 7.11 (dd, J = 8.6, 2.1 Hz, 1H), 5.35 - 5.22 (m, 1H), 2.93 - 2.80 (m, 2H), 2.60 - 2.50 (m, 2H), 2.08 - 1.97 (m, 1H), 1.94 - 1.80 (m, 1H). | 405.2 |
| 221 | (DMSO-d6) δ 11.07 (s, 1H), 8.80 (s, 1H), 7.92 ( d, J = 2.5 Hz, 1H), 7.85 (d, J = 2.1 Hz, 1H), 7 .55 (d, J = 1.7 Hz, 1H), 7.45 (d, J = 8.2 Hz, 1 H), 7.40 (dd, J = 8.7, 0.6 Hz, 1H), 7.34 - 7.30 (m, 1H), 7.11 (dd, J = 8.7, 2.1 Hz, 1H). | 368.1 |
| 222 | (DMSO-d6) δ 11.09 (d, J = 2.5 Hz, 1H), 8.68 (s , 1H), 7.90 (d, J = 2.5 Hz, 1H), 7.77 (d, J = 2. 1 Hz, 1H), 7.53 (d, J = 1.7 Hz, 1H), 7.47 (d, J = 8.3 Hz, 1H), 7.40 (dd, J = 8.7, 0.5 Hz, 1H), 7.33 - 7.29 (m, 1H), 7.11 (dd, J = 8.6, 2.1 Hz, 1H), 4.52 (t, J = 5.2 Hz, 2H), 3.72 (t, J = 5.2 Hz, 2H), 3.27 (s, 3H). | 409.1 |
| 223 | (DMSO-d6) δ 11.07 (s, 1H), 8.78 (s, 1H), 7.90 ( d, J = 2.4 Hz, 1H), 7.76 (d, J = 2.1 Hz, 1H), 7 .54 (d, J = 1.7 Hz, 1H), 7.47 (d, J = 8.3 Hz, 1 H), 7.40 (dd, J = 8.6, 0.5 Hz, 1H), 7.34 - 7.27 (m, 1H), 7.11 (dd, J = 8.7, 2.1 Hz, 1H), 5.29 (s , 1H), 4.39 (t, J = 5.2 Hz, 2H), 3.85 - 3.79 (m, 2H). | 395.1 |
| 224 | (DMSO-d6) δ 11.10 (s, 1H), 8.67 (s, 1H), 7.89 ( d, J = 1.9 Hz, 1H), 7.83 (d, J = 2.5 Hz, 1H), 7 .51 (d, J = 1.7 Hz, 1H), 7.46 (d, J = 8.2 Hz, 1 H), 7.37 (dd, J = 8.6, 0.6 Hz, 1H), 7.34 - 7.28 (m, 1H), 7.22 (dd, J = 8.6, 1.9 Hz, 1 H), 3.31 - 3.24 (m, 1H), 1.36 - 1.27 (m, 2H), 1.09 - 1.01 (m, 2H). | 437.0 |
| 225 | (DMSO-d6) δ 11.11 (s, 1H), 8.89 (s, 1H), 7.86 ( d, J = 2.5 Hz, 1H), 7.81 (d, J = 2.1 Hz, 1H), 7 .44 - 7.42 (m, 1H), 7.40 (d, J = 8.7 Hz, 1H), 7 .22 - 7.17 (m, 1H), 7.11 (dd, J = 8.7, 2.1 Hz, 1H), 3.93 (s, 3H). | 383.1 |
| 226 | (DMSO-d6) δ 11.07 (s, 1H), 8.77 (s, 1H), 7.95 ( d, J = 8.3 Hz, 1H), 7.90 (d, J = 2.5 Hz, 1H), 7 .82 (d, J = 2.1 Hz, 1H), 7.64 (d, J = 1.7 Hz, 1 H), 7.46 - 7.41 (m, 1H), 7.40 (dd, J = 8.6, 0.6 Hz, 1H), 7.11 (dd, J = 8.6, 2.1 Hz, 1H), 6.05 - 5.97 (m, 1H), 5.18 - 5.10 (m, 4H). | 407.1 |
| 227 | (DMSO-d6) δ 11.13 (s, 1H), 9.56 (s, 1H), 7.97 ( dd, J = 1.4, 0.6 Hz, 1H), 7.90 (d, J = 2.5 Hz, 1H), 7.79 (d, J = 1.4 Hz, 1H), 7.64 (d, J = 1.7 Hz, 1H), 7.56 (d, J = 2.1 Hz, 1H), 7.48 (d, J = 8.3 Hz, 1H), 7.42 (dd, J = 8.7, 0.6 Hz, 1H), 7.3 9 - 7.32 (m, 1H), 7.13 (dd, J = 8.6, 2.1 Hz, 1H ), 3.83 (s, 3H). | 431.2 |
| 228 | (DMSO-d6) δ 11.10 (s, 1H), 8.94 (s, 1H), 7.95 ( d, J = 2.5 Hz, 1H), 7.85 (d, J = 2.0 Hz, 1H), 7 .57 (d, J = 1.6 Hz, 1H), 7.51 (d, J = 8.2 Hz, 1 H), 7.41 (dd, J = 8.6, 0.6 Hz, 1H), 7.39 - 7.33 (m, 1H), 7.12 (dd, J = 8.6, 2.1 Hz, 1H), 5.22 (q , J = 2.3 Hz, 2H), 1.84 - 1.75 (m, 3H). | 403.2 |
| 229 | (DMSO-d6) δ 11.08 (d, J = 2.6 Hz, 1 H), 8.70 (s , 1H), 7.90 (d, J = 2.4 Hz, 1H), 7.75 (d, J = 2. 1 Hz, 1H), 7.53 (d, J = 1.7 Hz, 1H), 7.40 (d, J = 8.6 Hz, 1H), 7.35 (d, J = 8.2 Hz, 1H), 7.27 ( dd, J = 8.4, 1.7 Hz, 1H), 7.10 (dd, J = 8.7, 2.1 Hz, 1H), 5.26 (s, 2H), 3.18 (s, 3H), 2.91 (s, 3 H). | 436.2 |
| 230 | (DMSO-d6) δ 11.10 (d, J = 2.4 Hz, 1H), 8.84 (s , 1H), 7.87 (d, J = 2.4 Hz, 1H), 7.80 (d, J = 2. 1 Hz, 1H), 7.55 (d, J = 1.7 Hz, 1H), 7.49 (d, J = 8.2 Hz, 1H), 7.41 (dd, J = 8.7, 0.5 Hz, 1H), 7.38 - 7.32 (m, 1H), 7.11 (dd, J = 8.6, 2.1 Hz, 1 H), 4.78 (t, J = 7.0 Hz, 2H), 3.71 (t, J = 6.9 Hz, 2H), 3.09 (s, 3H). | 457.0 |
| 231 | (DMSO-d6) δ 11.07 (s, 1H), 8.56 (s, 1H), 7.82 ( d, J = 2.5 Hz, 1H), 7.73 (d, J = 2.1 Hz, 1H), 7 .40 (dd, J = 8.7, 0.6 Hz, 1H), 7.33 (d, J = 8.5 Hz, 1H), 7.19 - 7.17 (m, 1H), 7.11 (dd, J = 8.6, 2.1 Hz, 1H), 6.96 (ddd, J = 8.5, 2.4, 1.0 Hz, 1 H), 3.28 - 3.20 (m, 1H), 1.34 - 1.24 (m, 2H), 1 .07 - 0.99 (m, 2H). | 407.1 |
| 232 | (DMSO-d6) δ 11.08 (s, 1H), 8.66 (s, 1H), 8.07 ( d, J = 1.7 Hz, 1H), 7.79 (d, J = 2.5 Hz, 1H), 7 .51 (s, 1H), 7.45 (d, J = 8.2 Hz, 1H), 7.37 (dd, J = 8.5, 1.7 Hz, 1H), 7.32 (d, J = 8.6 Hz, 1H), 7.28 - 7.22 (m, 1H), 3.30 - 3.27 (m, 1H), 1.34 - 1.29 (m, 2H), 1.09 - 1.01 (m, 2H). | 483.0 |
| 233 | (DMSO-d6) δ 11.12 (s, 1H), 8.74 (s, 1H), 7.80 ( d, J = 2.5 Hz, 1H), 7.72 (d, J = 2.1 Hz, 1H), 7 .44 - 7.36 (m, 2H), 7.23 - 7.15 (m, 1H), 7.12 ( dd, J = 8.6, 2.1 Hz, 1H), 3.44 - 3.36 (m, 1H), 1.35 - 1.26 (m, 2H), 1.14 - 1.09 (m, 2H). | 409.2 |
| 234 | (DMSO-d6) δ 11.05 (d, J = 2.3 Hz, 1H), 9.19 (s , 1H), 7.91 (d, J = 2.5 Hz, 1H), 7.62 - 7.54 (m, 3H), 7.41 (dd, J = 8.7, 0.5 Hz, 1H), 7.35 - 7.2 8 (m, 1H), 7.12 (dd, J = 8.6, 2.0 Hz, 1H), 5.91 (s, 1H), 4.20 (s, 2H), 1.29 (s, 6H). | 423.2 |
| 235 | (DMSO-d6) δ 11.12 (s, 1H), 8.61 (s, 1H), 7.81 ( d, J = 2.5 Hz, 1H), 7.76 - 7.68 (m, 2H), 7.53 ( d, J = 1.7 Hz, 1H), 7.41 (dd, J = 8.6, 0.6 Hz, 1H), 7.28 (ddd, J = 8.3, 1.8, 0.8 Hz, 1H), 7.12 (dd, J = 8.7, 2.1 Hz, 1H), 3.97 - 3.88 (m, 4H), 3.75 - 3.64 (m, 2H), 3.15 - 3.08 (m, 2H). | 436.2 |
| 236 | (DMSO-d6) δ 11.11 (s, 1H), 8.83 (s, 1H), 7.84 ( d, J = 2.2 Hz, 1H), 7.75 (d, J = 2.0 Hz, 1H), 7 .45 - 7.38 (m, 2H), 7.19 (d, J = 11.6 Hz, 1H), 7.12 (dd, J = 8.6, 2.1 Hz, 1H), 5.26 (br.s, 1H), 4.48 (t, J = 5.2 Hz, 2H), 3.84 (t, J = 5.2 Hz, 2 H). | 413.1 |
| 237 | (DMSO-d6) δ 11.12 (s, 1H), 8.92 (s, 1H), 7.88 - 7.81 (m, 2H), 7.48 - 7.44 (m, 1H), 7.40 (dd, J = 8.7, 0.6 Hz, 1H), 7.35 - 7.27 (m, 1H), 7.11 ( dd, J = 8.6, 2.1 Hz, 1H), 2.96 - 2.93 (m, 6H). | 412.1 |
| 238 | (DMSO-d6) δ 11.15 (s, 1H), 8.85 (s, 1H), 7.83 ( d, J = 2.4 Hz, 1H), 7.72 (d, J = 2.1 Hz, 1H), 7 .45 - 7.38 (m, 2H), 7.25 - 7.17 (m, 1H), 7.12 ( dd, J = 8.6, 2.1 Hz, 1H), 4.36 (t, J = 7.2 Hz, 2 H), 1.83 (h, J = 7.3 Hz, 2H), 0.95 (t, J = 7.4 H z, 3H). | 411.1 |
| 239 | (DMSO-d6) δ 11.07 (s, 1H), 8.86 (s, 1H), 7.88 ( d, J = 2.4 Hz, 1H), 7.68 (d, J = 2.1 Hz, 1H), 7 .54 (d, J = 1.7 Hz, 1H), 7.49 (d, J = 8.3 Hz, 1 H), 7.41 (dd, J = 8.6, 0.6 Hz, 1H), 7.34 - 7.27 (m, 1H), 7.11 (dd, J = 8.6, 2.1 Hz, 1H), 5.53 (s , 1H), 4.32 - 4.12 (m, 3H), 1.21 (d, J = 6.0 Hz , 3H). | 409.1 |
| 240 | (DMSO-d6) δ 11.14 (s, 1H), 8.89 (s, 1H), 7.86 ( d, J = 2.5 Hz, 1H), 7.77 (d, J = 2.1 Hz, 1H), 7 .46 - 7.38 (m, 2H), 7.26 - 7.18 (m, 1H), 7.12 ( dd, J = 8.6, 2.1 Hz, 1H), 4.43 (q, J = 7.1 Hz, 2H), 1.39 (t, J = 7.0 Hz, 3H). | 397.1 |
| 241 | (DMSO-d6) δ 11.17 (s, 1H), 9.12 (s, 1H), 7.93 - 7.88 (m, 1H), 7.83 - 7.78 (m, 1H), 7.48 (s, 1H) , 7.45 - 7.39 (m, 1H), 7.31 - 7.24 (m, 1H), 7.16 - 7.09 (m, 1H), 5.30 (br.s, 2H), 3.50 (br.s, 1H). | 407.1 |
| 242 | (DMSO-d6) 5 11.12 (s, 1H), 8.88 (s, 1H), 7.87 ( d, J = 2.4 Hz, 1H), 7.78 (d, J = 2.1 Hz, 1H), 7 .49 - 7.44 (m, 1H), 7.40 (dd, J = 8.6, 0.6 Hz, 1 H), 7.29 - 7.21 (m, 1H), 7.11 (dd, J = 8.6, 2.1 Hz, 1H), 5.14 -5.04 (m, 1H), 1.58 - 1.53 (m, 6H ). | 411.1 |
| 243 | (DMSO-d6) δ 11.07 (s, 1 H), 8.85 (s, 1H), 7.88 ( d, J = 2.4 Hz, 1H), 7.67 (d, J = 2.1 Hz, 1H), 7 .56 - 7.53 (m, 1H), 7.49 (d, J = 8.3 Hz, 1H), 7. 41 (dd, J = 8.6, 0.6 Hz, 1H), 7.34 - 7.27 (m, 1 H), 7.11 (dd, J = 8.6, 2.1 Hz, 1H), 5.53 (s, 1H) , 4.32 - 4.11 (m, 3H), 1.21 (d, J = 6.0 Hz, 3H) | 409.1 |
| 244 | (DMSO-d6) δ 11.09 (d, J = 2.6 Hz, 1H), 8.65 (s , 1H), 7.86 (d, J = 2.5 Hz, 1H), 7.75 (d, J = 2. 1 Hz, 1H), 7.40 (dd, J = 8.7, 0.6 Hz, 1H), 7.35 (d, J = 8.5 Hz, 1H), 7.22 - 7.16 (m, 1H), 7.11 ( dd, J = 8.6, 2.1 Hz, 1H), 6.98 - 6.91 (m, 1H), 4.24 (t, J = 7.3 Hz, 2H), 1.77 (h, J = 7.3 Hz, 2 H), 0.94 (t, J = 7.4 Hz, 3H). | 409.1 |
| 245 | (DMSO-d6) δ 11.04 (d, J = 2.6 Hz, 1H), 8.70 (s , 1H), 7.88 (d, J = 2.4 Hz, 1H), 7.75 (d, J = 2. 1 Hz, 1H), 7.45 - 7.30 (m, 2H), 7.21 (dd, J = 2 .7, 1.3 Hz, 1H), 7.10 (dd, J = 8.6, 2.1 Hz, 1H), 6.97 - 6.92 (m, 1H), 5.29 (t, J = 5.0 Hz, 1H), 4 .34 (t, J = 5.3 Hz, 2H), 3.80 (q, J = 5.2 Hz, 2 H). | 411.0 |
| 246 | (DMSO-d6) δ 11.05 (s, 1H), 8.72 (s, 1H), 7.90 ( d, J = 2.5 Hz, 1H), 7.85 (d, J = 2.1 Hz, 1H), 7 .39 (dd, J = 8.7, 0.6 Hz, 1H), 7.32 (d, J = 8.5 Hz, 1H), 7.23 - 7.20 (m, 1H), 7.10 (dd, J = 8.7, 2.1 Hz, 1H), 6.97 (ddd, J = 8.5, 2.4, 1.0 Hz, 1 H), 3.76 (s, 3H). | 381.1 |
| 247 | (DMSO-d6) δ 11.09 (s, 1H), 8.69 (s, 1 H), 7.87 ( d, J = 2.4 Hz, 1H), 7.83 (d, J = 2.1 Hz, 1H), 7 .58 - 7.52 (m, 2H), 7.40 (dd, J = 8.6, 0.6 Hz, 1H), 7.33 - 7.26 (m, 1H), 7.11 (dd, J = 8.6, 2.1 Hz, 1H), 3.45 - 3.37 (m, 4H), 2.18 - 2.02 (m, 4H). | 420.1 |
| 248 | (DMSO-d6) δ 11.05 (s, 1H), 8.67 (s, 1H), 7.90 ( d, J = 2.5 Hz, 1H), 7.83 (d, J = 2.1 Hz, 1H), 7 .43 - 7.37 (m, 2H), 7.11 (dd, J = 8.6, 2.1 Hz, 1H), 7.06 (d, J = 1.5 Hz, 1H), 3.99 (s, 3H), 2.7 4 (s, 3H). | 379.1 |
| 249 | (DMSO-d6) δ 11.12 (s, 1H), 8.92 (s, 1H), 7.85 ( d, J = 2.6 Hz, 1H), 7.80 (d, J = 2.1 Hz, 1H), 7 .54 - 7.51 (m, 1H), 7.40 (d, J = 8.6 Hz, 1H), 7 .30 (s, 1H), 7.11 (dd, J = 8.7, 2.1 Hz, 1H), 4.0 6 (s, 3H). | 399.1 |
| 250 | (DMSO-d6) δ 11.12 (s, 1H), 9.03 (s, 1H), 7.95 ( d, J = 2.5 Hz, 1H), 7.86 (d, J = 2.1 Hz, 1H), 7 .68 (s, 1H), 7.45 - 7.37 (m, 2H), 7.12 (dd, J = 8.7, 2.1 Hz, 1H), 3.82 (s, 3H). | 399.0 |
| 251 | (DMSO-d6) 5 11.06 (s, 1H), 8.68 (s, 1H), 7.89 ( d, J = 2.5 Hz, 1H), 7.81 (d, J = 2.1 Hz, 1H), 7 .40 (d, J = 8.6 Hz, 1H), 7.35 (d, J = 8.5 Hz, 1 H), 7.22 - 7.19 (m, 1H), 7.10 (dd, J = 8.6, 2.1 Hz, 1H), 6.99 - 6.92 (m, 1H), 4.32 (q, J = 7.0 Hz, 2H), 1.32 (t, J = 7.0 Hz, 3H). | 395.1 |
| 252 | (DMSO-d6) δ 11.03 (s, 1H), 8.60 (s, 1H), 7.89 ( d, J = 2.5 Hz, 1H), 7.79 (d, J = 2.1 Hz, 1H), 7 .53 (d, J = 8.7 Hz, 1H), 7.39 (d, J = 8.6 Hz, 1 H), 7.22 - 7.20 (m, 1H), 7.10 (dd, J = 8.7, 2.1 Hz, 1H), 6.93 - 6.88 (m, 1H), 5.06 - 4.98 (m, 1H), 1.59 (d, J = 6.8 Hz, 6H). | 409.1 |
| 253 | (DMSO-d6) δ 11.10 - 11.06 (br.s, 1H), 8.89 (s, 1H), 7.93 (d, J = 2.5 Hz, 1H), 7.84 (d, J = 2.1 Hz, 1H), 7.40 (d, J = 8.6 Hz, 2H), 7.26 - 7.24 (m, 1H), 7.11 (dd, J = 8.7, 2.1 Hz, 1H), 7.03 - 6.99 (m, 1H), 5.24 (d, J = 2.5 Hz, 2H), 3.43 (t, J = 2.4 Hz, 1H). | 405.1 |
| 254 | (DMSO-d6) δ 11.10 (s, 1H), 8.86 (s, 1H), 7.91 ( d, J = 2.5 Hz, 1H), 7.85 (d, J = 2.1 Hz, 1H), 7 .56 - 7.48 (m, 2H), 7.40 (d, J = 8.7 Hz, 1H), 7 .11 (dd, J = 8.7, 2.1 Hz, 1H), 3.78 (s, 3H). | 383.1 |
| 255 | (Methanol-d4) δ 7.76 (d, J = 8.5 Hz, 1H), 7.73 - 7.66 (m, 1H), 7.64 (s, 1H), 7.59 - 7.55 (m, 2 H), 7.48 (dd, J = 8.7, 0.6 Hz, 1H), 7.23 (dd, J = 8.7, 2.0 Hz, 1H), 3.05 (s, 3H). | 380.1 |
| 256 | (DMSO-d6) δ 10.79 (s, 1H), 8.82 (s, 1H), 7.68 ( d, J = 2.5 Hz, 1H), 7.49 - 7.44 (m, 1H), 7.41 - 7.38 (m, 1H), 7.26 (d, J = 8.3 Hz, 1H), 7.19 ( d, J = 11.3 Hz, 1H), 6.97 - 6.91 (m, 1H), 3.92 (br.s, 3H), 2.38 (s, 3H). | 363.1 |
| 257 | (DMSO-d6) δ 11.50 (s, 1H), 9.04 (s, 1H), 8.37 - 8.35 (m, 1H), 8.02 (d, J = 2.4 Hz, 1H), 7.56 (dd, J = 8.4, 0.7 Hz, 1H), 7.48 - 7.44 (m, 2H), 7.23 (d, J = 11.7 Hz, 1H), 3.95 (br.s, 3H). | 374.2 |
| 258 | (DMSO-d6) δ 11.05 (s, 1H), 8.65 (s, 1H), 7.86 - 7.83 (m, 2H), 7.60 (d, J = 8.5 Hz, 1H), 7.38 (dd, J = 8.6, 0.5 Hz, 1H), 7.23 - 7.21 (m, 1H), 7.09 (dd, J = 8.6, 2.1 Hz, 1H), 6.95 - 6.90 (m, 1H), 3.05 (s, 6H). | 410.1 |
| 259 | (DMSO-d6) δ 11.11 (s, 1H), 8.86 (s, 1H), 7.87 ( d, J = 2.5 Hz, 1H), 7.83 (d, J = 2.1 Hz, 1H), 7 .62 (d, J = 8.3 Hz, 1H), 7.55 (d, J = 1.7 Hz, 1 H), 7.41 (dd, J = 8.7, 0.6 Hz, 1H), 7.37 - 7.30 (m, 1H), 7.11 (dd, J = 8.6, 2.1 Hz, 1H), 4.66 (t, J = 6.8 Hz, 2H), 3.07 (t, J = 6.8 Hz, 2H). | 404.1 |
| 260 | (DMSO-d6) δ 11.08 (s, 1H), 8.19 (s, 1H), 7.91 ( d, J = 2.5 Hz, 1H), 7.75 (d, J = 2.1 Hz, 1H), 7 .55 (d, J = 1.7 Hz, 1H), 7.47 (d, J = 8.2 Hz, 1 H), 7.40 (dd, J = 8.6, 0.5 Hz, 1H), 7.36 - 7.29 (m, 1H), 7.11 (dd, J = 8.6, 2.0 Hz, 1H), 4.99 (t, J = 4.9 Hz, 1H), 4.36 (t, J = 6.7 Hz, 2H), 3.4 8 (q, J = 5.9 Hz, 2H), 1.98 - 1.88 (m, 2H). | 409.1 |
| 261 | (DMSO-d6) δ 11.07 (s, 1H), 8.87 (s, 1H), 7.83 ( d, J = 2.5 Hz, 1H), 7.70 (dd, J = 11.5, 8.1 Hz, 1H), 7.45 - 7.35 (m, 2H), 7.23 - 7.17 (m, 1H), 3.94 - 3.91 (br.s, 3H). | 385.1 |
| 262 | (DMSO-d6) δ 11.10 (s, 1H), 8.83 (s, 1H), 8.00 ( d, J = 7.5 Hz, 1H), 7.93 (d, J = 2.5 Hz, 1H), 7 .58 - 7.55 (m, 1H), 7.45 (d, J = 8.3 Hz, 1H), 7 .39 (d, J = 10.2 Hz, 1H), 7.36 - 7.32 (m, 1H), 3.80 (s, 3H). | 383.1 |
| 263 | (DMSQ-cΣδ) δ 11.02 (s, 1H), 8.66 (s, 1H), 7.92 ( d, J = 2.5 Hz, 1H), 7.86 (d, J = 2.1 Hz, 1H), 7 .44 (s, 1H), 7.39 (d, J = 8.6 Hz, 1H), 7.22 (s, 1H), 7.10 (dd, J = 8.7, 2.1 Hz, 1H), 3.91 (s, 3H ), 3.78 (s, 2H). | 395.1 |
| 264 | (DMSO-d6) δ 11.18 (s, 1H), 9.17 (s, 1H), 7.88 ( d, J = 2.6 Hz, 1H), 7.85 - 7.83 (m, 1H), 7.82 ( d, J = 2.1 Hz, 1H), 7.80 - 7.78 (m, 1H), 7.41 ( dd, J = 8.7, 0.6 Hz, 1H), 7.12 (dd, J = 8.6, 2.1 Hz, 1H), 4.05 (s, 3H). | 390.1 |
| 265 | (DMSO-d6) δ 11.06 (s, 1H), 8.64 (s, 1H), 7.87 - 7.82 (m, 2H), 7.55 (d, J = 8.3 Hz, 1H), 7.38 (d, J = 8.6 Hz, 1H), 7.28 (d, J = 2.0 Hz, 1H), 7.09 (dd, J = 8.6, 2.1 Hz, 1H), 6.97 (dd, J = 8. 3, 2.0 Hz, 1H), 3.03 (s, 6H). | 360.1 |
| 266 | (DMSO-d6) δ 11.09 (s, 1H), 8.78 (s, 1H), 7.87 - 7.83 (m, 2H), 7.83 - 7.78 (m, 1H), 7.43 - 7.3 7 (m, 2H), 7.35 (dd, J = 8.4, 1.7 Hz, 1H), 7.10 (dd, J = 8.6, 2.1 Hz, 1H), 3.08 (s, 6H). | 394.1 |
| 267 | (DMSO-d6) δ 11.31 (s, 1H), 11.17 (s, 1H), 9.50 (s, 1H), 7.81 (d, J = 2.5 Hz, 1H), 7.65 (d, J = 2.1 Hz, 1H), 7.42 (d, J = 8.7 Hz, 1H), 7.34 (s, 1H), 7.19 (d, J = 11.0 Hz, 1H), 7.13 (dd, J = 8 .6, 2.1 Hz, 1H). | 369.1 |
| 268 | (DMSO-d6) δ 11.16 - 10.99 (m, 2H), 9.39 - 9.2 9 (m, 1H), 7.84 - 7.81 (m, 1H), 7.69 - 7.65 (m , 1H), 7.54 - 7.45 (m, 1H), 7.43 - 7.31 (m, 2H) , 7.31 - 7.21 (m, 1H), 7.15 - 7.09 (m, 1H). | 351.0 |
| 269 | (DMSO-d6) δ 11.09 (s, 1H), 8.83 (s, 1H), 7.86 - 7.81 (m, 2H), 7.43 - 7.36 (m, 2H), 7.21 - 7.1 4 (m, 1H), 7.10 (dd, J = 8.6, 2.1 Hz, 1H), 6.12 (s, 2H). | 384.1 |
| 270 | (DMSO-d6) δ 11.12 (s, 1H), 8.93 (s, 1H), 7.98 ( d, J = 2.0 Hz, 1H), 7.82 (d, J = 2.6 Hz, 1H), 7 .42 (d, J = 1.3 Hz, 1H), 7.38 - 7.32 (m, 1H), 7 .27 - 7.18 (m, 2H), 6.52 (q, J = 5.5 Hz, 1H), 2 .89 - 2.83 (m, 3H). | 444.0 |
| 271 | (DMSO-d6) δ 11.12 (s, 1H), 8.91 (s, 1H), 7.85 - 7.80 (m, 2H), 7.44 - 7.36 (m, 2H), 7.23 (d, J = 10.8 Hz, 1H), 7.10 (dd, J = 8.7, 2.1 Hz, 1H) , 6.52 (q, J = 5.4 Hz, 1H), 2.89 - 2.83 (m, 3H) | 398.1 |
| 272 | (DMSO-d6) δ 11.32 (s, 1H), 8.44 (s, 1H), 7.65 ( d, J = 2.6 Hz, 1H), 7.39 - 7.36 (m, 1H), 7.26 - 7.17 (m, 2H), 7.17 - 7.10 (m, 1H), 6.59 (q, J = 5.4 Hz, 1H), 2.87 - 2.81 (m, 3H). | 400.1 |
| 273 | (DMSO-d6) δ 11.05 (s, 1H), 8.87 (s, 1H), 7.81 ( d, J = 2.5 Hz, 1H), 7.75 (dd, J = 11.7, 8.1 Hz, 1H), 7.43 - 7.41 (m, 1H), 7.38 (dd, J = 11.3, 7. 0 Hz, 1H), 7.23 (d, J = 10.5 Hz, 1H), 6.51 (q, J = 5.4 Hz, 1H), 2.88 - 2.84 (m, 3H). | 400.1 |
| 274 | (DMSO-d6) δ 11.12 (s, 1H), 8.94 (s, 1H), 7.85 ( d, J = 2.5 Hz, 1H), 7.81 (d, J = 2.1 Hz, 1H), 7 .44 - 7.36 (m, 2H), 7.11 (dd, J = 8.7, 2.1 Hz, 1H), 3.94 - 3.92 (m, 3H). | 401.1 |
| 275 | (DMSO-d6) δ 11.07 (s, 1H), 8.75 (s, 1H), 7.88 - 7.78 (m, 3H), 7.52 (d, J = 6.4 Hz, 1H), 7.38 (d, J = 8.7 Hz, 1H), 7.10 (dd, J = 8.6, 2.1 Hz, 1H), 3.05 (s, 6H). | 412.1 |
| 276 | (DMSO-d6) δ 11.01 (s, 1H), 8.78 (s, 1H), 8.06 ( d, J = 2.5 Hz, 1H), 7.95 (d, J = 2.1 Hz, 1H), 7 .40 (d, J = 8.7 Hz, 1H), 7.31 (d, J = 8.4 Hz, 1 H), 7.24 (d, J = 8.3 Hz, 1H), 7.11 (dd, J = 8.6, 2.1 Hz, 1H), 3.77 (s, 3H), 2.60 - 2.56 (m, 3H) | 379.2 |
| 277 | (DMSO-d6) δ 10.78 - 10.74 (m, 1H), 8.62 (s, 1 H), 7.66 (d, J = 2.5 Hz, 1H), 7.44 (d, J = 1.5 Hz, 1H), 7.39 (d, J = 1.4 Hz, 1H), 7.26 (d, J = 8.2 Hz, 1H), 7.24 - 7.19 (m, 1H), 6.94 (dd, J = 8.3, 1.7 Hz, 1H), 6.53 (q, J = 5.4 Hz, 1H), 2.8 8 - 2.84 (m, 3H), 2.38 (s, 3H). | 378.1 |
| 278 | (DMSO-d6) δ 11.33 (s, 1H), 8.54 (s, 1H), 7.65 ( d, J = 2.6 Hz, 1H), 7.40 (s, 1H), 7.30 (d, J = 1 0.6 Hz, 1H), 7.20 (dd, J = 8.9, 3.5 Hz, 1H), 7.1 6 - 7.07 (m, 1H), 2.95 - 2.91 (m, 6H). | 414.1 |
| 279 | (DMSO-d6) δ 11.07 (s, 1H), 8.88 (s, 1H), 7.82 - 7.72 (m, 2H), 7.46 - 7.43 (m, 1H), 7.38 (dd, J = 11.2, 6.9 Hz, 1H), 7.34 - 7.27 (m, 1H), 2.9 6 - 2.92 (m, 6H). | 414.1 |
| 280 | (DMSO-d6) δ 10.77 (s, 1H), 8.68 (s, 1H), 7.67 ( d, J = 2.5 Hz, 1H), 7.48 - 7.41 (m, 2H), 7.32 - 7.24 (m, 2H), 6.94 (dd, J = 8.4, 1.7 Hz, 1H), 2.96 - 2.92 (m, 6H), 2.38 (s, 3H). | 392.2 |
| 281 | (DMSO-d6) δ 11.07 (d, J = 2.5 Hz, 1H), 8.81 (s , 1H), 7.88 (d, J = 2.5 Hz, 1H), 7.84 (d, J = 2. 1 Hz, 1H), 7.40 (d, J = 8.6 Hz, 1H), 7.28 (dd, J = 8.0, 1.0 Hz, 1H), 7.10 (dd, J = 8.6, 2.1 Hz, 1H), 3.74 (s, 3H). | 459.0 |
| 282 | (DMSO-d6) δ 11.30 (s, 1H), 8.33 (s, 1H), 7.70 ( d, J = 2.6 Hz, 1H), 7.39 - 7.36 (m, 1H), 7.23 - 7.08 (m, 3H), 6.25 (s, 2H), | 386.1 |
| 283 | (DMSO-d6) δ 11.04 (s, 1H), 8.85 (s, 1H), 7.83 ( d, J = 2.6 Hz, 1H), 7.76 (dd, J = 11.7, 8.1 Hz, 1H), 7.43 - 7.34 (m, 2H), 7.17 (d, J = 11.0 Hz, 1H), 6.14 (s, 2H). | 386.1 |
| 284 | (DMSO-d6) δ 10.74 (d, J = 2.6 Hz, 1H), 8.53 (s , 1H), 7.68 (d, J = 2.5 Hz, 1H), 7.47 (s, 1H), 7. 38 (d, J = 1.4 Hz, 1H), 7.26 (d, J = 8.3 Hz, 1H ), 7.15 (dd, J = 11.1, 1.5 Hz, 1H), 6.94 (dd, J = 8.3, 1.6 Hz, 1H), 6.14 (s, 2H), 2.38 (s, 3H). | 364.1 |
| 285 | (DMSO-d6) δ 11.03 (s, 1H), 8.73 (s, 1H), 7.86 - 7.73 (m, 3H), 7.51 (d, J = 6.4 Hz, 1H), 7.37 (dd, J = 11.3, 7.0 Hz, 1H), 3.05 (s, 6H). | 414.1 |
| 286 | (DMSO-d6) δ 11.29 (s, 1H), 8.32 (s, 1H), 7.83 ( d, J = 11.1 Hz, 1H), 7.66 (d, J = 2.6 Hz, 1H), 7.48 (d, J = 6.4 Hz, 1H), 7.19 (dd, J = 8.8, 3.4 Hz, 1H), 7.15 - 7.07 (m, 1H), 3.05 (s, 6H). | 414.1 |
| 287 | (DMSO-d6) δ 10.72 (s, 1H), 8.48 (s, 1H), 7.81 ( d, J = 11.1 Hz, 1H), 7.68 (d, J = 2.5 Hz, 1H), 7.50 (d, J = 6.4 Hz, 1H), 7.46 (s, 1H), 7.25 (d, J = 8.3 Hz, 1H), 6.93 (dd, J = 8.4, 1.7 Hz, 1H) , 3.05 (s, 6H), 2.38 (s, 3H). | 392.2 |
| 288 | (Methanol-d4) δ 7.70 - 7.60 (m, 2H), 7.58 (dd, J = 2.0, 0.6 Hz, 1H), 7.53 (d, J = 5.7 Hz, 1H), 7.48 (dd, J = 8.7, 0.6 Hz, 1H), 7.23 (dd, J = 8. 7, 2.0 Hz, 1H), 3.03 (s, 3H). | 398.1 |

### Test example 1

Inhibition test of intracellular human STING (hSTING) pathway using reporter cells Since STING activates the transcription factor IRF3 upon ligand stimulation, the activity of STING can be evaluated by a reporter assay using a secretory alkaline phosphatase (SEAP reporter) integrated downstream of an IRF-inducible promoter.

That is, the hSTING inhibitory activity of the test compound was evaluated using HEK-Blue^{™} ISG cells (manufactured by Invivogen, #hkb-isg-1) incorporating a SEAP reporter. Activation of hSTING was performed by stimulation with the small molecule ligand Compound 3 as described in the literature (Ramanjulu, J. M., et al., Nature. 2018, 564 (7736), 439-443).

HEK-Blue^{™} ISG cells were seeded in a 96-well plate and cultured overnight at 37°C in a 5% CO₂ incubator. To each well of this cell culture plate, a test compound solution adjusted to a final concentration of 0.1 to 10 µM of the test compound was added, cultured for 1 hour in a c incubator, and then Compound 3 (final concentration 10 nM) was added and further cultured in a CO₂ incubator for 21 hours. After collecting the culture supernatant of each well, the reporter activity was measured by color development reaction with alkaline phosphatase.

### (Evaluation method for inhibitory activity)

The reporter activity of the test compound non-addition and Compound 3 addition group was set to 100%, and the reporter activity of the test compound non-addition and Compound 3 non-addition group was set to 0%. The IC₅₀ value was determined by regression analysis of the inhibitory rate determined from the reporter activity at each compound concentration and the test compound concentration (logarithm).

### (Evaluation results)

Table 5 shows the inhibitory activity against hSTING of representative compounds of the present invention. The hSTING inhibitory action is indicated by *** for less than 0.1 µM, ** for 0.1 µM to less than 1 µM, * for 1 µM to less than 10 µM, and 10 µM or more is indicated by -.

### [Table 5]

**[Table 5]**

| Test compound (Embodiment number) | hSTING inhibitory activity |
|---|---|
| 1 | ∗ ∗ |
| 2 | - |
| 3 | ∗ ∗ |
| 4 | ∗ ∗ ∗ |
| 5 | ∗ ∗ |
| 6 | ∗ ∗ |
| 7 | ∗ ∗ ∗ |
| 8 | ∗ ∗ |
| 9 | - |
| 10 | ∗ ∗ |
| 11 | ∗ |
| 12 | ∗ ∗ |
| 13 | ∗ ∗ |
| 14 | - |
| 15 | ∗ |
| 16 | ∗ ∗ |
| 17 | - |
| 18 | ∗ ∗ |
| 19 | ∗ ∗ |
| 20 | ∗ ∗ |
| 21 | - |
| 22 | ∗ ∗ |
| 23 | ∗ ∗ |
| 24 | ∗ ∗ |
| 25 | ∗ ∗ |
| 26 | ∗ ∗ |
| 27 | - |
| 28 | - |
| 29 | - |
| 30 | ∗ ∗ |
| 31 | ∗ ∗ |
| 32 | ∗ ∗ |
| 33 | ∗ ∗ ∗ |
| 34 | ∗ ∗ |
| 35 | ∗ ∗ ∗ |
| 36 | ∗ ∗ |
| 37 | - |
| 38 | ∗ ∗ |
| 39 | ∗ ∗ |
| 40 | ∗ ∗ ∗ |
| 41 | ∗ ∗ ∗ |
| 42 | - |
| 43 | ∗ ∗ ∗ |
| 44 | ∗ ∗ |
| 45 | ∗ ∗ |
| 46 | - |
| 47 | ∗ ∗ |
| 48 | ∗ |
| 49 | ∗ |
| 50 | ∗ |
| 51 | ∗ ∗ |
| 52 | ∗ ∗ |
| 53 | ∗ ∗ |
| 54 | ∗ ∗ |
| 55 | ∗ ∗ |
| 56 | ∗ ∗ ∗ |
| 57 | ∗ ∗ ∗ |
| 58 | ∗ ∗ |
| 59 | ∗ ∗ ∗ |
| 60 | ∗ ∗ ∗ |
| 61 | ∗ |
| 62 | ∗ ∗ |
| 63 | ∗ ∗ ∗ |
| 64 | ∗ ∗ |
| 65 | ∗ ∗ |
| 66 | ∗ |
| 67 | ∗ ∗ |
| 63 | - |
| 69 | ∗ ∗ |
| 70 | - |
| 71 | ∗ ∗ |
| 72 | ∗ ∗ |
| 73 | ∗ ∗ ∗ |
| 74 | ∗ |
| 75 | ∗ ∗ |
| 76 | - |
| 77 | ∗ ∗ |
| 78 | ∗ ∗ |
| 79 | ∗ ∗ |
| 80 | ∗ ∗ ∗ |
| 81 | ∗ |
| 82 | ∗ ∗ |
| 83 | - |
| 84 | ∗ ∗ |
| 85 | ∗ ∗ |
| 86 | ∗ ∗ |
| 87 | ∗ ∗ |
| 88 | ∗ ∗ ∗ |
| 89 | ∗ ∗ ∗ |
| 90 | ∗ |
| 91 | ∗ ∗ ∗ |
| 92 | ∗ |
| 93 | ∗ ∗ ∗ |
| 94 | ∗ ∗ ∗ |
| 95 | ∗ ∗ |
| 96 | ∗ ∗ |
| 97 | ∗ |
| 98 | - |
| 99 | ∗ ∗ |
| 100 | ∗ ∗ ∗ |
| 101 | ∗ ∗ ∗ |
| 102 | - |
| 103 | ∗ ∗ |
| 104 | ∗ ∗ |
| 105 | ∗ ∗ |
| 106 | ∗ |
| 107 | - |
| 108 | ∗ ∗ |
| 109 | ∗ ∗ |
| 110 | ∗ |
| 111 | ∗ ∗ ∗ |
| 112 | ∗ ∗ |
| 113 | - |
| 114 | ∗ ∗ ∗ |
| 115 | ∗ ∗ |
| 116 | ∗ ∗ ∗ |
| 117 | ∗ ∗ ∗ |
| 118 | ∗ ∗ ∗ |
| 119 | ∗ ∗ ∗ |
| 120 | ∗ ∗ ∗ |
| 121 | ∗ |
| 122 | ∗ |
| 123 | ∗ ∗ ∗ |
| 124 | ∗ ∗ |
| 125 | ∗ ∗ |
| 126 | ∗ ∗ |
| 127 | ∗ ∗ |
| 128 | ∗ ∗ |
| 129 | ∗ ∗ |
| 130 | ∗ ∗ |
| 131 | ∗ ∗ |
| 132 | ∗ |
| 133 | ∗ ∗ |
| 134 | - |
| 135 | - |
| 136 | ∗ ∗ |
| 137 | ∗ ∗ ∗ |
| 138 | - |
| 139 | |
| 140 | - |
| 141 | ∗ |
| 142 | - |
| 143 | - |
| 144 | ∗ ∗ |
| 145 | ∗ ∗ |
| 146 | - |
| 147 | - |
| 148 | - |
| 149 | ∗ |
| 150 | - |
| 151 | ∗ ∗ |
| 152 | - |
| 153 | - |
| 154 | - |
| 155 | - |
| 156 | - |
| 157 | ∗ ∗ |
| 158 | ∗ ∗ |
| 159 | ∗ |
| 160 | ∗ ∗ ∗ |
| 161 | - |
| 162 | ∗ ∗ |
| 163 | - |
| 164 | ∗ |
| 165 | ∗ |
| 166 | - |
| 167 | - |
| 168 | - |
| 169 | ∗ |
| 170 | - |
| 171 | * |
| 172 | - |
| 173 | ∗ |
| 174 | - |
| 175 | ∗ ∗ |
| 176 | ∗ |
| 177 | ∗ |
| 178 | - |
| 179 | - |
| 180 | - |
| 181 | - |
| 182 | - |
| 183 | - |
| 184 | - |
| 185 | ∗ ∗ |
| 186 | ∗ |
| 187 | ∗ ∗ |
| 188 | ∗ ∗ |
| 189 | ∗ ∗ |
| 190 | ∗ |
| 191 | ∗ ∗ |
| 192 | ∗ ∗ |
| 193 | ∗ ∗ |
| 194 | ∗ ∗ |
| 195 | ∗ ∗ ∗ |
| 196 | ∗ ∗ ∗ |
| 197 | ∗ ∗ |
| 198 | - |
| 199 | - |
| 200 | - |
| 201 | - |
| 202 | - |
| 203 | - |
| 204 | - |
| 205 | - |
| 206 | - |
| 207 | - |
| 208 | ∗ |
| 209 | ∗ |
| 210 | ∗ ∗ |
| 211 | - |
| 212 | ∗ ∗ ∗ |
| 213 | ∗ ∗ ∗ |
| 214 | ∗ ∗ ∗ |
| 215 | ∗ ∗ ∗ |
| 216 | ∗ ∗ ∗ |
| 217 | ∗ ∗ ∗ |
| 218 | ∗ ∗ ∗ |
| 219 | ∗ ∗ |
| 220 | ∗ ∗ |
| 221 | ∗ ∗ ∗ |
| 222 | ∗ ∗ ∗ |
| 223 | ∗ ∗ ∗ |
| 224 | ∗ ∗ ∗ |
| 225 | ∗ ∗ ∗ |
| 226 | ∗ ∗ |
| 227 | - |
| 228 | ∗ ∗ |
| 229 | - |
| 230 | * |
| 231 | ∗ ∗ ∗ |
| 232 | ∗ ∗ |
| 233 | ∗ ∗ ∗ |
| 234 | ∗ |
| 235 | ∗ |
| 236 | ∗ ∗ ∗ |
| 237 | ∗ ∗ ∗ |
| 238 | ∗ ∗ ∗ |
| 239 | ∗ ∗ |
| 240 | ∗ ∗ ∗ |
| 241 | ∗ ∗ ∗ |
| 242 | ∗ ∗ ∗ |
| 243 | ∗ ∗ |
| 244 | ∗ ∗ ∗ |
| 245 | ∗ ∗ |
| 246 | ∗ ∗ ∗ |
| 247 | ∗ ∗ |
| 248 | ∗ ∗ ∗ |
| 249 | ∗ ∗ ∗ |
| 250 | ∗ ∗ |
| 251 | ∗ ∗ ∗ |
| 252 | ∗ ∗ ∗ |
| 253 | ∗ ∗ ∗ |
| 254 | ∗ ∗ ∗ |
| 255 | ∗ ∗ ∗ |
| 256 | ∗ ∗ ∗ |
| 257 | ∗ ∗ ∗ |
| 258 | ∗ ∗ ∗ |
| 259 | ∗ ∗ |
| 260 | ∗ ∗ |
| 261 | ∗ ∗ ∗ |
| 262 | ∗ ∗ ∗ |
| 263 | ∗ ∗ |
| 264 | ∗ ∗ ∗ |
| 265 | ∗ ∗ ∗ |
| 266 | ∗ ∗ |
| 267 | ∗ ∗ |
| 268 | ∗ ∗ |
| 269 | ∗ ∗ ∗ |
| 270 | ∗ ∗ ∗ |
| 271 | ∗ ∗ ∗ |
| 272 | ∗ ∗ ∗ |
| 273 | ∗ ∗ ∗ |
| 274 | ∗ ∗ ∗ |
| 275 | ∗ ∗ ∗ |
| 276 | ∗ ∗ ∗ |
| 277 | ∗ ∗ ∗ |
| 278 | ∗ ∗ |
| 279 | ∗ ∗ |
| 280 | ∗ ∗ ∗ |

This result indicates that the compound (I) of the present invention has strong STING pathway inhibitory activity.

### Test example 2

Cytokine production suppression test using STING agonist-stimulated mouse model

CMA (10-Carboxymethyl-9-acridanone), a mouse STING agonist, was administered to mice to stimulate the STING pathway, and the amount of cytokines (IFN-β, IL-6, TNF-α) released into blood was evaluated for the inhibitory action of the compounds of the present invention (Compounds of Embodiments 3, 56, 57 and 58)

### (Adjustment of test compound solution)

DMSO, polyethylene glycol #400 and 30% (w/v) hydroxypropyl-β-cyclodextrin were sequentially added to the test compound and mixed well (5:20:75 solvent composition) to prepare a test compound solution. For the solvent-administered group, a solution having the same solvent composition but not containing the test compound was used.

### (CMA stimulated response)

C57BL/6N mice (female, 6-9 weeks old) were orally dosed with vehicle or test compound solutions adjusted to the test dose (4 mice per group). One hour after administration, CMA (Tokyo Kasei Kogyo) suspended in 0.5% methylcellulose solution was intraperitoneally administered to the mice at a dosage of 224 mg/kg. Two hours after CMA administration, blood was collected from each mouse, and plasma concentrations of IFN-β, IL-6 and TNF-α were measured using Duoset ELISA Kit (R&D Systems).

### (Evaluation results)

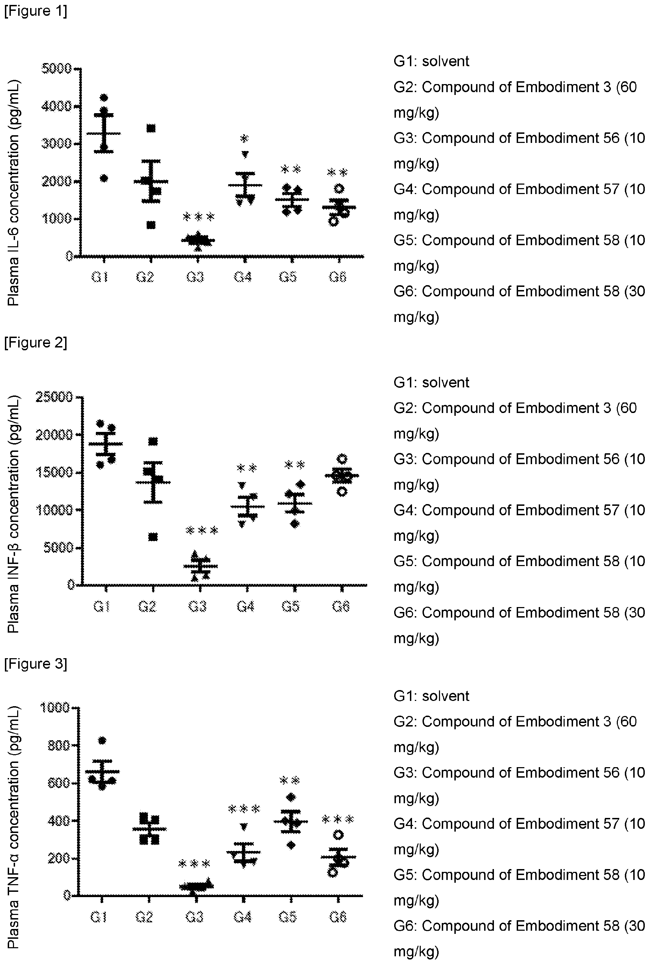

The results are shown in Figures 1-3. All test results are indicated by *, ** or ***.

Dunnett's multiple comparison tests (comparison to vehicle group)

| | |
|---|---|
| *** | p < 0. 001 |
| ** | p < 0. 01 |
| * | p < 0. 05 |

As shown in FIGS. 1 to 3, the representative compounds of the present invention significantly suppressed or tended to suppress the production of cytokines induced by STING stimulation as compared with the solvent group. This result indicates that compound (I) of the present invention has an inhibitory effect on IFN-β, IL-6, and TNF-α production induced by STING activation in vivo in mice.

### Test example 3

### Human IFN-β production inhibition test by cGAMP stimulation

The inhibitory activity of test compounds against STING activation was evaluated by measuring the amount of IFN-β produced when human monocytic cell line THP-1 cells were stimulated with the endogenous ligand cGAMP.

After seeding THP-1 cells (ATCC) in a 96-well plate, PMA (Santa Cruz Biotechnology) adjusted to a concentration of 100 nM after addition was added, and incubated at 37°C in a 5% CO₂ incubator. It was cultured overnight (RPM11640 medium containing 10% FBS, 50 U/mL penicillin/50 µg/mL streptomycin). A test compound solution adjusted to a final concentration of 0.001 to 1 µM was added to each well of this plate and cultured for 1 hour in a CO₂ incubator (DMSO final concentration 0.1%). 0.12 µg/well of 2'3'-cGAMP (Invivogen, #tlrl-nacga23) was introduced into the cells by transfection using Lipofectamine 2000 (Invitrogen) and cultured in a CO incubator for an additional 18 hours. After collecting the culture supernatant from each well, the amount of human IFN-β produced in the culture supernatant was measured by ELISA using R&D human IFN-β Duoset (R&D Systems).

### (Evaluation method for inhibitory activity)

Assuming that the human IFN-β production amount of the test compound-free and cGAMP-added group is 100%, and the human IFN-β production amount of the test compound-free and cGAMP-free group is 0%, the IC₅₀ value was determined by regression analysis of the inhibitory rate determined from the amount of human IFN-β production at each compound concentration and the concentration of the test compound (logarithm).

### (Evaluation results)

Table 6 shows the IFN-β production inhibitory activity of representative compounds of the present invention. As for the IFN-β production inhibitory activity, the IC₅₀ value of less than 0.01 µM is marked with ***, 0.01 µM or more and less than 0.1 µM are marked with **, 0.1 µM or more and less than 1 µM are marked with *, and 1 µM or more is indicated by -.

### [Table 6]

**[Table 6]**

| Test compound (Embodiment number) | IFN-β bioinhibitory activity |
|---|---|
| 3 | ∗ ∗ |
| 4 | ∗ ∗ |
| 6 | ∗ |
| 7 | ∗ ∗ |
| 8 | ∗ ∗ |
| 12 | ∗ |
| 18 | - |
| 19 | ∗ |
| 24 | ∗ ∗ |
| 25 | ∗ ∗ |
| 26 | ∗ ∗ |
| 31 | ∗ ∗ ∗ |
| 32 | ∗ ∗ |
| 33 | ∗ |
| 35 | ∗ |
| 36 | ∗ ∗ |
| 38 | - |
| 44 | ∗ |
| 45 | ∗ |
| 47 | ∗ ∗ |
| 52 | ∗ |
| 53 | - |
| 54 | - |
| 56 | ∗ ∗ ∗ |
| 57 | ∗ ∗ |
| 58 | ∗ |
| 59 | ∗ ∗ |
| 60 | ∗ |
| 64 | ∗ |
| 72 | ∗ |
| 73 | ∗ |
| 77 | - |
| 78 | - |
| 79 | ∗ |
| 80 | ∗ ∗ ∗ |
| 82 | ∗ ∗ |
| 84 | - |
| 86 | - |
| 87 | ∗ |
| 88 | ∗ |
| 89 | ∗ |
| 91 | ∗ |
| 93 | ∗ |
| 94 | ∗ |
| 95 | ∗ ∗ ∗ |
| 100 | ∗ ∗ ∗ |
| 101 | ∗ ∗ |
| 103 | ∗ |
| 104 | ∗ ∗ |
| 108 | - |
| 109 | ∗ |
| 111 | ∗ ∗ |
| 112 | ∗ |
| 114 | |
| 115 | ∗ |
| 116 | ∗ |
| 117 | ∗ |
| 11.8 | ∗ |
| 119 | ∗ |
| 120 | ∗ |
| 123 | ∗ |
| 124 | ∗ ∗ ∗ |
| 125 | ∗ |
| 127 | ∗ ∗ |
| 128 | ∗ ∗ ∗ |
| 129 | ∗ ∗ |
| 130 | - |
| 131 | ∗ ∗ |
| 133 | ∗ ∗ ∗ |
| 136 | ∗ |
| 144 | - |
| 145 | ∗ |
| 149 | ∗ |
| 151 | ∗ |
| 157 | ∗ |
| 158 | ∗ |
| 159 | ∗ |
| 160 | ∗ |
| 169 | ∗ ∗ |
| 178 | - |
| 185 | ∗ ∗ ∗ |
| 186 | ∗ |
| 187 | ∗ |
| 188 | ∗ |
| 189 | ∗ |
| 190 | ∗ |
| 191 | ∗ |
| 192 | * |
| 195 | ∗ ∗ |
| 196 | ∗ ∗ |
| 210 | ∗ ∗ |
| 212 | ∗ ∗ ∗ |
| 213 | ∗ ∗ |
| 214 | ∗ ∗ ∗ |
| 21.5 | ∗ ∗ |
| 216 | ∗ ∗ ∗ |
| 217 | ∗ ∗ |
| 218 | ∗ ∗ |
| 219 | ∗ ∗ |
| 220 | ∗ |
| 221 | ∗ ∗ |
| 222 | ∗ ∗ |
| 223 | ∗ ∗ |
| 224 | ∗ ∗ |
| 225 | ∗ ∗ |
| 226 | ∗ ∗ |
| 228 | ∗ ∗ |
| 231 | ∗ ∗ |
| 232 | ∗ ∗ |
| 233 | ∗ ∗ |
| 236 | ∗ ∗ |
| 237 | ∗ ∗ |
| 238 | ∗ ∗ |
| 240 | ∗ ∗ |
| 241 | ∗ ∗ |
| 242 | ∗ ∗ |
| 244 | ∗ ∗ |
| 245 | ∗ |
| 246 | ∗ ∗ ∗ |
| 247 | ∗ |
| 248 | ∗ ∗ |
| 249 | ∗ ∗ |
| 250 | ∗ ∗ |
| 251 | ∗ ∗ |
| 252 | ∗ ∗ |
| 253 | ∗ ∗ |
| 254 | ∗ ∗ |
| 255 | ∗ ∗ |
| 256 | ∗ ∗ |
| 257 | ∗ ∗ |
| 258 | ∗ ∗ |
| 259 | ∗ |
| 260 | ∗ |
| 261 | ∗ ∗ |
| 262 | ∗ ∗ |
| 263 | ∗ |
| 264 | ∗ |
| 265 | ∗ ∗ |
| 266 | ∗ |
| 267 | ∗ |
| 268 | ∗ ∗ |
| 269 | ∗ ∗ |

This result indicates that the compound (I) of the present invention exhibits strong IFN-β production inhibitory activity, indicating that it strongly inhibits the activation of the STING pathway.

### Industrial applicability

The compounds provided by the present invention are effective as prophylactic or therapeutic pharmaceuticals (pharmaceutical compositions) for diseases known to be associated with STING-mediated cell responses, such as inflammatory diseases, autoimmune diseases, cancer, and the like. In addition, by combining with therapeutic agents for other inflammatory diseases, autoimmune diseases, and cancer, an effect on immune response and the like can be expected, and it is useful as therapeutic pharmaceuticals (pharmaceutical compositions). Furthermore, it is useful as a STING inhibitor and as a reagent for experimental research.

## Claims

1. A benzimidazole derivative or a pharmaceutically acceptable salt thereof represented by the following formula (I): 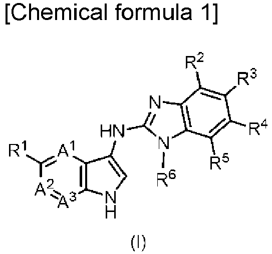
(where A¹ represents a nitrogen atom or C-R⁷, A² represents a nitrogen
atom or C-R⁸, A³ represents a nitrogen atom or C-R⁹,
R¹ is a hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted cycloalkenyl group, a substituted or unsubstituted alkoxy group, a substituted or unsubstituted aryl group, a substituted or unsubstituted carbamoyl group, a 4-morpholine carbonyl group, a cyano group, a carboxy group or an alkoxycarbonyl group,
R², R³, R⁴ and R⁵ are each independently and optionally selected from the group consisting of a hydrogen atom, a halogen atom, a substituted or unsubstituted alkyl group, a substituted or unsubstituted alkenyl group, a substituted or unsubstituted alkynyl group, a substituted or unsubstituted cycloalkyl group, a substituted or unsubstituted alkoxy group, a substituted or unsubstituted aryl group, a substituted or unsubstituted heteroaryl group, a substituted or unsubstituted heterocyclo group, a substituted or unsubstituted aryloxy group, a substituted or unsubstituted cycloalkyloxy group, a substituted or unsubstituted heteroaryloxy group, a substituted or unsubstituted heterocyclooxy group, a substituted or unsubstituted arylsulfonyl group, a substituted or unsubstituted carbamoyl group, a substituted or unsubstituted amino group, a substituted or unsubstituted aminosulfonyl groups, cyano groups, carboxy groups, alkoxycarbonyl groups and nitro groups, wherein R² and R³, or R³ and R⁴, or R⁴ and R⁵ may be bonded to each other to form a ring,
R⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted heteroaryl group, a substituted or unsubstituted heterocyclo group, or a substituted or unsubstituted amino group,
R⁷, R⁸ and R⁹ are each independently and optionally selected from the group consisting of a hydrogen atom, a halogen atom, a substituted or unsubstituted alkyl group, a substituted or unsubstituted alkenyl group, a substituted or unsubstituted cycloalkenyl group, a substituted or unsubstituted alkoxy group, a substituted or unsubstituted aryl group, a substituted or unsubstituted heteroaryl group, a hydroxyl group, a substituted or unsubstituted amino group and a substituted or unsubstituted carbamoyl group.)

2. The benzimidazole derivative or a pharmaceutically acceptable salt thereof according to claim 1, wherein, in formula (I), 1) A¹ is C-R7, A² is C-R⁸, A³ is C-R⁹; 2) A¹ is a nitrogen atom, A² is C-R⁸, A³ is C-R⁹; 3) A¹ is C-R⁷, A² is a nitrogen atom, A³ is C-R⁹; 4) A¹ is C-R⁷, A² is C-R⁸, A³ is a nitrogen atom, or 5) both A¹ and A³ represent a nitrogen atom and A² represents C-R⁸.

3. The benzimidazole derivative or a pharmaceutically acceptable salt thereof according to claim 1 or 2, wherein in formula (I), A² represents C-R⁸, 1) A¹ is C-R⁷, A³ is C-R⁹; 2) A¹ is a nitrogen atom, A³ is C-R⁹; or 3) 3. A¹ represents C-R⁷ and A³ represents a nitrogen atom, respectively.

4. The benzimidazole derivative or a pharmaceutically acceptable salt thereof according to claim 1 or 2, wherein in formula (I), A¹, A² and A³ are represented by C-R⁷, C-R⁸ and C-R⁹, respectively.

5. The benzimidazole derivative or a pharmaceutically acceptable salt thereof according to claim 1 or 2, wherein in formula (I), A¹ is a nitrogen atom and A² and A³ are represented by C-R⁸ and C-R⁹, respectively.

6. The benzimidazole derivative or a pharmaceutically acceptable salt thereof according to claim 1 or 2, wherein in formula (I), A¹ is C-R⁷, A² is a nitrogen atom, and A³ is C-R⁹.

7. The benzimidazole derivative or a pharmaceutically acceptable salt thereof according to claim 1 or 2, wherein in formula (I), A¹ is C-R⁷, A² is C-R⁸, and A³ is a nitrogen atom.

8. The benzimidazole derivative or a pharmaceutically acceptable salt thereof according to claim 1 or 2, wherein in formula (I), both A¹ and A³ are nitrogen atoms and A² is C-R⁸.

9. A compound or a pharmaceutically acceptable salt thereof according to any one of Embodiments 1 to 288.
